# EUROPEAN PATENT APPLICATION

(11) **EP 4 431 508 A1**
(43) Date of publication of application: **18.09.2024**
(21) Application number: 22892123.5
(22) Date of filing: 11.11.2022
(51) Int. Cl.: C07D 409/04, C07D 409/14, C07D 241/10, A61K 31/497, A61K 31/381, A61P 35/00, A61P 37/00, A61P 3/10

(54) **DRAK2 INHIBITOR, AND PREPARATION METHOD THEREFOR AND USE THEREOF**

(30) Priority: 12.11.2021 CN 202111342457
(71) Applicant: Biopolar Youtang (Guangdong) Pharmaceutical Co., Ltd., Zhongshan, Guangdong 528400 (CN)
(72) Inventor: LV, Zhijian, Zhongshan, Guangdong 528400 (CN); ZHONG, Li, Zhongshan, Guangdong 528400 (CN)
(74) Representative: Lavoix
(86) International application number: PCT/CN2022/131513
(87) International publication number: WO 2023/083330

(57) **Abstract**

Provided are a compound as represented by formula I serving as a DRK2 inhibitor, or a stereoisomer or optical isomer, a pharmaceutically acceptable salt, a prodrug or a solvate thereof. Further provided are a preparation method for the compound, a pharmaceutical composition thereof, and the use thereof as a Drak2 inhibitor and in the preparation of a drug for preventing and/or treating a Drak2-related disease.

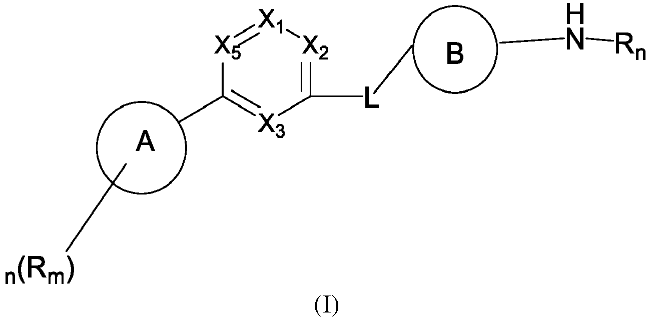

## Description

### Technical field

The present invention belong to the field of pharmaceutical chemistry, specifically relate to Drak2 inhibitor, and preparation method therefor and use therof.

### Background

Drak2 (Death-associated protein-related apoptotic kinase-2) is a member of death related protein kinases DAPK family, which is abundantly expresses in the thymus, spleen, and lymph nodes, and mainly participates in the regulation of cell apoptosis.

It has been reported that both gene and protein levels of Drak2 were dramatically increased in pancreatic β-cells treated with free fatty acid (FFA) and inflammatory cytokine (IL-1β, IFNγ, TNFα), along with anti-apoptotic factors such as Bcl-2, Bcl-xL, and Flip were increased. Up-regulations of these genes and protein levels promote an increase of apoptosis of pancreatic β-cells and reduces insulin secretion. Meanwhile, Drak2 transgenic mice showed worse glucose tolerance after obesity being induced by high-fat diet, confirming that Drak2 is not conducive to pancreatic β-cell survival under simulated inflammatory and high-fat pathological conditions. Therefore, Drak2 may become a novel target for preventing apoptosis of pancreatic β-cells and restoring the function thereof to achieve the fundamental treatment of type 2 diabetes.

In addition to participating in the regulation of cell apoptosis, Drak2 also has an immunomodulatory function, which is responsible for the negative regulation of T cell activation, and is closely related to T cell survival and differentiation, tumor immune regulation, and autoimmunity and the like.

Therefore, it is necessary to develop new Drak2 inhibitors.

### SUMMARY OF THE INVENTION

The purpose of the present invention is to provide a novel Drak2 inhibitor with broad clinical application prospects.

In the first aspect of the present invention, provided is a compound of formula I, or a stereoisomer or optical isomer, a pharmaceutically acceptable salt, a prodrug or a solvate thereof, 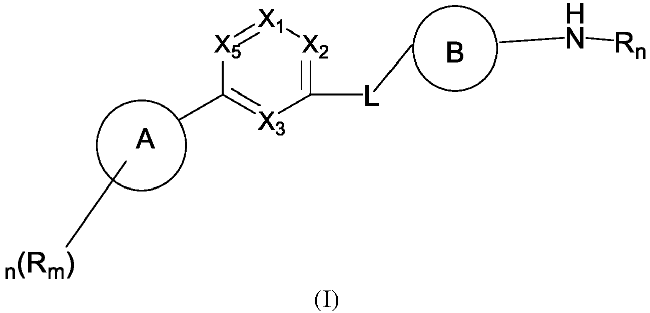 wherein,
X₁ is selected from N and CRₘ₁;
X₂ is selected from N and CRₘ₂;
X₃ is selected from N and CRₘ₃;
X₅ is selected from N and CR'ₘ₃;
L is selected from: bond, -O-, -S-, NRₘ₄-, -C(O)- and -C(O)NRₘ₄-;
Ring A and ring B are each independently selected from: C3-C12 cycloalkyl, 3-12 membered heterocyclyl, C6-C10 aryl, and 5-12 membered heteroaryl; wherein, C3-C12 cycloalkyl, 3-12 membered heterocyclyl, C6-C10 aryl, or 5-12 membered heteroaryl is optionally substituted with 1 to 3 R;
each Rₘ is independently selected from: H, D, halogen, cyano, hydroxyl, carboxyl,-S(O)ₜRₘ₅, -C(O)Rₘ₅, -C(O)ORₘ₅, -C(O)NRₘ₆Rₘ₇, -S(O)ₜNRₘ₆Rₘ₇, -C(O)NRₘ₈S(O)ₜNRₘ₆Rₘ₇, -(CH₂)_{q}S(O)ₜRₘ₅, -(CH₂)_{q}C(O)Rₘ₅, -(CH₂)_{q}C(O)ORₘ₅, -(CH₂)_{q}C(O)NRₘ₆Rₘ₇, -(CH₂)_{q}(CH₂)_{q}S (O)ₜNRₘ₆Rₘ₇, -C(O)NRₘ₈S(O)ₜNRₘ₆Rₘ₇, -(CH₂)_{q}Rₘ₉, -C1-C6 alkyl, -C1-C6 alkoxy, -C1-C6 alkylamino, C3-C12 cycloalkyl, C2-C6 alkenyl, C2-C6 alkynyl, 3-12 membered heterocyclyl, C6-C10 aryl and 5-12 membered heteroaryl; wherein, C1-C6 alkyl, C1-C6 alkoxy, C3-C12 cycloalkyl, 3-12 membered heterocyclyl, C6-C10 aryl and 5-12 membered heteroaryl are optionally substituted with 1 to 3 R;
alternatively, two Rₘ located on adjacent ring atoms together with their adjacent ring atoms form C3-C12 cycloalkyl or 3-12 membered heterocyclyl, wherein C3-C12 cycloalkyl or 3-12 membered heterocyclyl is optionally substituted with 1 to 3 R;
Rₙ is selected from: H, C1-C15 alkyl, C2-C15 alkenyl, C2-C15 alkynyl, and C(O)Rₚ; wherein, Rₚ is selected from: NH₂, C1-C15 alkyl, C1-C15 alkoxy, C1-C15 alkylamino, C2-C15 alkenyl, C2-C15 alkynyl, C3-C12 cycloalkyl, 3-12 membered heterocyclyl, C6-C10 aryl and 5-12 membered heteroaryl; wherein, C1-C15 alkyl, C1-C15 alkoxy, C1-C15 alkylamino, C3-C12 cycloalkyl, 3-12 membered heterocyclyl, C6-C10 aryl or 5-12 membered heteroaryl is optionally substituted with 1 to 3 R;
Rₘ₁, Rₘ₂, Rₘ₃, and R'ₘ₃ are each independently selected from: H, cyano, halogen, C(O)NH₂, carboxyl, S(O)₂NH₂, C(O)OC1-C6 alkyl and S(O)₂C1-C6 alkyl;
Rₘ₄ is selected from: H and C1-C6 alkyl;
Rₘ₅ is selected from: C1-C6 alkyl, C1-C6 alkoxy, C1-C6 alkylamino, C3-C12 cycloalkyl, 3-12 membered heterocyclyl, C6-C10 aryl and 5-12 membered heteroaryl; wherein, C1-C6 alkyl, C1-C6 alkoxy, C3-C12 cycloalkyl, 3-12 membered heterocyclyl, C6-C10 aryl and 5-12 membered heteroaryl is optionally substituted with 1 to 3 R;
Rₘ₆, Rₘ₇, Rₘ₈, and Rₘ₉ are each independently selected from: H, C1-C6 alkyl, C1-C6 alkoxy, C1-C6 alkylamino, C3-C12 cycloalkyl, 3-12 membered heterocyclyl, C6-C10 aryl, and 5-12 membered heteroaryl; alternatively, Rₘ₆ and Rₘ₇ together with the adjacent N atom form a 3-12 membered heterocyclyl; wherein, C1-C6 alkyl, C1-C6 alkoxy, C3-C12 cycloalkyl, 3-12 membered heterocyclyl, C6-C10 aryl, or 5-12 membered heteroaryl is optionally substituted with 1 to 3 R;
the H atom in (CH₂)_{q} is optionally substituted with R;
R is selected from: H, D, halogen, cyano, hydroxyl, carboxyl, NH₂, oxo- (=O), C1-C6 alkyl, C1-C6 alkoxy, C1-C6 alkylamino, C(O)OC1-C6 alkyl, S(O)₂C1-C6 alkyl, C2-C6 alkenyl, C2-C6 alkynyl, C3-C12 cycloalkyl, 3-12 membered heterocyclyl, C6-C10 aryl and 5-12 membered heteroaryl; wherein, C1-C6 alkyl, C1-C6 alkoxy, C3-C12 cycloalkyl, 3-12 membered heterocyclyl, C6-C10 aryl and 5-12 membered heteroaryl are optionally substituted with 1 to 3 substituents selected from the group consisting of: halogen, cyano, hydroxyl, carboxyl, C1-C6 alkyl, C1-C6 alkoxy, C1-C6 alkylamino, C(O)OC1-C6 alkyl, and S(O)₂C1-C6 alkyl;
t is 0, 1 or 2;
q is 1, 2, 3, 4, 5 or 6; and
n is 1, 2, 3, 4, 5 or 6.

In another preferred embodiment, the compound, or a stereoisomer, an optical isomer, a pharmaceutically acceptable salt, a prodrug or a solvate thereof, has a structure as represented by formula II 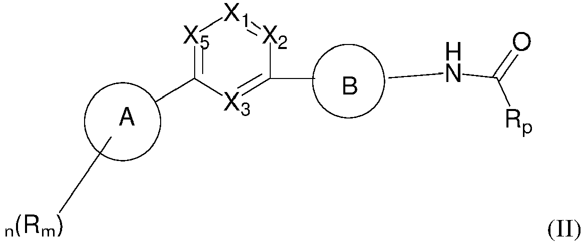 wherein, X₁, X₂, X₃, X₅, ring A, ring B, Rₘ, Rₚ, and n are described as above.

In another preferred embodiment, Ring A is selected from: phenyl, C5-C6 cycloalkyl, 5-6-membered heteroaryl and 5-6-membered heteroaryl, preferably phenyl.

In another preferred embodiment, ring B is 5-6-membered heteroaryl, preferably thienyl or thiazolyl.

In another preferred embodiment, the compound, or a stereoisomer, an optical isomer, a pharmaceutically acceptable salt, a prodrug or a solvate thereof, has a structure as represented by formula III 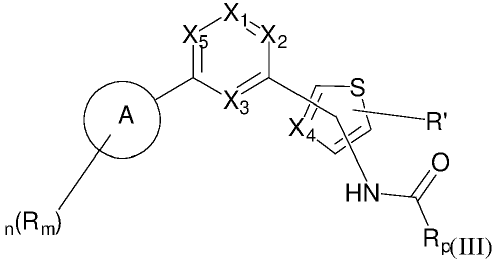
wherein, X₄ is selected from: N and CRₘ₁₀; wherein, Rₘ₁₀ is selected from: H, C1-C6 alkyl, C1-C6 alkoxy, C1-C6 alkylamino, C3-C12 cycloalkyl, 3-12 membered heterocyclyl, C6-C10 aryl and 5-12 membered heteroaryl; or, Rₘ₆ and Rₘ₇ together with the adjacent N atom form a 3-12 membered heterocyclyl; wherein, C1-C6 alkyl, C1-C6 alkoxy, C3-C12 cycloalkyl, 3-12 membered heterocyclyl, C6-C10 aryl, and 5-12 membered heteroaryl are optionally substituted with 1 to 3 R;
R' is selected from: H, D, halogen, cyano, hydroxyl, carboxyl, NH₂, C1-C6 alkyl, C1-C6 alkoxy, C1-C6 alkylamino, preferablyis H;
R, X₁, X₂, X₃, X₅, ring A, Rₘ, Rₚ, and n are described as above.

In another preferred embodiment, 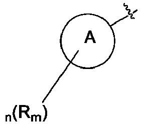 is 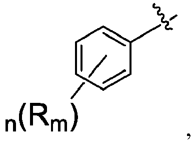 preferably,

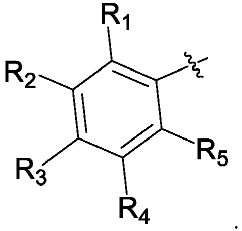

In another preferred embodiment, the compound, or a stereoisomer, an optical isomer, a pharmaceutically acceptable salt, a prodrug or a solvate thereof, has a structure as represented by formula IV 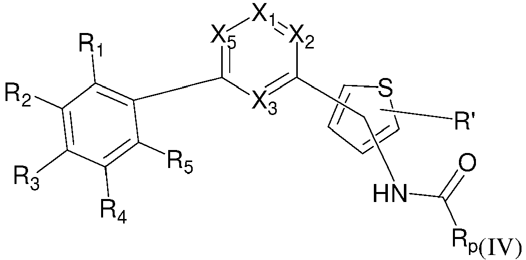 wherein,
R₁, R₂, R₃, R₄, and R₅ are each independently selected from: H, D, halogen, cyano, hydroxyl, carboxyl, S(O)ₜRₘ₅, C(O)Rₘ₅, C(O)ORₘ₅, C(O)NRₘ₆Rₘ₇, S(O)ₜNRₘ₆Rₘ₇, C(O)NRₘ₈S(O)ₜNRₘ₆Rₘ₇, (CH₂)_{q}S(O)ₜRₘ₅, (CH₂)_{q}C(O)Rₘ₅, (CH₂)_{q}C(O)ORₘ₅, (CH₂)_{q}C(O)NRₘ₆Rₘ₇, (CH₂)_{q}(CH₂)_{q}S(O)ₜNRₘ₆Rₘ₇, C(O)NRₘ₈S(O)ₜNRₘ₆Rₘ₇, (CH₂)_{q}Rₘ₉, C1-C6 alkyl, C1-C6 alkoxy, C1-C6 alkylamino, C3-C12 cycloalkyl, 3-12 membered heterocyclyl, C6-C10 aryl and 5-12 membered heteroaryl; wherein, C1-C6 alkyl, C1-C6 alkoxy, C3-C12 cycloalkyl, 3-12 membered heterocyclyl, C6-C10 aryl and 5-12 membered heteroaryl are optionally substituted with 1 to 3 R;
or R₁ and R₂, or R₂ and R₃, or R₃ and R₄, or R₄ and R₅ together with their adjacent C atoms form a 5-6-membered heterocyclyl, wherein the 5-6-membered heterocyclyl is optionally substituted with 1 to 3 R;
R' is selected from: H, D, halogen, cyano, hydroxyl, carboxyl, NH₂, C1-C6 alkyl, C1-C6 alkoxy, C1-C6 alkylamino, preferably H;
t, q, R, Rₘ₅, Rₘ₆, Rₘ₇, Rₘ₈, Rₘ₉, X₁, X₂, X₃, X₅, Rₚ, and n are described as above.

In another preferred embodiment, R₁ is H.

In another preferred embodiment, R₄ is H.

In another preferred embodiment, R₅ is H.

In another preferred embodiment, R₁ is H, R₄ is H and R₅ is H.

In another preferred embodiment, R₂ is C1-6 alkoxy (preferably, methoxy).

In another preferred embodiment, R₃ is C1-C6 alkoxy (preferably methoxy), C(O)NRₘ₆Rₘ₇, or S(O)₂NRₘ₆Rₘ₇; wherein, Rₘ₆ and Rₘ₇ are described as above.

In another preferred embodiment, R₃ is C1-C6 alkoxy-3-6 membered heterocyclyl (preferably, -O-CH₂-3-6-membered heterocyclyl, -O-CH₂CH₂-3-6-membered heterocyclyl, etc.), wherein C1-C6 alkoxy-3-6 membered heterocyclyl is substituted with 1 to 4 substituents selected from halogen and C1-C6 alkyl.

In another preferred embodiment, the compound, or a stereoisomer, an optical isomer, a pharmaceutically acceptable salt, a prodrug or a solvate thereof, has a structure as represented by formula A 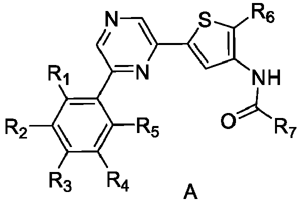 wherein,
R₆ is selected from: H, D, halogen, cyano, hydroxyl, carboxyl, NH₂, C1-C6 alkyl, C1-C6 alkoxy, C1-C6 alkylamino, preferably H;
R₇ is selected from: C1-C15 alkyl, C1-C15 alkoxy, C1-C15 alkylamino, C3-C12 cycloalkyl, 3-12 membered heterocyclyl, C6-C10 aryl and 5-12 membered heteroaryl; wherein, C1-C15 alkyl, C1-C15 alkoxy, C1-C15 alkylamino, C3-C12 cycloalkyl, 3-12 membered heterocyclyl, C6-C10 aryl and 5-12 membered heteroaryl are optionally substituted with 1 to 3 substituents selected from the group consisting of: D, halogen, cyano, hydroxyl, carboxyl, NH₂, oxo-(=O), C1-C6 alkyl, C1-C6 alkoxy, C1-C6 alkylamino, C(O)OC1-C6 alkyl, S(O)₂C1-C6 alkyl, C3-C12 cycloalkyl, 3-12 membered heterocyclyl, C6-C10 aryl and 5-12 membered heteroaryl;
R₁, R₂, R₃, R₄, and R₅ are described as above.

In another preferred embodiment, R₂ and R₃ together with the adjacent C atoms form a 5-6-membered heterocyclyl; preferably, R₂ and R₃ together with the adjacent C atoms form

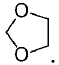

In another preferred embodiment, the compound, or a stereoisomer, an optical isomer, a pharmaceutically acceptable salt, a prodrug or a solvate thereof, has a structure as represented by formula B 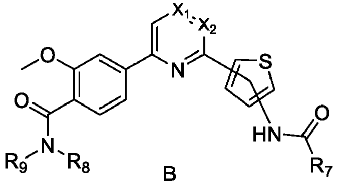 wherein,
R₇ is selected from: C1-C15 alkyl, C1-C15 alkoxy, C1-C15 alkylamino, C3-C12 cycloalkyl, 3-12 membered heterocyclyl, C6-C10 aryl and 5-12 membered heteroaryl; wherein, C1-C15 alkyl, C1-C15 alkoxy, C1-C15 alkylamino, C3-C12 cycloalkyl, 3-12 membered heterocyclyl, C6-C10 aryl and 5-12 membered heteroaryl are optionally substituted with 1 to 3 substituents selected from the group consisting of: D, halogen, cyano, hydroxyl, carboxyl, NH₂, oxo-(=O), C1-C6 alkyl, C1-C6 alkoxy, C1-C6 alkylamino, C(O)OC1-C6 alkyl, S(O)₂C1-C6 alkyl, C3-C12 cycloalkyl, 3-12 membered heterocyclyl, C6-C10 aryl and 5-12 membered heteroaryl;
R₈ and R₉ are each independently selected from: H, S(O)₂NR₁₁R₁₂, C1-C6 alkyl, C1-C6 alkoxy, C1-C6 alkylamino, C3-C12 cycloalkyl, 3-12 membered heterocyclyl, C6-C10 aryl, and 5-12 membered heteroaryl; alternatively, R₈ and R₉ together with the adjacent N atom form a 3-12 membered heterocyclyl (preferably 5-6 membered heterocyclyl); wherein, C1-C6 alkyl, C1-C6 alkoxy, C3-C12 cycloalkyl, 3-12 membered heterocyclyl, 5-6 membered heterocyclyl, C6-C10 aryl, or 5-12 membered heteroaryl is optionally substituted with 1 to 3 substituents selected from the group consisting of: C1-C6 alkyl, C1-C6 alkoxy, C1-C6 alkylamino, C(O)OC1-C6 alkyl, S(O)₂C1-C6 alkyl, C3-C12 cycloalkyl, C1-C6 alkyl-C3-C12 cycloalkyl, 3-12 membered heterocyclyl, C1-C6 alkyl-3-12 membered heterocyclyl, C6-C10 aryl and 5-12 membered heteroaryl;
R₁₁ and R₁₂ are each independently selected from: H, C1-C6 alkyl, C3-C12 cycloalkyl, 3-12 membered heterocyclyl, C6-C10 aryl and 5-12 membered heteroaryl.

In another preferred example, 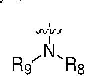 is selected from:

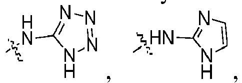

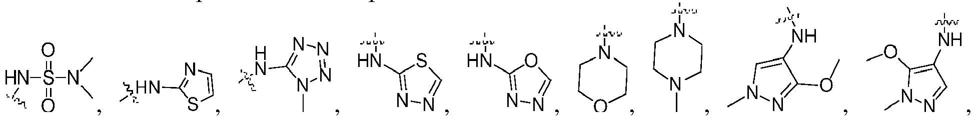

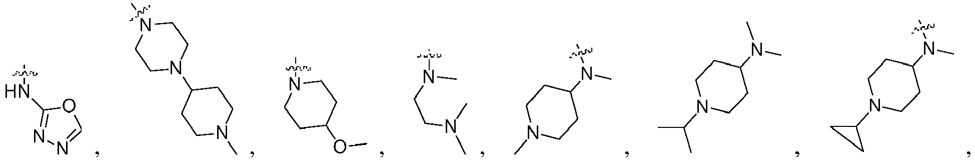

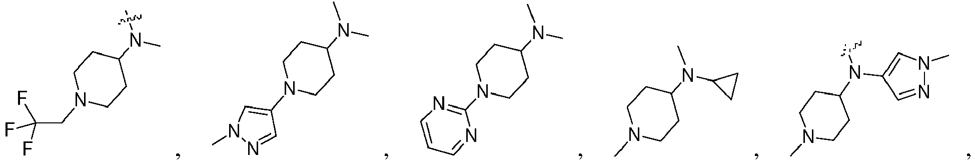

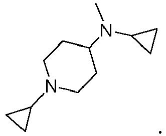

In another preferred embodiment, the compound, or a stereoisomer, an optical isomer, a pharmaceutically acceptable salt, a prodrug or a solvate thereof, has a structure as represented by formula C 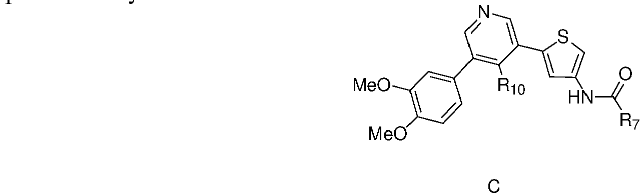 wherein,
R₁₀ is selected from: H, cyano, and CONH₂;
R₇ is selected from: C1-C15 alkyl, C1-C15 alkoxy, C1-C15 alkylamino, C3-C12 cycloalkyl, 3-12 membered heterocyclyl, C6-C10 aryl and 5-12 membered heteroaryl; wherein, C1-C15 alkyl, C1-C15 alkoxy, C1-C15 alkylamino, C3-C12 cycloalkyl, 3-12 membered heterocyclyl, C6-C10 aryl and 5-12 membered heteroaryl are optionally substituted with 1 to 3 substituents selected from the group consisting of: D, halogen, cyano, hydroxyl, carboxyl, NH₂, oxo-(=O), C1-C6 alkyl, C1-C6 alkoxy, C1-C6 alkylamino, C(O)OC1-C6 alkyl, S(O)₂C1-C6 alkyl, C3-C12 cycloalkyl, 3-12 membered heterocyclyl, C6-C10 aryl and 5-12 membered heteroaryl.

In another preferred embodiment, X₁ is N or CH.

In another preferred embodiment, X₂ is N or CH.

In another preferred embodiment, X₃ is N, C-C(=O)NH₂ or C-CN.

In another preferred embodiment, X₅ is N or CH.

In another preferred embodiment, 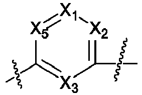 is

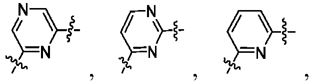

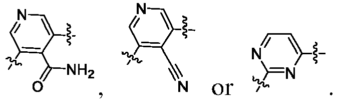

In another preferred embodiment, L is bond.

In another preferred embodiment, L is NH.

In another preferred embodiment, ring A is C6-C10 aryl or 3-12 membered heterocyclyl; wherein, the 3-12 membered heterocyclyl is preferably 5-7 membered heterocyclyl, and more preferably 6-membered heterocyclyl; the definition of substituents on ring A is the same as that of Rₘ mentioned above (the number of substituents is the same as n).

In another preferred embodiment, ring A is phenyl, 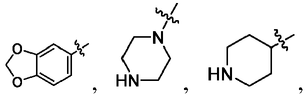 or 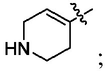 the definition of substituents on ring A (the position of substituent can be any position of H, including H in -NH-) is the same as that of above-mentioned Rm (the number of substituents is defined as n).

In another preferred embodiment, ring B is 5-12 membered heteroaryl or C3-C12 cycloalkyl, preferably 5-membered heteroaryl or 6-membered cycloalkyl; the definition of substituents on ring B is the same as that of R mentioned above.

In another excellent embodiment, ring B is 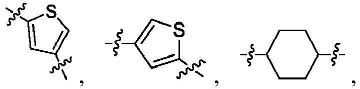 or 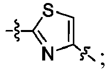 the definition of substituents on ring B is the same as that of R mentioned above.

In another preferred embodiment, Rₘ is methoxy, F, Cl, cyano, hydroxyl, carboxyl, formamide, 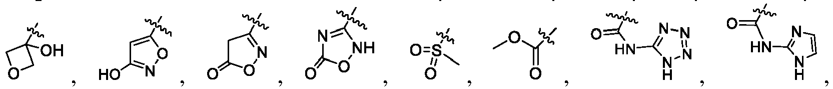 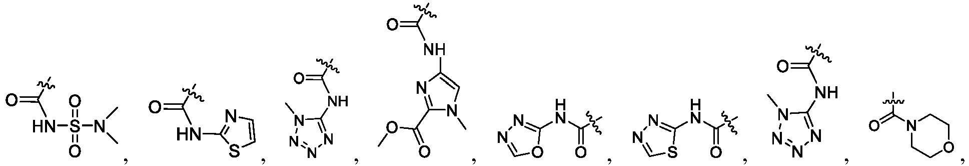 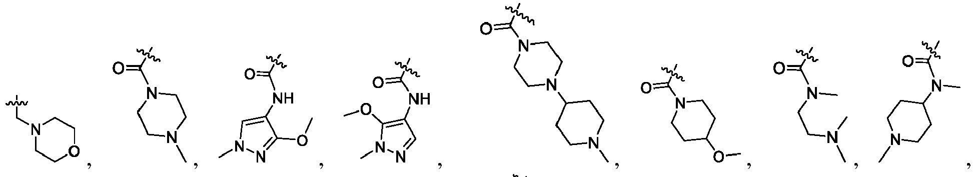 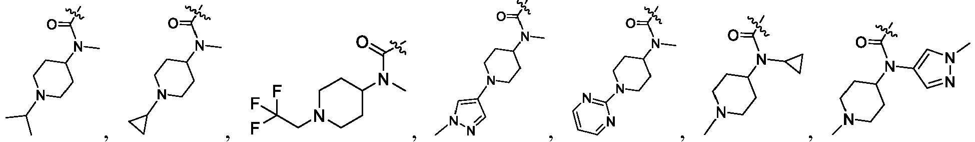 or

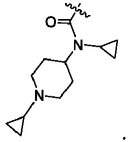

In another preferred embodiment, Rₙ is 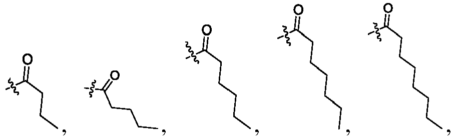 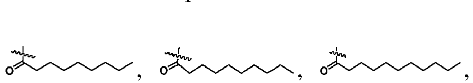 H, acetyl 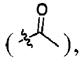 isobutyryl 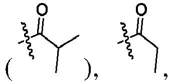 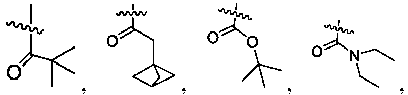 cyclopropylcarbonyl 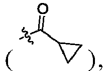 cyclobutylcarbonyl 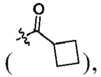 cyclopentyl carbonyl 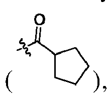 cyclohexyl carbonyl 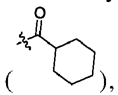 phenyl carbonyl 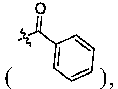 cyclopropyl methylene carbonyl 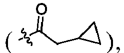 cyclobutyl methylene carbonyl 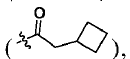 cyclopentyl methylene carbonyl 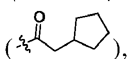 cyclohexyl methylene carbonyl

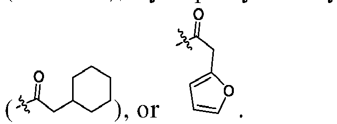

In another preferred embodiment, the compound has a structure as represented by formula I': 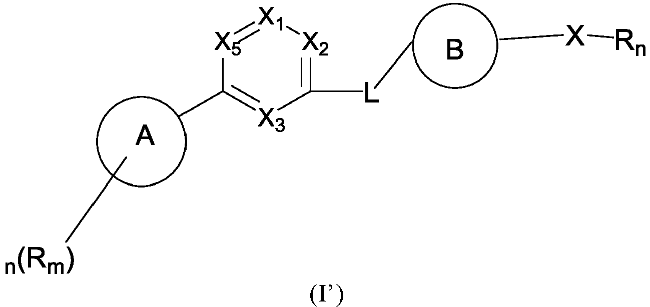 wherein,
X is selected from O and CH₂;
X₁ is selected from N and CRₘ₁;
X₂ is selected from N and CRₘ₂;
X₃ is selected from N and CRₘ₃;
X₅ is selected from N and CR'ₘ₃;
L is selected from bond, -O-, -S, -NRₘ₄-, -C(O)- and -C(O)NRₘ₄-;
Ring A and ring B are each independently selected from: C3-C12 cycloalkyl, 3-12 membered heterocyclyl, C6-C10 aryl, and 5-12 membered heteroaryl; wherein, C3-C12 cycloalkyl, 3-12 membered heterocyclyl, C6-C10 aryl, and 5-12 membered heteroaryl are optionally substituted with 1 to 3 R;
each Rₘ is independently selected from: H, D, halogen, cyano, hydroxyl, carboxyl,-S(O)ₜRₘ₅, -C(O)Rₘ₅, -C(O)ORₘ₅, -C(O)NRₘ₆Rₘ₇, -S(O)ₜNRₘ₆Rₘ₇, -C(O)NRₘ₈S(O)ₜNRₘ₆Rₘ₇, -(CH₂)_{q}S(O)ₜRₘ₅, -(CH₂)_{q}C(O)Rₘ₅, -(CH₂)_{q}C(O)ORₘ₅, -(CH₂)_{q}C(O)NRₘ₆Rₘ₇, -(CH₂)_{q}(CH₂)_{q}S(O)ₜNRₘ₆Rₘ₇, -C(O)NRₘ₈S(O)ₜNRₘ₆Rₘ₇, -(CH₂)_{q}Rₘ₉, -C1-C6 alkyl, -C1-C6 alkoxy, -C1-C6 alkylamino, C3-C12 cycloalkyl, C2-C6 alkenyl, C2-C6 alkynyl, 3-12 membered heterocyclyl, C6-C10 aryl and 5-12 membered heteroaryl; wherein, -C1-C6 alkyl, -C1-C6 alkoxy, C3-C12 cycloalkyl, 3-12 membered heterocyclyl, C6-C10 aryl and 5-12 membered heteroaryl are optionally substituted with 1 to 3 R;
alternatively, two Rₘ located on adjacent ring atoms together with their adjacent ring atoms form a C3-C12 cycloalkyl or a 3-12 membered heterocyclyl, wherein C3-C12 cycloalkyl or 3-12 membered heterocyclyl is optionally substituted with 1 to 3 R;
Rₙ is selected from: H, C1-C15 alkyl, C2-C15 alkenyl, C2-C15 alkynyl, and C(O)Rₚ; wherein, Rₚ is selected from: NH₂, C1-C15 alkyl, C1-C15 alkoxy, C1-C15 alkylamino, C2-C15 alkenyl, C2-C15 alkynyl, C3-C12 cycloalkyl, 3-12 membered heterocyclyl, C6-C10 aryl and 5-12 membered heteroaryl; wherein, C1-C15 alkyl, C1-C15 alkoxy, C1-C15 alkylamino, C3-C12 cycloalkyl, 3-12 membered heterocyclyl, C6-C10 aryl and 5-12 membered heteroaryl are optionally substituted with 1 to 3 R;
Rₘ₁, Rₘ₂, Rₘ₃, and R'ₘ₃ are each independently selected from: H, cyano, halogen, C(O)NH₂, carboxyl, S(O)₂NH₂, C(O)OC1-C6 alkyl and S(O)₂C1-C6 alkyl;
Rₘ₄ is selected from: H and C1-C6 alkyl;
Rₘ₅ is selected from: C1-C6 alkyl, C1-C6 alkoxy, C1-C6 alkylamino, C3-C12 cycloalkyl, 3-12 membered heterocyclyl, C6-C10 aryl and 5-12 membered heteroaryl; wherein, C1-C6 alkyl, C1-C6 alkoxy, C3-C12 cycloalkyl, 3-12 membered heterocyclyl, C6-C10 aryl and 5-12 membered heteroaryl are optionally substituted with 1 to 3 R;
Rₘ₆, Rₘ₇, Rₘ₈, and Rₘ₉ are each independently selected from: H, C1-C6 alkyl, C1-C6 alkoxy, C1-C6 alkylamino, C3-C12 cycloalkyl, 3-12 membered heterocyclyl, C6-C10 aryl, and 5-12 membered heteroaryl; alternatively, Rₘ₆ and Rₘ₇ together with the adjacent N atom form a 3-12 membered heterocyclyl; wherein, C1-C6 alkyl, C1-C6 alkoxy, C3-C12 cycloalkyl, 3-12 membered heterocyclyl, C6-C10 aryl, or 5-12 membered heteroaryl is optionally substituted with 1 to 3 R;
the H atoms in (CH₂)_{q} are optionally substituted with R;
R is selected from: H, D, halogen, cyano, hydroxyl, carboxyl, NH₂, oxo- (=O), C1-C6 alkyl, C1-C6 alkoxy, C1-C6 alkylamino, C(O)OC1-C6 alkyl, S(O)₂C1-C6 alkyl, C2-C6 alkenyl, C2-C6 alkynyl, C3-C12 cycloalkyl, 3-12 membered heterocyclyl, C6-C10 aryl and 5-12 membered heteroaryl; wherein, C1-C6 alkyl, C1-C6 alkoxy, C3-C12 cycloalkyl, 3-12 membered heterocyclyl, C6-C10 aryl and 5-12 membered heteroaryl are optionally substituted with 1 to 3 substituents selected from the group consisting of: halogen, cyano, hydroxyl, carboxyl, C1-C6 alkyl, C1-C6 alkoxy, C1-C6 alkylamino, C(O)OC1-C6 alkyl, and S(O)₂C1-C6 alkyl;
t is 0, 1 or 2;
q is 1, 2, 3, 4, 5 or 6; and
n is 1, 2, 3, 4, 5 or 6.

In another preferred embodiment, 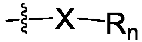 is ORₚ; wherein, Rₚ is selected from C1-C15 alkyl (preferably C1-C6 alkyl); wherein, C1-C15 alkyl (preferably C1-C6 alkyl) is optionally substituted with 1, 2, or 3 R; R is selected from C1-C15 alkylamino; the C1-C15 alkylamino is optionally substituted with 1, 2, or 3 substituents selected from group consisting of: halogen, cyano, hydroxyl, carboxyl, C1-C6 alkyl, halogenated C1-C6 alkyl, C6-C10 aryl substituted with halogenated C1-C6 alkyl, halogenated C1-C6 alkoxy, C6-C10 aryl substituted with halogenated C1-C6 alkoxy, C(O)C1-C6 alkyl, C1-C6 alkoxy, C1-C6 alkylamino, C(O)OC1-C6 alkyl, and S(O)₂C1-C6 alkyl; preferably, 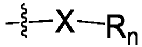 is

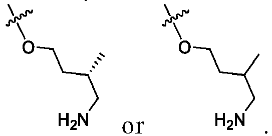

In another preferred embodiment, the compound satisfies one or more of the following conditions:
(1)X₁ is N or CH;
(2)X₂ is N or CH;
(3)X₃ is N, C-C(=O)NH₂ or C-CN;
(4)Xs is N or CH;
(5) L is bond or NH; and
(6) ring A is C6-C10 aryl, or 3-12 membered heterocyclyl; wherein, the 3-12 membered heterocyclyl is preferably 5-7 membered heterocyclyl, and more preferably 6-membered heterocyclyl; preferably, ring A is phenyl, 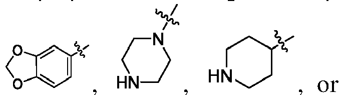 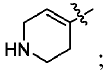 the definition of substituents on ring A the position of substituents can be any position where H locates, including H in -NH-) is the same as that of Rₘ mentioned above (the number of substituents is the same as n).
(7) ring B is 5-12 membered heteroaryl or C3-C12 cycloalkyl, preferably 5-membered heteroaryl or 6-membered cycloalkyl; preferably, ring B is 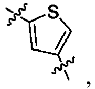 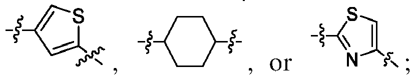 the substituents on ring B are the same as R mentioned above;
(8) Rₘ is methoxy, F, Cl, cyano, hydroxyl, carboxyl, formamide, 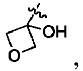 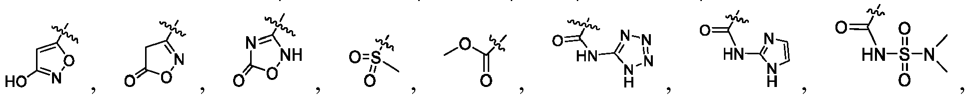 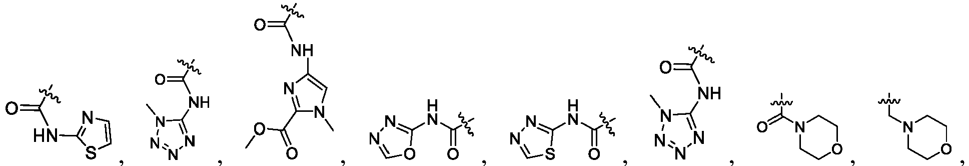 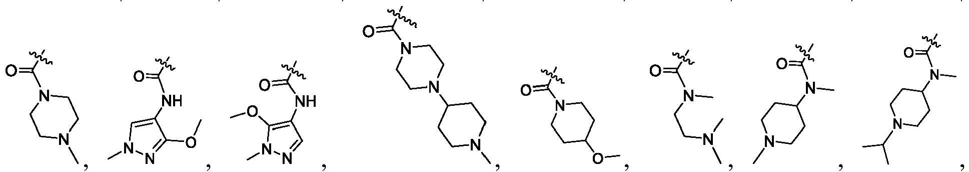 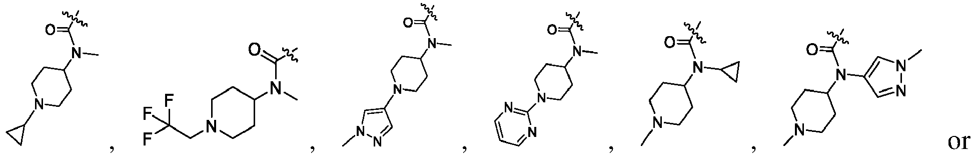 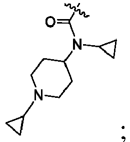
(9) Rₙ is 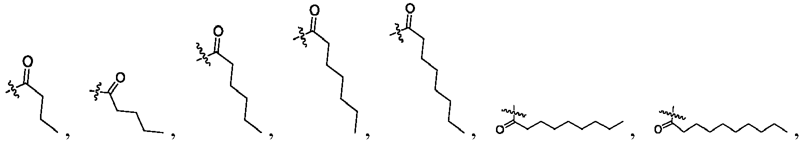 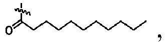 H, acetyl, isobutyryl, 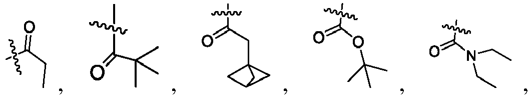 cyclopropylcarbonyl, cyclobutylcarbonyl, cyclopentyl carbonyl, cyclohexyl carbonyl, phenyl carbonyl, cyclopropyl methylene carbonyl, cyclobutyl methylene carbonyl, cyclopentyl methylene carbonyl, cyclohexyl methylene carbonyl, or 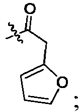 particularly,

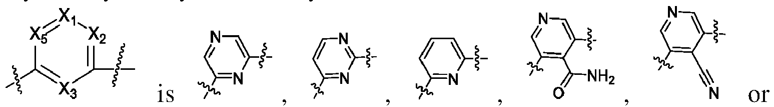

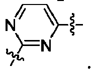

In another preferred embodiment, X₁, X₂, X₃, X₅, ring A, ring B, Rₘ, Rₚ, n, R₁, R₂, R₃, R₄, R₅, R₆, R₇, R₈, R₉, and R₁₀ are corresponding groups in each specific compound of the examples.

In another preferred embodiment, R is selected from: C1-C6 alkyl, C1-C6 alkoxy, C1-C6 alkylamino, C(O)OC1-C6 alkyl, S(O)₂C1-C6 alkyl, C2-C6 alkenyl, C2-C6 alkynyl, C3-C12 cycloalkyl, 3-12 membered heterocyclyl, C6-C10 aryl and 5-12 membered heteroaryl; wherein, C1-C6 alkyl, C1-C6 alkoxy, C3-C12 cycloalkyl, 3-12 membered heterocyclyl, C6-C10 aryl and 5-12 membered heteroaryl are optionally substituted with 1 to 3 substituents selected from the group consisting of: halogen, cyano, hydroxyl, carboxyl, C1-C6 alkyl, halogenated C1-C6 alkyl, C6-C10 aryl substituted with halogenated C1-C6 alkyl, C1-C6 alkoxy, halogenated C1-C6 alkoxy, C6-C10 aryl substituted with halogenated C1-C6 alkoxy, C(O)C1-C6 alkyl, C1-C6 alkylamino, C(O)OC1-C6 alkyl, and S(O)₂C1-C6 alkyl.

In another preferred example, Rn is selected from: C(O)Rₚ; wherein, Rₚ is selected from: C1-C15 alkyl (preferably C1-C6 alkyl); wherein, C1-C15 alkyl (preferably C1-C6 alkyl) is optionally substituted with 1, 2, or 3 R; R is selected from: C3-C12 cycloalkyl; wherein, C3-C12 cycloalkyl is optionally substituted with 1, 2, or 3 substituents selected from the group consisting of: C1-C6 alkyl, halogenated C1-C6 alkyl, C6-C10 aryl substituted with halogenated C1-C6 alkyl, halogenated C1-C6 alkoxy, C6-C10 aryl substituted with halogenated C1-C6 alkoxy, and C(O)C1-C6 alkyl.

In another preferred example, Rn is selected from: ORₚ; wherein, Rₚ is selected from: C1-C15 alkyl; wherein, C1-C15 alkyl is optionally substituted with 1, 2, or 3 R; R is selected from: C1-C15 alkylamino; C1-C15 alkylamino is optionally substituted with 1, 2, or 3 substituents selected from the group consisting of: halogen, cyano, hydroxyl, carboxyl, C1-C6 alkyl, halogenated C1-C6 alkyl, C6-C10 aryl substituted with halogenated C1-C6 alkyl, halogenated C1-C6 alkoxy, C6-C10 aryl substituted with halogenated C1-C6 alkoxy, C(O)C1-C6 alkyl, C1-C6 alkoxy, C1-C6 alkylamino, C(O)OC1-C6 alkyl, and S(O)₂C1-C6 alkyl.

In another preferred embodiment, ring A is selected from 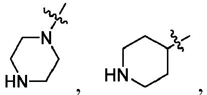 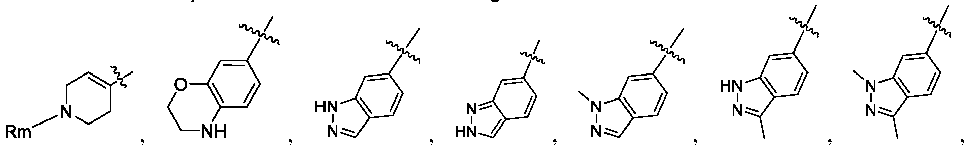 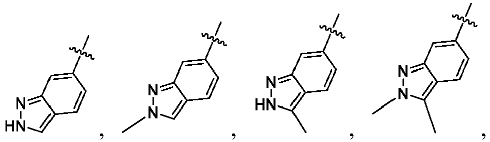 phenyl, 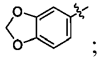 the substituents on ring A (the position of substituent can be any position of H, including H in -NH-) is defined as above-mentioned Rm the number of substituents is defined as n).

In another preferred example, 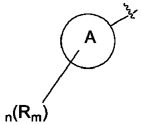 is selected from 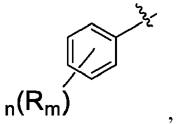 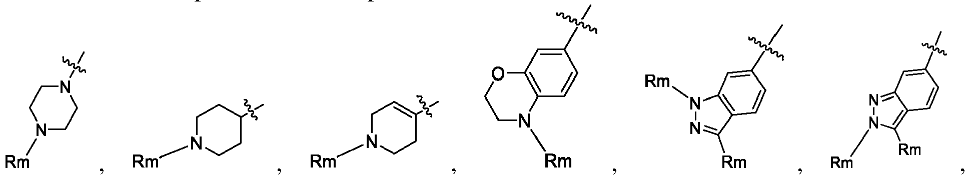 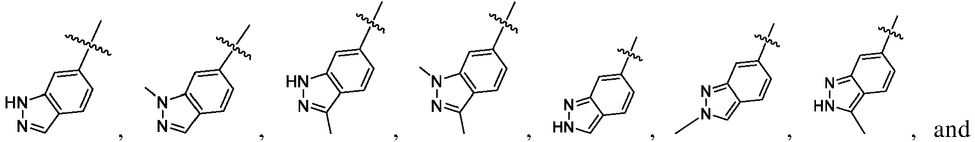 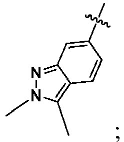 wherein, Rₘ is deined as above.

In another preferred embodiment, ring B selected from on ring B is defined as R mentioned above. 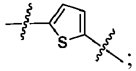 the substituents

In another preferred embodiment, Rm is selected from: 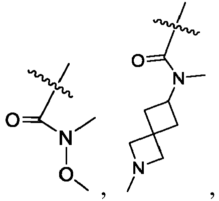 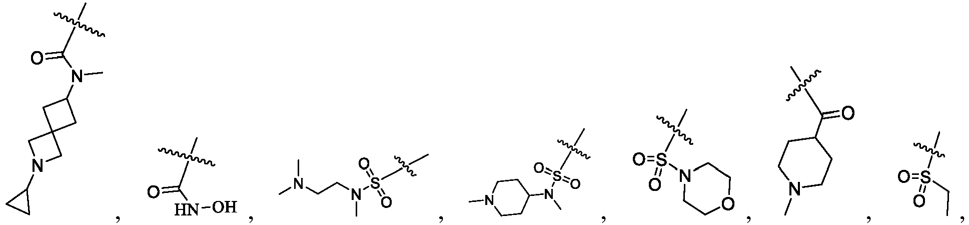 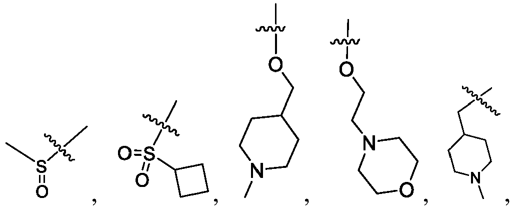 methyl and

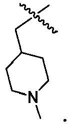

In another preferred embodiment, Rn is selected from:

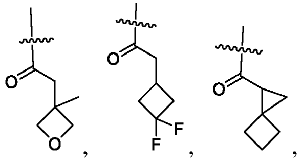

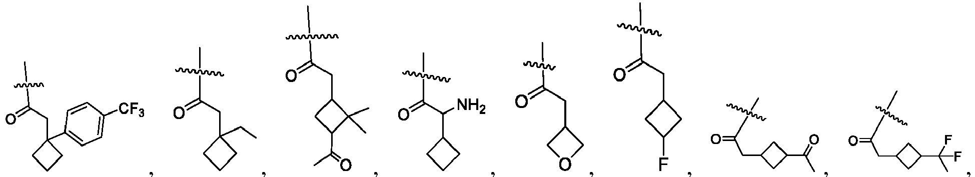

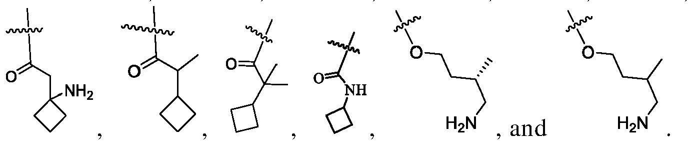

In another preferred embodiment, n is 0.

In another preferred embodiment, the compound is selected from the group consisting of:

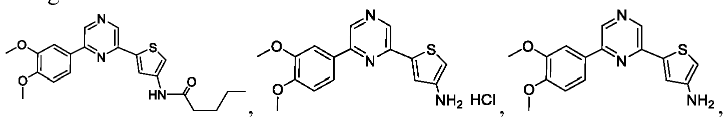

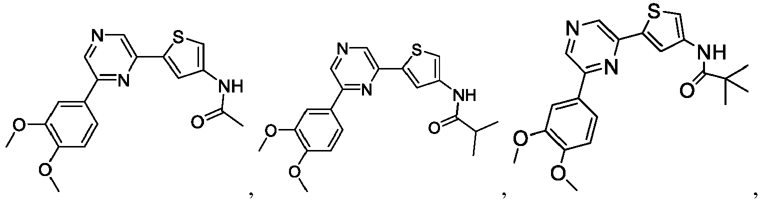

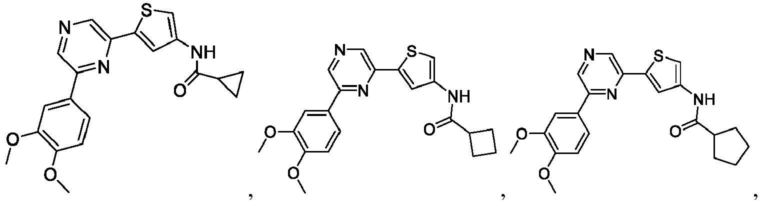

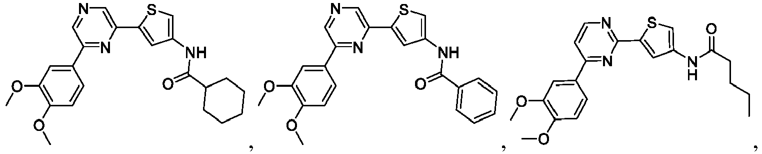

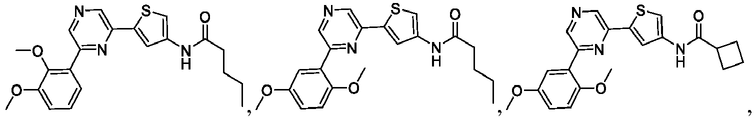

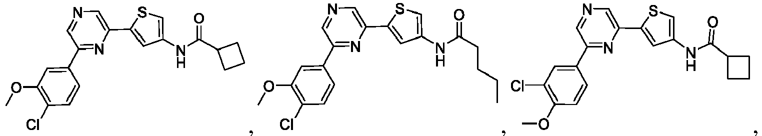

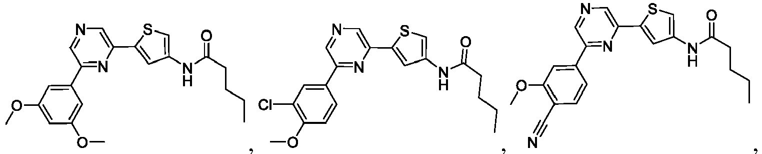

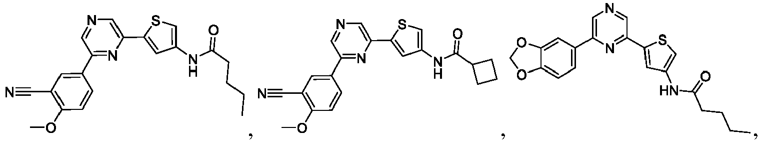

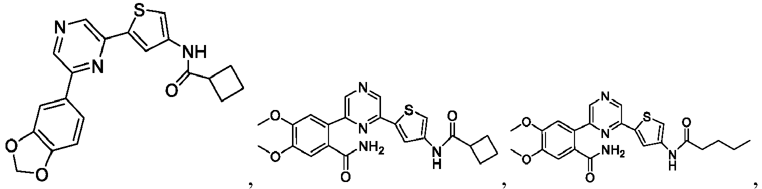

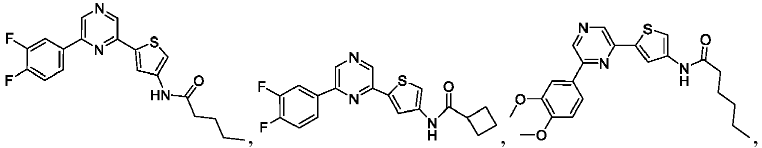

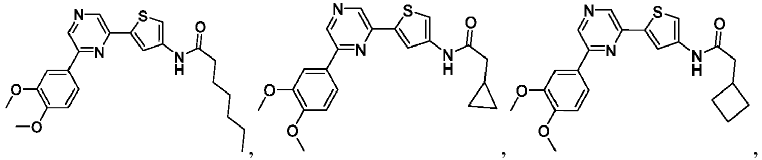

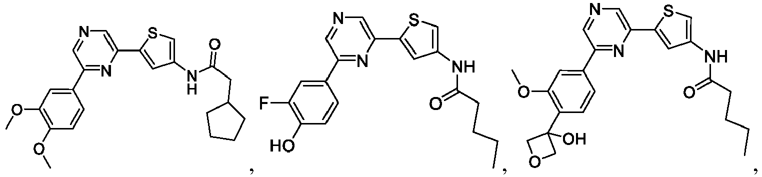

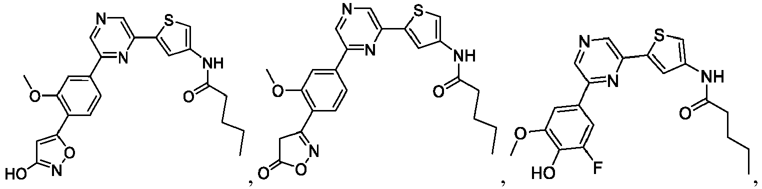

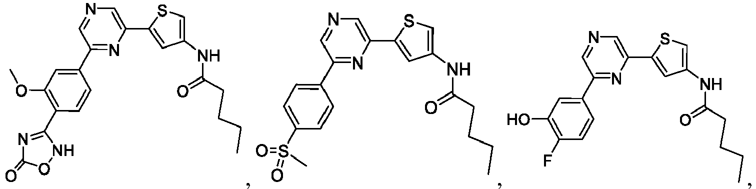

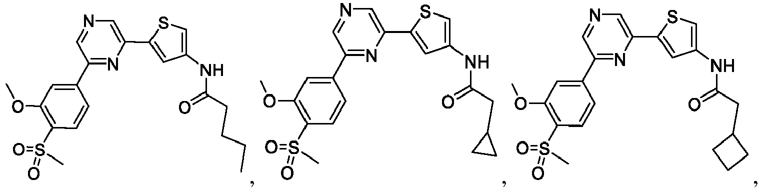

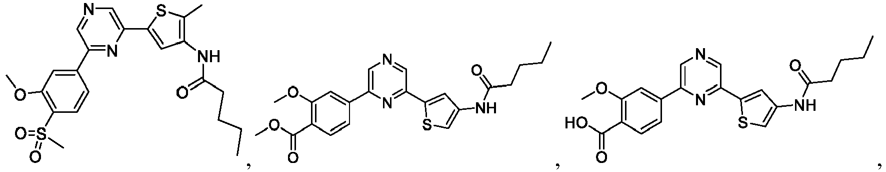

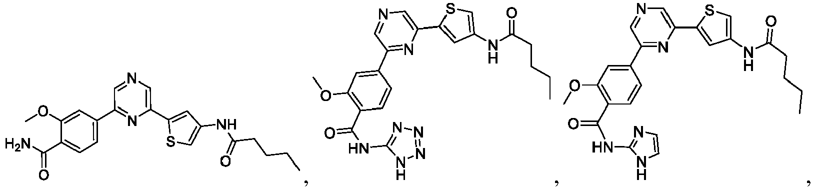

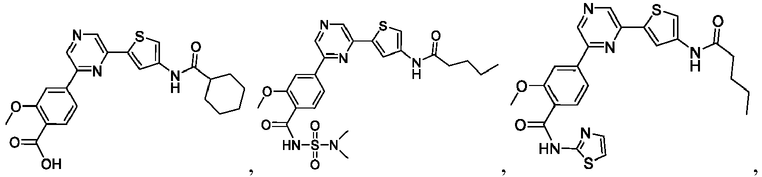

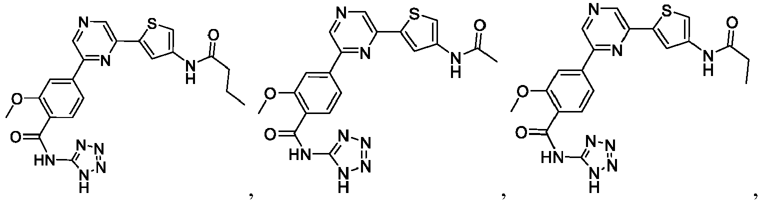

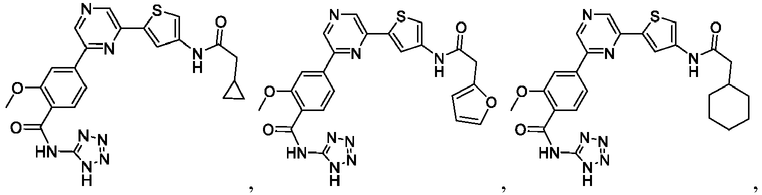

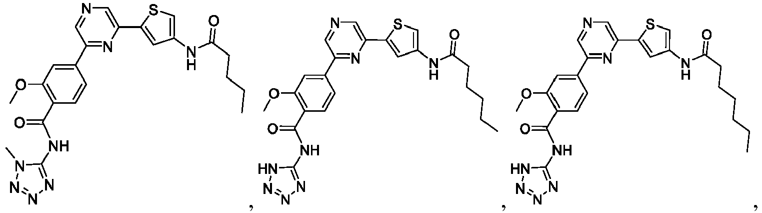

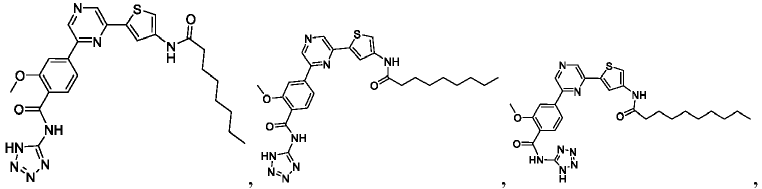

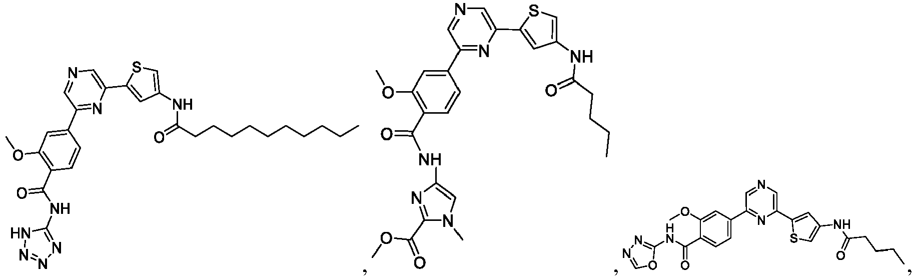

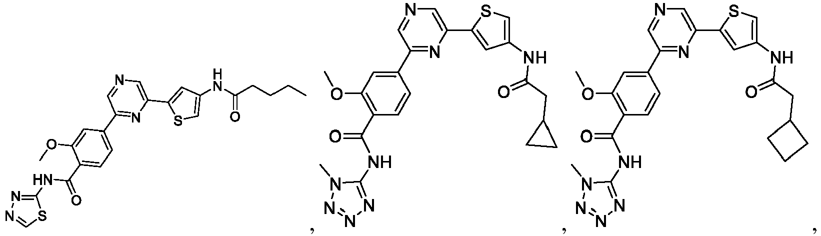

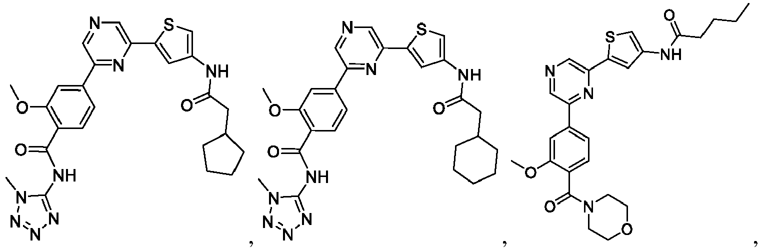

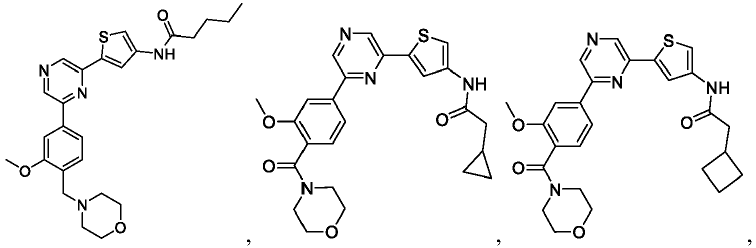

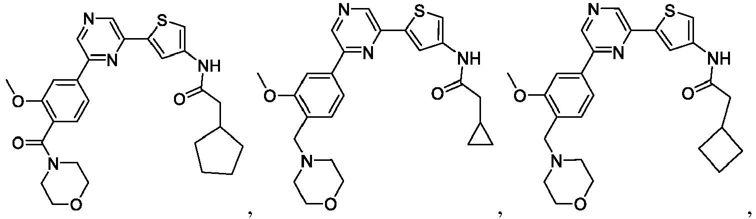

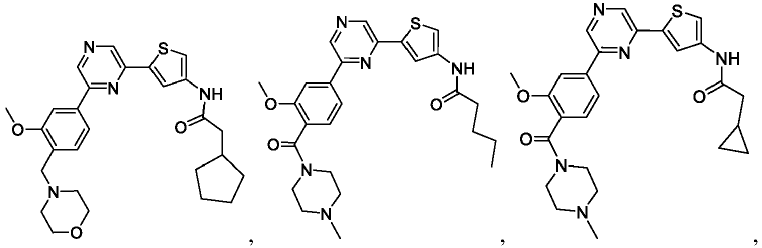

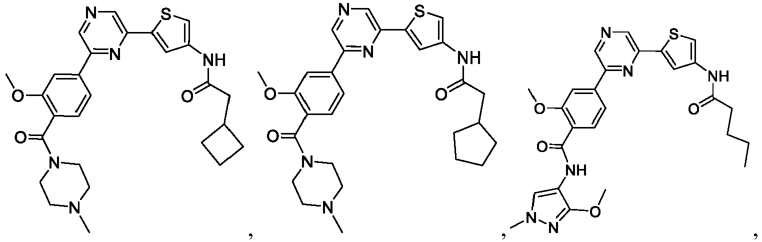

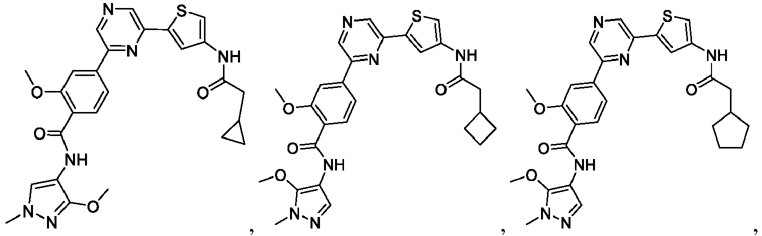

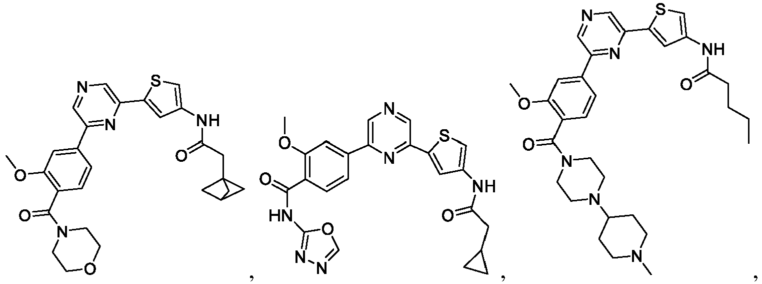

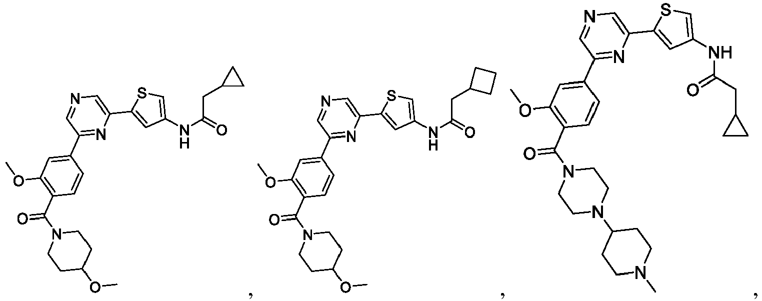

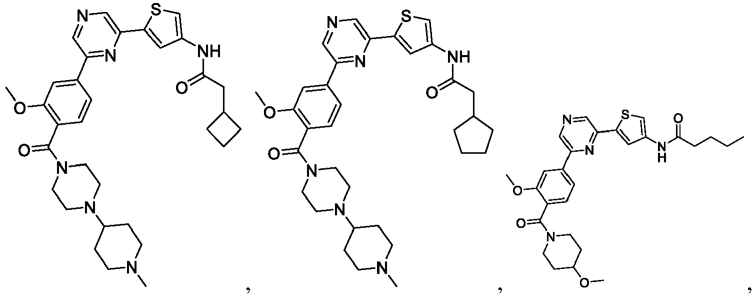

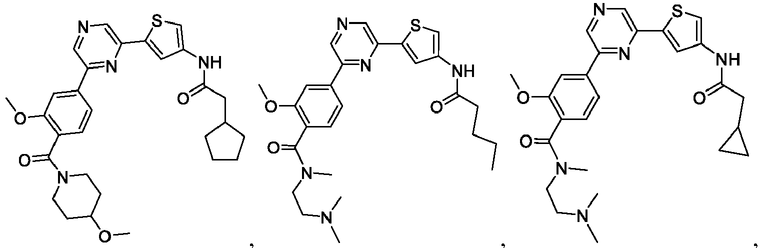

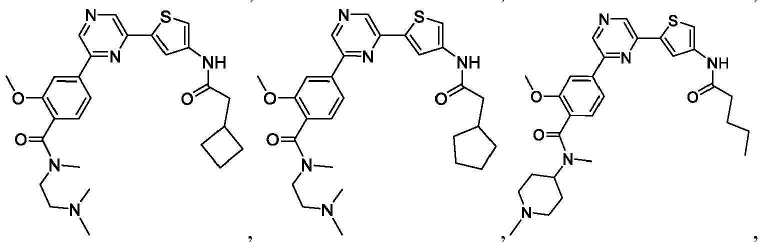

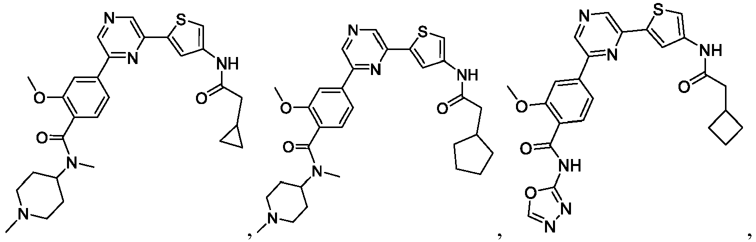

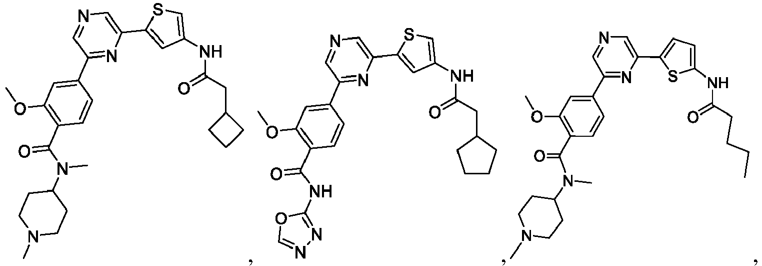

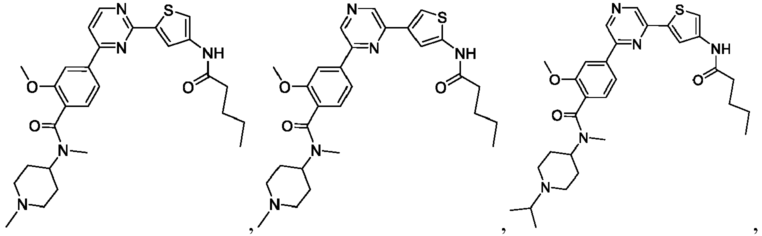

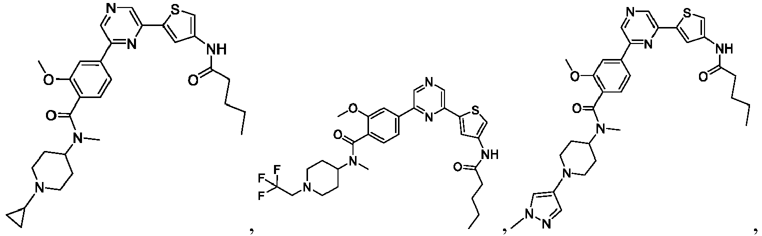

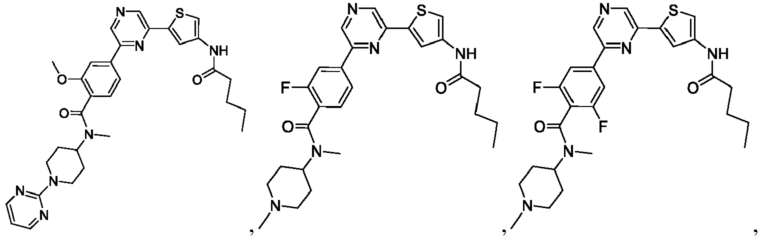

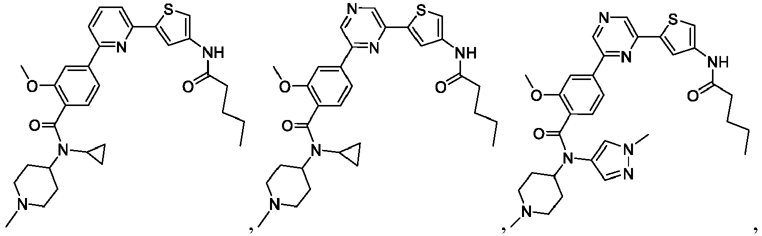

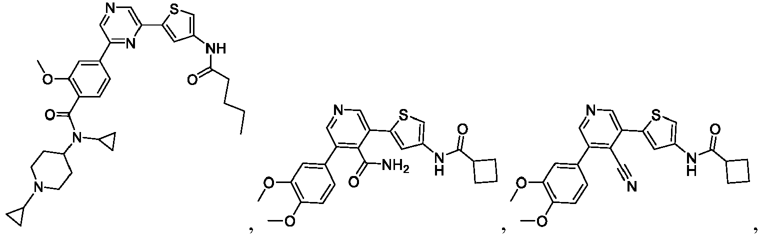

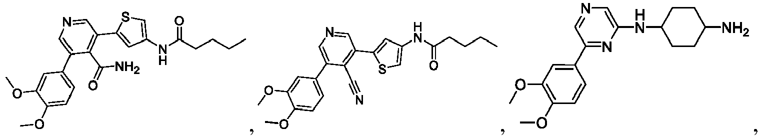

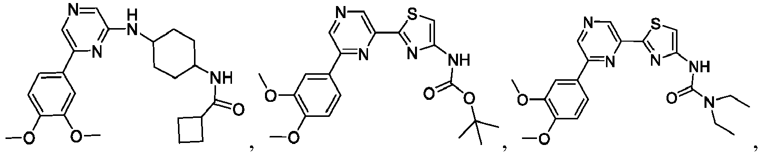

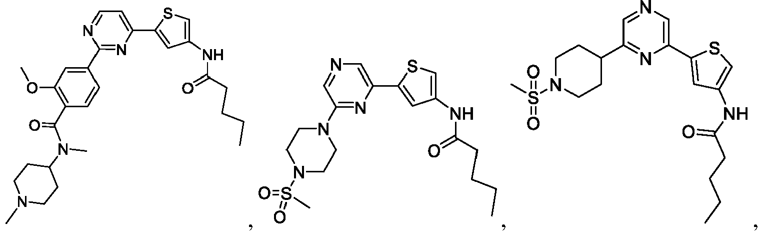

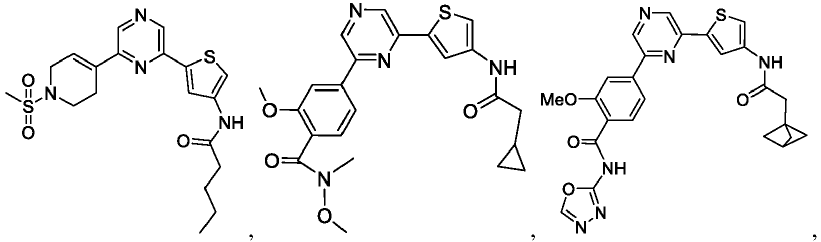

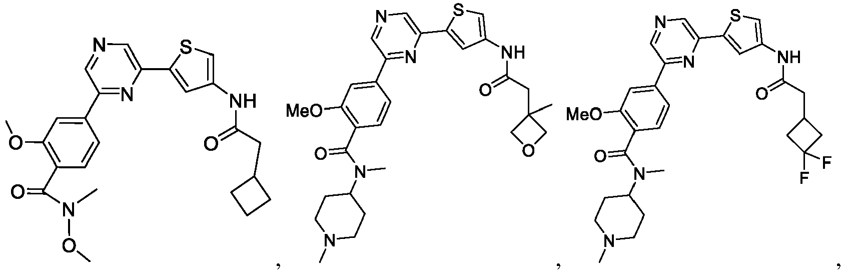

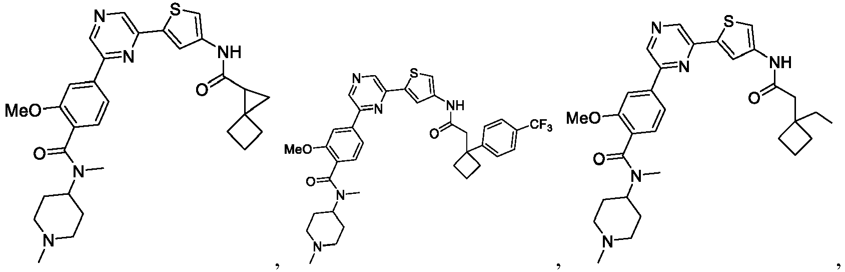

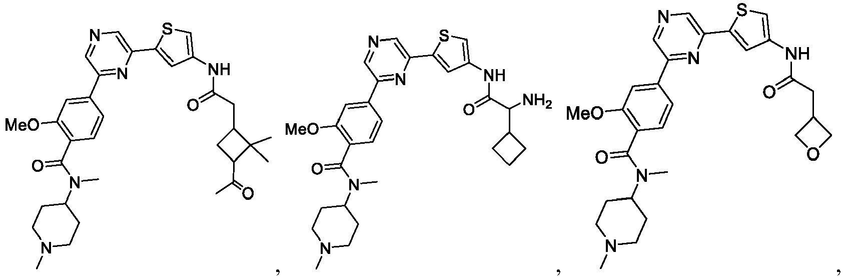

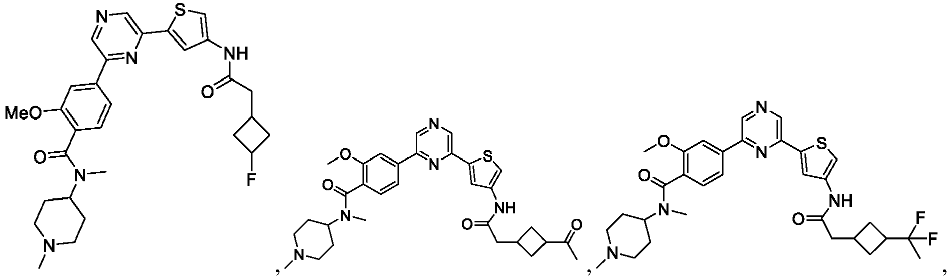

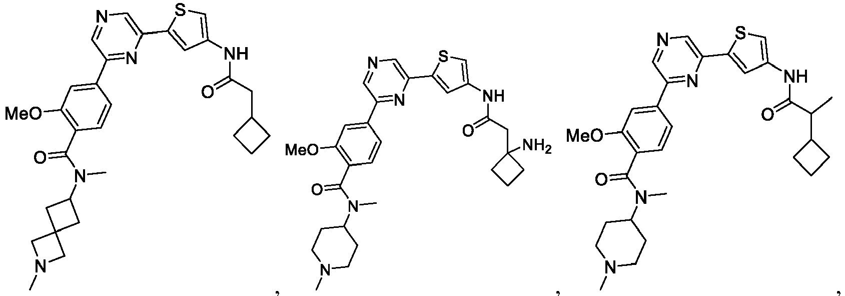

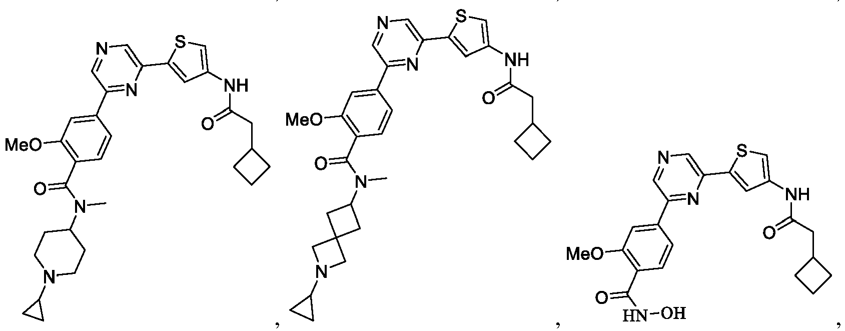

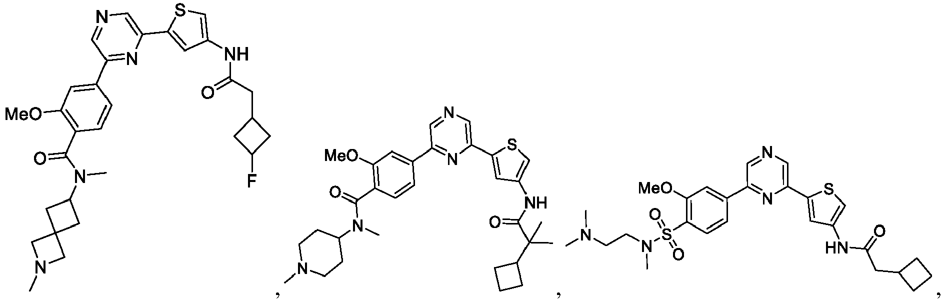

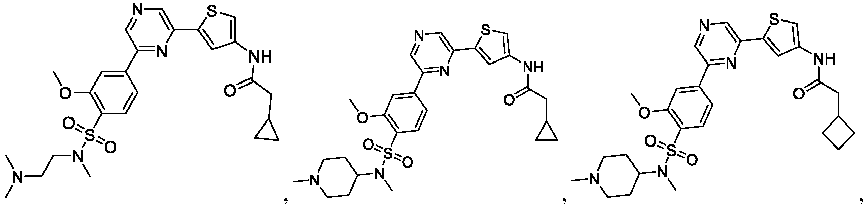

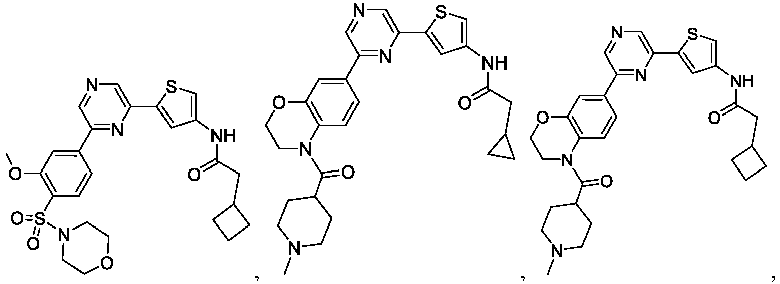

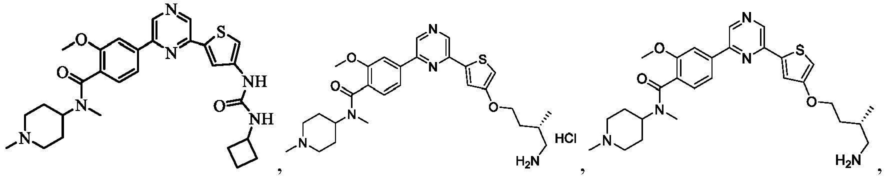

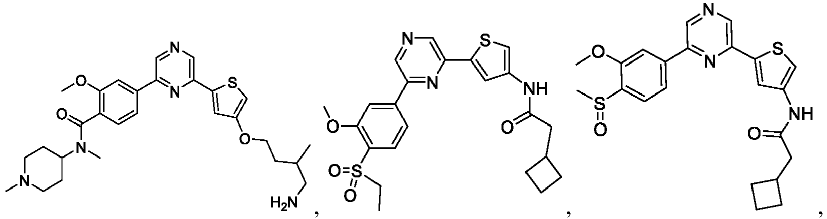

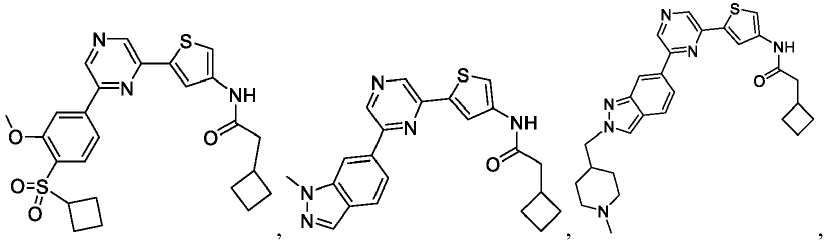

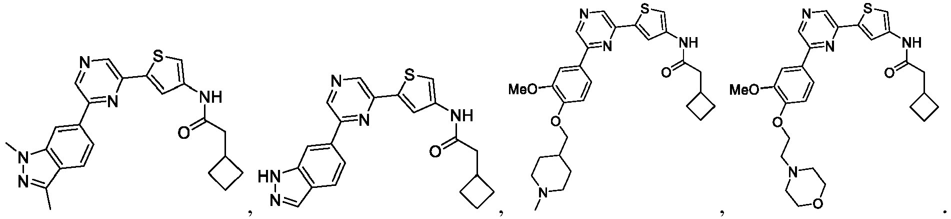

In the second aspect of the present invention, provided is a pharmaceutical composition, comprising the compound of formula I as described in the first aspect, or a stereoisomer, an optical isomer, a pharmaceutically acceptable salt, a prodrug or a solvate thereof, and pharmaceutically acceptable carriers.

In another preferred embodiment, provided is a preparation method for a pharmaceutical composition, comprising a step of: mixing pharmaceutically acceptable carriers with the compound as described in the first aspect of the present invention, or a stereoisomer, an optical isomer, a pharmaceutically acceptable salt, a prodrug, or a solvate thereof, thereby forming the pharmaceutical composition.

In another preferred example, the compound of the present invention can be prepared into powders, tablets, granules, capsules, solutions, emulsions, suspensions, etc.

In the third aspect of the present invention, provided is a use of the compound as described in the first aspect, or a stereoisomer, an optical isomer, a pharmaceutically acceptable salt, a prodrug or a solvate thereof, or the pharmaceutical composition as described in the second aspect, in the preparation of a drug for treating or preventing a disease associated with the activity or expression of Drak2 kinase, or in the preparation of a drug for inhibiting the activity of Drak2 kinase.
preferably, the disease is cancer, autoimmune disease, or metabolic disease;
wherein, the cancer is preferably selected from: malignant lymphoma, acute myeloid leukemia, acute lymphocytic leukemia, chronic lymphocytic leukemia, chronic myeloid leukemia, diffuse large B-cell lymphoma, multiple myeloma, non Hodgkin lymphoma, pseudomyxoma, intrahepatic cholangiocarcinoma, hepatoblastoma, liver cancer, thyroid cancer, colon cancer, testicular cancer, myelodysplastic syndrome, glioblastoma, basal cell carcinoma, breast cancer, brain cancer, adrenal cancer, renal cancer, nephroblastoma, stomach cancer, gastrointestinal stromal cancer, pituitary adenoma, pancreatic cancer, gallbladder cancer, cholangiocarcinoma, colon cancer, rectal cancer, small intestine cancer, duodenal carcinoma, retinoblastoma, choroidal melanoma, ampullary cancer, bladder cancer, peritoneal cancer, parathyroid carcinoma, non sinus cancer, small cell lung cancer, non small cell lung cancer (lung adenocarcinoma, lung squamous cell carcinoma), astrocytoma, esophageal cancer, glioma, neuroblastoma, malignant soft tissue tumor, malignant bone tumor, malignant mesothelioma, malignant melanoma, eye cancer, vulvar cancer, ureteral cancer, urethral cancer, tumors with unknown primary sites, carcinoma of penis, oral cancer, lip cancer, pharyngeal cancer, epithelial ovarian cancer, ovarian gernital carcinoma, cervical cancer, endometrial cancer, uterine sarcoma, prostate cancer, vaginal cancer, Paget's disease, tonsil cancer, anal cancer, rhabdomyosarcoma, Kaposi sarcoma, sarcoma, tongue cancer, laryngeal cancer, pleural cancer, thymic cancer, and combinations thereof;
the autoimmune disease is preferably selected from: inflammatory colitis, Crohn's disease, Behcet's disease, multiple sclerosis, macular degeneration, arthritis, encephalitis, viral meningitis, and combinations thereof;
the metabolic disease is preferably selected from diabetes, and the diabetes is preferably type I diabetes or type II diabetes, more preferably type II diabetes.

It should be understood that, within the scope of the present invention, each of the above technical features of the present invention and each of the technical features specifically described in the following (such as the embodiments) can be combined with each other to constitute a new or preferred technical solution. Due to space limitations, It will not be repeated herein.

### EMBODIMENTS FOR CARRYING OUT THE INVENTION

The inventor, through extensive and in-depth research, unexpectedly discovered for the first time a new Drak2 inhibitor with a novel structure and excellent biological activity and safety. The present invention was completed on this basis.

### TERMS

As used herein, unless otherwise specified, the terms used have a general meaning known to those skilled in the art.

When a substituent is described by a conventional formula written from left to right, the substituent likewise includes the chemically equivalent substituent obtained when the formula is written from right to left. For example, -CH₂O- is equivalent to -OCH₂-.

As used herein, term "about" means that the value may change by no more than 1% from the enumerated value when used in reference to a specific enumerated value. For example, as used herein, the expression "about 100" includes all values between 99 and 101 and (e. g., 99.1, 99.2, 99.3, 99.4, etc.).

As used herein, term "contain" or "include (comprise)" may be open, semi-closed, and closed. In other words, said term also includes "consisting essentially of" or "consisting of".

As used herein, the term "alkyl" includes a linear or branched alkyl. For example, C1-C6 alkyl represents a straight or branched alkyl group with 1-6 carbon atoms. The alkyl group is preferably C1-C15 alkyl, more preferably C1-C10 alkyl, more preferably C1-C6 alkyl, more preferably C1-C4 alkyl, and more preferably C1-C3 alkyl. Alkyl includes but are not limited to methyl, ethyl, propyl, iso-propyl, butyl, isobutyl, tert-butyl, etc. As used hererin, alkyl may be optionally substituted or unsubstituted. Substituted alkyl includes halogenated alkyl, benzyl, etc.

As used herein, the term "alkenyl" includes a linear or branched alkenyl. For example, C2-C6 alkenyl refers to a straight or branched alkenyl with 2-6 carbon atoms, such as vinyl, allyl, 1-propenyl, isopropenyl, 1-butenyl, 2-butenyl, or similar groups.

As used herein, the term "alkynyl" includes a linear or branched alkynyl. For example, C₂-C₆ alkynyl refers to a straight or branched alkynyl containing 2-6 carbon atoms, for example ethynyl, propynyl, butynyl, or similar groups.

As used herein, the term "cycloalkyl" refers to a cyclic alkyl group having a specified number of carbon atoms. Such as, "C3-C10 cycloalkyl" refers to a cycloalkyl group having 3-10 carbon atoms (preferably 3, 4, 5, 6, 7 or 8). It may be monocyclic, such as cyclopropyl, cyclobutyl, cyclopentyl, cyclohexyl, or the like. It may be bicyclic, such as a bridged ring or a spiro ring. In the present invention, the cycloalkyl group is intended to include substituted cycloalkyl group.

As used herein, the term "C1-C6 alkoxy" refers to a linear or branched alkoxy group having 1-6 carbon atoms, with a formula represented by C1-C6 alkyl-O-, for example, methoxy, ethoxy, propoxy, isopropoxy, butoxy, isobutyloxy, tert-butoxy, etc.

As used in this article, the term "cycloalkyl" refers to a cyclic alkyl group containing a specified number of C atoms, for example, "C3-C12 cycloalkyl" refers to a cycloalkyl group with 3-12 (e.g. 3, 4, 5, 6, 7, 8, 9, 10, 11, or 12) carbon atoms. It may be monocyclic, such as cyclopropyl, cyclobutyl, cyclopentyl, cyclohexyl, or the like. It may be bicyclic, such as a bridged ring or a spiro ring. The cycloalkyl group can also be fused with aryl, heteroarl or heterocyclyl, wherein the ring connected to the parent structure is the cycloalkyl group, such as 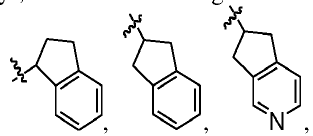 etc. As used herein, the cycloalkyl group is intended to include substituted cycloalkyl group.

As used herein, "heterocyclyl" refers to a saturated or partially saturated cyclic group with heteroatoms selected from N, S, and O, and "3-12 membered heterocyclyl" refers to a saturated or partially saturated cyclic group with 3-12 (such as 3, 4, 5, 6, 7, 8, 9, 10, 11, or 12) atoms, and wherein 1-3 (such as 1, 2, or 3) atoms are heteroatoms selected from the group consisting of N, S, and O. It can be a monocyclic ring, or can also be a bicyclic ring, such as a bridged ring or a spiro ring. The 3-12-membered heterocyclyl is preferably 3-8-membered heterocyclyl, and more preferably 3-6 membered or 6-8-membered heterocyclyl. Specific examples may be oxetanyl, azetidinyl, tetrahydro-2H-pyranyl, piperidinyl, piperazinyl, tetrahydrofuryl, morpholinyl and pyrrolidinyl, etc.. The heterocyclyl can be fused with heteroaryl, aryl, or cycloalkyl rings, wherein the ring connected to the parent structure is heterocyclyl, such as 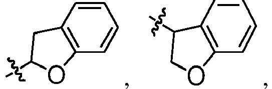 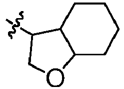 etc.

As used herein, "aryl" refers to an aromatic ring group which does not contain heteroatoms on the ring, and "C6-C10 aryl" means an aromatic ring group with 6 to 10 carbon atoms which does not contain heteroatoms on the ring. Said aryl can be fused with heteroaryl, heterocyclyl, and cycloalkyl rings, wherein the ring connected to the parent structure is the aryl ring. Such as phenyl (i.e. six membered aromatic ring), naphthyl, etc., wherein six membered aromatic ring is also intended to include six membered aromatic group fused with 5-6-membered cycloalkyl and six membered aromatic group fused 5-6-membered heterocyclic alkyl. Aryl may be optionally substituted or unsubstituted.

As used herein, the term "heteroaryl" refers to a cyclic aromatic group having 1-3 (e.g., 1, 2 or 3) heteroatoms selected from the group consisting of: N, S and O; "5-12 membered heteroaryl" refers to a cyclic aromatic group having 5-12 (e.g., 5, 6, 7, 8, 9, 10, 11, or 12) atoms and wherein 1-3 (e.g., 1, 2 or 3) atoms are heteroatoms selected from N, S and O. It can be monocyclic ring, or it can also be fused. Specific examples may be pyridyl, pyridazinyl, pyrimidinyl, pyrazinyl, triazinyl, pyrrolyl, pyrazolyl, imidazolyl, (1,2,3)-triazolyl, and (1,2,4)-triazolyl, tetrazolyl, furyl, thienyl, isoxazolyl, thiazolyl, oxazolyl, etc.. Heteroaryl may be fused with an aromatic, heterocyclic, or cycloalkyl ring, wherein the ring connected to the parent structure is a heteroaryl ring. Heteroaryl may be optionally substituted or unsubstituted. When substituted, the substituent may be one or more of the following groups, and independently selected from the group consisting of: alkyl, deuterated alkyl, halogenated alkyl, alkoxyl, halogenated alkoxyl, alkenyl, alkenyl, alkylthiol, alkylamino, halogen, amino, nitro, hydroxyl, thiol, cyano, cycloalkyl, heterocyclyl, aryl, heteroaryl, cycloalkylthiol, oxo, amide, sulfonamido, formyl, formamido, carboxyl, and carboxylate, etc..

As used herein, "halogen" or "halogen atom" refers to F, Cl, Br and I. More preferably, the halogen or halogen atom is selected from F, Cl and Br.

As used herein, "amino" refers to -NH₂.

As used herein, "carboxyl" refers to -COOH.

As used herein, the term "amido" refers to a group having a structure of -CONRR', wherein, R and R' can independently represent hydrogen, alkyl, cycloalkyl, aryl, heterocyclyl, as defined above. R and R' any be the same or different in dialkylamino fragments.

As used herein, the term "sulfonamido" refers to a group having a structure of -SO₂NRR', wherein, R and R' can independently represent hydrogen, alkyl, cycloalkyl, aryl, and heterocyclyl, as defined above. R and R 'can be the same or different in dialkylamino fragments.

As used herein, the term "formyl" refers to a group containing -CHO.

As used herein, the term "formamido" refers to a group containing 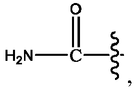 and formamido is also intended to include substituted formamido having a structure represented by formula 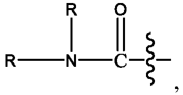 wherein R independently represents hydrogen, alkyl, cycloalkyl, cycloalkenyl, aryl, heteroaryl, and heterocyclyl, as defined above. Each R can be the same or different.

In the present invention, "alkylamino" refers to a structure having -N(R)(R') or -alkyl-N(R)(R'), wherein R and R' each independently represent hydrogen, alkyl, cycloalkyl, aryl, heteroaryl, and heterocycly 1, as defined above, and R and R' may be the same or different. "C1-C6 alkylamino" includes but is not limited to: NHCH₃, N(CH₃)₂, NHCH₂CH₃, N(CH₂CH₃)₂, N(CH₃)(CH₂CH₃), CH₂NHCH₃, CH₂CH₂NHCH₃, CH₂CH₂CH₂NHCH₃, CH₂N(CH₃)₂, CH₂CH₂N(CH₃)₂, CH₂CH₂CH₂N(CH₃)₂, CH₂N(CH₃)(CH₂CH₃), CH₂CH₂N(CH₃)(CH₂CH₃), CH₂CH₂CH₂N(CH₃)(CH₂CH₃).

As used herein, "sulfoxide group" refers to a structure having -S(O)-R, wherein R independently represents hydrogen, alkyl, cycloalkyl, aryl, heteroaryl, and heterocyclyl, as defined above.

As used herein, "sulfonyl" refers to a structure having -S(O)₂-R, wherein R independently represents hydrogen, alkyl, cycloalkyl, aryl, heteroaryl, and heterocyclyl, as defined above.

As used herein, "ester group" refers to a structure having -C(O)-O-R or R-C(O)-O-, wherein, R each independently represent hydrogen, alkyl, cycloalkyl, aryl, heteroaryl, and heterocyclyl, as defined above.

As used herein, "C3-C12 cycloalkyl-C1-C6 alkyl" refers to -C3-C12 cycloalkyl-C1-C6 alkyl or C3-C12 cycloalkyl-C1-C6 alkyl-. "3-12 membered heterocyclyl-C1-C6 alkyl" has a similar meaning.

As used herein, "optional" refers to that the event or condition subsequently described may, but is not must occur, and the description includes situations in which said event or condition occurs, and situations in which said event or condition does not occur.

As used herein, the term "substituted" indicates that one or more hydrogen atoms on a specific group are replaced by specific substituents. The specific substituent is the substituent described above, or the substituent appeared in each example. Unless otherwise specified, a substituted group may have a substituent selected from a specific group at any substitutable site of that group, and the substituent may be the same or different in each position. It should be understood by those skilled in the art that the combinations of substituents contemplated by the present invention are those that are stable or chemically achievable.

Unless the groups described in the present invention being specifically stated as "substituted or unsubstituted", the groups of the present invention can be substituted by substituents selected from the group consisting of deuterium, halogen, cyano, nitro, hydroxyl, amino, C1-C6 alkyl, C2-C6 alkenyl, C2-C6 alkynyl, C1-C6 alkoxy, 3-10 membered heterocycloalkyl, C3-C10 cycloalkyl, 5-12 membered heteroaryl, C6-C12 aryl.

As used herein, the term "more" independently refers to 2, 3, 4, and 5.

Unless otherwise specified, the structural formula described in the present invention is intended to include all isomeric forms (e. g., enantiomeric, diastereomeric, and geometric (or conformational) isomers): for example, R and S configurations of asymmetric centers, (Z) and (E) isomers of double bonds, etc. Thus, a single stereochemical isomer of the compound of the invention or a mixture of its enantiomers, diastereomers or geometric isomers (or conformational isomers) is within the scope of the invention.

As used herein, the term "tautomeric isomer" indicates that structural isomers with different energies can exceed low energy barriers and thus transform into each other. For example, proton tautomers (i.e. proton shift) include tautomerism through proton transfer, such as 1H-indazole and 2H-indazole. Valence tautomers include mutual transformations through the recombination of some bonding electrons.

As used herein, the term "solvate" refers to complexes formed of the compound of the present invention and solvent molecules in any specific ratio.

### Active Ingredients

As used herein, "the compound of the present invention" refers to a compound of formula I, and also includes a stereoisomer, a pharmaceutically acceptable salt, a prodrug or a solvate of the compound of formula I.

The compound of the present invention may contain one or more chiral carbon atoms and may therefore produce enantiomers, diastereomers, and other stereoisomeric forms. Each chiral carbon atom may be defined as (R)- or (S)- based on stereochemistry. The present invention is intended to include all possible isomers, as well as racemates and optically pure forms thereof. Racemates, diastereomers or enantiomers can be selected as starting materials or intermediates in the preparation of the compounds of the present invention. Isomers with optical activity can be prepared using chiral synthons or chiral reagents, or resolved using conventional techniques, such as crystallization and chiral chromatography.

Conventional techniques for the preparation/separation of individual optical isomers (i.e., enantiomers and diastereomers) include chiral synthesis from appropriate optically pure precursors, or resolution of the racemate (or racemate of salts or derivatives) using, for example, chiral high performance liquid chromatography, see, for example, Gerald Gübitz and Martin G. Schmid (Eds.), Chiral Separations, Methods and Protocols, Methods in Molecular Biology, Vol. 243, 2004;A.M. Stalcup, Chiral Separations. Annu. Rev. Anal. Chem. 3:341-63, 2010; Fumiss et al. (eds.), VOGEL'S ENCYCLOPEDIA OF PRACTICAL ORGANIC CHEMISTRY 5.sup.TH ED., Longman Scientific and Technical Ltd., Essex, 1991, 809-816; Heller, Acc. Chem. Res. 1990, 23, 128.

The present invention also includes isotopically labeled compounds (i.e. isotopic derivatives), equivalent to the original compounds disclosed herein. However, in reality, it is common for one or more atoms to be replaced by atoms with different atomic weights or mass numbers. Examples of isotopes in the isotopic derivatives of the present invention include hydrogen, carbon, nitrogen, oxygen, phosphorus, sulfur, fluorine, and chlorine isotopes, such as ²H, ³H, ¹³C, ¹¹C, ¹⁴C, ¹⁵N, ¹⁸O, ¹⁷O, ³¹P, ³²P, ³⁵S, ¹⁸F, and ³⁶Cl, respectively. The isotopic derivatives of the compound of the present invention are all within the scope of protection of the present invention. In this article, compounds labeled with ³H and compounds labeled with ¹⁴C are useful in tissue distribution experiments of drugs and substrates. The preparation and detection of tritium (i.e. ³H) labeled compounds and carbon-14 (i.e. ¹⁴C) labeled compounds are relatively easy, making them the preferred isotopes. In addition, substitution of heavier isotopes such as deuterium, also known as ²H, has advantages in certain therapies due to good metabolic stability, such as increasing half-life in vivo or reducing dosage. Therefore, in some cases, they can be given priority consideration. Isotope labeled compounds can be prepared using general methods by replacing non isotope reagents with readily available isotope labeled reagents, using the scheme disclosed in the example.

As used herein, the term "pharmaceutically acceptable salt" includes pharmaceutically acceptable acid addition salts and pharmaceutically acceptable base addition salts.

The term "pharmaceutically acceptable acid addition salts" refers to salts formed with inorganic or organic acids that retain bioavailability of the free base without exerting other adverse effects. The inorganic acid salts include but are not limited to hydrochloride, hydrobromide, sulfate, nitrate, phosphate, etc.; the organic acid salts include but are not limited to formate, acetate, 2,2-dichloro acetate, trifluoroacetate, propionate, caproate, caprylate, caprate, undecylenate, glycolate, gluconate, lactate, sebacate, adipate, glutarate, malonate, oxalate, maleate, succinate, fumarate, tartrate, citrate, palmitate, stearate, oleate, cinnamate, lauroleate, malate, glutamate, pyroglutamate, aspartate, benzoate, mesylate, benzene sulfonate, p-toluenesulfonate, alginate, ascorbate, salicylate, 4-aminosalicylate, naphthalene disulfonate, etc. These salts can be prepared by methods known in the art.

The term "pharmaceutically acceptable base addition salts" refers to salts formed with inorganic or organic bases that retain bioavailability of the free acid without exerting other adverse effects. Salts derived from inorganic bases include, but are not limited to, sodium salts, potassium salts, lithium salts, ammonium salts, calcium salts, magnesium salts, ferric salts, zinc salts, copper salts, manganese salts, aluminum salts, and the like. Preferred inorganic salts are ammonium salts, sodium salts, potassium salts, calcium salts and magnesium salts. Salts derived from organic bases include, but are not limited to, primary, secondary and tertiary amines; substituted amines, including natural substituted amines, cyclic amines and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucamine, methylglucamine, theobromine, purine, piperazine, piperidine, N-ethylpiperidine, polyamine resin, and the like. Preferred organic bases include isopropylamine, diethylamine, ethanolamine, trimethylamine, dicyclohexylamine, choline and caffeine. These salts can be prepared by methods known in the art.

If a synthesis of a specific enantiomer of the compound of the present invention is to be designed, it can be asymmetrically synthesized or derivatized using chiral excipients, whereby the resulting diastereoisomeric mixture is isolated, and then the chiral excipients is removed to obtain the pure enantiomer. In addition, if the molecule contains a basic functional group, such as an amino acid, or an acidic functional group, such as carboxyl group, a suitable optically active acid or base can be used to form a diastereoisomeric salt with it, which can then be separated by conventional means, such as separation crystallization or chromatography to obtain pure enantiomer.

As used herein, the compound of the present invention can be substituted by any number of substituents or functional groups to expand the scope of coveragethereof. Usually, the term "substitution" refers to the substitution of hydrogen radicals with a specified substituent. When a plurality of positions in a particular structure are replaced by a plurality of particular substituents, the substituents at each position may be the same or different. The term "substitution" used herein includes all allowed organic compound substitutions. Broadly speaking, the allowed substituents include non-cyclic, cyclic, branched, non-branched, carbocyclic, and heterocyclic, aromatic, and non aromatic organic compounds. In this article, heteroatoms such as nitrogen may have hydrogen substituents or any allowed organic compounds as described above to complement its valence. Furthermore, the present invention is not intended to limit in any way the permissible substitution of organic compounds. The present invention considers that the combination of substituents and variable groups in the form of stabilized compounds is good in the treatment of diseases. The term "stabilized" herein refers to a compound with stability which is sufficient to maintain the integrity of the compound structure when being detected over a sufficient long period of time, preferably is effective over a sufficient long period of time, and is used herein for the foregoing purposes.

The metabolites of the compound shown in formula I and its pharmaceutically acceptable salt, as well as prodrugs that can convert in vivo into the compound shown in formula I and its pharmaceutically acceptable salt are also included in the scope of protection of the present invention.

### Preparation Method of the Compound

The method for preparing the compound shown in formula I is described in the following schemes. In some cases, the order of steps for executing the reaction scheme can be changed to promote the reaction or avoid unnecessary side reaction products. The compound of the present invention can also be conveniently prepared by combining various synthesis methods described in this specification or known in the art, and such combinations can be easily carried out by those skilled in the art to which the present invention belongs.

Usually, in the preparation process, each reaction is carried out in an inert solvent at from room temperature to reflux temperature (such as 0 °C -150 °C, preferably 10 °C -100 °C). The reaction time is usually 0.1-60 hours, preferably 0.5-48 hours.

Preferably, the compound of the present invention can be prepared by the following methods:

### Method 1

### Method A:

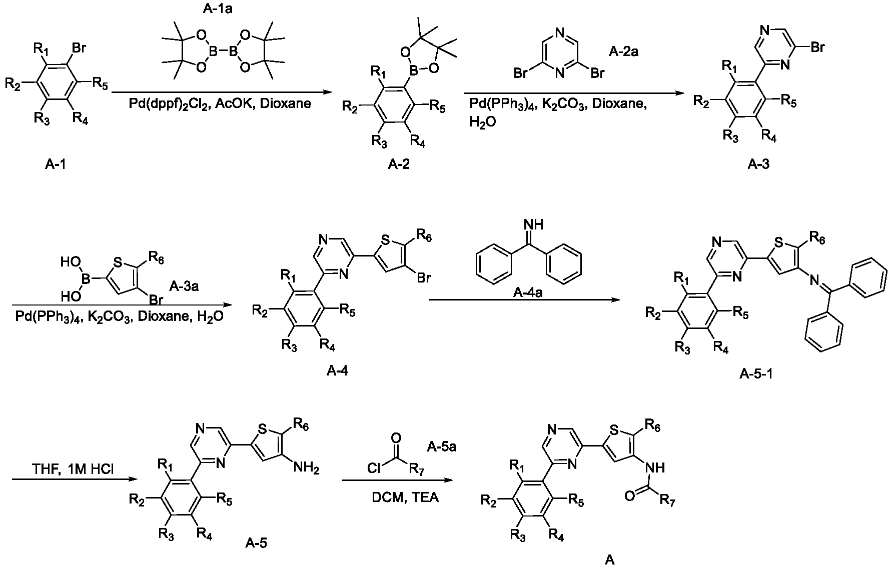

S1) Under appropriate alkaline conditions (such as but not limited to potassium acetate, etc.), selecting appropriate solvents (such as but not limited to Dioxane, etc.), and at an appropriate temperature (such as but not limited to 80 °C), under the catalysis with appropriate coupling catalysts (such as but not limited to Pd (dppf)₂), reacting compound (A-1)with corresponding boron ester or boric acid compounds (A-1a) (such as but not limited to Pinacolboronates) to obtain compound (A-2);
S2) Under appropriate alkaline conditions (such as but not limited to potassium carbonate, etc.), selecting suitable solvent (such as but not limited to a mixed solvent of Dioxane and water, etc.), and at an appropriate temperature (such as but not limited to 80 °C), under the catalysis with an appropriate coupling catalyst (such as but not limited to tetrakis(triphenylphosphine)palladium), reacting compound (A-2) with the corresponding aryl halide compound (A-2a) (such as but not limited to 2,6-dibromopyrazine) to obtain compound (A-3);
S3) Under appropriate alkaline conditions (such as but not limited to potassium carbonate, etc.), selecting appropriate solvents (such as but not limited to Dioxane, etc.), and at an appropriate temperature (such as but not limited to 60 °C), under the catalysis with appropriate coupling catalysts (such as but not limited to tetrakis(triphenylphosphine)palladium), reacting the compound (A-3) with the corresponding boric acid or boron ester compound (A-3a) to obtain compound (A-4);
S4) Under appropriate alkaline conditions (such as but not limited to cesium carbonate, etc.), selecting appropriate solvents (such as but not limited to toluene, etc.), and at an appropriate temperature (such as but not limited to 110°C), selecting appropriate ligand (such as but not limited to BINAP), under the catalysis with appropriate coupling catalysts (such as but not limited to Pd₂(dba)₃), reacting compound (A-4) with corresponding nitrogen-containing compound (A-4a) to obtain compound (A-5-1);
S5) Selecting appropriate solvents (such as but not limited to tetrahydrofuran, etc.), selecting appropriate acids (such as but not limited to 1M hydrochloric acid), and subjecting compound (A-5-2) to deprotecting reaction to obtain compound (A-5);
S6) Selecting suitable solvent (such as but not limited to dichloromethane, etc.), selecting suitable base (such as but not limited to triethylamine), and reacting compound (A-5) with corresponding acyl chloride (A-5a) to obtain compound (A);

### Method B:

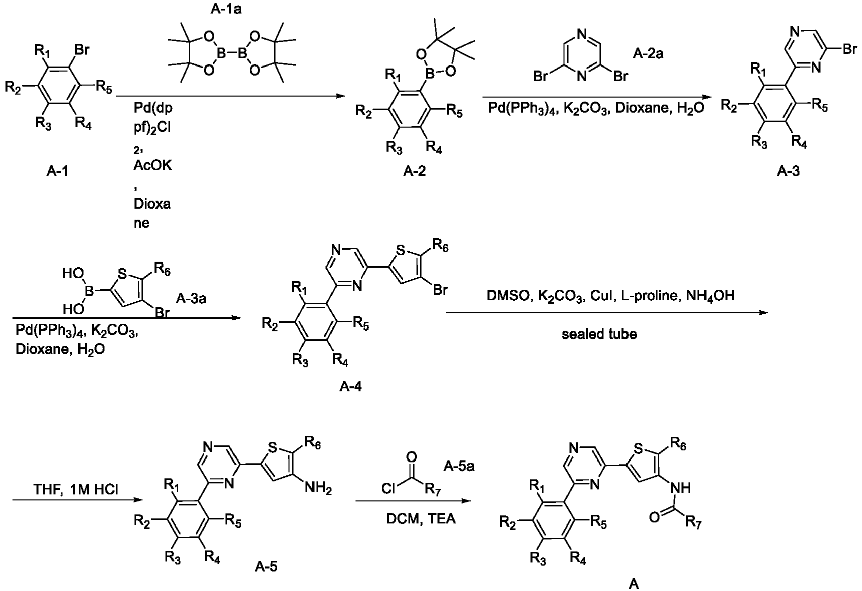

S1) Under appropriate alkaline conditions (such as but not limited to potassium acetate, etc.), selecting appropriate solvents (such as but not limited to Dioxane, etc.), and at an appropriate temperature (such as but not limited to 80 °C), under the catalysis with appropriate coupling catalysts (such as but not limited to Pd (dppf)₂), reacting compound (A-1) with corresponding boron ester or boric acid compounds (A-1a) (such as but not limited to Pinacolboronates) to obtain compound (A-2);
S2) Under appropriate alkaline conditions (such as but not limited to potassium carbonate, etc.), selecting suitable solvent (such as but not limited to a mixed solvent of Dioxane and water, etc.), and at an appropriate temperature (such as but not limited to 80 °C), under the catalysis with an appropriate coupling catalyst (such as but not limited to tetrakis(triphenylphosphine)palladium), reacting compound (A-2) with the corresponding aryl halide compound (A-2a) (such as but not limited to 2,6-dibromopyrazine) to obtain compound (A-3);
S3) Under appropriate alkaline conditions (such as but not limited to potassium carbonate, etc.), selecting appropriate solvents (such as but not limited to Dioxane, etc.), and at an appropriate temperature (such as but not limited to 60 °C), under the catalysis with appropriate coupling catalysts (such as but not limited to tetrakis(triphenylphosphine)palladium), reacting compound (A-3) with the corresponding boric acid or boron ester compound (A-3a) to obtain compound (A-4);
S4) Under appropriate alkaline conditions (such as but not limited to potassium carbonate, etc.), selecting appropriate solvents (such as but not limited to DMSO, etc.), and at an appropriate temperature (such as but not limited to 110°C), selecting appropriate ligand (such as but not limited to L-proline, etc.), under the catalysis with appropriate coupling catalysts (such as but not limited to cuprous iodide), reacting compound (A-4) with corresponding nitrogen-containing compound ammonia under pipe sealing condition to obtain compound (A-5);
S5) Selecting suitable solvent (such as but not limited to dichloromethane, etc.), selecting suitable base (such as but not limited to triethylamine), and reacting compound (A-5) with corresponding acyl chloride (A-5a) to obtain compound (A);

### Method 2

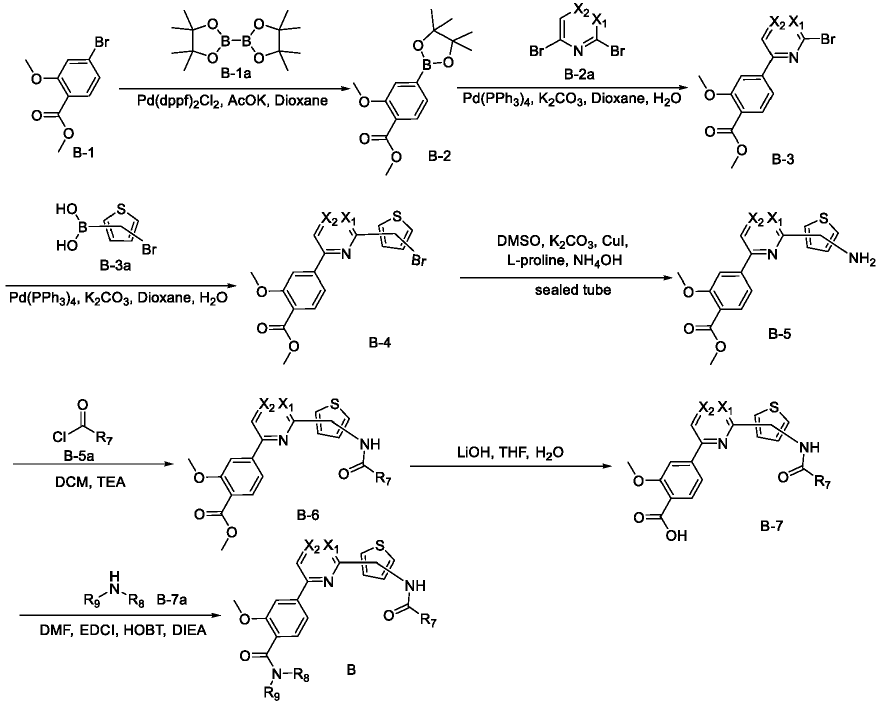

S1) Under appropriate alkaline conditions (such as but not limited to potassium acetate, etc.), selecting appropriate solvents (such as but not limited to Dioxane, etc.), and at an appropriate temperature (such as but not limited to 80 °C), under the catalysis with appropriate coupling catalysts (such as but not limited to Pd (dppf)₂), reacting compound (B-1) with corresponding boron esters or boric acid compounds (B-1a) (such as but not limited to pinacolboronates) to obtain compound (B-2);
S2) Under appropriate alkaline conditions (such as but not limited to potassium carbonate, etc.), selecting appropriate solvents (such as but not limited to a mixed solvent of Dioxane and water, etc.), and at an appropriate temperature (such as but not limited to 80 °C), under the catalysis with appropriate coupling catalysts (such as but not limited to tetrakis(triphenylphosphine)palladium), reacting compound (B-2) with the corresponding aryl halide compound (B-2a) to obtain compound (B-3);
S3) Under appropriate alkaline conditions (such as but not limited to potassium carbonate, etc.), selecting suitable solvent (such as but not limited to a mixed solvent of Dioxane and water, etc.), and at an appropriate temperature (such as but not limited to 60 °C), under the catalysis with an appropriate coupling catalyst (such as but not limited to tetrakis(triphenylphosphine)palladium), reacting compound (B-3) with the corresponding boric acid or boron ester compound (B-3a) to obtain compound (B-4);
S4) Under appropriate alkaline conditions (such as but not limited to cesium carbonate, etc.), selecting appropriate solvents (such as but not limited to DMSO, etc.), and at an appropriate temperature (such as but not limited to 110°C), selecting appropriate ligand (such as but not limited to L-proline, etc.), under the catalysis with appropriate coupling catalysts (such as but not limited to cuprous iodide), reacting compound (B-4) with corresponding nitrogen-containing compound ammonia under pipe sealing condition to obtain compound (B-5);
S5) Selecting suitable solvent (such as but not limited to dichloromethane), selecting suitable base (such as but not limited to triethylamine), and reacting compound (B-5) with corresponding acyl chloride (B -5a) to obtain compound (B-6);
S6) Selecting appropriate solvents (such as but not limited to a mixed solvent of tetrahydrofuran and water, etc.), selecting appropriate bases (such as but not limited to lithium hydroxide), and subjecting compound (B-6) to hydrolytic reaction to obtain carboxyl compound (B-7);
S7) Selecting suitable solvent (such as but not limited to DMF, etc.), selecting suitable base (such as but not limited to DIEA), selecting suitable coupling reagents (such as but not limited to EDCI+HOBT), and reacting compound (B-7) with corresponding amine (B -7a) to obtain compound (B-8);

### Method 3

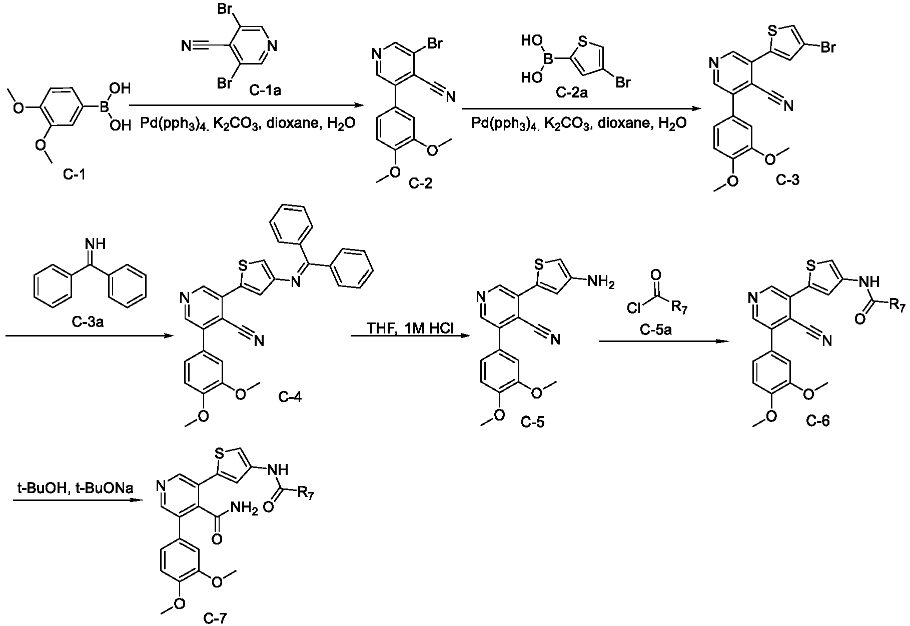

S1) Under appropriate alkaline conditions (such as but not limited to potassium carbonate, etc.), selecting suitable solvent (such as but not limited to a mixed solvent of Dioxane and water, etc.), and at an appropriate temperature (such as but not limited to 80 °C), under the catalysis with an appropriate coupling catalyst (such as but not limited to tetrakis(triphenylphosphine)palladium), reacting compound (C-1) with the corresponding aryl halide compound (C-1a) to obtain compound (C-2);
S2) Under appropriate alkaline conditions (such as but not limited to potassium carbonate, etc.), selecting suitable solvent (such as but not limited to a mixed solvent of Dioxane and water, etc.), and at an appropriate temperature (such as but not limited to 60 °C), under the catalysis with an appropriate coupling catalyst (such as but not limited to tetrakis(triphenylphosphine)palladium), reacting compound (C-2) with corresponding boric acid or boron ester compounds (C-2a) to obtain compound (C-3);
S3) Under appropriate alkaline conditions (such as but not limited to cesium carbonate, etc.), selecting appropriate solvents (such as but not limited to toluene, etc.), at appropriate temperatures (such as but not limited to 110 °C), selecting appropriate ligands (such as but not limited to BINAP), and under the catalysis with appropriate coupling catalysts (such as but not limited to Pd₂(dba)₃), reacting compound (C-3) with corresponding nitrogen-containing compound (C-3a) to obtain compound (C-4);
S4) Selecting appropriate solvents (such as but not limited to tetrahydrofuran, etc.), selecting appropriate acids (such as but not limited to 1M hydrochloric acid), and subjecting compound (C-4) to deprotecting reaction to obtain compound (C-5);
S5) Selecting suitable solvent (such as but not limited to dichloromethane, etc.), selecting suitable base (such as but not limited to triethylamine), and reacting compound (C-5) with corresponding acyl chloride (C-5a) to obtain compound (C-6);
S6) Selecting appropriate solvents (such as but not limited to tert butanol, etc.), selecting appropriate bases (such as but not limited to sodium tert-butoxide), and subjecting compound (C-6) to hydrolytic reaction to obtain amide compound (C-7).

### Pharmaceutical composition and method of administration

Due to the excellent inhibitory activity of the compound of the present invention on Drak2 kinase, a pharmaceutical composition with the compound of the present invention as the main active ingredient can be used for the prevention and/or treatment (stabilization, alleviation, or cure) of Drak2 kinase related diseases.

The pharmaceutical composition of the present invention comprises a safe and effective amount of the compound of the present invention and a pharmaceutically acceptable excipient or carrier. Wherein "safe and effective amount" refers to the amount of compound which is sufficient to significantly improve the condition, and not to generate severe side effects. Generally, the pharmaceutical composition contains 1-2000 mg the compound of present invention per dose, preferably, 10-200mg the compound of present invention per dose. Preferably, the "one dose" is one capsule or one pill.

"Pharmaceutically acceptable carrier" means one or more compatible solid or liquid fillers, or gelatinous materials which are suitable for human use and should be of sufficient purity and sufficiently low toxicity. "Compatible" used herein refers to the ability of each component of a pharmaceutical composition can be mixed with the compound of the present invention and with each other without appreciably reducing the efficacy of the compound. Some examples of pharmaceutically acceptable carrier include cellulose and derivatives thereof (such as sodium carboxymethylcellulose, sodium ethylcellulose, cellulose acetate, etc.), gelatin, talc, solid lubricant (such as stearic acid, magnesium stearate), calcium sulfate, vegetable oil (such as soybean oil, sesame oil, peanut oil, olive oil, etc.), polyol (such as propylene glycol, glycerol, mannitol, sorbitol, etc.), emulsifier (such as Tween^{®}), wetting agent (such as lauryl sodium sulfate), colorant, flavoring, stabilizer, antioxidant, preservative, pyrogen-free water, etc.

There is no special limitation on the administration mode of the compound or pharmaceutical compositions of the present invention, and the representative administration mode includes (but is not limited to): oral, parenteral (intravenous, intramuscular or subcutaneous) administration.

The solid dosage forms used for oral administration include capsules, tablets, pills, powders, and granules. In these solid dosage forms, the compound of the present invention are mixed with at least one conventional inert excipient (or carrier), such as sodium citrate or dicalcium phosphate, or mixed with any of the following components: (a) fillers or compatibilizer, for example, starch, lactose, sucrose, glucose, mannitol and silicic acid; (b) binders, for example, hydroxymethyl cellulose, alginates, gelatin, polyvinylpyrrolidone, sucrose and arabic gum; (c) humectant, such as, glycerol; (d) disintegrating agents such as agar, calcium carbonate, potato starch or tapioca starch, alginic acid, certain composite silicates, and sodium carbonate; (e) dissolution-retarding agents, such as paraffin; (f) absorption accelerators, for example, quaternary ammonium compounds; (g) wetting agents, such as cetyl alcohol and glyceryl monostearate; (h) adsorbents, for example, kaolin; and (i) lubricants such as talc, stearin calcium, magnesium stearate, solid polyethylene glycol, sodium lauryl sulfate, or the mixtures thereof. In capsules, tablets, and pills, the dosage form may also include buffers.

Solid dosage forms such as tablets, sugar pills, capsules, pills, and granules can be prepared using coating and shell materials, such as casings and other materials well-known in the art. They can contain opacifiers, and the compound of the present invention in the pharmaceutical composition can be released in a delayed mode in a certain part of the digestive tract. Examples of embedding components that may be employed are polymeric substances and waxes. If necessary, the compound of the present invention may also be formed into a microcapsules with one or more of the above excipients.

Liquid dosage forms for oral administration include pharmaceutically acceptable lotion, solutions, suspensions, syrups or tinctures. In addition to the compound of the present invention, the liquid dosage forms may contain any conventional inert diluents known in the art such as water or other solvents, solubilizers and emulsifiers, for example, ethanol, isopropanol, ethyl carbonate, ethyl acetate, propylene glycol, 1,3-butanediol, dimethyl formamide, as well as oil, in particular, cottonseed oil, peanut oil, corn germ oil, olive oil, castor oil and sesame oil, or the combination thereof.

In addition to these inert diluents, the pharmaceutical composition can also include additives such as wetting agents, emulsifiers and suspensions, sweeteners, correctors, and spices.

In addition to the compound of the present invention, suspensions can include suspending agents such as ethoxylated isooctadecanol, polyoxyethylene sorbitol and dehydrated sorbitol esters, microcrystalline cellulose, methanol aluminum and agar, or mixtures of these substances.

Pharmaceutical compositions for parenteral injection may include physiologically acceptable sterile aqueous or anhydrous solutions, dispersion liquid, suspensions or lotions, and sterile powders for re-dissoluting into sterile injectable solutions or dispersions. Suitable aqueous and non-aqueous carriers, diluents, solvents or excipients include water, ethanol, polyols, and their suitable mixtures.

The compound of the present invention can be administered alone or in combination with other pharmaceutically acceptable compound (such as EGFR inhibitor).

When co-administered, the pharmaceutical composition further comprises one or more (2, 3, 4, or more) other pharmaceutically acceptable compounds (such as Drak2 inhibitor). One or more (2, 3, 4, or more) of other pharmaceutically acceptable compounds (such as Drak2 inhibitor) may be used simultaneously, separately, or sequentially with the compounds of the present invention for preventing and/or treating diseases related with the activity or expression level of Drak2 kinase.

When the pharmaceutical compositions are used, a safe and effective amount of compound of the present invention is applied to a mammal (such as human) in need thereof, wherein the dose of administration is a pharmaceutically effective dose. For a person weighed 60 kg, the daily dose is usually 1-2000mg, preferably 20-500 mg. Of course, the particular dose should also depend on various factors, such as the route of administration, patient healthy status, which are within the skills of an experienced physician.

### The main advantages of the present invention include:

1. The compound of the present invention has a novel structure and excellent Drak2 kinase inhibitory effect;
2. The compound of the present invention has good pharmacokinetic and pharmacological properties.

### EMBODIMENTS FOR CARRYING OUT THE INVENTION

The present invention was further described hereafter in combination with specific embodiments. It should be understood that these examples are only used to illustrate the and not to limit the scope of the invention. The experimental methods without specific conditions in the following examples generally follow the conventional conditions or the conditions suggested by the manufacturer. Unless otherwise stated, percentages and parts are by weight.

### Example

As used herein, all temperatures were represented by °C, unless otherwise specified.

As used herein, percentages for yield were all mass percentages.

As used herein, all portions were by volume, and all percentages were by volume, unless otherwise specified.

In this article, the preparative chromatography of PTLC or TLC (thin layer chromatography) was carried out on a 20 × 20cm plate (500 µm thick silica gel); Biotage rapid chromatography system was used for silica gel chromatography.

In this article, ¹H NMR (hydrogen spectrum) was performed using a Bruker Ascend TM400 spectrometer at 400MHz, 298 ° K, and the chemical shift (ppm) of residual protons in deuterated reagents was given as a reference: δ (Chemical shift) for CDCl₃ was 7.26 ppm, δ for CD₃OD was 3.31 ppm, δ for DMSO-d6 was 2.50 ppm.

In this article, in LCMS (liquid chromatography-mass spectrometry) testing, Agilent Technologies 1200 series or 6120 quadrupole spectrometers were used for liquid chromatography. For liquid chromatography, the mobile phase consists of acetonitrile (A), water (B), and 0.01% formic acid. The eluent gradient is 5-95% A for 6.0 minutes, 60-95% A for 5.0 minutes, 80-100% A for 5.0 minutes, and 85-100% A for 10 minutes, a SBC1850 mm × 4.6 mm × 2.7 µm capillary column is used; mass spectrometry (MS) is determined by electrospray ionization mass spectrometry (ESI).

As used herein, the analysis conditions for high-performance liquid chromatography (HPLC) - mass spectrometry (MS) were as follows:
LC1 column: SB-C18 50 mm×4.6 mm×2.7 µm;
Temperature: 50°C
Eluent: acetonitrile/water from 5:95 to 95:5 (the above ratio was by volume)+0.01% formic acid, 6 minutes;
Flow: 1.5 mL/min, Injection: 5 µL;
Detection: PDA detector, 200-600 nm;
MS: Quality range 150-750 amu; Positive ion spray ionization.
LC2 column: SB-C18 50 mm×4.6 mm×2.7 µm;
Temperature: 50°C
Eluent: Acetonitrile/water from 5:95 to 95:5 (the above ratio was by volume)+0.05% TFA (trifluoroacetic acid) gradient, over 3.00 minutes;
Flow: 1.5 mL/min, Injection: 5 µL
Detection: PDA detector, 200-600 nm;
MS: Quality range 150-750 amu; Positive ion spray ionization.
LC3 column: SB-C18 50 mm×4.6 mm×2.7 µm;
Temperature: 50°C
Eluent: Acetonitrile/water from 10:90 to 98:2 (the above ratio was by volume)+0.05% TFA gradient, over 3.75 minutes;
Flow rate: 1.0 mL/min, Injection: 10 µL
Detection: PDA detector, 200-600 nm;
MS: Quality range 150-750 amu; Positive ion spray ionization.

As used herein, the meaning of each abbreviation was as follows:
AcOH=acetic acid; Alk represents an alkyl group; AR represents an aryl group; Boc=tert-butoxycarbonyl; CH₂Cl₂=dichloromethane; DBU=1,8-diazabicyclo [5.4.0] undecyl-7-ene; DCM=dichloromethane; DEAD=Diethyl azodicarboxylate; DMF=N, N-dimethylformamide; DMSO=dimethyl sulfoxide; EA=ethyl acetate; Et=Ethyl; EtOAc=ethyl acetate; EtOH=ethanol; HOAc=acetic acid; LiOH=lithium hydroxide; Me=methyl; MeCN=acetonitrile; MeOH=methanol; MgSO₄=magnesium sulfate; NaCl=sodium chloride; NaOH=sodium hydroxide; Na₂SO₄=sodium sulfate; PE=petroleum ether; Ph=phenyl; PG=protecting group; TFA=trifluoroacetic acid; THF=tetrahydrofuran; Ts=p-toluene sulfonyl group; rt=room temperature; h=hour; min=minute; bs=broad peak; s=single peak; d=bimodal; dd=double double peak; t=triple peak; m=multiple peak.

### General Method

### Method 1

### Method A:

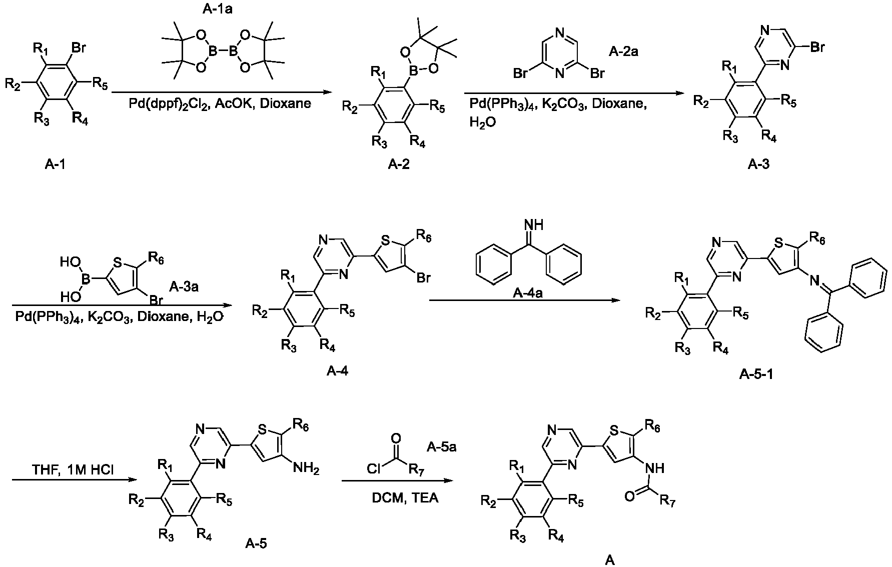

S1) Under appropriate alkaline conditions (such as but not limited to potassium acetate, etc.), selecting appropriate solvents (such as but not limited to Dioxane, etc.), and at an appropriate temperature (such as but not limited to 80 °C), under the catalysis with appropriate coupling catalysts (such as but not limited to Pd (dppf)₂), reacting compound (A-1) with corresponding boron ester or boric acid compounds (A-1a) (such as but not limited to Pinacolboronates) to obtain compound (A-2);
S2) Under appropriate alkaline conditions (such as but not limited to potassium carbonate, etc.), selecting suitable solvent (such as but not limited to a mixed solvent of Dioxane and water, etc.), and at an appropriate temperature (such as but not limited to 80 °C), under the catalysis with an appropriate coupling catalyst (such as but not limited to tetrakis(triphenylphosphine)palladium), reacting compound (A-2) with the corresponding aryl halide compound (A-2a) (such as but not limited to 2,6-dibromopyrazine) to obtain compound (A-3);
S3) Under appropriate alkaline conditions (such as but not limited to potassium carbonate, etc.), selecting appropriate solvents (such as but not limited to Dioxane, etc.), and at appropriate temperatures (such as but not limited to 60 °C), under the catalysis with appropriate coupling catalysts (such as but not limited to tetrakis(triphenylphosphine)palladium), reacting the compound (A-3) with the corresponding boric acid or boron ester compounds (A-3a) to obtain compound (A-4);
S4) Under appropriate alkaline conditions (such as but not limited to cesium carbonate, etc.), selecting appropriate solvents (such as but not limited to toluene, etc.), and at an appropriate temperature (such as but not limited to 110°C), selecting appropriate ligand (such as but not limited to BINAP, etc.), under the catalysis with appropriate coupling catalysts (such as but not limited to Pd₂(dba)₃), reacting compound (A-4) with corresponding nitrogen-containing compound (A-4a) to obtain compound (A-5-1);
S5) Selecting appropriate solvents (such as but not limited to tetrahydrofuran, etc.), selecting appropriate acids (such as but not limited to 1M hydrochloric acid), and making compound (A-5-2) undergo deprotecting reaction to obtain compound (A-5);
S6) Selecting suitable solvent (such as but not limited to dichloromethane, etc.), selecting suitable base (such as but not limited to triethylamine), and reacting compound (A-5) with corresponding acyl chloride (A-5a) to obtain compound (A);

### Method B:

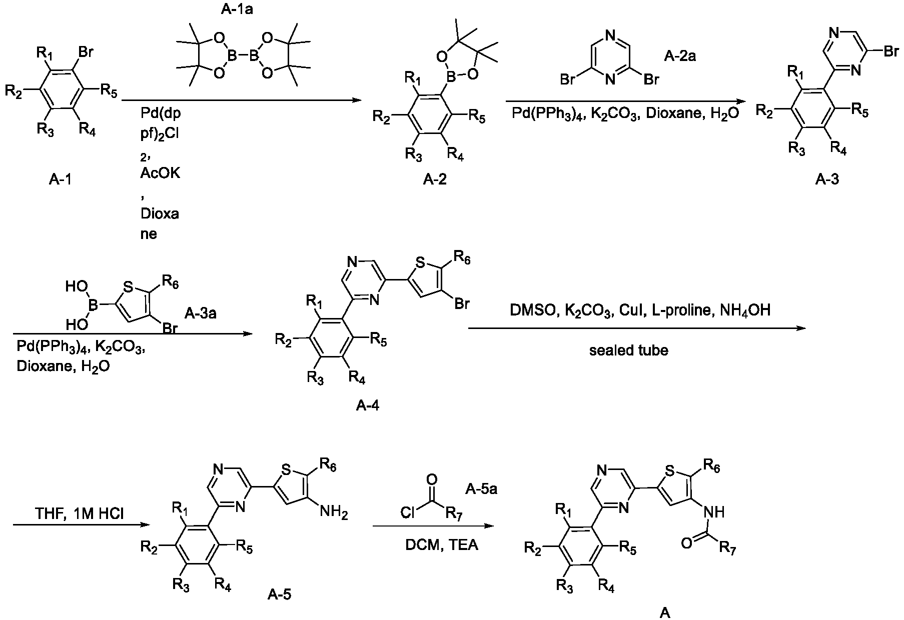

S1) Under appropriate alkaline conditions (such as but not limited to potassium acetate, etc.), selecting appropriate solvents (such as but not limited to Dioxane, etc.), and at an appropriate temperature (such as but not limited to 80 °C), under the catalysis with appropriate coupling catalysts (such as but not limited to Pd (dppf)₂), reacting compound (A-1) with corresponding boron ester or boric acid compounds (A-1a) (such as but not limited to Pinacolboronates) to obtain compound (A-2);
S2) Under appropriate alkaline conditions (such as but not limited to potassium carbonate, etc.), selecting suitable solvent (such as but not limited to a mixed solvent of Dioxane and water, etc.), and at an appropriate temperature (such as but not limited to 80 °C), under the catalysis with an appropriate coupling catalyst (such as but not limited to tetrakis(triphenylphosphine)palladium), reacting compound (A-2) with the corresponding aryl halide compound (A-2a) (such as but not limited to 2,6-dibromopyrazine) to obtain compound (A-3);
S3) Under appropriate alkaline conditions (such as but not limited to potassium carbonate, etc.), appropriate solvents (such as but not limited to Dioxane, etc.), and at appropriate temperatures (such as but not limited to 60 °C), under the catalysis with appropriate coupling catalysts (such as but not limited to tetrakis(triphenylphosphine)palladium), reacting the compound (A-3) with the corresponding boric acid or boron ester compounds (A-3a) to obtain compound (A-4);
S4) Under appropriate alkaline conditions (such as but not limited to potassium carbonate, etc.), selecting appropriate solvents (such as but not limited to DMSO, etc.), and at an appropriate temperature (such as but not limited to 110°C), selecting appropriate ligand (such as but not limited to L-proline, etc.), under the catalysis with appropriate coupling catalysts (such as but not limited to cuprous iodide), reacting compound (A-4) with corresponding nitrogen-containing compound ammonia under pipe sealing condition to obtain compound (A-5);
S5) Selecting suitable solvent (such as but not limited to dichloromethane, etc.), selecting suitable base (such as but not limited to triethylamine), and reacting compound (A-5) with corresponding acyl chloride (A-5a) to obtain compound (A);

### Method 2

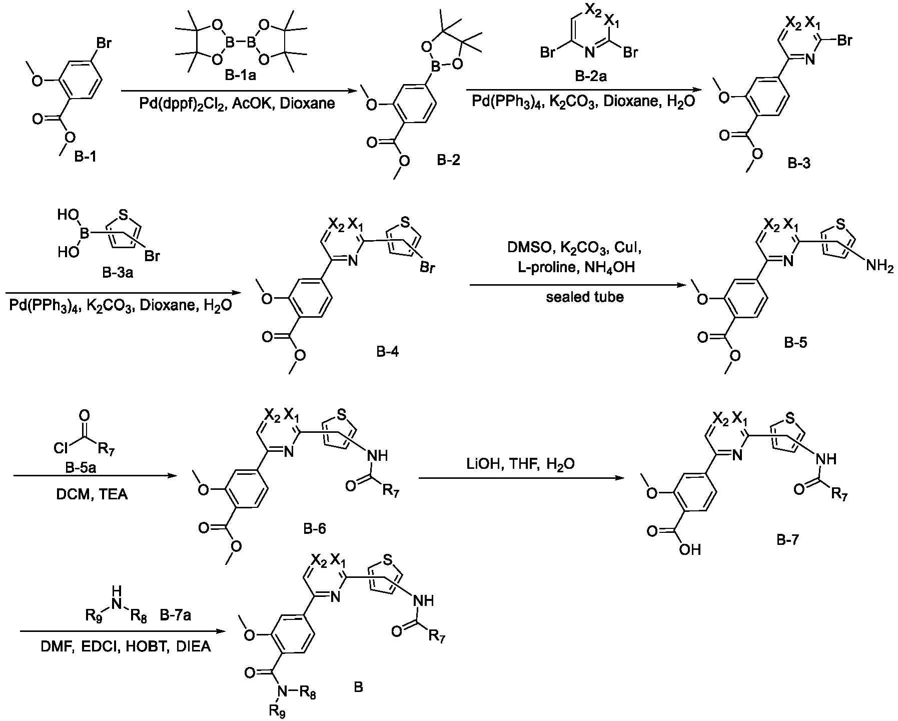

S1) Under appropriate alkaline conditions (such as but not limited to potassium acetate, etc.), selecting appropriate solvents (such as but not limited to Dioxane, etc.), and at appropriate temperatures (such as but not limited to 80 °C), under the catalysis with appropriate coupling catalysts (such as but not limited to Pd (dppf)₂), reacting compound (B-1) with corresponding boron ester or boric acid compounds (B-1a) (such as but not limited to pinacolboronates) to obtain compound (B-2);
S2) Under appropriate alkaline conditions (such as but not limited to potassium carbonate, etc.), selecting appropriate solvents (such as but not limited to a mixed solvent of Dioxane and water, etc.), and at appropriate temperatures (such as but not limited to 80 °C), under the catalysis with appropriate coupling catalysts (such as but not limited to tetrakis(triphenylphosphine)palladium), reacting compound (B-2) with the corresponding aryl halide compound (B-2a) to obtain compound (B-3);
S3) Under appropriate alkaline conditions (such as but not limited to potassium carbonate, etc.), selecting suitable solvent (such as but not limited to a mixed solvent of Dioxane and water, etc.), and at an appropriate temperature (such as but not limited to 60 °C), under the catalysis with an appropriate coupling catalyst (such as but not limited to tetrakis(triphenylphosphine)palladium), reacting compound (B-3) with the corresponding boric acid or boron ester compound (B-3a) to obtain compound (B-4);
S4) Under appropriate alkaline conditions (such as but not limited to cesium carbonate, etc.), selecting appropriate solvents (such as but not limited to DMSO, etc.), and at an appropriate temperature (such as but not limited to 110°C), selecting appropriate ligand (such as but not limited to L-proline, etc.), under the catalysis with appropriate coupling catalysts (such as but not limited to cuprous iodide), reacting compound (B-4) with corresponding nitrogen-containing compound ammonia under pipe sealing condition to obtain compound (B-5);
S5) Selecting suitable solvent (such as but not limited to dichloromethane), selecting suitable base (such as but not limited to triethylamine), and reacting compound (B-5) with corresponding acyl chloride (B -5a) to obtain compound (B-6);
S6) Selecting appropriate solvents (such as but not limited to a mixed solvent of tetrahydrofuran and water, etc.), appropriate bases (such as but not limited to lithium hydroxide), and making compound (B-6) undergo hydrolytic reaction to obtain carboxyl compound (B-7);
S7) Selecting suitable solvent (such as but not limited to DMF), selecting suitable base (such as but not limited to DIEA), suitable coupling reagents (such as but not limited to EDCI+HOBT), and reacting compound (B-7) with corresponding amine (B -7a) to obtain compound (B-8);

### Method 3

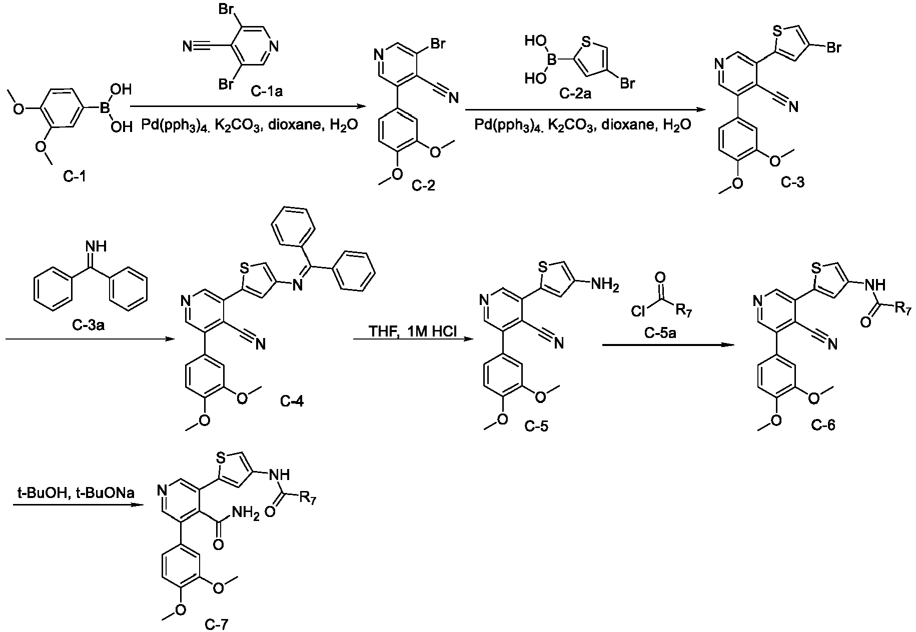

S1) Under appropriate alkaline conditions (such as but not limited to potassium carbonate, etc.), selecting suitable solvent (such as but not limited to a mixed solvent of Dioxane and water, etc.), and at an appropriate temperature (such as but not limited to 80 °C), under the catalysis with an appropriate coupling catalyst (such as but not limited to tetrakis(triphenylphosphine)palladium), reacting compound (C-1) with the corresponding aryl halide compound (C-1a) to obtain compound (C-2);
S2) Under appropriate alkaline conditions (such as but not limited to potassium carbonate, etc.), selecting suitable solvent (such as but not limited to a mixed solvent of Dioxane and water, etc.), and at an appropriate temperature (such as but not limited to 60 °C), under the catalysis with an appropriate coupling catalyst (such as but not limited to tetrakis(triphenylphosphine)palladium), reacting compound (C-2) with corresponding boric acid or boron ester compounds (C-2a) to obtain compound (C-3);
S3) Under appropriate alkaline conditions (such as but not limited to cesium carbonate, etc.), selecting appropriate solvents (such as but not limited to toluene, etc.), at appropriate temperatures (such as but not limited to 110 °C), selecting appropriate ligands (such as but not limited to BINAP), and under the catalysis with appropriate coupling catalysts (such as but not limited to Pd₂(dba)₃), reacting compound (C-3) with corresponding nitrogen-containing compound (C-3a) to obtain compound (C-4);
S4) Selecting appropriate solvents (such as but not limited to tetrahydrofuran, etc.), selecting appropriate acids (such as but not limited to 1M hydrochloric acid), and making compound (C-4) undergo deprotecting reaction to obtain compound (C-5);
S5) Selecting suitable solvent (such as but not limited to dichloromethane), selecting suitable base (such as but not limited to triethylamine), and reacting compound (C-5) with corresponding acyl chloride (C-5a) to obtain compound (C-6);
S6) Selecting appropriate solvents (such as but not limited to tert butanol, etc.), selecting appropriate bases (such as but not limited to sodium tert-butoxide, etc.), and making compound (C-6) undergo hydrolytic reaction to obtain amide compound (C-7);

### Example 1: Preparation of N- (5- (6- (3,4-dimethoxyphenyl) pyrazin-2-yl) thiophen-3-yl) pentanamide (A1) Method A)

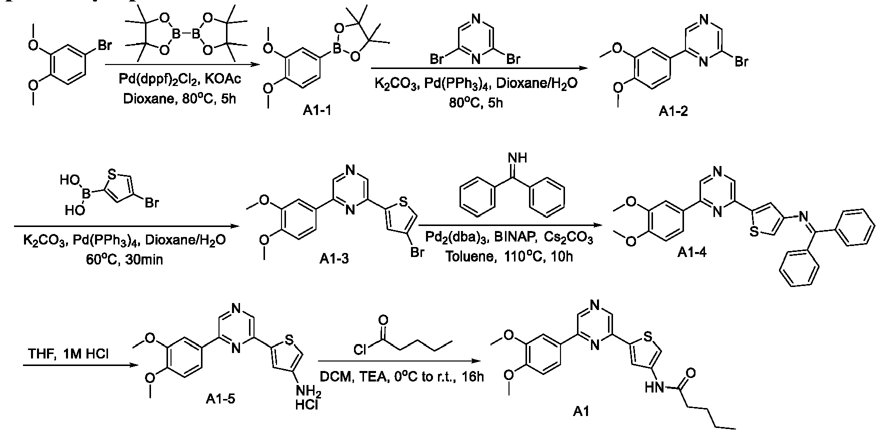

### Step 1: 3,4-dimethoxyphenyl boronic acid pinacol ester (A1-1)

3,4-Dimethoxybromobenzene (910 mg, 4.20 mmol), potassium acetate (1.03 g, 10.50 mmol), 1,1'-Bis(diphenylphosphino)ferrocene]dichloropalladium(II)(154 mg, 0.21 mmol), Bis(pinacolato)diboron (1.28 g, 5.00 mmol) and, 1,4-dioxane (20 mL) were added successively to a reaction flask. The mixture was reacted under nitrogen protection at 80°C for 5 hours, and TLC monitoring showed that the reaction was complete. The reaction solution was filtered, and the filter cake was washed with ethyl acetate. The reaction solution and the washing solution were combined, and spin dried to obtain 3,4-dimethoxyphenyl boronic acid pinacol ester (A1-1, crude, 4.20 mmol), which was directly used in the next reaction without any purification.

### Step 2: 2-bromo-6- (3,4-dimethoxyphenyl) pyrazine (A1-2)

2,6-Dibromopyrazine(1.0 g, 4.2 mmol), 3,4-dimethoxyphenyl boronic acid pinacol ester (A1-1, crude, 4.20mmol), potassium carbonate(1.38 g, 10.00 mmol), Tetrakis(triphenylphosphine)palladium (243 mg, 0.21 mmol), 1,4-dioxane (40 mL), and water (10mL) were added successively to a reaction flask. The mixture was reacted under nitrogen protection at 80°C for 5 hours, and TLC monitoring showed that the reaction was complete. The reaction solution was evaporated to remove the most of 1,4-dioxane, and re-dissolved with ethyl acetate(50 mL). The organic phase was extracted successively with water and saturated saline, dried over anhydrous sodium sulfate, spin-dried and purified by column chromatography to obtain 2-bromo-6- (3,4-dimethoxyphenyl) pyrazine (A1-2, 900 mg, 72.6%). ¹HNMR(400 MHz, CDC13) δ 8.92 (s, 1H), 8.55 (s, 1H), 7.63 (d, *J* = 2.0 Hz, 1H), 7.60 (dd, *J* = 8.4, 2.1 Hz, 1H), 7.00 (d, *J* = 8.4 Hz, 1H), 4.02 (s, 3H), 3.98 (s, 3H).

### Step 3: 2- (4-bromothiophen-2-yl) -6- (3,4-dimethoxyphenyl) pyrazine(A1-3)

2-bromo-6- (3,4-dimethoxyphenyl) pyrazine (A1-2, 488 mg, 1.65 mmol), (4-bromothiophen-2-yl) boronic acid (377 mg, 1.82 mmol), tetrakis(phosphorus)palladium (38 mg, 0.033 mmol), potassium carbonate(524 mg,3.79 mmol), 1,4-dioxane (16 mL), and water (2.5mL) were successively added to a reaction flask. The mixture was reacted under nitrogen protection at 80°C for 4 hours, and TLC monitoring showed that the reaction was complete. The reaction solution was evaporated to remove the most of 1,4-dioxane, and then added with dichloromethane (30 mL) and water (10 mL), and fractioned. The aqueous phase was extracted with dichloromethane (20 mL*2), and the organic phases were combined, washed with saturated salt water (20 mL * 1), dried over anhydrous sodium sulfate, spin-dried and purified by column chromatography to obtain 2-(4-bromothiophen-2-yl) -6- (3,4-dimethoxyphenyl) pyrazine(A1-3, 463 mg, 74.4%). ¹H NMR (400 MHz, CDCl₃) δ 8.89 (s, 1H), 8.78 (s, 1H), 7.74 (d, *J* = 1.9 Hz, 1H), 7.67 (dd, *J* = 8.4, 2.0 Hz, 1H), 7.64 (d, *J* = 1.2 Hz, 1H), 7.40 (d, *J* = 1.1 Hz, 1H), 7.02 (d, *J* = 8.4 Hz, 1H), 4.04 (s, 3H), 3.99 (s, 3H).

### Step 4: N- (5- (6- (3,4-dimethoxyphenyl) pyrazin-2yl) thiazol-3yl) -1,1-diphenylimine (A1-4)

2- (4-bromothiophen-2-yl) -6- (3,4-dimethoxyphenyl) pyrazine (A1-3, 106 mg, 0.28 mmol), benzophenone imine (76.4 mg, 0.42 mmol), 1.1'-binaphthyl-2.2'-diphenyl phosphine (17.4 mg, 0.028 mmol), Tris(dibenzylideneacetone)dipalladium (12.8 mg, 0.014 mmol), cesium carbonate (136.8 mg, 0.42 mmol), and toluene (10 mL) were sequentially added to a reaction flask. The mixture was reacted under nitrogen protection at 110°C for 16 hours, and TLC monitoring showed that the reaction was complete. The reaction solution was directly spin-dried and purified by column chromatography to afford N- (5- (6- (3,4-dimethoxyphenyl) pyrazin-2yl) thiazol-3yl) -1,1-diphenylimine (A1-4, 59 mg, 43.9%). ¹H NMR (400 MHz, CDCl₃) δ 8.82 (d, *J* = 9.7 Hz, 1H), 8.56 (s, 1H), 7.82 (d, *J* = 7.3 Hz, 2H), 7.74 (d, *J* = 1.8 Hz, 1H), 7.65 (dd, *J* = 8.4, 1.9 Hz, 1H), 7.57-7.49 (m, 1H), 7.43 (dt, *J* = 9.2, 5.8 Hz, 5H), 7.29 (m, 3H), 7.20 (d, *J* = 1.3 Hz, 1H), 7.01 (d, *J* = 8.4 Hz, 1H), 4.03 (s, 3H), 3.98 (s, 3H).

### Step 5: 5- (6- (3,4-dimethoxyphenyl) pyrazin-2- yl)thiazol-3-amine hydrochloride(A1-5)

N- (5- (6- (3,4-dimethoxyphenyl) pyrazin-2yl) thiazol-3yl) -1,1-diphenylimine (A1-4, 59 mg, 0.12 mmol) was dissolved in tetrahydrofuran(10 mL), to the reaction solution was added 1M concentrated hydrochloric acid (0.5 mL) dropwise and the mixture was reacted at room temperature for 4 hours. White solid precipitated. The solid was collected by filtration, and dried to obtain 5- (6- (3,4-dimethoxyphenyl) pyrazin-2- yl)thiazol-3-amine hydrochloride (A1-5, 34 mg, 78.5%). 1H NMR (400 MHz, CDC13) δ 8.83 (s, 1H), 8.74 (s, 1H), 7.77 (s, 1H), 7.67 (d, *J* = 8.4 Hz, 1H), 7.33 (s, 1H), 7.02 (d, *J* = 8.4 Hz, 1H), 6.38 (s, 1H), 4.05 (s, 3H), 3.99 (s, 3H).

### Step 6: N - (5- (6- (3,4-dimethoxyphenyl) pyrazin-2-yl) thiophen-3-yl) pentanamide (A1)

5- (6- (3,4-Dimethoxyphenyl) pyrazin-2- yl)thiazol-3-amine hydrochloride (A1-5, 32 mg, 0.09 mmol) was dissolved in dichloromethane (5 mL), and the solution was cooled under an ice water bath and added with triethylamine (18.5 mg, 0.183 mmol). Additionally, pentanoyl chloride (14.9 mg, 0.123mmol) was taken and dissolved in dichloromethane (0.5 mL) and the resulting mixture was slowly dropped into the above reaction system. Upon addition, the reaction system was warmed to room temperature and reacted for 16 hours. LCMS detection showed that the reaction was complete. The reaction solution was directly spin-dried and purified by column chromatography to afford N - (5- (6-(3,4-dimethoxyphenyl) pyrazin-2-yl) thiophen-3-yl) pentanamide (A1, 19 mg, 53.1%). 1H NMR (400 MHz, DMSO) δ 10.39 (s, 1H), 9.13 (s, 1H), 8.97 (s, 1H), 7.86 (d, *J* = 1.2 Hz, 1H), 7.80 (dd, *J* = 8.4, 1.9 Hz, 1H), 7.77 (d, *J* = 1.8 Hz, 1H), 7.71 (d, *J* = 1.2 Hz, 1H), 7.15 (d, *J* = 8.4 Hz, 1H), 3.90 (s, 3H), 3.83 (d, *J* = 19.3 Hz, 3H), 2.32 (t, *J* = 7.4 Hz, 2H), 1.69-1.51 (m, 2H), 1.41-1.29 (m, 2H), 0.91 (t, *J* = 7.3 Hz, 3H).

### Compounds A2-A34 of examples 2-34 were prepared using experimental steps similar to those in Example 1 above, and compounds A1-A34 were summarized in Table 1.

**Table 1**

| Exam ple | Compo und No. | Structure | MS(cald) [M+H]⁺/ MS (found) | Name and characterization |
|---|---|---|---|---|
| 1 | A1 | 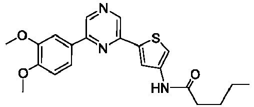 | 398.15/N/A (HNMR) | N-(5-(6-(3,4-dimethoxyphenyl) pyrazin-2-yl)thiophen-3-yl)pent anamide |
| | | | | ¹H NMR (400 MHz, DMSO) δ 10.39 (s, 1H), 9.13 (s, 1H), 8.97 (s, 1H), 7.86 (d, *J* = 1.2 Hz, 1H), 7.80 (dd, *J* = 8.4, 1.9 Hz, 1H), 7.77 (d, *J* = 1.8 Hz, 1H), 7.71 (d, *J* = 1.2 Hz, 1H), 7.15 (d, *J* = 8.4 Hz, 1H), 3.90 (s, 3H), 3.83 (d, *J* = 19.3 Hz, 3H), 2.32 (t, *J* = 7.4 Hz, 2H), 1.69-1.51 (m, 2H), 1.41-1.29 (m, 2H), 0.91 (t, *J* = 7.3 Hz, 3H). |
| 2 | A2 | 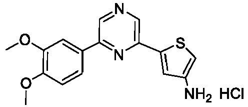 | 314.09/N/A (HNMR) | 5-(6-(3,4-dimethoxyphenyl)pyr azin-2-yl)thiophen-3-amine hydrochloride |
| | | | | ¹H NMR (400 MHz, CDC13) δ 8.83 (s, 1H), 8.74 (s, 1H), 7.77 (s, 1H), 7.67 (d, *J* = 8.4 Hz, 1H), 7.33 (s, 1H), 7.02 (d, *J* = 8.4 Hz, 1H), 6.38 (s, 1H), 4.05 (s, 3H), 3.99 (s, 3H). |
| 3 | A3 | 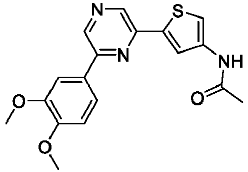 | 356.10/356. 34 | N-(5-(6-(3,4-dimethoxyphenyl) pyrazin-2-yl)thiophen-3-yl)acet amide |
| 4 | A4 | 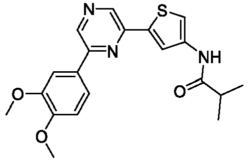 | 384.13/384. 76 | N-(5-(6-(3,4-dimethoxyphenyl) pyrazin-2-yl)thiophen-3-yl)isob utyramide |
| 5 | A5 | 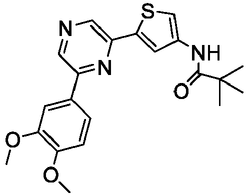 | 398.15/398. 86 | N-(5-(6-(3,4-dimethoxyphenyl) pyrazin-2-yl)thiophen-3-yl)piva lamide |
| 6 | A6 | 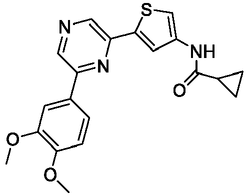 | 382.11/382. 46 | N-(5-(6-(3,4-dimethoxyphenyl) pyrazin-2-yl)thiophen-3-yl)cycl opropanecarboxamide |
| 7 | A7 | 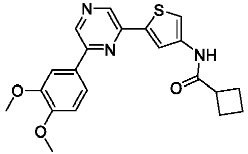 | 396.13/396. 20 | N-(5-(6-(3,4-dimethoxyphenyl) pyrazin-2-yl)thiophen-3-yl)cycl obutanecarboxamide |
| 8 | A8 | 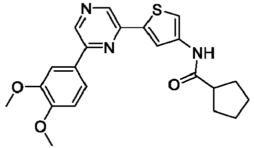 | 410.15/410. 49 | N-(5-(6-(3,4-dimethoxyphenyl) pyrazin-2-yl)thiophen-3-yl)cycl opentanecarboxamide |
| 9 | A9 | 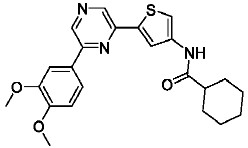 | 424.16/424. 33 | N-(5-(6-(3,4-dimethoxyphenyl) pyrazin-2-yl)thiophen-3-yl)cycl ohexanecarboxamide |
| 10 | A10 | 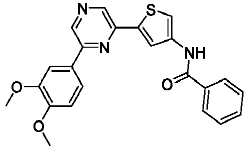 | 418.11/418. 37 | N-(5-(6-(3,4-dimethoxyphenyl) pyrazin-2-yl)thiophen-3-yl)benz amide |
| 11 | A11 | 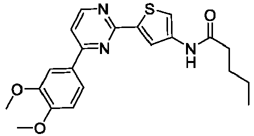 | 398.15/398. 43 | N-(5-(4-(3,4-dimethoxyphenyl) pyrimidin-2-yl)thiophen-3-yl)pe ntanamide |
| 12 | A12 | 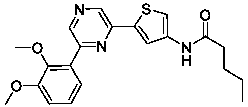 | 398.15/397. 97 | N-(5-(6-(2,3-dimethoxyphenyl) pyrazin-2-yl)thiophen-3-yl)pent anamide |
| 13 | A13 | 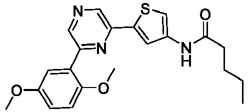 | 398.15/398. 66 | N-(5-(6-(2, 5-dimethoxyphenyl) pyrazin-2-yl)thiophen-3-yl)pent anamide |
| 14 | A14 | 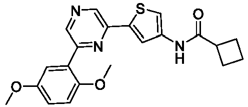 | 396.13/396. 63 | N-(5-(6-(2, 5-dimethoxyphenyl) pyrazin-2-yl)thiophen-3-yl)cycl obutanecarboxamide |
| 15 | A15 | 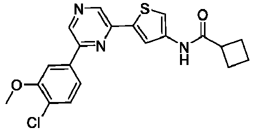 | 400.08/399. 94 | N-(5-(6-(4-chloro-3-methoxyph enyl)pyrazin-2-yl)thiophen-3-yl )cyclobutanecarboxamide |
| 16 | A16 | 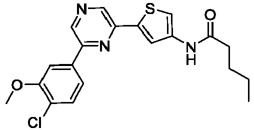 | 402.10/401. 94 | N-(5-(6-(4-chloro-3-methoxyph enyl)pyrazin-2-yl)thiophen-3-yl )pentanamide |
| 17 | A17 | 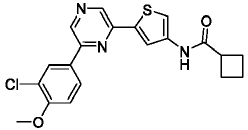 | 400.08/399. 87 | N-(5-(6-(3-chloro-4-methoxyph enyl)pyrazin-2-yl)thiophen-3-yl )cyclobutanecarboxamide |
| 18 | A18 | 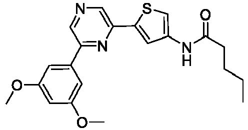 | 398.15/398. 24 | N-(5-(6-(3, 5-dimethoxyphenyl) pyrazin-2-yl)thiophen-3-yl)pent anamide |
| 19 | A19 | 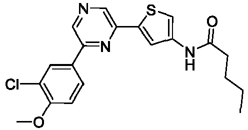 | 402.10/401. 91 | N-(5-(6-(3-chloro-4-methoxyph enyl)pyrazin-2-yl)thiophen-3-yl )pentanamide |
| 20 | A20 | 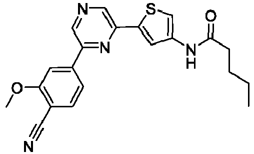 | 393.13/N/A (HNMR) | N-(5-(6-(4-cyano-3-methoxyph enyl)pyrazin-2-yl)thiophen-3-yl )pentanamide |
| | | | | ¹H NMR (400 MHz, DMSO) δ 10.40 (s, 1H), 9.30 (s, 1H), 9.16 (s, 1H), 8.03-7.87 (m, 4H), 7.73 (s, 1H), 4.08 (s, 3H), 2.32 (t*, J* = 7.4 Hz, 2H), 1.68-1.54 (m, 2H), 1.37-1.31 (m, 2H), 0.92 (t, *J* = 7.3 Hz, 3H). |
| 21 | A21 | 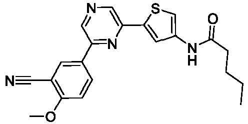 | 393.13/393. 60 | N-(5-(6-(3-cyano-4-methoxyph enyl)pyrazin-2-yl)thiophen-3-yl )pentanamide |
| 22 | A22 | 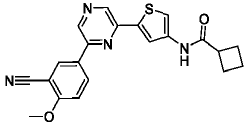 | 391.12/N/A (HNMR) | N-(5-(6-(3-cyano-4-methoxyph enyl)pyrazin-2-yl)thiophen-3-yl )cyclobutanecarboxamide |
| | | | | ¹H NMR (400 MHz, CDCl₃) δ 8.83 (s, 2H), 8.38 (d, *J* = 2.0 Hz, 1H), 8.30 (dd, *J* = 8.8, 2.0 Hz, 1H), 7.82 (s, 1H), 7.69 (s, 1H), 7.47 (s, 1H), 7.15 (d, *J* = 8.8 Hz, 1H), 4.05 (s, 3H), 3.28-3.17 (m, 1H), 2.54-2.38 (m, 2H), 2.36-2.21 (m, 2H), 2.13-1.97 (m, 2H). |
| 23 | A23 | 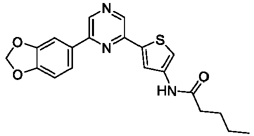 | 382.11/N/A (HNMR) | N-(5-(6-(benzo[d][1,3]dioxol-5-yl)pyrazin-2-yl)thiophen-3-yl)p entanamide |
| | | | | ¹H NMR (400 MHz, DMSO) δ 10.39 (s, 1H), 9.09 (d, *J* = 9.6 Hz, 1H), 8.98 (s, 1H), 7.86 (d, *J* = 1.2 Hz, 1H), 7.83-7.66 (m, 3H), 7.12 (d, *J* = 8.1 Hz, 1H), 6.14 (s, 2H), 2.32 (t, J = 7.4 Hz, 2H), 1.67-1.54 (m, 2H), 1.37-1.28 (m, 2H), 0.91 (t, *J* = 7.3 Hz, 3H). |
| 24 | A24 | 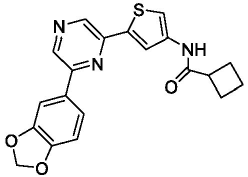 | 380.10/N/A (HNMR) | N-(5-(6-(benzo[d][1,3]dioxol-5-yl)pyrazin-2-yl)thiophen-3-yl)c yclobutanecarboxamide |
| | | | | ¹H NMR (400 MHz, DMSO) δ 10.24 (s, 1H), 9.08 (s, 1H), 8.96 (s, 1H), 7.89 (s, 1H), 7.76 (dd, *J* = 18.8, 11.4 Hz, 3H), 7.12 (d, *J* = 8.1 Hz, 1H), 6.14 (s, 2H), 3.22 (dd, *J* = 16.5, 8.3 Hz, 1H), 2.27 (dd, *J* = 27.4, 17.4 Hz, 2H), 2.13 (d, *J* = 8.9 Hz, 2H), 2.01-1.80 (m, 2H). |
| 25 | A25 | 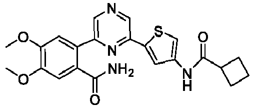 | 439.14/ N/A (HNMR) | 2-(6-(4-(cyclobutanecarboxami do)thiophen-2-yl)pyrazin-2-yl)-4,5-dimethoxybenzamide |
| | | | | ¹H NMR (400 MHz, CDCl₃) δ 8.78 (s, 1H), 8.64 (s, 1H), 7.70 (d, *J* = 29.4 Hz, 3H), 7.27 (s, 1H), 7.16 (s, 1H), 4.01 (d, *J* = 4.3 Hz, 6H), 3.17-3.09 (m, 1H), 2.45-2.35 (m, 2H), 2.28-2.19 (m, 2H), 2.10-1.97 (m, 2H). |
| 26 | A26 | 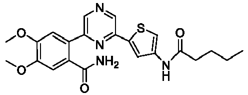 | 441.15/441. 70 | 4,5-dimethoxy-2-(6-(4-pentana midothiophen-2-yl)pyrazin-2-yl )benzamide |
| 27 | A27 | 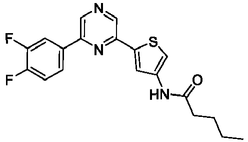 | 374.11/ N/A | N-(5-(6-(3,4-difluorophenyl)pyr azin-2-yl)thiophen-3-yl)pentana mide |
| | | | | ¹H NMR (400 MHz, CDC13) δ 8.86 (s, 1H), 8.84 (s, 1H), 8.07-7.95 (m, 1H), 7.91-7.83 (m, 1H), 7.81 (s, 1H), 7.67 (s, 1H), 7.46 (s, 1H), 7.34 (dd, *J* = 18.2, 8.5 Hz, 1H), 2.41 (t, *J* = 7.6 Hz, 2H), 1.81-1.73 (m, 2H), 1.51-1.40 (m, 2H), 1.06-0.94 (m, 3H). |
| 28 | A28 | 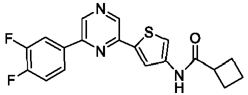 | 372.09/ N/A | N-(5-(6-(3,4-difluorophenyl)pyr azin-2-yl)thiophen-3-yl)cyclobu tanecarboxamide |
| | | | | ¹H NMR (400 MHz, CDCl₃) δ 8.86 (s, 1H), 8.84 (s, 1H), 8.07-7.97 (m, 1H), 7.85 (s, 1H), 7.82 (s, 1H), 7.68 (s, 1H), 7.36 (d, *J* = 8.0 Hz, 1H), 7.31 (d, *J* = 8.5 Hz, 1H), 3.24-3.16 (m, 1H), 2.52-2.38 (m, 2H), 2.38-2.22 (m, 2H), 2.13-1.99 (m, 2H). |
| 29 | A29 | 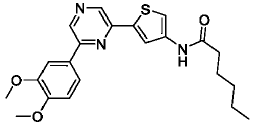 | 412.16/412. 20 | N-(5-(6-(3,4-dimethoxyphenyl) pyrazin-2-yl)thiophen-3-yl)hexa namide |
| 30 | A30 | 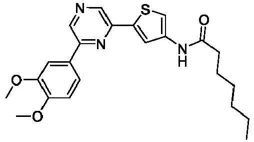 | 426.18/426. 20 | N-(5-(6-(3,4-dimethoxyphenyl) pyrazin-2-yl)thiophen-3-yl)hept anamide |
| 31 | A31 | 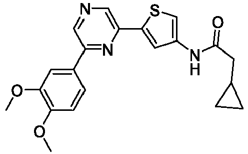 | 396.13/396. 10 | 2-cyclopropyl-N-(5-(6-(3,4-dim ethoxyphenyl)pyrazin-2-yl)thio phen-3-yl)acetamide |
| 32 | A32 | 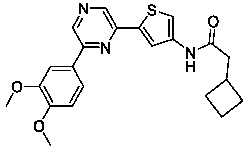 | 410.15/410. 20 | 2-cyclobutyl-N-(5-(6-(3,4-dime thoxyphenyl)pyrazin-2-yl)thiop hen-3-yl)acetamide |
| 33 | A33 | 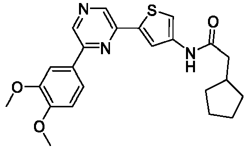 | 424.16/424. 20 | 2-cyclopentyl-N-(5-(6-(3,4-dim ethoxyphenyl)pyrazin-2-yl)thio phen-3-yl)acetamide |
| 34 | A34 | 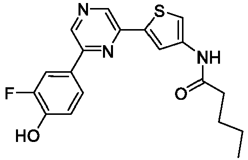 | 372.11/372. 10 | N-(5-(6-(3-fluoro-4-hydroxyphe nyl)pyrazin-2-yl)thiophen-3-yl) pentanamide |

### Example 35: Preparation of N-(5- (6-(4-(3-hydroxyoxetan-3-yl) -3-methoxyphenyl)pyrazin-2-yl) thiophen-3-yl) pentanamide (A35) (Method B)

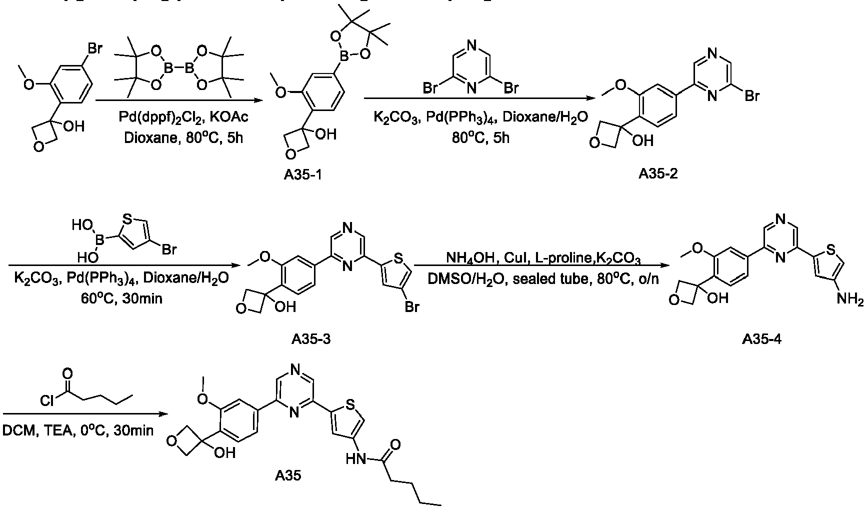

### Step 1: 3- (2-Methoxy-4-borate-phenyl)oxetan-3-ol (A35-1)

3- (4-bromo-2-methoxyphenyl) oxetan-3-ol (400 mg, 1.54 mmol), potassium acetate (379 mg, 3.86 mmol), 1,1'-bis(diphenylphosphino)ferrocene]dichloropalladium(II) (57 mg, 0.08 mmol), and bis(pinacolato)diboron (471 mg, 1.85 mmol) were added to 1,4-dioxane (15 mL) and the mixture was reacted under nitrogen protection at 80°C for 5 hours. TLC monitoring showed that the reaction was complete. The reaction solution was filtered and the filter cake was washed with ethyl acetate. The reaction solution and the washing solution were combined, and spin dried to obtain 3-(2-methoxy-4-borate-phenyl)oxetan-3-ol (A35-1, crude, 1.54 mmol in theory), which was directly used in the next reaction without any purification.

### Step 2: 3- (4- (6-bromopyrazin-2-yl) -2-methoxyphenyl)oxetan-3-ol (A35-2)

3- (2-Methoxy-4-borate-phenyl)oxetan-3-ol (A35-1, crude, 1.54 mmol), potassium carbonate (512 mg, 3.70 mmol), 2,6-dibromopyrazine (551 mg, 2.32 mmol), and tetrakis(triphenylphosphine)palladium (89 mg, 0.08 mmol) were added to 1,4-dioxane/water (4:1, 15 mL) and the mixture was reacted under nitrogen protection at 80 °C for 5 hours. TLC monitoring showed that the reaction was complete. The reaction solution was cooled to room temperature, added with 45 mL of water and extracted with ethyl acetate (20 mL*3). The organic phases were combined, washed with saturated salt water (20 mL*1), dried over anhydrous sodium sulfate, spin-dried and purified by column chromatography to obtain 3- (4- (6-bromopyrazin-2-yl) -2-methoxyphenyl)oxetan-3-ol (A35-2, 310 mg, 59.6%). MS (ESI) m/z: calcd 337.01 (M+H⁺), found 337.00

### Step 3: 3- (4- (6- (4-bromothiophen-2-yl) pyrazin-2-yl)-2-methoxyphenyl) oxetan-3-ol (A35-3)

3- (4- (6-bromopyrazin-2-yl) -2-methoxyphenyl)oxetan-3-ol (A35-2, 310 mg, 0.92 mmol), (4-bromothiophen-2-yl) boronic acid (191 mg, 0.93 mmol), tetrakis(triphenylphosphine)palladium (55 mg, 0.05 mmol) and potassium carbonate(305 mg, 2.21mmol) were added to 1,4-dioxane/water (4:1, 15 mL) and the mixture was reacted under nitrogen protection at 60°C for 0.5 hour. TLC monitoring showed that the reaction was complete. The reaction solution was cooled to room temperature, added with water (45 mL) and extracted with ethyl acetate(20 mL*3). The organic phases were combined, washed with saturated saline (20 mL*1), dried over anhydrous sodium sulfate, and spin-dried. The residue was purified by column chromatography to afford 3-(4-(6-(4-bromothiophen-2-yl) pyrazin-2-yl)-2-methoxyphenyl) oxetan-3-ol (A35-3, 160 mg, 41.6%). MS (ESI) m/z: calcd 419.29 (M+H), found 419.00

### Step 4: 3- (4- (6- (4-aminothiophen-2-yl) pyrazin-2-yl)-2-methoxyphenyl) oxetan-3-ol (A35-4)

3-(4-(6- (4-bromothiophen-2-yl) pyrazin-2-yl)-2-methoxyphenyl) oxetan-3-ol (A35-3, 160 mg, 0.38mmol), cuprous iodide (15 mg, 0.08 mmol), L-proline (35 mg, 0.31 mmol), dimethyl sulfoxide (3 mL), and ammonia (25% wt, 360 mg, 2.57 mmol) were sequentially added to a sealed tube, and the reaction was sealed and reacted at 80 °C for 10 hours. TLC monitoring showed that the reaction was complete. The reaction solution was cooled to room temperature, added with 20 mL of water, and extracted with ethyl acetate (10 mL * 3). The organic phases were combined, washed with saturated ammonium chloride (10 mL * 2), dried over anhydrous sodium sulfate, and spin-dried to obtain 3- (4- (6-(4-aminothiophen-2-yl) pyrazin-2-yl)-2-methoxyphenyl) oxetan-3-ol (A35-4, crude, theoretical 0.38 mmol), which was directly used in the next reaction step without any purification.

### Step 5: N-(5- (6-(4-(3-hydroxyoxetan-3-yl) -3-methoxyphenyl)pyrazin-2-yl) thiophen-3-yl) pentanamide (A35)

3- (4- (6- (4-Aminothiophen-2-yl) pyrazin-2-yl)-2-methoxyphenyl) oxetan-3-ol (A35-4, crude, 0.38 mmol) was dissolved in dichloromethane(10 mL). The solution was cooled under an ice water bath, and added with triethylamine (117 mg, 1.16 mmol). Pentanoyl chloride (93 mg, 0.77 mol) was taken and dissolved in dichloromethane (0.5 mL) and the resulting mixture was slowly dropped into the above reaction system. Upon addition, the reaction continued for 1 hour under an ice water bath. TLC monitoring showed that the reaction was complete. The reaction solution was directly spin-dried and purified by column chromatography to afford N-(5- (6-(4-(3-hydroxyoxetan-3-yl) -3-methoxyphenyl)pyrazin-2-yl) thiophen-3-yl) pentanamide (A35, 32 mg, 19.1%). MS (ESI) m/z: calcd 440.16 (M+H⁺), found 440.10 ¹H NMR (400 MHz, DMSO) δ 10.39 (s, 1H), 9.19 (s, 1H), 9.06 (s, 1H), 7.89 (d, *J* = 1.4 Hz, 1H), 7.76 (dd, *J* = 13.5, 7.3 Hz, 2H), 7.72 (d, *J* = 1.3 Hz, 1H), 7.42 (d, *J* = 7.8 Hz, 1H), 5.97 (s, 1H), 5.02 (d, *J* = 6.9 Hz, 2H), 4.67 (d, *J* = 6.9 Hz, 2H), 3.94 (d, *J* = 6.9 Hz, 3H), 2.36-2.29 (m, 2H), 1.64-1.56 (m, 2H), 1.38-1.30 (m, 2H), 0.92 (t, *J* = 7.3 Hz, 3H).

### Compounds A36-A45 of examples 36-45 were prepared using experimental steps similar to those in Example 35 above, and compounds A35-A45 were summarized in Table 2.

**Table 2**

| Examp le | Comp ound No. | Structure | MS(cald) [M+H]⁺/ MS (found) | Name and characterization |
|---|---|---|---|---|
| 35 | A35 | 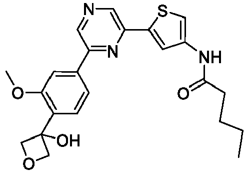 | 440.16/440. 40 | N-(5-(6-(4-(3-hydroxyoxeta n-3-yl)-3-methoxyphenyl)py razin-2-yl)thiophen-3-yl)pen tanamide |
| 36 | A36 | 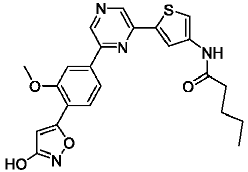 | 451.14/451. 20 | N-(5-(6-(4-(3-hydroxyisoxaz ol-5-yl)-3-methoxyphenyl)p yrazin-2-yl)thiophen-3-yl)pe ntanamide |
| 37 | A37 | 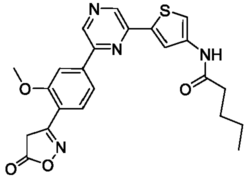 | 451.14/451. 20 | N-(5-(6-(3-methoxy-4-(5-ox o-4,5-dihydroisoxazol-3-yl)p henyl)pyrazin-2-yl)thiophen-3-yl)pentanamide |
| 38 | A38 | 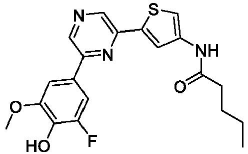 | 402.12/402. 30 | N-(5-(6-(3-fluoro-4-hydroxy -5-methoxyphenyl)pyrazin-2 -yl)thiophen-3-yl)pentanami de |
| 39 | A39 | 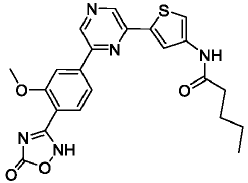 | 452.13/No MS signal (HNMR) | N-(5-(6-(3-methoxy-4-(5-ox o-2,5-dihydro-1,2,4-oxadiaz ol-3-yl)phenyl)pyrazin-2-yl)t hiophen-3-yl)pentanamide ¹H NMR (400 MHz, DMSO) δ 10.76 (s, 1H), 9.29 (d, *J* = 4.3 Hz, 2H), 8.16 (d, *J* = 1.4 Hz, 1H), 7.95 (d, *J* = 1.4 Hz, 1H), 7.88 (s, 1H), 6.52 (s, 1H), 4.00 (s, 3H), 2.35-2.32 (m, 2H), 1.20-1.16 (m, 2H), 1.09-1.03 (m, 2H), 0.91 (t, *J*=8.0 Hz, 3H). |
| 40 | A40 | 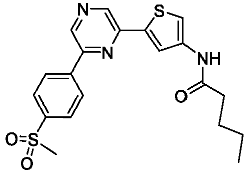 | 416.10/416. 30 | N-(5-(6-(4-(methylsulfonyl) phenyl)pyrazin-2-yl)thiophe n-3-yl)pentanamide |
| 41 | A41 | 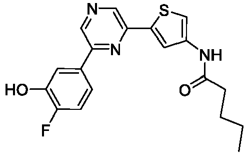 | 372.11/372. 10 | N-(5-(6-(4-fluoro-3-hydroxy phenyl)pyrazin-2-yl)thiophe n-3-yl)pentanamide |
| 42 | A42 | 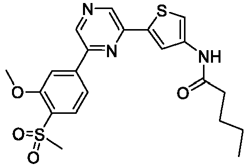 | 446.11/446. 00 | N-(5-(6-(3-methoxy-4-(meth ylsulfonyl)phenyl)pyrazin-2-yl)thiophen-3 -yl)pentanamid e |
| 43 | A43 | 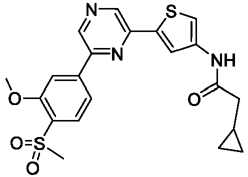 | 444.10/444. 10 | 2-cyclopropyl-N-(5-(6-(3-m ethoxy-4-(methylsulfonyl)ph enyl)pyrazin-2-yl)thiophen-3 -yl)acetamide |
| 44 | A44 | 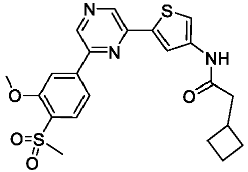 | 458.11/458. 10 | 2-cyclobutyl-N-(5-(6-(3-met hoxy-4-(methylsulfonyl)phe nyl)pyrazin-2-yl)thiophen-3-yl)acetamide |
| 45 | A45 | 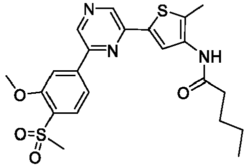 | 460.13/460. 10 | N-(5-(6-(3-methoxy-4-(meth ylsulfonyl)phenyl)pyrazin-2-yl)-2-methylthiophen-3 -yl)p entanamide |

### Example 46: Preparation of 2-methoxy-4- (6-(4-pentamidothiophen-2-yl) pyrazin-2-yl)- N-(1H-tetrazol-5-yl) benzamide (B4)

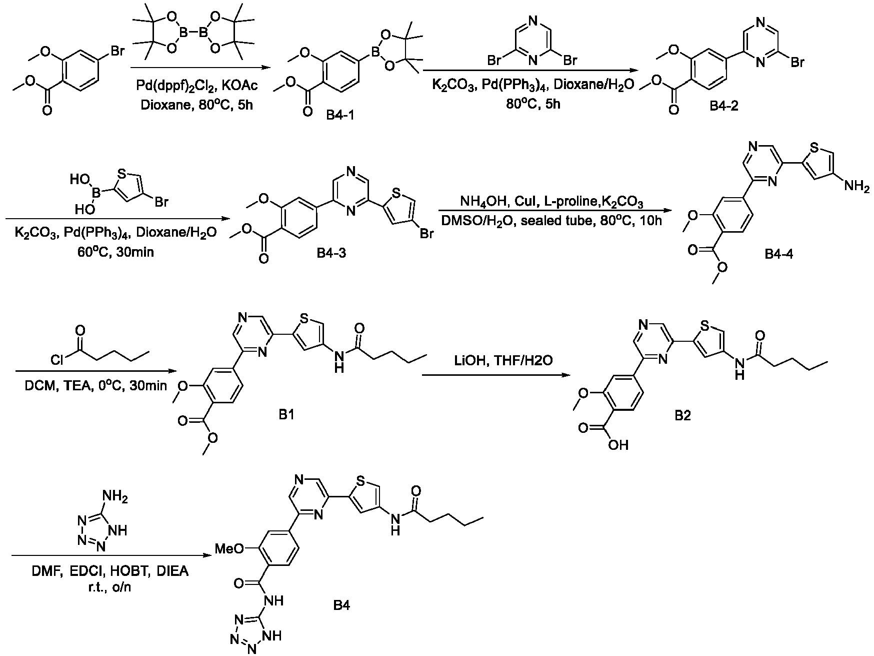

### Step 1: 3-methoxy-4-methoxycarbonylphenyl boronic acid pinacol ester (B4-1)

Methyl 4-Bromo-2-methoxybenzoate (10 g, 40.8 mmol), potassium acetate (10.1 g, 103 mmol), 1,1'-bis(diphenylphosphino)ferroceneldichloropalladium(II) (1.51 g, 2.06 mmol), bis(pinacolato)diboron (12.44 g, 49.0 mmol) and 1,4-dioxane (100 mL) were successively added to a reaction flask and the mixture was reacted under nitrogen protection at 80°C for 5 hours. TLC monitoring showed that the reaction was complete. The reaction solution was dried by rotary dryer directly, and purified by column chromatography to afford 3-methoxy-4-methoxycarbonylphenyl boronic acid pinacol ester (B4-1, 11.0 g, 92.3%). ¹H NMR (400 MHz, CDC13) δ 7.78 (d, *J* = 7.6 Hz, 1H), 7.43 (d, *J* = 7.6 Hz, 1H), 7.40 (s, 1H), 3.97 (s, 3H), 3.91 (s, 3H), 1.37 (s, 12H).

### Step 2: methyl 2-methoxy-4-(6-bromopyrazin-2-yl) benzoate (B4-2)

2,6-dibromopyrazine (11.6 g, 48.8 mmol), 3-methoxy-4-methoxycarbonylphenyl boronic acid pinacol ester (B4-1, 11.0 g, 37.6 mmol), potassium carbonate(13.52 g, 98.0 mmol), tetrakis(triphenylphosphine)palladium(2.37 g, 2.05 mmol), 1,4-dioxane (120 mL), and water (10mL) were added successively to a reaction flask. The mixture was reacted under nitrogen protection at 100°C for 5 hours, and TLC monitoring showed that the reaction was complete. The most of 1,4-dioxane was removed by rotary evaporation, the residue was added with water (50 mL), and extracted with ethyl acetate (30 mL*3). The organic phases were combined, washed successively with water and saturated saline, dried over anhydrous sodium sulfate, spin-dried and purified by column chromatography to obtain methyl 2-methoxy-4-(6-bromopyrazin-2-yl) benzoate (B4-2, 6.67 g, 54.7%). MS (ESI) m/z: calcd 323.00(M+H⁺), found 322.90; ¹H NMR (400 MHz, CDCl₃) δ 9.00 (s, 1H), 8.68 (s, 1H), 7.94 (d, *J* = 8.1 Hz, 1H), 7.71 (d, *J* = 1.4 Hz, 1H), 7.59 (dd, *J* = 8.1, 1.6 Hz, 1H), 4.05 (s, 3H), 3.95 (s, 3H).

### Step 3: methyl 2-methoxy-4-(6-bromothiophen-2-yl) pyrazin-2-yl)benzoate (B4-3)

Methyl 2-methoxy-4-(6-bromopyrazin-2-yl) benzoate (B4-2, 6.67 g, 20.8 mmol), (4-bromothiophen-2-yl) boronic acid (4.30 g, 20.8 mmol), tetrakis(triphenylphosphorus)palladium (1.3 g, 0.86 mmol), potassium carbonate(10.25 g,74.3 mmol), 1,4-dioxane (120 mL), and water (2.5mL) were successively added to a reaction flask. The mixture was reacted under nitrogen protection at 60°C for 0.5 hour, and TLC monitoring showed that the reaction was complete. The most of 1,4-dioxane was removed by rotary evaporation, the residue was added with dichloromethane (60 mL) and water (30 mL), and fractioned. The organic phases were combined, washed with saturated salt water (30 mL * 1), dried over anhydrous sodium sulfate, spin-dried and purified by column chromatography to obtain methyl 2-methoxy-4-(6-bromothiophen-2-yl) pyrazin-2-yl)benzoate (B4-3, 3.96 g, 47.3%). MS (ESI) m/z: calcd 404.98(M+H⁺), found 404.90; ¹H NMR (400 MHz, CDC13) δ 8.97 (s, 1H), 8.90 (s, 1H), 7.97 (d, *J* = 8.1 Hz, 1H), 7.82 (s, 1H), 7.68 (d, *J* = 6.5 Hz, 1H), 7.44 (s, 1H), 6.98 (s, 1H), 4.08 (s, 3H), 3.96 (s, 3H).

### Step 4: methyl 2-methoxy-4-(6-(4-aminothiophen-2-yl) pyrazin-2-yl)benzoate (B4-4)

Methyl 2-methoxy-4-(6-bromothiophen-2-yl) pyrazin-2-yl)benzoate(B4-3, 400 mg, 0.99 mmol), cuprous iodide (47 mg, 0.25 mmol), L-proline (57 mg, 0.50 mmol), dimethyl sulfoxide (25 mL), and ammonia (25% wt, 1.0g, 5.92 mmol) were sequentially added to a sealed tube, and the reaction was sealed and reacted at 80 °C for 10 hours. TLC monitoring showed that the reaction was complete. The reaction solution was cooled to room temperature, added with water (100 mL), and extracted with ethyl acetate (40 mL * 3). The organic phases were combined , washed with saturated ammonium chloride (40 mL * 2), dried over anhydrous sodium sulfate, and spin-dried to obtain methyl 2-methoxy-4-(6-(4-aminothiophen-2-yl) pyrazin-2-yl)benzoate (B4-4, 450mg crude, theoretical 0.99 mmol), which was directly used in the next reaction without any purification. MS (ESI) m/z: calcd 342.08 (M+H⁺), found 342.10_{∘}

### Step 5: methyl 2-methoxy-4-(6-(4-pentamidothiophen-2-yl) pyrazin-2-yl)benzoate (B1)

Methyl 2-methoxy-4-(6-(4-aminothiophen-2-yl) pyrazin-2-yl)benzoate (B4-4, 450 mg crude, theoretical 0.99 mmol) was dissolved in dichloromethane (5 mL). The solution was cooled under an ice water bath, and added with triethylamine (666 mg, 6.59 mmol). Additionally, pentanoyl chloride (318 mg, 2.64 mmol) was taken and dissolved in dichloromethane (1 mL) and the resulting mixture was slowly dropped into the above system. Upon addition, the reaction was warmed to room temperature and reacted for 0.5 hour. TLC monitoring showed that the reaction was complete. The reaction solution was directly spin dried and purified by column chromatography to obtain methyl 2-methoxy-4-(6-(4-pentamidothiophen-2-yl) pyrazin-2-yl)benzoate (B1, 295 mg, 70.0%). MS (ESI) m/z: calcd 426.14(M+H⁺), found 426.10. ¹H NMR (400 MHz, DMSO) δ 10.42 (s, 1H), 9.27 (s, 1H), 9.13 (s, 1H), 7.91 (d, *J* = 3.2 Hz, 2H), 7.85 (s, 2H), 7.74 (s, 1H), 3.98 (s, 3H), 3.84 (s, 3H), 2.32 (t, *J* = 7.4Hz, 2H), 1.67-1.54 (m, 2H), 1.39-1.30(m, 2H), 0.92 (t, *J* = 7.3 Hz, 3H).

### Step 6: 2-Methoxy-4- (6- (4-pentamidothiophen-2-yl) pyrazin-2-yl)benzoic acid (B2)

Methyl 2-methoxy-4-(6-(4-pentamidothiophen-2-yl) pyrazin-2-yl)benzoate (B1, 295 mg, 0.69 mmol), lithium hydroxide monohydrate (146 mg, 3.48 mmol), tetrahydrofuran(4 mL) and water (2 mL) were added successively to a reaction flask and the mixture was reacted at room temperature for 16 hours. TLC monitoring showed that the reaction was complete. The reaction solution was evaporated by rotary dryer to remove tetrahydrofuran, diluted by adding 5 mL of water, acidified to pH=3-4 using 1M hydrochloric acid and extracted with ethyl acetate (10 mL*3). The organic phases were combined, washed successively with water and saturated salt water, dried over anhydrous sodium sulfate, spin-dried and purified by column chromatography to obtain 2-methoxy-4- (6-(4-pentamidothiophen-2-yl) pyrazin-2-yl)benzoic acid (B2, 258 mg, 90.4%). MS (ESI) m/z: calcd 412.13(M+H⁺), found 412.10.

### Step 7: 2-methoxy-4- (6-(4-pentamidothiophen-2-yl) pyrazin-2-yl)-N-(1H-tetrazol-5-yl) benzamide (B4)

2-Methoxy-4- (6- (4-pentamidothiophen-2-yl) pyrazin-2-yl)benzoic acid (B4-6, 258 mg, 0.63 mmol), 5-aminotetrazole (56 mg, 0.66 mmol), 1-(3-dimethylaminopropyl) -3-ethylcarbodiimide hydrochloride (241 mg, 1.26 mmol), 1-hydroxybenzotriazole (170 mg, 1.26 mmol), N,N-diisopropylethylamine (324 mg, 2.51 mmol), and N, N-dimethylformamide (5 mL) were sequentially added to a reaction flask and the mixture was reacted at room temperature for 16 hours. TLC monitoring showed that the reaction was complete. The reaction solution was diluted with water (50 mL) and extracted with ethyl acetate (20 mL*3). The organic phases were combined, washed successively with water and saturated salt water, dried over anhydrous sodium sulfate, spin-dried and purified by column chromatography to obtain 2-methoxy-4- (6-(4-pentamidothiophen-2-yl) pyrazin-2-yl)- N-(1H-tetrazol-5-yl) benzamide (B4, 115 mg, 38.3%). MS (ESI) m/z: calcd 477.15(M-H⁺), found 477.10; ¹H NMR (400 MHz, DMSO) δ 10.40 (s, 1H), 9.29 (s, 1H), 9.13 (s, 1H), 8.05-7.82 (m, 3H), 7.73 (d, *J* = 1.3 Hz, 1H), 4.05 (s, 3H), 2.36-2.26 (m, 2H), 1.64-1.57(m, 2H), 1.44-1.28 (m, 2H), 0.92 (t, *J* = 7.3 Hz, 3H).

### Examples 47-121 (compounds B1-B78) are prepared using experimental steps similar to those in Example 46 above, and compounds B1-B78 are summarized in Table 3.

**Table 3**

| Exam ple | Compoun d No. | Structure | MS(calcd) [M+H]⁺/M S (found) | Name and characterization |
|---|---|---|---|---|
| 46 | B1 | 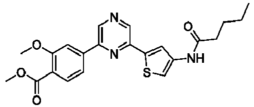 | 426.14/426 .10 | Methyl 2-methoxy-4-(6-(4-pentana midothiophen-2-yl)pyrazin-2-yl)benzoate |
| 46 | B2 | 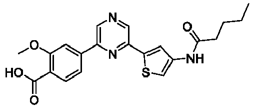 | 412.13/412 .00 | 2-methoxy-4-(6-(4-pentana midothiophen-2-yl)pyrazin-2-yl)benzoic acid |
| 47 | B3 | 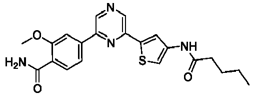 | 411.14/410. 96 | 2-methoxy-4-(6-(4-pentana midothiophen-2-yl)pyrazin-2-yl)benzamide |
| 46 | B4 | 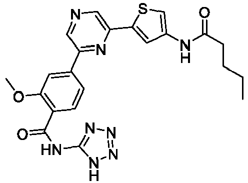 | 479.15/477 .10 (-) | 2-methoxy-4-(6-(4-pentana midothiophen-2-yl)pyrazin-2-yl)-N-(1H-tetrazol-5-yl)be nzamide |
| 48 | B5 | 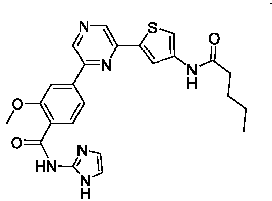 | 477.16/477 .10 | 2-methoxy-4-(6-(4-pentana midothiophen-2-yl)pyrazin-2-yl)-N-(1H-imidazol-2-yl) benzamide |
| 49 | B6 | 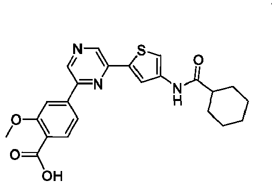 | 438.14/438 .10 | 4-(6-(4-(cyclohexanecarbox amido)thiophen-2-yl)pyrazi n-2-yl)-2-methoxybenzoic acid |
| 50 | B7 | 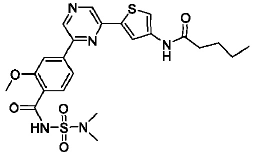 | 518.15/518 .00 | 2-methoxy-4-(6-(4-pentana midothiophen-2-yl)pyrazin-2-yl)-N-(N,N-dimethylsulfa moyl)benzamide |
| 51 | B8 | 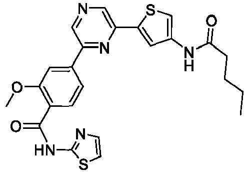 | 494.12/494 .20 | 2-methoxy-4-(6-(4-pentana midothiophen-2-yl)pyrazin-2-yl)-N-(thiazol-2-yl)benza mide |
| 52 | B9 | 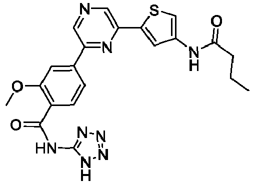 | 465.14/464 .80 | 2-methoxy-4-(6-(4-butyrami dothiophen-2-yl)pyrazin-2-y 1)-N-(1H-tetrazol-5-yl)benza mide |
| 53 | B10 | 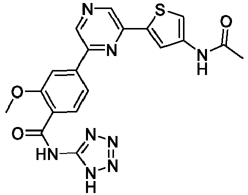 | 437.11/435. 10 (-) | 2-methoxy-4-(6-(4-acetamid othiophen-2-yl)pyrazin-2-yl )-N-(1H-tetrazol-5-yl)benza mide |
| 54 | B11 | 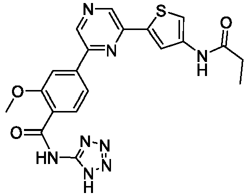 | 451.12/449 .10 (-) | 2-methoxy-4-(6-(4-propiona midothiophen-2-yl)pyrazin-2-yl)-N-(1H-tetrazol-5-yl)be nzamide |
| 55 | B12 | 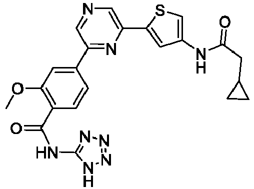 | 477.14/475 .10 (-) | 2-methoxy-4-(6-(4-(2-cyclo propylacetamido)thiophen-2 -yl)pyrazin-2-yl)-N-(1H-tetr azol-5-yl)benzamide 2- 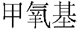 -4-(6-(4-(2- 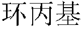 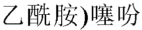 -2- 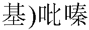 -2- 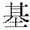 )- N- (1H - 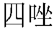 -5- 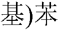 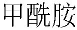 |
| 56 | B13 | 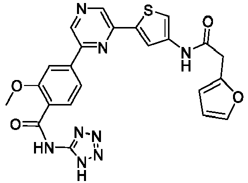 | 503.12/No MS signals (HNMR) | 2-methoxy-4-(6-(4-(2-(furan -2-yl)acetamido)thiophen-2-yl)pyrazin-2-yl)-N-(1H-tetra zol-5-yl)benzamide |
| | | | | ¹H NMR (400 MHz, DMSO) δ 10.74 (s, 1H), 9.29 (s, 1H), 9.16 (s, 1H), 7.93 (d, *J* = 14.2 Hz, 4H), 7.75 (s, 1H), 7.59 (s, 1H), 6.43 (s, 1H), 6.31 (s, 1H), 5.97 (d, *J* = 16.9 Hz, 1H), 4.07 (s, 3H), 3.75 (s, 2H). |
| 57 | B14 | 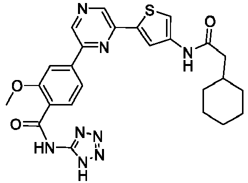 | 519.18/No MS signals (HNMR) | 2-methoxy-4-(6-(4-(2-cyclo hexylacetamido)thiophen-2-yl)pyrazin-2-yl)-N-(1H-tetra zol-5-yl)benzamide |
| | | | | ¹H NMR (400 MHz, DMSO) δ 10.40 (s, 1H), 9.28 (s, 1H), 9.12 (s, 1H), 7.93 (d, *J* = 12.5 Hz, 3H), 7.75 (s, 1H), 5.93 (d, *J* = 14.2 Hz, 1H), 4.08 (s, 3H), 2.21 (d, *J* = 6.8 Hz, 2H), 1.71-1.61 (m, 5H), 1.27-1.17(m, 6H). |
| 58 | B15 | 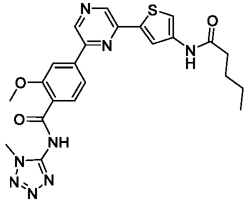 | 493.17/493 .10 | 2-methoxy-4-(6-(4-pentana midothiophen-2-yl)pyrazin-2-yl)-N-(1-methyl-1H-tetraz ol-5-yl)-benzamide |
| 59 | B16 | 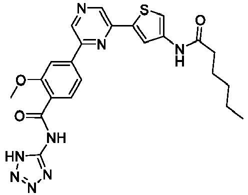 | 493.17/493 .20 | 2-methoxy-4-(6-(4-hexanam idothiophen-2-yl)pyrazin-2-yl)-N-(1H-tetrazol-5-yl)ben zamide |
| 60 | B17 | 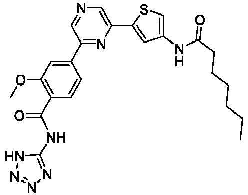 | 507.18/ No MS signals (HNMR) | 2-methoxy-4-(6-(4-heptana midothiophen-2-yl)pyrazin-2-yl)-N-(1H-tetrazol-5-yl)be nzamide |
| | | | | ¹H NMR (400 MHz, DMSO) δ 10.41 (s, 1H), 9.29 (s, 1H), 9.13 (s, 1H), 7.93 (d, *J* = 13.1 Hz, 3H), 7.74 (s, 1H), 4.07 (s, 3H), 2.39-2.27 (m, 2H), 1.66-1.58 (m, 2H), 1.36-1.25 (m, 4H), 1.17 (t, *J* = 7.2 Hz, 3H), 0.92-0.84 (m, 2H). |
| 61 | B18 | 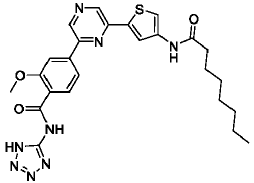 | 521.20/519 .10 (-) | 2-methoxy-4-(6-(4-octanami dothiophen-2-yl)pyrazin-2-y 1)-N-(1H-tetrazol-5-yl)benza mide |
| 62 | B19 | 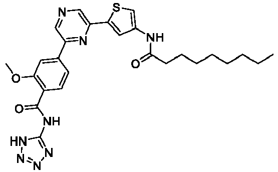 | 535.22/ No MS signals (HNMR) | 2-methoxy-4-(6-(4-nonanam idothiophen-2-yl)pyrazin-2-yl)-N-(1H-tetrazol-5-yl)ben zamide |
| | | | | ¹H NMR (400 MHz, DMSO) δ 10.41 (s, 1H), 9.30 (s, 1H), 9.14 (s, 1H), 7.90 (dd, *J* = 28.4, 11.5 Hz, 4H), 7.74 (s, 1H), 4.06 (s, 3H), 2.39-2.26 (m, 2H), 1.68-1.57 (m, 2H), 1.34-1.23 (m, 10H), 0.87 (s, 3H). |
| 63 | B20 | 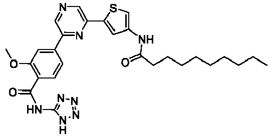 | 549.23/ No MS signals (HNMR) | 2-methoxy-4-(6-(4-decanam idothiophen-2-yl)pyrazin-2-yl)-N-(1H-tetrazol-5-yl)ben zamide |
| | | | | ¹H NMR (400 MHz, DMSO) δ 10.40 (s, 1H), 9.29 (s, 1H), 9.13 (s, 1H), 7.93 (d, *J* = 13.4 Hz, 4H), 7.74 (s, 1H), 4.06 (s, 3H), 2.35-2.28 (m, 2H), 1.66-1.58 (m, 2H), 1.30-1.24 (m, 10H), 1.18 (t, *J* = 7.3 Hz, 2H), 0.87 (t, *J* = 6.4 Hz, 3H). |
| 64 | B21 | 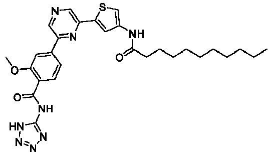 | 563.25/ No MS signals (HNMR) | 2-methoxy-4-(6-(4-undecan amidothiophen-2-yl)pyrazin -2-yl)-N-(1H-tetrazol-5-yl)b enzamide |
| | | | | ¹H NMR (400 MHz, DMSO) δ 10.41 (s, 1H), 9.29 (s, 1H), 9.13 (s, 1H), 7.91 (dd, *J* = 22.8, 11.0 Hz, 4H), 7.74 (s, 1H), 4.06 (s, 3H), 2.35-2.28 (m, 2H), 1.61 (s, 2H), 1.30-1.24 (m, 14H), 0.86 (s, 3H). |
| 65 | B22 | 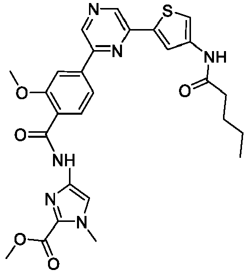 | 549.18/549 .20 | methyl 4-(2-methoxy-4-(6-(4-penta namidothiophen-2-yl)pyrazi n-2-yl)benzamido)-1-methyl -1H-imidazole-2-carboxylat e |
| 66 | B23 | 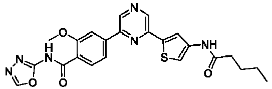 | 479.14/479 .10 | 2-methoxy-4-(6-(4-pentana midothiophen-2-yl)pyrazin-2-yl)-N-(1,3,4-oxadiazol-2-yl)benzamide |
| 67 | B24 | 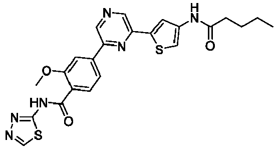 | 495.12/495 .10 | 2-methoxy-4-(6-(4-pentana midothiophen-2-yl)pyrazin-2-yl)-N-(1,3,4-thiadiazol-2-yl)benzamide |
| 68 | B25 | 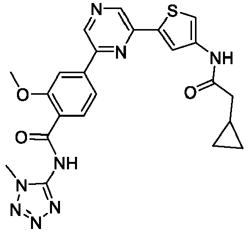 | 491.15/491 .10 | 2-methoxy-4-(6-(4-(2-cyclo propylacetamido)thiophen-2 -yl)pyrazin-2-yl)-N-(1-meth yl-1H-tetrazol-5-yl)benzami de |
| 69 | B26 | 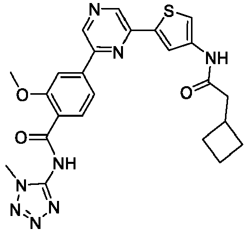 | 505.17/505 .30 | 2-methoxy-4-(6-(4-(2-cyclo butylacetamido)thiophen-2-yl)pyrazin-2-yl)-N-(1-methy 1-1H-tetrazol-5-yl)benzamid e |
| 70 | B27 | 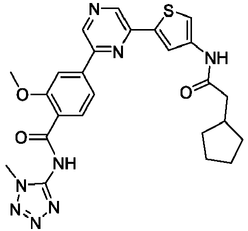 | 519.18/519 .10 | 2-methoxy-4-(6-(4-(2-cyclo pentylacetamido)thiophen-2 -yl)pyrazin-2-yl)-N-(1-meth yl-1H-tetrazol-5-yl)benzami de |
| 71 | B28 | 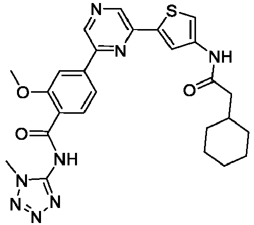 | 533.20/533 .20 | 2-methoxy-4-(6-(4-(2-cyclo hexylacetamido)thiophen-2-yl)pyrazin-2-yl)-N-(1-methy 1-1H-tetrazol-5-yl)benzamid e |
| 72 | B29 | 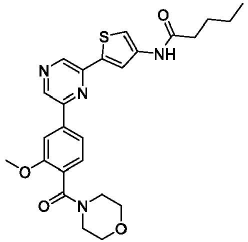 | 481.18/481 .20 | N-(5-(6-(3-methoxy-4-(mor pholine-4-carbonyl)phenyl) pyrazin-2-yl)thiophen-3-yl) pentanamide |
| 73 | B30 | 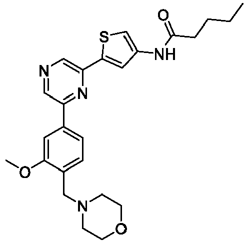 | 467.20/467 .10 | N-(5-(6-(3-methoxy-4-(mor pholinomethyl)phenyl)pyraz in-2-yl)thiophen-3-yl)pentan amide |
| 74 | B31 | 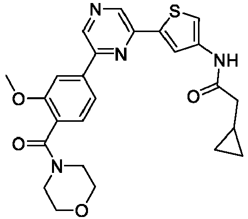 | 479.17/479 .20 | 2-cyclopropyl-N-(5-(6-(3 -m ethoxy-4-(morpholine-4-car bonyl)phenyl)pyrazin-2-yl)t hiophen-3-yl)acetamide |
| 75 | B32 | 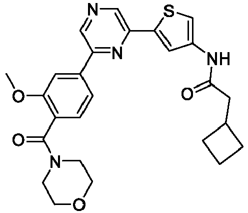 | 493.18/493 .30 | 2-cyclobutyl-N-(5-(6-(3-met hoxy-4-(morpholine-4-carbo nyl)phenyl)pyrazin-2-yl)thio phen-3-yl)acetamide |
| 76 | B33 | 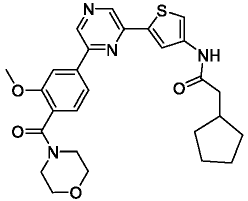 | 507.20/507 .20 | 2-cyclopentyl-N-(5-(6-(3-m ethoxy-4-(morpholine-4-car bonyl)phenyl)pyrazin-2-yl)t hiophen-3-yl)acetamide |
| 77 | B34 | 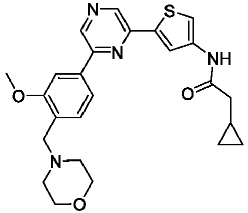 | 465.19/465 .20 | 2-cyclopropyl-N-(5-(6-(3 -m ethoxy-4-(morpholinomethy l)phenyl)pyrazin-2-yl)thioph en-3-yl)acetamide |
| 78 | B35 | 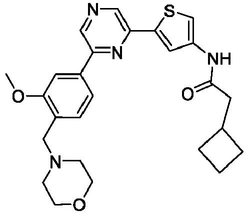 | 479.20/479 .20 | 2-cyclobutyl-N-(5-(6-(3-met hoxy-4-(morpholinomethyl) phenyl)pyrazin-2-yl)thiophe n-3-yl)acetamide |
| 79 | B36 | 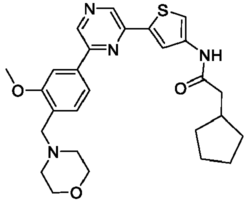 | 493.22/493 .20 | 2-cyclopentyl-N-(5-(6-(3-m ethoxy-4-(morpholinomethy l)phenyl)pyrazin-2-yl)thioph en-3-yl)acetamide |
| 80 | B37 | 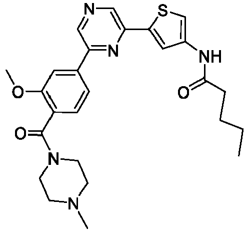 | 494.21/494 .20 | N-(5-(6-(3-methoxy-4-(4-m ethylpiperazine-1-carbonyl) phenyl)pyrazin-2-yl)thiophe n-3-yl)pentanamide |
| 81 | B38 | 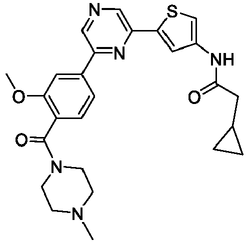 | 492.20/492 .20 | 2-cyclopropyl-N-(5-(6-(3 -m ethoxy-4-(4-methylpiperazi ne-1-carbonyl)phenyl)pyrazi n-2-yl)thiophen-3-yl)acetam ide |
| 82 | B39 | 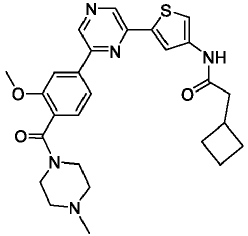 | 506.21/506 .10 | 2-cyclobutyl-N-(5-(6-(3-met hoxy-4-(4-methylpiperazine -1-carbonyl)phenyl)pyrazin-2-yl)thiophen-3-yl)acetamid e |
| 83 | B40 | 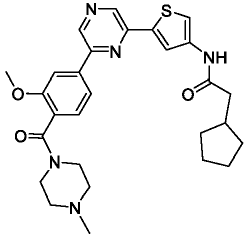 | 520.23/520 .20 | 2-cyclopentyl-N-(5-(6-(3-m ethoxy-4-(4-methylpiperazi ne-1-carbonyl)phenyl)pyrazi n-2-yl)thiophen-3-yl)acetam ide |
| 84 | B41 | 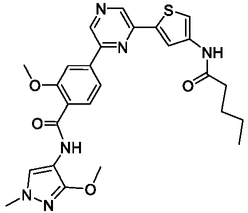 | 521.19/521 .20 | 2-methoxy-N-(3-methoxy-1 -methyl-1H-pyrazol-4-yl)-4-(6-(4-pentanamidothiophen-2-yl)pyrazin-2-yl)benzamid e |
| 85 | B42 | 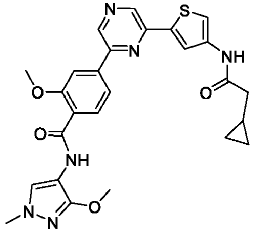 | 519.17/519 .10 | 4-(6-(4-(2-cyclopropylaceta mido)thiophen-2-yl)pyrazin-2-yl)-2-methoxy-N-(3-meth oxy-1-methyl-1H-pyrazol-4-yl)benzamide |
| 86 | B43 | 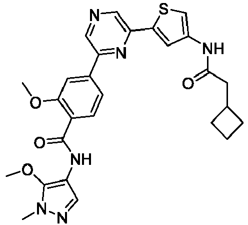 | 533.19/533 .10 | 4-(6-(4-(2-cyclobutylacetam ido)thiophen-2-yl)pyrazin-2 -yl)-2-methoxy-N-(5-metho xy-1-methyl-1H-pyrazol-4-y l)benzamide |
| 87 | B44 | 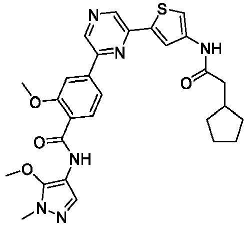 | 547.20/547 .30 | 4-(6-(4-(2-cyclopentylaceta mido)thiophen-2-yl)pyrazin-2-yl)-2-methoxy-N-(5-meth oxy-1-methyl-1H-pyrazol-4-yl)benzamide |
| 88 | B45 | 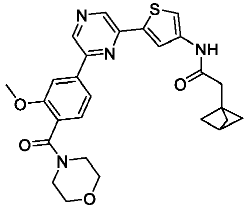 | 505.18/505 .30 | 2-(bicyclo[1.1.1]pentan-1-yl )-N-(5-(6-(3-methoxy-4-(mo rpholine-4-carbonyl)phenyl) pyrazin-2-yl)thiophen-3-yl)a cetamide |
| 89 | B46 | 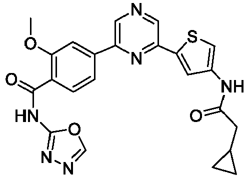 | 477.13/477 .20 | 4-(6-(4-(2-cyclopropylaceta mido)thiophen-2-yl)pyrazin-2-yl)-2-methoxy-N-(1,3,4-o xadiazol-2-yl)benzamide |
| 90 | B47 | 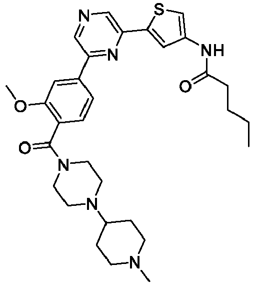 | 577.29/577 .20 | N-(5-(6-(3-methoxy-4-(4-(1 -methylpiperidin-4-yl)pipera zine-1-carbonyl)phenyl)pyra zin-2-yl)thiophen-3-yl)penta namide |
| 91 | B48 | 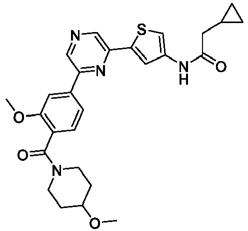 | 507.20/507 .30 | 2-cyclopropyl-N-(5-(6-(3 -m ethoxy-4-(4-methoxypiperid ine-1-carbonyl)phenyl)pyraz in-2-yl)thiophen-3-yl)aceta mide |
| 92 | B49 | 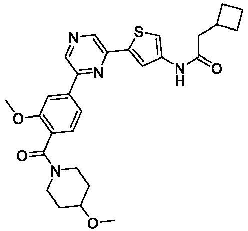 | 521.21/521 .30 | 2-cyclobutyl-N-(5-(6-(3-met hoxy-4-(4-methoxypiperidin e-1-carbonyl)phenyl)pyrazin -2-yl)thiophen-3-yl)acetami de |
| 93 | B50 | 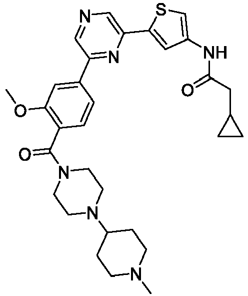 | 575.27/575 .40 | 2-cyclopropyl-N-(5-(6-(3 -m ethoxy-4-(4-(1-methylpiperi din-4-yl)piperazine-1-carbo nyl)phenyl)pyrazin-2-yl)thio phen-3-yl)acetamide |
| 94 | B51 | 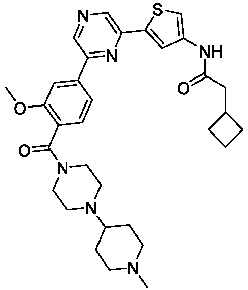 | 589.29/589 .30 | 2-cyclobutyl-N-(5-(6-(3-met hoxy-4-(4-(1-methylpiperidi n-4-yl)piperazine-1-carbony l)phenyl)pyrazin-2-yl)thioph en-3-yl)acetamide |
| 95 | B52 | 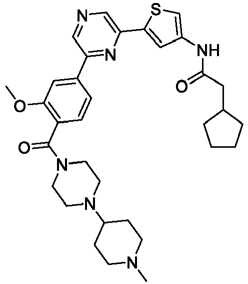 | 603.30/603 .40 | 2-cyclopentyl-N-(5-(6-(3-m ethoxy-4-(4-(1-methylpiperi din-4-yl)piperazine-1 -carbo nyl)phenyl)pyrazin-2-yl)thio phen-3-yl)acetamide |
| 96 | B53 | 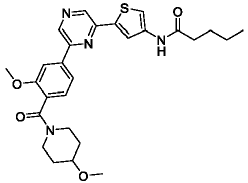 | 509.21/509 .20 | N-(5-(6-(3-methoxy-4-(4-m ethoxypiperidine-1-carbonyl )phenyl)pyrazin-2-yl)thioph en-3-yl)pentanamide |
| 97 | B54 | 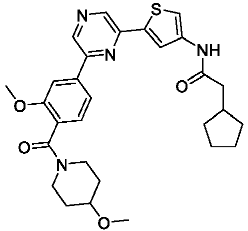 | 535.23/535 .30 | 2-cyclopentyl-N-(5-(6-(3-m ethoxy-4-(4-methoxypiperid ine-1-carbonyl)phenyl)pyraz in-2-yl)thiophen-3-yl)aceta mide |
| 98 | B55 | 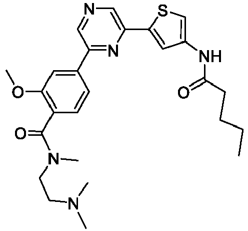 | 496.23/496 .30 | N-(2-(dimethylamino)ethyl) -2-methoxy-N-methyl-4-(6-( 4-pentanamidothiophen-2-yl )pyrazin-2-yl)benzamide |
| 99 | B56 | 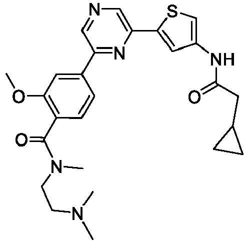 | 494.21/494 .30 | 4-(6-(4-(2-cyclopropylaceta mido)thiophen-2-yl)pyrazin-2-yl)-N-(2-(dimethylamino) ethyl)-2-methoxy-N-methyl benzamide |
| 100 | B57 | 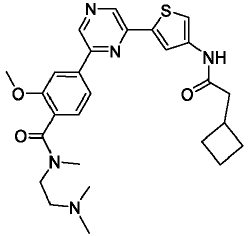 | 508.23/508 .20 | 4-(6-(4-(2-cyclobutylacetam ido)thiophen-2-yl)pyrazin-2 -yl)-N-(2-(dimethylamino)et hyl)-2-methoxy-N-methylbe nzamide |
| 101 | B58 | 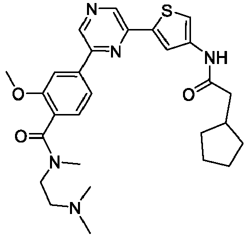 | 522.25/522 .30 | 4-(6-(4-(2-cyclopentylaceta mido)thiophen-2-yl)pyrazin-2-yl)-N-(2-(dimethylamino) ethyl)-2-methoxy-N-methyl benzamide |
| 102 | B59 | 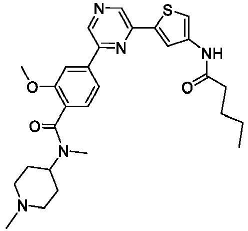 | 522.25/522 .40 | 2-methoxy-N-methyl-N-(1-methylpiperidin-4-yl)-4-(6-( 4-pentanamidothiophen-2-yl )pyrazin-2-yl)benzamide |
| 103 | B60 | 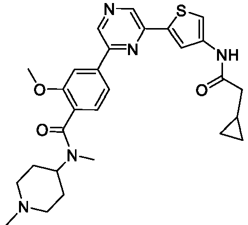 | 520.23/520 .30 | 4-(6-(4-(2-cyclopropylaceta mido)thiophen-2-yl)pyrazin-2-yl)-2-methoxy-N-methyl-N-(1-methylpiperidin-4-yl)b enzamide |
| 104 | B61 | 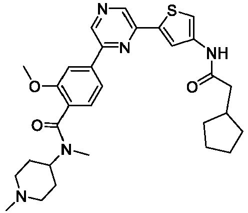 | 548.26/548 .30 | 4-(6-(4-(2-cyclopentylaceta mido)thiophen-2-yl)pyrazin-2-yl)-2-methoxy-N-methyl-N-(1-methylpiperidin-4-yl)b enzamide |
| 105 | B62 | 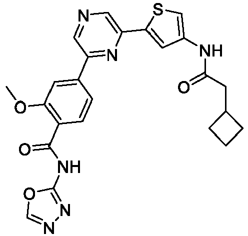 | 491.14/491 .20 | 4-(6-(4-(2-cyclobutylacetam ido)thiophen-2-yl)pyrazin-2 -yl)-2-methoxy-N-(1,3,4-ox adiazol-2-yl)benzamide |
| 106 | B63 | 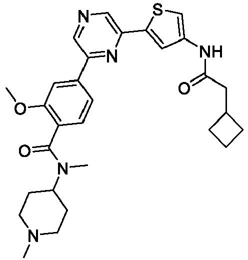 | 534.25/534 .40 | 4-(6-(4-(2-cyclobutylacetam ido)thiophen-2-yl)pyrazin-2 -yl)-2-methoxy-N-methyl-N -(1-methylpiperidin-4-yl)be nzamide |
| 107 | B64 | 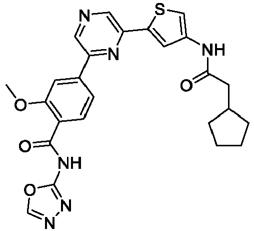 | 505.16/505 .10 | 4-(6-(4-(2-cyclopentylaceta mido)thiophen-2-yl)pyrazin-2-yl)-2-methoxy-N-(1,3,4-o xadiazol-2-yl)benzamide |
| 108 | B65 | 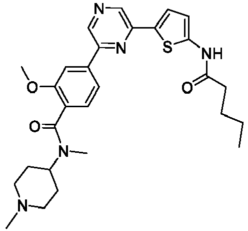 | 522.25/522 .20 | 2-methoxy-N-methyl-N-(1-methylpiperidin-4-yl)-4-(6-( 5-pentanamidothiophen-2-yl )pyrazin-2-yl)benzamide |
| 109 | B66 | 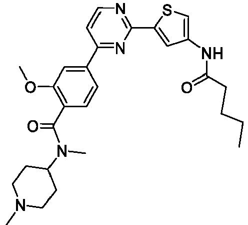 | 522.25/522 .30 | 2-methoxy-N-methyl-N-(1-methylpiperidin-4-yl)-4-(2-( 4-pentanamidothiophen-2-yl )pyrimidin-4-yl)benzamide |
| 110 | B67 | 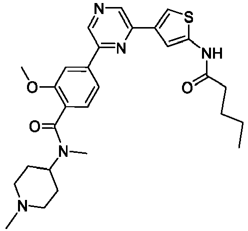 | 522.25/522 .20 | 2-methoxy-N-methyl-N-(1-methylpiperidin-4-yl)-4-(6-( 5-pentanamidothiophen-3-yl )pyrazin-2-yl)benzamide |
| 111 | B68 | 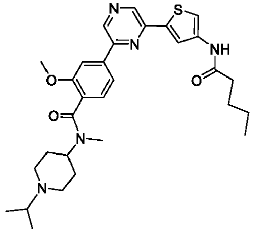 | 550.28/550 .20 | N-(1-isopropylpiperidin-4-y l)-2-methoxy-N-methyl-4-(6 -(4-pentanamidothiophen-2-yl)pyrazin-2-yl)benzamide |
| 112 | B69 | 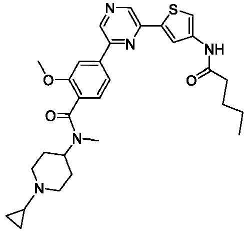 | 548.26/548 .30 | N-(1-cyclopropylpiperidin-4 -yl)-2-methoxy-N-methyl-4-(6-(4-pentanamidothiophen-2-yl)pyrazin-2-yl)benzamid e |
| 113 | B70 | 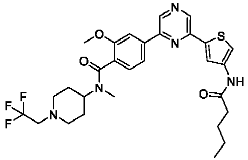 | 590.23/590 .10 | 2-methoxy-N-methyl-4-(6-( 4-pentanamidothiophen-2-yl )pyrazin-2-yl)-N-(1-(2,2,2-tr ifluoroethyl)piperidin-4-yl)b enzamide |
| 114 | B71 | 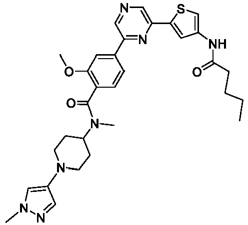 | 588.27/588 .20 | 2-methoxy-N-methyl-N-(1-( 1-methyl-1H-pyrazol-4-yl)p iperidin-4-yl)-4-(6-(4-penta namidothiophen-2-yl)pyrazi n-2-yl)benzamide |
| 115 | B72 | 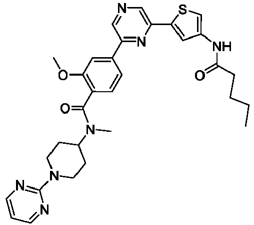 | 586.25/586 .30 | 2-methoxy-N-methyl-4-(6-( 4-pentanamidothiophen-2-yl )pyrazin-2-yl)-N-(1-(pyrimi din-2-yl)piperidin-4-yl)benz amide |
| 116 | B73 | 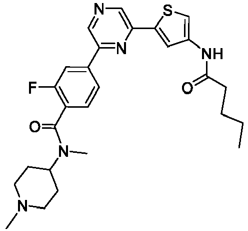 | 510.23/510 .40 | 2-fluoro-N-methyl-N-(1-met hylpiperidin-4-yl)-4-(6-(4-p entanamidothiophen-2-yl)py razin-2-yl)benzamide |
| 117 | B74 | 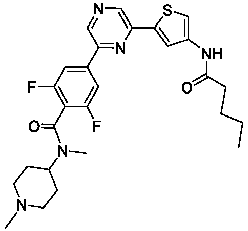 | 528.22/528 .20 | 2,6-difluoro-N-methyl-N-(1 -methylpiperidin-4-yl)-4-(6-(4-pentanamidothiophen-2-y l)pyrazin-2-yl)benzamide |
| 118 | B75 | 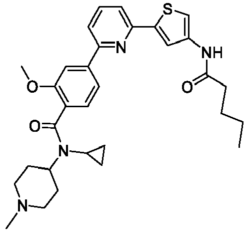 | 547.27/547 .50 | N-cyclopropyl-2-methoxy-N-(1-methylpiperidin-4-yl)-4-(6-(4-pentanamidothiophe n-2-yl)pyridin-2-yl)benzami de |
| 119 | B76 | 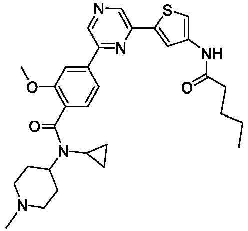 | 548.26/548 .40 | N-cyclopropyl-2-methoxy-N-(1-methylpiperidin-4-yl)-4-(6-(4-pentanamidothiophe n-2-yl)pyrazin-2-yl)benzami de |
| 120 | B77 | 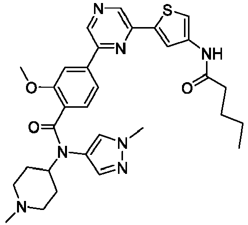 | 588.27/588 .40 | 2-methoxy-N-(1-methyl-1H -pyrazol-4-yl)-N-(1-methylp iperidin-4-yl)-4-(6-(4-penta namidothiophen-2-yl)pyrazi n-2-yl)benzamide |
| 121 | B78 | 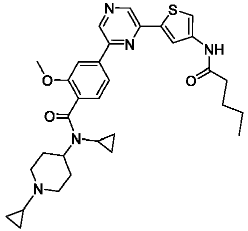 | 574.28/574 .40 | N-cyclopropyl-N-(1-cyclopr opylpiperidin-4-yl)-2-metho xy-4-(6-(4-pentanamidothio phen-2-yl)pyrazin-2-yl)benz amide |

### Example 122: Preparation of 3- (4- (cyclobutylformamido) thiophen-2-yl) -5-(3,4-dimethoxyphenyl) isonicotinamide (C1)

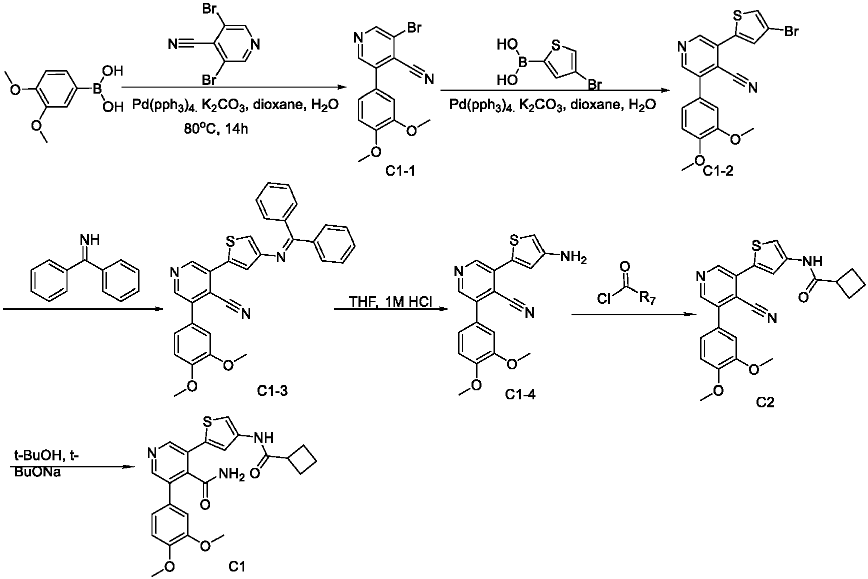

### Step 1: 3-Bromo-5- (3,4-dimethoxyphenyl)isonicotinonitrile(C1-1)

3,5-dibromo-4-cyanopyridine (1.5 g, 5.73 mmol), 3,4-dimethoxyphenylboronic acid (1.15 g, 6.30 mmol), tetrakis(triphenylphosphine)palladium(132 mg, 0.12 mmol), potassium carbonate (1.82 g, 13.17 mmol), 1,4-dioxane (20 mL), and water (5mL) were added to a reaction flask. The mixture was reacted under nitrogen protection at 80°C for 14 hours, and TLC monitoring showed that the reaction was complete. The most of 1,4-dioxane was removed by rotary evaporation, the residue was diluted by adding 25 mL of water, and extracted with dichloromethane (15 mL*3). The organic phases were combined, washed with saturated salt water (15 mL * 1), dried over anhydrous sodium sulfate, and purified by column chromatography after rotary drying to obtain 3-bromo-5-(3,4-dimethoxyphenyl)isonicotinonitrile (C1-1, 676 mg, 37.0%). ¹H NMR (400 MHz, DMSO) δ 9.01 (s, 1H), 8.89 (s, 1H), 7.34 (d, *J* = 2.0 Hz, 1H), 7.26 (dd, *J* = 8.3, 2.0 Hz, 1H), 7.16 (d, *J* = 8.4 Hz, 1H), 3.85 (s, 3H), 3.84 (s, 3H).

### Step 2: 3- (4-bromothiophen-2-yl) -5- (3,4-dimethoxy) isonicotinonitrile (C1-2)

3-Bromo-5- (3,4-dimethoxyphenyl)isonicotinonitrile (C1-1, 470 mg, 0.48mmol), (4-bromothiophen-2-yl) boronic acid (335.1 mg, 1.62 mmol), tetrakis(triphenylphosphine)palladium (34 mg, 0.029 mmol), potassium carbonate(610 mg,4.42 mmol), 1,4-dioxane (10 mL), and water (2.5mL) were added to a reaction flask. The mixture was reacted under nitrogen protection at 80°C for 14 hours, and TLC monitoring showed that the reaction was complete. The most of 1,4-dioxane was removed by rotary evaporation, the residue was diluted by adding water(15 mL), and extracted with dichloromethane (10 mL*3). The organic phases were combined, washed with saturated salt water (5 mL * 1), dried over anhydrous sodium sulfate, and purified by column chromatography after rotary drying to obtain 3- (4-bromothiophen-2-yl) -5-(3,4-dimethoxy) isonicotinonitrile(C1-2, 190.5 mg, 30.8%). ¹H NMR (400 MHz, DMSO) δ 8.94 (s, 1H), 8.90 (s, 1H), 8.02 (d, *J* = 1.3 Hz, 1H), 7.77 (d, *J* = 1.4 Hz, 1H), 7.36 (d, *J* = 2.0 Hz, 1H), 7.29 (dd, *J* = 8.3, 2.0 Hz, 1H), 7.17 (d, *J* = 8.4 Hz, 1H), 3.86 (s, 3H), 3.85 (s, 3H).

### Step 3: 3- (3,4-Dimethoxyphenyl) -5- (4- ((diphenylmethylene) amino) thiophen-2-yl) isonicotinonitrile (C1-3)

3- (4-bromothiophen-2-yl) -5- (3,4-dimethoxy) isonicotinonitrile (C1-2, 100 mg, 0.238 mmol), benzophenone imine (64.83 mg, 0.358 mmol), 1.1'-binaphthyl-2.2'-diphenyl phosphine (29.70 mg, 0.048 mmol), tris(dibenzylideneacetone)dipalladium (21.84 mg, 0.024 mmol), sodium tert-butoxide (68.76 mg, 0.715 mmol), and toluene (5 mL) were sequentially added to a reaction flask. The mixture was reacted under nitrogen protection at 100 °C for 16 hours, and TLC monitoring showed that the reaction was complete. The reaction solution was directly spin-dried and purified by column chromatography to afford 3- (3,4-Dimethoxyphenyl) -5- (4- ((diphenylmethylene) amino) thiophen-2-yl) isonicotinonitrile (C1-3, 110mg, 92.1%). ¹H NMR (400 MHz, CDC13) δ 8.72 (s, 1H), 8.69 (s, 1H), 7.82 (d, *J* = 6.2 Hz, 2H), 7.47-7.40 (m, 10H), 6.82 (d, *J* = 3.5 Hz, 1H), 6.77 (s, 1H), 6.65 (s, 1H), 3.99 (s, 3H), 3.98 (s, 3H).

### Step 4: 3- (4-aminothiophen-2-yl) -5- (3,4-dimethoxyphenyl) isonicotinonitrile hydrochloride (C1-4)

3- (3,4-Dimethoxyphenyl) -5- (4- ((diphenylmethylene) amino) thiophen-2-yl) isonicotinonitrile (C1-3, 110mg, 0.219mmol) was dissolved in dichloromethane. Two drops of concentrated hydrochloric acid were added and the mixture was reacted at room temperature for 4 hours. White solid precipitated, the reaction solution was filtered and the solid was collected, and dried to obtain 3- (4-aminothiophen-2-yl) -5-(3,4-dimethoxyphenyl) isonicotinonitrile hydrochloride (C1-4, 47mg, 57.4%). MS (ESI) m/z: calcd 338.09 (M+H⁺), found 338.10

### Step 5: N - (5- (4-cyano-5- (3,4-dimethoxyphenyl) pyridin-3-yl) thiophen-3-yl) cyclobutylformamide (C2)

3- (4-aminothiophen-2-yl) -5- (3,4-dimethoxyphenyl) isonicotinonitrile hydrochloride (C1-4, 30 mg, 0.08 mmol) was dissolved in 3mL of dichloromethane. The system was cooled under an ice water bath, and added with triethylamine (40.4 mg, 0.40 mmol). Additionally, cyclobutylcarbonyl chloride (11.61 mg, 0.098 mmol) was taken and dissolved in dichloromethane (0.5 mL) and the resulting mixture was slowly dropped into the reaction system. Upon addition, the reaction was warmed to room temperature and reacted for 3 hours. TLC monitoring showed that the reaction was complete. The reaction solution was directly spin-dried and purified by column chromatography to afford N - (5-(4-cyano-5- (3,4-dimethoxyphenyl) pyridin-3-yl) thiophen-3-yl) cyclobutylformamide (C2, 32 mg, 95%). ¹H NMR (400 MHz, CDC13) δ 8.88 (s, 1H), 8.75 (s, 1H), 7.91 (s, 1H), 7.61 (s, 1H), 7.48 (s, 1H), 7.19 (dd, *J* = 8.3, 2.0 Hz, 1H), 7.14 (d, *J* = 1.9 Hz, 1H), 7.06 (d, *J* = 8.3 Hz, 1H), 3.99 (s, 6H), 3.25-3.13 (m, 1H), 2.51-2.37 (m, 2H), 2.31-2.25 (m, 2H).

### Step 6: 3- (4- (cyclobutylformamido) thiophen-2-yl) -5- (3,4-dimethoxyphenyl) isonicotinamide (C1)

N - (5- (4-cyano-5- (3,4-dimethoxyphenyl) pyridin-3-yl) thiophen-3-yl) cyclobutylformamide (C2, 2 mg, 0.0048 mmol), sodium tert-butoxide (1.68 mg, 0.015 mmol), and tert butanol (3 mL) were added to a reaction flask, heated to 50°C and reacted for 2 hours. TLC monitoring showed that the reaction was complete. The reaction solution was cooled to room temperature, quenched by adding ice water (10 mL) and extracted with ethyl acetate (5 mL*3). The organic phases were combined, washed successively with saturated salt water, dried over anhydrous sodium sulfate, and spin-dried to obtain 3-(4- (cyclobutylformamido) thiophen-2-yl) -5- (3,4-dimethoxyphenyl) isonicotinamide (C1, 1.8 mg,). ¹H NMR (400 MHz, CDC13) δ 8.74 (s, 1H), 8.64 (s, 1H), 7.78 (s, 1H), 7.60 (s, 1H), 7.12 (d, *J* = 1.5 Hz, 1H), 7.09 (dd, *J* = 8.0, 1.7 Hz, 1H), 6.97 (d, *J* = 8.3 Hz, 1H), 3.95 (s, 3H), 3.93 (s, 3H), 3.20-3.12 (m, 1H), 2.44-2.36 (m, 2H), 2.28-2.20 (m, 2H).

### Example 123-124 were prepared using experimental steps similar to those in Example 122 above, and compounds C1-C4 were summarized in Table 4.

**Table 4**

| Example | Compound No. | Structure | MS(calcd) [M+H]⁺/ MS (found) | Name and characterization |
|---|---|---|---|---|
| 122 | C1 | 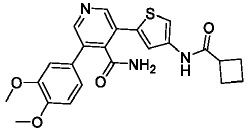 | 438.14/N/ A (HNMR) | 3-(4-(cyclobutanecarboxamido)t hiophen-2-yl)-5-(3,4-dimethoxy phenyl)isonicotinamide |
| | | | | ¹H NMR (400 MHz, CDC13) δ 8.74 (s, 1H), 8.64 (s, 1H), 7.78 (s, 1H), 7.60 (s, 1H), 7.12 (d, *J* = 1.5 Hz, 1H), 7.09 (dd, *J* = 8.0, 1.7 Hz, 1H), 6.97 (d, *J* = 8.3 Hz, 1H), 3.95 (s, 3H), 3.93 (s, 3H), 3.20-3.12 (m, 1H), 2.44-2.36 (m, 2H), 2.28-2.20 (m, 2H). |
| 122 | C2 | 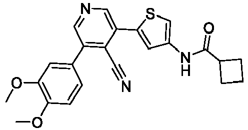 | 420.13/ N/A (HNMR) | N-(5-(4-cyano-5-(3,4-dimethoxy phenyl)pyridin-3-yl)thiophen-3-yl)cyclobutanecarboxamide |
| | | | | ¹H NMR (400 MHz, CDC13) δ 8.88 (s, 1H), 8.75 (s, 1H), 7.91 (s, 1H), 7.61 (s, 1H), 7.48 (s, 1H), 7.19 (dd, *J* = 8.3, 2.0 Hz, 1H), 7.14 (d, *J* = 1.9 Hz, 1H), 7.06 (d, *J* = 8.3 Hz, 1H), 3.99 (s, 6H), 3.25-3.13 (m, 1H), 2.51-2.37 (m, 2H), 2.28 (dd, *J* = 17.3, 8.6 Hz, 2H). |
| 123 | C3 | 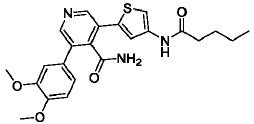 | 440.16/ N/A (HNMR) | 3-(3,4-dimethoxyphenyl)-5-(4-p entanamidothiophen-2-yl)isonic otinamide |
| | | | | ¹H NMR (400 MHz, CDC13) δ 8.74 (s, 1H), 8.64 (s, 1H), 7.77 (s, 1H), 7.69 (s, 1H), 7.27 (s, 1H), 7.12 (s, 1H), 7.09 (d, *J* = 8.2 Hz, 1H), 6.97 (d, *J* = 8.2 Hz, 1H), 3.95 (s, 3H), 3.93 (s, 3H), 2.36 (t, *J* = 7.5 Hz, 2H), 1.76-1.71 (m, 2H), 1.45-1.39 (m, 2H), 0.97 (t, *J* = 7.2 Hz, 3H). |
| 124 | C4 | 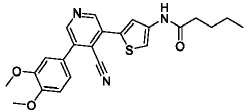 | 422.15 N/A (HNMR) | N-(5-(4-cyano-5-(3,4-dimethoxy phenyl)pyridin-3-yl)thiophen-3-yl)pentanamide |
| | | | | ¹H NMR (400 MHz, CDC13) δ 8.89 (s, 1H), 8.75 (s, 1H), 7.89 (s, 1H), 7.61 (s, 1H), 7.59 (s, 1H), 7.19 (dd, *J* = 8.2, 1.9 Hz, 1H), 7.14 (d, *J* = 1.8 Hz, 1H), 7.06 (d, *J* = 8.3 Hz, 1H), 3.99 (s, 6H), 2.40 (t, *J* = 7.6 Hz, 2H), 1.79-1.74 (m, 2H), 1.49-1.40 (m, 2H), 0.99 (t, *J* = 7.3 Hz, 3H). |

### Example 125: Preparation of N - (4- (6- (3,4-dimethoxyphenyl) pyrazin-2-yl) amino) cyclohexyl) cyclobutylformamide (D2)

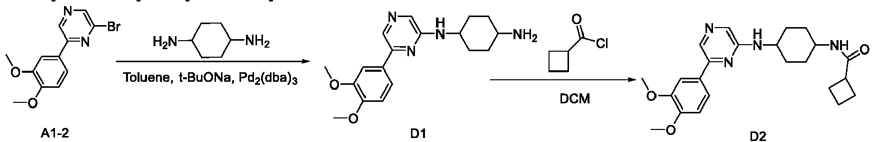

### Step 1: N¹- (6- (3,4-Dimethoxyphenyl) pyrazin-2-yl) cyclohexan-1,4-diamine (D1)

2-bromo-6- (3,4-dimethoxyphenyl) pyrazine (A1-2, 50 mg, 0.17 mmol), 1,4-cyclohexanediamine (97 mg, 0.85 mmol), sodium tert-butoxide (33 mg, 0.34 mmol), 1,1'-binaphthyl-2.2'-diphenyl phosphine (10.6 mg, 0.017 mmol), tris(dibenzylideneacetone)dipalladium (7.3 mg, 0.008 mmol), and toluene (10 mL) were sequentially added to a reaction flask. The mixture was reacted under nitrogen protection at 110 °C for 4 hours. TLC monitoring showed that the reaction was complete. The reaction solution was cooled to room temperature, spin-dried to remove solvent and purified by column chromatography to afford N¹- (6- (3,4-dimethoxyphenyl) pyrazin-2-yl) cyclohexan-1,4-diamine (D1, 25 mg, 45.0%). ¹H NMR (400 MHz, DMSO) δ 8.23 (s, 1H), 7.78 (s, 1H), 7.64 (s, 1H), 7.61 (d, *J* = 8.3 Hz, 1H), 7.05 (d, *J* = 8.4 Hz, 1H), 6.96 (d, *J* = 7.0 Hz, 1H), 3.84 (s, 3H), 3.81 (s, 3H), 3.04 (dt, *J* = 13.9, 7.0 Hz, 2H), 2.05 (s, 2H), 1.87 (s, 2H), 1.26 (s, 4H).

### Step 2: N - (4- ((6- (3,4-dimethoxyphenyl) pyrazin-2-yl) amino) cyclohexyl) cyclobutylformamide (D2)

N¹- (6- (3,4-Dimethoxyphenyl) pyrazin-2-yl) cyclohexan-1,4-diamine (D1, 14 mg, 0.043 mmol) was dissolved in dichloromethane (5 mL) and then cyclobutylcarbonyl chloride (6.1 mg, 0.051 mmol) was added. The mixture was reacted at room temperature for 2 hours. TLC monitoring showed that the reaction was complete. The reaction system was quenched by adding water(10 mL) and fractioned. The aqueous phase was extracted again with dichloromethane (5 mL). The organic phases were combined, washed with saturated salt water, dried over anhydrous sodium sulfate, and purified by column chromatography after rotary drying to obtain N - (4- ((6- (3,4-dimethoxyphenyl) pyrazin-2-yl) amino) cyclohexyl) cyclobutylformamide (D2, 8 mg, 45.3%). MS (ESI) m/z: calcd 411.23 (M+H⁺), found 411.57; ¹H NMR (400 MHz, CDC13) δ 8.26 (s, 1H), 7.80 (s, 1H), 7.60 (d, *J* = 1.8 Hz, 1H), 7.55 (dd, *J* = 8.3, 1.9 Hz, 1H), 6.97 (d, *J* = 8.4 Hz, 1H), 5.20 (d, *J* = 7.6 Hz, 1H), 4.70 (s, 1H), 4.00 (s, 3H), 3.96 (s, 3H), 3.37-3.26 (m, 1H), 3.03-2.94 (m, 1H), 2.34-2.31 (m, 4H), 2.21-2.07 (m, 4H), 1.93-1.86 (m, 1H), 1.35-1.22 (m, 4H), 0.96-0.77 (m, 2H).

### Example 126: Preparation of 3- (2- (6- (3,4-dimethoxyphenyl) pyrazin-2-yl) thiazol-4-yl) -1,1-diethylurea (D4)

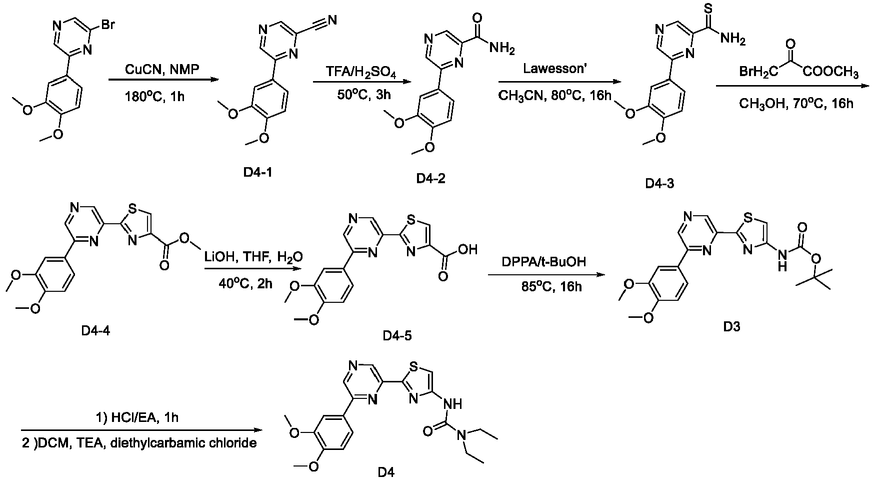

### Step 1: 6- (3,4-Dimethoxyphenyl) pyrazin-2-nitrile (D4-1)

2-bromo-6- (3,4-dimethoxyphenyl) pyrazine (A1-2, 520 mg, 1.76 mmol), cuprous cyanide (189 mg, 2.11mmol), and N-methylpyrrolidone (14 mL) were added successively to a reaction flask and the mixture was reacted at 180°C for 1 hours. TLC monitoring showed that the reaction was complete. The reaction solution was cooled to room temperature, diluted with water (70 mL) and extracted with ethyl acetate (30 mL*3). The organic phases were combined, washed successively with water and saturated salt water, dried over anhydrous sodium sulfate to obtain 6- (3,4-dimethoxyphenyl) pyrazin-2-nitrile (D4-1, 268 mg, 63.1%). ¹H NMR (400 MHz, DMSO) δ 9.58 (s, 1H), 9.09 (s, 1H), 7.82 (d, *J* = 8.4 Hz, 1H), 7.73 (s, 1H), 7.16 (d, *J* = 8.5 Hz, 1H), 3.89 (s, 3H), 3.86 (s, 3H).

### Step 2: 6- (3,4-Dimethoxyphenyl) pyrazin-2-formamide (D4-2)

6- (3,4-Dimethoxyphenyl) pyrazin-2-nitrile (D4-1, 268 mg, 1.11 mmol) was dissolved in trifluoroacetic acid/concentrated sulfuric acid (4/1, 10mL) and reacted at 50°C for 3 hours. TLC monitoring showed that the reaction was complete. The reaction solution was cooled under an ice water bath, and alkalized to pH=8-11 using 2 M sodium hydroxide solution. Solid precipitates were observed; the reaction solution was filtered, and the solid was collected and dried to obtain 6- (3,4-dimethoxyphenyl) pyrazin-2-formamide (D4-2, 201 mg, 69.8%). ¹H NMR (400 MHz, DMSO) δ 9.44 (s, 1H), 9.04 (s, 1H), 8.48 (s, 1H), 8.07-7.87 (m, 3H), 7.12 (d, *J* = 8.4 Hz, 1H), 3.92 (s, 3H), 3.86 (s, 3H).

### Step 3: 6- (3,4-Dimethoxyphenyl) pyrazin-2-thioamide (D4-3)

6- (3,4-Dimethoxyphenyl) pyrazin-2-formamide (D4-2, 201 mg, 0.775 mmol), lawesson's reagent (188 mg, 0.465 mmol), and acetonitrile (10 mL) were added to a reaction flask and the mixture was reacted at 80°C for 16 hours. TLC monitoring showed that the reaction was complete. The reaction solution was cooled to room temperature, spin-dried and purified by column chromatography to afford 6- (3,4-dimethoxyphenyl) pyrazin-2-thioamide (D4-3, 157mg, 73.5%). ¹H NMR (400 MHz, DMSO) δ 10.41 (s, 1H), 10.18 (s, 1H), 9.44 (d, *J* = 6.2 Hz, 2H), 8.09-7.83 (m, 2H), 7.11 (d, *J* = 8.5 Hz, 1H), 3.92 (s, 3H), 3.86 (s, 3H).

### Step 4: methyl 2- (6- (3,4-dimethoxyphenyl) pyrazin-2-yl) thiazol-4-carboxylate (D4-4)

6- (3,4-Dimethoxyphenyl) pyrazin-2-thioamide (D4-3, 142 mg, 0.52 mmol), methyl bromopyruvate (140mg, 0.62 mmol), and methanol (15 mL) were added successively to a reaction flask and the mixture was reacted at 70°C for 16 hours. TLC monitoring showed that the reaction was complete. The reaction solution was cooled to room temperature. Solid precipitates were observed; the reaction solution was filtered, and the solid was collected and dried to obtain methyl 2- (6- (3,4-dimethoxyphenyl) pyrazin-2-yl) thiazol-4-carboxylate (D4-4, 82 mg, 44.1%). ¹H NMR (400 MHz, DMSO) δ 9.42 (s, 1H), 9.20 (s, 1H), 8.80 (s, 1H), 7.87 (dd, *J* = 8.5, 1.9 Hz, 1H), 7.82 (d, *J* = 1.9 Hz, 1H), 7.18 (d, *J* = 8.5 Hz, 1H), 3.92 (s, 6H), 3.87 (s, 3H).

### Step 5: 2- (6- (3,4-dimethoxyphenyl) pyrazin-2-yl) thiazol-4-carboxylic acid (D4-5)

Methyl 2- (6- (3,4-dimethoxyphenyl) pyrazin-2-yl) thiazol-4-carboxylate (D4-4, 80 mg, 0.224 mmol), lithium hydroxide monohydrate (47 mg, 1.12 mmol), and tetrahydrofuran/water (3/1, 4 mL) were added successively to a reaction flask and the mixture was reacted at 40°C for 2 hours. TLC monitoring showed that the reaction was complete. The reaction solution was cooled under an ice water bath, acidified with 2 M hydrochloric acid, and adjusted to pH=3-4. Solid precipitates were observed; the reaction solution was filtered, and the solid was collected and dried to obtain 2- (6-(3,4-dimethoxyphenyl) pyrazin-2-yl) thiazol-4-carboxylic acid (D4-5, 75.0 mg, 97.5%). ¹H NMR (400 MHz, DMSO) δ 9.41 (s, 1H), 9.19 (s, 1H), 8.70 (s, 1H), 7.87 (dd, *J* = 8.4, 1.9 Hz, 1H), 7.82 (d, *J* = 1.9 Hz, 1H), 7.18 (d, *J* = 8.5 Hz, 1H), 3.92 (s, 3H), 3.87 (s, 3H).

### Step 6: Tert-Butyl 2- (6- (3,4-dimethoxyphenyl) pyrazin-2-yl) thiazol-4-carbamate(D3)

2- (6- (3,4-dimethoxyphenyl) pyrazin-2-yl) thiazol-4-carboxylic acid (D4-5, 50.0 mg, 0.146 mmol), triethylamine (44.3 mg, 0.438 mmol), and tert butanol (5 mL) were added successively to a reaction flask and diphenyl azidophosphate (80.1 mg, 0.292 mmol, diluted with 0.5 mL tert butanol) was added dropwis. Upon addition, the reaction system was heated to 85°C and reacted for 16 hours. TLC monitoring showed that the reaction was complete. The reaction solution was directly spin-dried and purified by column chromatography to afford tert-butyl 2- (6- (3,4-dimethoxyphenyl) pyrazin-2-yl) thiazol-4-carbamate (D3, 25 mg, 41.3%). ¹H NMR (400 MHz, CDC13) δ 9.20 (s, 1H), 9.02 (s, 1H), 7.77 (d, *J* = 1.9 Hz, 1H), 7.73-7.61 (m, 2H), 7.47 (s, 1H), 7.04 (d, *J* = 8.4 Hz, 1H), 4.06 (s, 3H), 4.00 (s, 3H), 1.59 (s, 9H).

### Step 7: 3- (2- (6- (3,4-dimethoxyphenyl) pyrazin-2-yl) thiazol-4-yl) -1,1-diethylurea (D4)

Tert-Butyl 2- (6- (3,4-dimethoxyphenyl) pyrazin-2-yl) thiazol-4-carbamate (D3, 18 mg, 0.043 mmol) and hydrogen chloride ethyl acetate solution (3 M, 5 mL) were sequentially added to a reaction flask, and the mixture was reacted at room temperature for 1 hour. Solid precipitates were observed, and the solid was collected by filtration and transferred to a reaction flask. Dichloromethane (2 mL) and triethylamine (27.3 mg, 0.27 mmol) were added. Additionally, N,N-diethylchloroformamide (14.9 mg, 0.11 mmol) was taken and dissolved in dichloromethane (0.5 mL) and added dropwise to the above system. Upon addition, the reaction was carried out at room temperature for 2 hours, and TLC monitoring showed that the reaction was complete. The reaction solution was directly spin-dried and purified by column chromatography to afford 3- (2- (6-(3,4-dimethoxyphenyl) pyrazin-2-yl) thiazol-4-yl) -1,1-diethylurea (D4, 0.8mg, 4.5%). MS (ESI) m/z: calcd 414.15 (M+H⁺), found 414.10; ¹H NMR (400 MHz, CDC13) δ 9.16 (s, 1H), 9.01 (s, 1H), 7.77 (d, *J* = 1.9 Hz, 1H), 7.70 (dd, *J* = 8.3, 1.9 Hz, 1H), 7.64 (s, 1H), 7.44 (s, 1H), 7.04 (d, *J* = 8.4 Hz, 1H), 4.06 (s, 3H), 4.00 (s, 3H), 3.46 (q, *J* = 7.2 Hz, 4H), 1.31 (t, *J* = 7.2 Hz, 6H).

### Example 127: Preparation of 2-methoxy-4- (4- (4-Pentamidothiophen) pyrimidin-2-yl) - N-methyl-N - (1-methylpiperidin-4-yl) benzamide (D5)

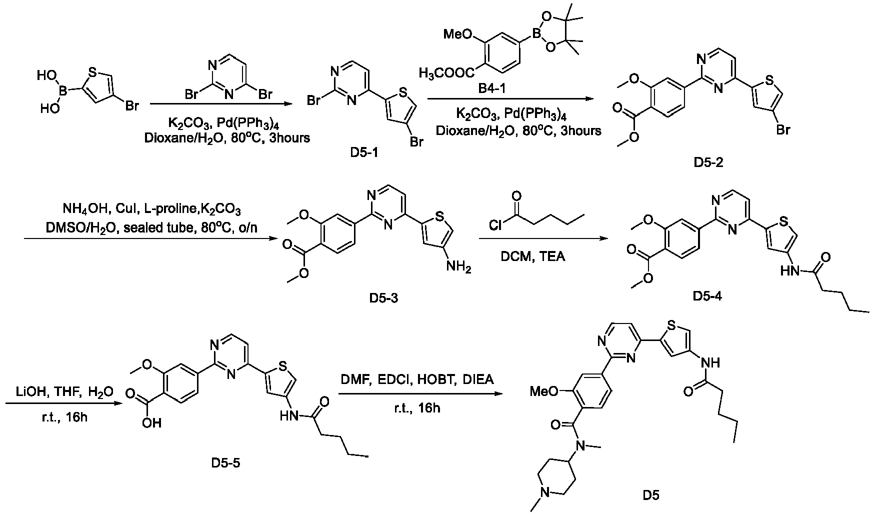

### Step 1: 2-bromo-4- (4-bromothiophen-2-yl) pyrimidine (D5-1)

2,4-dibromopyrimidine (100 mg, 0.42 mmol), (4-bromothiophen-2-yl) boronic acid (87 mg, 0.42 mmol), tetrakis(triphenylphosphine)palladium (24 mg, 0.021 mmol), potassium carbonate (87 mg, 0.63 mmol), 1,4-dioxane (1.6 mL), and water (0.4mL) were added sequentially. The mixture was reacted under nitrogen protection at 80°C for 4 hours, and TLC monitoring showed that the reaction was complete. The reaction solution was directly spin dried and purified by column chromatography to obtain 2-bromo-4-(4-bromothiophen-2-yl) pyrimidine (D5-1, 90 mg, 67.0%). MS (ESI) m/z: calcd 318.85 (M+H⁺), found 318.90

### Step 2: methyl 4- (4- (4-bromothiophen-2-yl) pyrimidin-2-yl) -2-methoxybenzoate (D5-2)

2-bromo-4- (4-bromothiophen-2-yl) pyrimidine (D5-1, 90 mg, 0.28mmol), 3-methoxy-4-methoxycarbonylphenylborate pinacol ester (B4-1, 98 mg, 0.34 mmol), tetrakis(triphenylphosphine)palladium (32 mg, 0.028 mmol), potassium carbonate(58 mg,0.42mmol), 1,4-dioxane (2 mL), and water (0.5mL) were added. The mixture was reacted under nitrogen protection at 80°C for 2 hours, and TLC monitoring showed that the reaction was complete. The reaction solution was directly spin-dried and purified by column chromatography to obtain methyl 4- (4- (4-bromothiophen-2-yl) pyrimidin-2-yl) -2-methoxybenzoate (D5-2, 101 mg, 88.9%). ¹H NMR (400 MHz, DMSO) δ 9.00 (d, *J* = 5.3 Hz, 1H), 8.27 (s, 1H), 8.15 (s, 1H), 8.08 (d, *J* = 8.1 Hz, 1H), 8.04 (d, *J* = 6.7 Hz, 2H), 7.84 (d, *J* = 8.0 Hz, 1H), 3.96 (s, 3H), 3.84 (s, 3H).

### Step 3: methyl 4- (4- (4-aminothiophen-2-yl) pyrimidin-2-yl) -2-methoxybenzoate (D5-3)

Methyl 4- (4- (4-bromothiophen-2-yl) pyrimidin-2-yl) -2-methoxybenzoate (D5-2, 100 mg, 0.247 mmol), cuprous iodide (9.4 mg, 0.049 mmol), L-proline (11.4 mg, 0.10 mmol), dimethyl sulfoxide (2 mL), and ammonia (25% wt, 200 mg, 1.48 mmol) were sequentially added to a sealed tube, and the reaction was sealed and reacted at 80 °C for 16 hours. TLC monitoring showed that the reaction was complete. The reaction solution was cooled to room temperature, added with water (10 mL), and extracted with ethyl acetate (5 mL * 3). The organic phases were combined , washed with saturated ammonium chloride (5 mL * 2), dried over anhydrous sodium sulfate, and spin-dried to obtain methyl 4- (4-(4-aminothiophen-2-yl) pyrimidin-2-yl) -2-methoxybenzoate (D5-3, 105 mg crude, theoretical 0.247 mmol), which was directly used in the next reaction without any purification. MS (ESI) m/z: calcd 342.08 (M+H⁺), found 342.10_{∘}

### Step 4: methyl 2-Methoxy-4- (4- (4-pentamidothiophen-2-yl) pyrimidin-2-yl) benzoate (D5-4)

Methyl 4- (4- (4-aminothiophen-2-yl) pyrimidin-2-yl) -2-methoxybenzoate (D5-3, 105 mg crude, theoretical 0.247 mmol) was dissolved in dichloromethane (5 mL). The reaction system was cooled under an ice water bath, and added with triethylamine (31 mg, 0.31 mmol). Additionally, pentanoyl chloride (56 mg, 0.46 mmol) was taken and dissolved in dichloromethane (1 mL) and the resulting mixture was slowly dropped into the reaction system. Upon addition, the reaction was warmed to room temperature and reacted for 0.5 hour. TLC monitoring showed that the reaction was complete. The reaction solution was directly spin dried and purified by column chromatography to obtain methyl 2-methoxy-4-(4- (4-pentamidothiophen-2-yl) pyrimidin-2-yl) benzoate (D5-4, 67 mg, 63.8%). MS (ESI) m/z: calcd 426.14(M+H⁺), found 426.10.

### Step 5: 2-Methoxy-4- (4- (4-pentamidothiophen-2-yl) pyrimidin-2-yl)benzoic acid (D5-5)

Methyl 2-methoxy-4- (4- (4-pentamidothiophen-2-yl) pyrimidin-2-yl) benzoate (D5-4, 67 mg, 0.158 mmol), lithium hydroxide monohydrate (33 mg, 0.786 mmol), tetrahydrofuran (4 mL) and water (1 mL) were added successively to a reaction flask and the mixture was reacted at room temperature for 16 hours. TLC monitoring showed that the reaction was complete. Tetrahydrofuran was removed by rotary evaporation, the residue was diluted with water (5 mL), acidified to pH=3-4 using saturated citric acid and extracted with ethyl acetate (10 mL*3). The organic phases were combined, washed successively with water and saturated salt water, dried over anhydrous sodium sulfate, spin-dried and purified by column chromatography to obtain 2-methoxy-4- (4-(4-pentamidothiophen-2-yl) pyrimidin-2-yl)benzoic acid (D5-5, 56 mg, 86.1%). MS (ESI) m/z: calcd 412.13(M+H⁺), found 412.10.

### Step 6: 2-Methoxy-4- (4- (4-pentamidothiophen) pyrimidin-2-yl) - N-methyl-N - (1-methylpiperidin-4-yl) benzamide (D5)

2-Methoxy-4- (4- (4-pentamidothiophen-2-yl) pyrimidin-2-yl)benzoic acid (D5-5, 10 mg, 0.024 mmol), 1-methyl-4- (methylamino) piperidine (4.7 mg, 0.036 mmol), 1-(3-dimethylaminopropyl)-3-ethylcarbodiimide hydrochloride (9.3 mg, 0.048 mmol), 1-hydroxybenzotriazole (6.6 mg, 0.048 mmol), N, N-diisopropylethylamine (9.4 mg, 0.073 mmol), and N,N-dimethylformamide (1 mL) were sequentially added to a reaction flask and the mixture was reacted at room temperature for 5 hours. TLC monitoring showed that the reaction was complete. The reaction solution was diluted with water (10 mL) and extracted with ethyl acetate (5 mL*3). The organic phase phases were combined, washed successively with water and saturated salt water, dried over anhydrous sodium sulfate, spin-dried and purified by column chromatography to obtain 2-methoxy-4- (4-(4-pentamidothiophen) pyrimidin-2-yl) - N-methyl-N - (1-methylpiperidin-4-yl) benzamide (D5, 5 mg, 39.4%). MS (ESI) m/z: calcd 522.25 (M+H⁺), found 522.40; ¹H NMR (400 MHz, DMSO) δ 10.42 (s, 1H), 8.92 (dd, *J* = 5.3, 2.2 Hz, 1H), 8.16 - 8.06 (m, 2H), 8.01 (dd, *J* = 5.6, 1.4 Hz, 1H), 7.84 (dd, *J* = 5.3, 2.6 Hz, 1H), 7.78 (dd, *J* = 3.2, 1.3 Hz, 1H), 7.38 (dd, *J* = 10.3, 8.2 Hz, 1H), 3.93 (s, 3H), 3.52 (d, *J* = 8.0 Hz, 2H), 3.19 (d, *J* = 12.6 Hz, 2H), 2.99 (s, 1H), 2.92-2.79 (m, 3H), 2.66 (d, *J* = 7.1 Hz, 3H), 2.32 (t, *J* = 7.4 Hz, 2H), 2.08-1.77 (m, 4H), 1.60 (dt, *J* = 15.0, 7.5 Hz, 2H), 1.34 (dq, *J* = 14.5, 7.3 Hz, 2H), 0.91 (t, *J* = 7.3 Hz, 3H).

### Example 128: Preparation of N-(5- (6- (4- (methylsulfonyl) piperazin-1-yl) pyrazin 2-yl) thiophen-3-yl) pentanamide (D6)

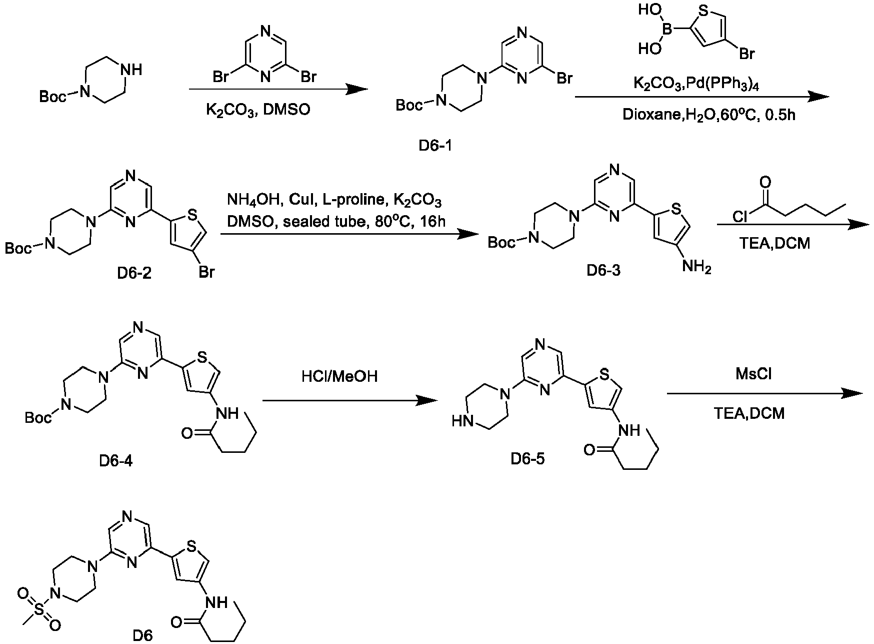

### Step 1: Tert butyl 4- (6-bromopyrazin -2-yl) piperazin -1-carboxylate (D6-1)

1-tert butyl carboxylate piperazine (300 mg, 1.61 mmol), 2,6-dibromopyrazine (460 mg, 1.93 mmol), potassium carbonate (445 mg, 3.22 mmol), and dimethyl sulfoxide (9 mL) were added, and the mixture was reacted under nitrogen protection for 5 hours at 120 °C. TLC monitoring showed that the reaction was complete. The reaction solution was cooled to room temperature, diluted with 50 mL of water and extracted with ethyl acetate (20 mL*3). The organic phases were combined, washed with saturated salt water (20 mL*1), dried over anhydrous sodium sulfate and spin-dried to obtain Tert butyl 4-(6-bromopyrazin -2-yl) piperazin -1-carboxylate (D6-1, 250 mg, 45.2%). MS (ESI) m/z: calcd 343.07 (M+H), found 343.10.

### Step 2: Tert butyl 4- (6- (4-bromothiophen-2-yl) pyrazin-2-yl) piperazin-1-carboxylate (D6-2)

Tert butyl 4- (6-bromopyrazin-2-yl) piperazin-1-carboxylate (250 mg, 0.73 mmol), (4-bromothiophen-2-yl) boronic acid (200 mg, 0.97 mmol), tetrakis(triphenylphosphine)palladium (42 mg, 0.036 mmol), potassium carbonate (242 mg,1.75 mmol) and 1,4-dioxane/water (4:1, 15 mL) were successively added to a reaction flask, and the mixture was reacted under nitrogen protection at 60°C for 0.5 hour. TLC monitoring showed that the reaction was complete. The reaction solution was added with water (25 mL) and extracted with ethyl acetate (20 mL*3). The organic phases were combined, washed with saturated saline, dried over anhydrous sodium sulfate, and spin-dried. The residue was purified by column chromatography to afford tert butyl 4- (6-(4-bromothiophen-2-yl) pyrazin-2-yl) piperazin-1-carboxylate (D6-2, 105 mg, 33.8%). 1H NMR (400 MHz, DMSO) δ 8.46 (s, 1H), 8.26 (s, 1H), 7.91 (d, *J* = 1.3 Hz, 1H), 7.81 (d, *J* = 1.3 Hz, 1H), 3.64-3.60 (m, 4H), 3.48-3.45 (m, 4H), 1.44 (s, 9H).

### Step 3: Tert butyl 4- (6- (4-aminothiophen-2-yl) pyrazin-2-yl) piperazin-1-carboxylate (D6-3)

Tert butyl 4- (6- (4-bromothiophen-2-yl) pyrazin-2-yl) piperazin-1-carboxylate (105 mg, 0.25 mmol), cuprous iodide (10mg, 0.052 mmol), L-proline (23 mg, 0.2 mmol), dimethyl sulfoxide (2 mL), and ammonia (25% wt, 59mg, 1.69 mmol) were sequentially added to a sealed tube, and the reaction system was sealed and reacted at 80 °C for 16 hours. TLC monitoring showed that the reaction was complete. The reaction solution was cooled to room temperature, added with water (10 mL), and extracted with ethyl acetate (5 mL * 3). The organic phases were combined , washed with saturated ammonium chloride (5 mL * 2), dried over anhydrous sodium sulfate, and spin-dried to obtain tert butyl 4- (6-(4-aminothiophen-2-yl) pyrazin-2-yl) piperazin-1-carboxylate (D6-3, 90 mg, theoretical 0.25 mmol), which was directly used in the next reaction without any purification. MS (ESI) m/z: calcd 362.16 (M+H), found 362.10.

### Step 4: Tert butyl 4- (6- (4-pentamidothiophen-2-yl) pyrazin-2-yl) piperazin-1-carboxylate (D6-4)

Tert butyl 4- (6- (4-aminothiophen-2-yl) pyrazin-2-yl) piperazin-1-carboxylate (D6-3, 90 mg, theoretical 0.25 mmol) was dissolved in dichloromethane (5 mL). The reaction system was cooled under an ice water bath, and added with triethylamine (76 mg, 0.75 mmol). Additionally, pentanoyl chloride (60 mg, 0.498 mmol) was taken and dissolved in dichloromethane (0.5 mL) and the resulting mixture was slowly dropped into the above reaction system. The reaction system was reacted under ice water bath for 1 hour. TLC monitoring monitoring that the reaction was complete. The reaction solution was purified by column chromatography to afford tert butyl 4- (6- (4-pentamidothiophen-2-yl) pyrazin-2-yl) piperazin-1-carboxylate (D6-4, 65 mg, 58.3%). MS (ESI) m/z: calcd 446.21 (M+H⁺), found 446.30_{∘}

### Step 5: N- (5- (6- (piperazin-1-yl) pyrazin-2-yl) thiophen-3-yl) pentanamide (D6-5)

Tert butyl 4- (6- (4-pentamidothiophen-2-yl) pyrazin-2-yl) piperazin-1-carboxylate (65 mg, 0.146 mmol) was dissolved in methanol (1mL). To the system then was added with hydrogen chloride methanol solution (3 M, 1 mL), and the reaction was carried out at room temperature for 16 hours. TLC monitoring showed that the reaction was complete. The reaction solution was directly spin dried without any treatment to afford N- (5- (6-(piperazin-1-yl) pyrazin-2-yl) thiophen-3-yl) pentanamide (D6-5, theoretical 0.146 mmol). MS (ESI) m/z: calcd 346.21 (M+H⁺), found 346.30

### Step 6: N- (5- (6- (4- (methylsulfonyl) piperazin-1-yl) pyrazin-2-yl) thiophen-3-yl) pentanamide (D6)

N- (5- (6- (piperazin-1-yl) pyrazin-2-yl) thiophen-3-yl) pentanamide (D6-5, theoretical 0.146 mmol) was dissolved in dichloromethane (5 mL). The reaction system was cooled under an ice water bath, and added with triethylamine (73 mg, 0.72 mmol). Additionally, methanesulfonyl chloride (25 mg, 0.22mmol) was taken and dissolved in dichloromethane (0.5 mL) and the resulting mixture was slowly dropped into the above system. Upon addition, the reaction was warmed to room temperature and reacted for 0.5 hour. TLC monitoring monitoring that the reaction was complete. The reaction solution was directly spin-dried and purified by column chromatography to afford N- (5- (6- (4-(methylsulfonyl) piperazin-1-yl) pyrazin-2-yl) thiophen-3-yl) pentanamide (D6, 21 mg, 34.0%). MS (ESI) m/z: calcd 423.55 (M+H⁺), found 424.30; 1H NMR (400 MHz, DMSO) δ 10.31 (s, 1H), 8.31 (s, 1H), 8.27 (s, 1H), 7.69 (d, *J* = 1.4 Hz, 1H), 7.62 (s, 1H), 3.77 (s, 4H), 3.26 (d, *J* = 4.9 Hz, 4H), 2.92 (s, 3H), 2.31 - 2.27 (m, 2H), 1.58 (d, *J* = 7.4 Hz, 2H), 1.35-1.30 (m, 2H), 0.91 (t, *J* = 7.3 Hz, 3H).

### Example 129: Preparation of N- (5- (6- (1- (methylsulfonyl) piperidin-4-yl) pyrazin-2-yl) thiophen-3-yl) pentanamide (D7)

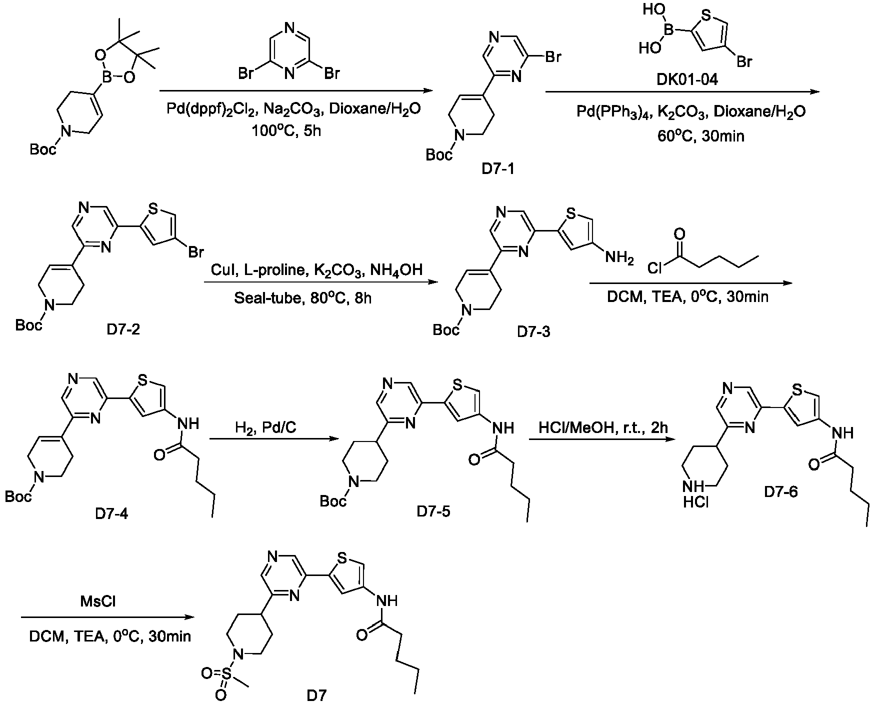

### Step 1: Tert butyl 4- (6-bromopyrazin-2-yl) -3,6-dihydropyridin-1(2H) -carboxylate (D7-1)

N-Boc-1,2,5,6-tetrahydropyridin-4-boronic acid pinacol ester (500 mg, 1.62 mmol), 2,6-dibromopyrazine (385 mg, 1.62 mmol), sodium carbonate (429 mg, 4.05 mmol), [1,1'-bis(diphenylphosphino)ferrocene]dichloropalladium(II) (60 mg, 0.082 mmol), and 1,4-dioxane/water (5:1, 12 mL) were sequentially added to a reaction flask. The reaction was carried out at 100 °C for 5 hours under nitrogen protection. TLC monitoring showed that the reaction was complete. The reaction solution was directly spin dried and purified by column chromatography to obtain tert butyl 4- (6-bromopyrazin-2-yl) -3,6-dihydropyridin-1(2H) -carboxylate (D7-1, 310 mg, 56.3%). ¹H NMR (400 MHz, DMSO) δ 8.88 (s, 1H), 8.72 (s, 1H), 6.88 (s, 1H), 4.10 (d, *J* = 2.3 Hz, 2H), 3.60-3.49 (m, 2H), 2.54 (d, *J* = 1.7 Hz, 2H), 1.43 (s, 9H).

### Step 2: Tert butyl 4- (6- (4-bromothiophen-2-yl) pyrazin-2-yl) -3,6-dihydropyridin-1(2H)-carboxylate (D7-2)

Tert butyl 4- (6-bromopyrazin-2-yl) -3,6-dihydropyridin-1(2H) -carboxylate (D7-1, 220 mg, 0.647 mmol), (4-bromothiophen-2-yl) boronic acid (134 mg, 0.647 mmol), tetrakis(triphenylphosphine)palladium (75 mg, 0.065 mmol), potassium carbonate (214 mg,1.75mmol) and 1,4-dioxane/water (5:1, 6 mL) were successively added to a reaction flask, and the mixture was reacted under nitrogen protection at 60°C for 0.5 hour. TLC monitoring showed that the reaction was complete. The reaction solution was added with water (25 mL) and extracted with ethyl acetate (20 mL*3). The organic phase phases were combined, washed with saturated salt water, dried over anhydrous sodium sulfate, spin-dried and purified by column chromatography to obtain tert butyl 4- (6-(4-bromothiophen-2-yl) pyrazin-2-yl) -3,6-dihydropyridin-1(2H)-carboxylate (D7-2, 158 mg, 57.8%). ¹H NMR (400 MHz, DMSO) δ 9.12 (s, 1H), 8.78 (s, 1H), 8.07 (d, *J* = 1.4 Hz, 1H), 7.89 (d, *J* = 1.4 Hz, 1H), 6.90 (s, 1H), 4.12 (s, 2H), 3.66-3.52 (m, 3H), 2.62 (s, 2H), 1.44 (s, 9H).

### Step 3: Tert butyl 4- (6- (4-aminothiophen-2-yl) pyrazin-2-yl) -3,6-dihydropyridin-1(2H)-carboxylate (D7-3)

Tert butyl 4- (6- (4-bromothiophen-2-yl) pyrazin-2-yl) -3,6-dihydropyridin-1(2H)-carboxylate (D7-2, 158 mg, 0.375mmol), cuprous iodide (14 mg, 0.074 mmol), L-proline (17 mg, 0.148 mmol), dimethyl sulfoxide (2 mL), and ammonia (25% wt, 315 mg, 2.25 mmol) were sequentially added to a sealed tube, and the reaction system was sealed and reacted at 80 °C for 16 hours. TLC monitoring showed that the reaction was complete. The reaction solution was cooled to room temperature, added with water (10 mL), and extracted with ethyl acetate (5 mL * 3). The organic phases were combined , washed with saturated ammonium chloride (5 mL * 2), dried over anhydrous sodium sulfate, and spin-dried to obtain tert butyl 4- (6- (4-aminothiophen-2-yl) pyrazin-2-yl) -3,6-dihydropyridin-1(2H)-carboxylate (D7-3, theoretical 0.375 mmol), which was directly used in the next reaction without any purification. MS (ESI) m/z: calcd 359.15 (M+H⁺), found 359.10_{∘}

### Step 4: Tert butyl 4- (6- (4-pentamidothiophen-2-yl) pyrazin-2-yl) -3,6-dihydropyridin-1(2H)-carboxylate (D7-4)

Tert butyl 4- (6- (4-aminothiophen-2-yl) pyrazin-2-yl) -3,6-dihydropyridin-1(2H)-carboxylate (D7-3, theoretical 0.375 mmol) was dissolved in dichloromethane (5 mL). The reaction system was cooled under an ice water bath, and added with triethylamine (73 mg, 0.72 mmol). Additionally, pentanoyl chloride (134mg, 1.11 mmol) was taken and dissolved in dichloromethane (0.5 mL) and the resulting mixture was slowly dropped into the above system. Upon addition, the reaction system was warmed to room temperature and reacted for 0.5 hour. TLC monitoring showed that the reaction was complete. The reaction solution was directly spin-dried and purified by column chromatography to afford tert butyl 4- (6- (4-pentamidothiophen-2-yl) pyrazin-2-yl) -3,6-dihydropyridin-1(2H)-carboxylate (D7-4, 35 mg, 21.1%). MS (ESI) m/z: calcd 443.20 (M+H⁺), found 443.10

### Step 5: Tert butyl 4- (6- (4-pentamidothiophen-2-yl) pyrazin-2-yl)piperidin-1-carbonate (D7-5)

Tert butyl 4- (6- (4-pentamidothiophen-2-yl) pyrazin-2-yl) -3,6-dihydropyridin-1(2H)-carboxylate (D7-4, 35 mg, 0.079 mmol) was dissolved in methanol (2 mL), the solution was added palladium carbon (35 mg), and the reaction was carried out at room temperature under hydrogen atmosphere (1 atm) for 16 hours. TLC monitoring showed that the reaction was complete. The reaction solution was filtered to remove palladium carbon and spin-dried to afford tert butyl 4- (6-(4-pentamidothiophen-2-yl) pyrazin-2-yl)piperidin-1-carbonate (D7-5, 35 mg, 100%). MS (ESI) m/z: calcd 445.22 (M+H⁺), found 445.20_{∘}

### Step 6: N - (5- (6- (piperidin-4-yl) pyrazin-2-yl) thiophen-3-yl) pentanamide hydrochloride (D7-6)

Tert butyl 4- (6- (4-pentamidothiophen-2-yl) pyrazin-2-yl)piperidin-1-carbonate (D7-5, 35 mg, 0.079 mmol) was dissolved in methanol (4mL), the solution was added with hydrogen chloride methanol solution (3 M, 4 mL), and the mixture was reacted at room temperature for 1 hour. LCMS detection showed that the reaction was complete. The reaction solution was spin-dried and used directly in the next reaction to afford N - (5- (6-(piperidin-4-yl) pyrazin-2-yl) thiophen-3-yl) pentanamide hydrochloride (D7-6, 30 mg, 100%). MS (ESI) m/z: calcd 345.17 (M+H⁺), found 345.10_{∘}

### Step 7: N- (5- (6- (1- (methylsulfonyl) pyridin -4-yl) pyrazin-2yl) thiophen-3-yl) pentanamide (D7)

N - (5- (6- (piperidin-4-yl) pyrazin-2-yl) thiophen-3-yl) pentanamide hydrochloride (D7-6, 30 mg, 0.079 mmol) was dissolved in dichloromethane (5 mL). The solution was added with triethylamine (24 mg, 0.238 mmol) and cooled under an ice water bath. Additionally, methylsulfonyl chloride (13.6mg, 0.119 mmol) was taken and dissolved in dichloromethane (2 mL) and the resulting mixture was slowly dropped into the above system. Upon addition, the reaction system was warmed to room temperature and reacted for 0.5 hour. LCMS detection showed that the reaction was complete. The reaction solution was directly spin-dried and purified by column chromatography to afford N- (5-(6- (1- (methylsulfonyl) pyridin -4-yl) pyrazin-2yl) thiophen-3-yl) pentanamide (D7, 1.6 mg, 4.8%). MS (ESI) m/z: calcd 423.14 (M+H⁺), found 423.10; ¹H NMR (400 MHz, DMSO) δ 10.35 (s, 1H), 8.94 (s, 1H), 8.51 (d, *J* = 3.7 Hz, 1H), 7.81 (d, *J* = 1.4 Hz, 1H), 7.66 (d, *J* = 1.3 Hz, 1H), 3.70 (d, *J* = 11.9 Hz, 2H), 3.01-2.83 (m, 5H), 2.34-2.28 (m, 2H), 2.08-2.01 (m, 2H), 1.88-1.77 (m, 2H), 1.63-1.55 (m, 2H), 1.38-1.30 (m, 2H), 1.24 (s, 1H), 0.91 (t, *J* = 7.3 Hz, 3H).

### Example 130 was prepared using experimental steps similar to those in Examples 125-129 above, and compounds D1-D9 were summarized in Table 5.

**Table 5**

| Exam ple | Compo und No. | Structure | MS(calcd) [M+H]⁺/ MS (found) | Name and characterization |
|---|---|---|---|---|
| 125 | D1 | 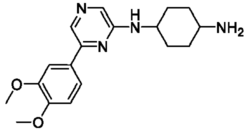 | 329.19/N/A (HNMR) | N¹-(6-(3,4-dimethoxyphenyl)p yrazin-2-yl)cyclohexane-1,4-di amine |
| | | | | ¹H NMR (400 MHz, DMSO) δ 8.23 (s, 1H), 7.78 (s, 1H), 7.64 (s, 1H), 7.61 (d, *J* = 8.3 Hz, 1H), 7.05 (d, *J* = 8.4 Hz, 1H), 6.96 (d, *J* = 7.0 Hz, 1H), 3.84 (s, 3H), 3.81 (s, 3H), 3.04 (dt, *J* = 13.9, 7.0 Hz, 2H), 2.05 (s, 2H), 1.87 (s, 2H), 1.26 (s, 4H). |
| 125 | D2 | 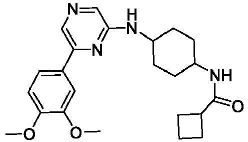 | 411.23/411.5 7 | N-(4-((6-(3,4-dimethoxyphenyl )pyrazin-2-yl)amino)cyclohexy l)cyclobutanecarboxamide |
| 126 | D3 | 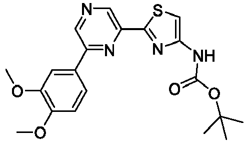 | 415.14/No MS signals (HNMR) | tert-butyl (2-(6-(3,4-dimethoxyphenyl)py razin-2-yl)thiazol-4-yl)carbama te |
| | | | | ¹H NMR (400 MHz, CDC13) δ 9.20 (s, 1H), 9.02 (s, 1H), 7.77 (d, *J* = 1.9 Hz, 1H), 7.73-7.61 (m, 2H), 7.47 (s, 1H), 7.04 (d, *J* = 8.4 Hz, 1H), 4.06 (s, 3H), 4.00 (s, 3H), 1.59 (s, 9H). |
| 126 | D4 | 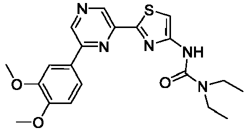 | 414.15/ 414.10 | 3-(2-(6-(3,4-dimethoxyphenyl) pyrazin-2-yl)thiazol-4-yl)-1,1-diethylurea |
| 127 | D5 | 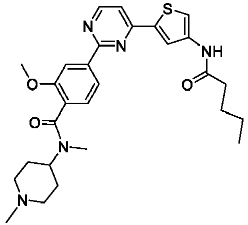 | 522.25/522.4 0 | 2-methoxy-N-methyl-N-(1-met hylpiperidin-4-yl)-4-(4-(4-pent anamidothiophen-2-yl)pyrimidi n-2-yl)benzamide |
| 128 | D6 | 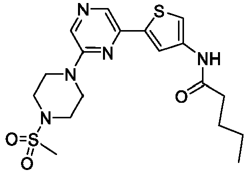 | 424.14/424.3 0 | N-(5-(6-(4-(methylsulfonyl)pip erazin-1-yl)pyrazin-2-yl)thioph en-3-yl)pentanamide |
| 129 | D7 | 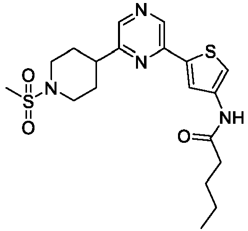 | 423.14/423.1 0 | N-(5-(6-(1-(methylsulfonyl)pip eridin-4-yl)pyrazin-2-yl)thioph en-3-yl)pentanamide |
| 130 | D8 | 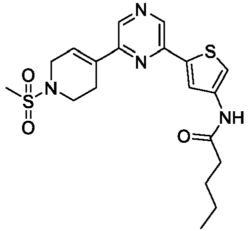 | 421.13/421.1 0 | N-(5-(6-(1-(methylsulfonyl)-1, 2,3,6-tetrahydropyridin-4-yl)py razin-2-yl)thiophen-3-yl)penta namide |

### Examples 131-152 (compounds B79-100) were prepared using experimental steps similar to those in Example 46 above, and compounds B79-100 were summarized in Table 6.

**Table 6**

| Exam ple | Compo und No. | Structure | MS(calcd) [M+H]⁺/ MS (found) | Name and characterization |
|---|---|---|---|---|
| 131 | B79 | 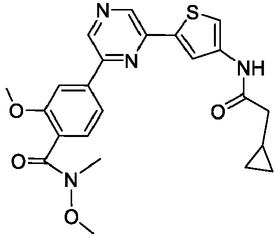 | 453.15/45 3.30 | 4-(6-(4-(2-cyclopropylacetamido )thiophen-2-yl)pyrazin-2-yl)-N,2 -dimethoxy-N-methylbenzamide ¹H NMR (400 MHz, DMSO) δ 10.34 (s, 1H), 9.23 (s, 1H), 9.10 (s, 1H), 7.91 (d, *J* = 1.3 Hz, 1H), 7.83 (d, *J* = 8.9 Hz, 2H), 7.74 (d, *J* = 1.3 Hz, 1H), 7.44 (d, *J* = 7.7 Hz, 1H), 3.94 (s, 3H), 3.49 (s, 3H), 3.26 (s, 3H), 2.22 (d, *J* = 7.0 Hz, 2H), 1.13-1.03 (m, 1H), 0.56-0.44 (m, 2H), 0.30-0.17 (m, 2H). |
| 132 | B80 | 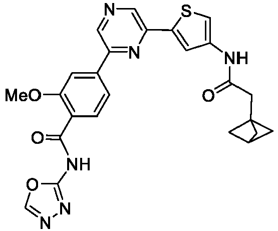 | 503.14/50 3.30 | 4-(6-(4-(2-(bicyclo[1.1.1]pentan-1-yl)acetamido)thiophen-2-yl)py razin-2-yl)-2-methoxy-N-(1,3,4-oxadiazol-2-yl)benzamide |
| | | | | ¹H NMR (400 MHz, DMSO) δ 12.05 (s, 1H), 10.34 (s, 1H), 9.25 (s, 1H), 9.12 (s, 1H), 8.11 (d, *J* = 0.9 Hz, 1H), 7.90 (d, *J* = 1.4 Hz, 1H), 7.88-7.83 (m, 2H), 7.73 (d, *J* = 1.4 Hz, 1H), 7.58 (d, *J* = 8.3 Hz, 1H), 3.91 (s, 3H), 1.78 (s, 4H), 1.24 (s, 2H), 1.19-1.17 (m, 1H). |
| 133 | B81 | 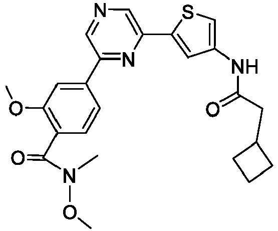 | 467.17/46 7.30 | 4-(6-(4-(2-cyclobutylacetamido)t hiophen-2-yl)pyrazin-2-yl)-N,2-dimethoxy-N-methylbenzamide |
| | | | | ¹H NMR (400 MHz, DMSO) δ 10.37 (s, 1H), 9.23 (s, 1H), 9.09 (s, 1H), 7.89 (d, *J* = 1.4 Hz, 1H), 7.87-7.79 (m, 2H), 7.71 (d, *J* = 1.3 Hz, 1H), 7.44 (d, *J* = 7.7 Hz, 1H), 3.94 (s, 3H), 3.50 (s, 3H), 3.25 (s, 3H), 2.43 (d, *J* = 7.6 Hz, 2H), 2.08 (dt, *J* = 19.4, 7.5 Hz, 2H), 1.94-1.70 (m, 4H), 1.27-1.22 (m, 1H). |
| 134 | B82 | 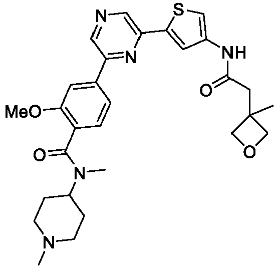 | 550.24/55 0.00 | 2-methoxy-N-methyl-4-(6-(4-(2-(3-methyloxetan-3-yl)acetamido )thiophen-2-yl)pyrazin-2-yl)-N-( 1-methylpiperidin-4-yl)benzami de |
| 135 | B83 | 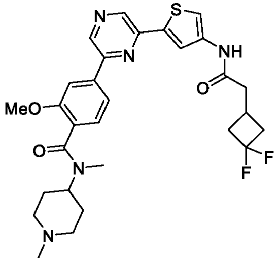 | 570.23/57 0.40 | 4-(6-(4-(2-(3,3-difluorocyclobut yl)acetamido)thiophen-2-yl)pyra zin-2-yl)-2-methoxy-N-methyl-N-(1-methylpiperidin-4-yl)benza mide |
| 136 | B84 | 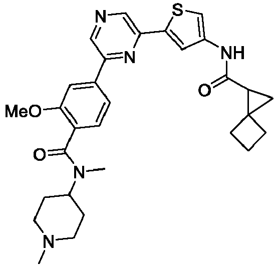 | 546.25/54 6.30 | N-(5-(6-(3-methoxy-4-(methyl(1 -methylpiperidin-4-yl)carbamoyl )phenyl)pyrazin-2-yl)thiophen-3 -yl)spiro[2.3]hexane-1-carboxa mide |
| | | | | ¹H NMR (400 MHz, DMSO) δ 10.61 (s, 1H), 9.24 (d, *J* = 10.0 Hz, 1H), 9.10 (d, *J* = 2.3 Hz, 1H), 7.92 (dd, *J* = 3.8, 1.3 Hz, 1H), 7.85 (t, *J* = 7.4 Hz, 2H), 7.69 (s, 1H), 7.35 (t, *J* = 7.5 Hz, 1H), 4.48-4.35 (m, 1H), 3.93 (d, *J* = 2.5 Hz, 3H), 3.11 (d, *J* = 97.3 Hz, 2H), 2.85 (d, *J* = 29.2 Hz, 3H), 2.61 (d, *J* = 46.9 Hz, 3H), 2.33 (s, 2H), 2.05-1.95 (m, 2H), 1.83 (d, *J* = 11.5 Hz, 3H), 1.73 (dd, *J* = 8.1, 5.4 Hz, 1H), 1.63 (s, 2H), 1.47 (s, 1H), 1.27 (dd, *J* = 14.8, 11.1 Hz, 2H), 1.10 (t, *J* = 4.7 Hz, 1H), 1.00 (dd, *J* = 8.2, 4.2 Hz, 1H). |
| 137 | B85 | 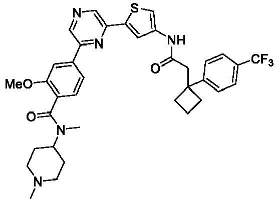 | 678.26/67 8.30 | 2-methoxy-N-methyl-N-(1-meth ylpiperidin-4-yl)-4-(6-(4-(2-(1-( 4-(trifluoromethyl)phenyl)cyclo butyl)acetamido)thiophen-2-yl)p yrazin-2-yl)benzamide |
| | | | | ¹H NMR (400 MHz, DMSO) δ 10.23 (s, 1H), 9.22 (d, *J* = 10.2 Hz, 1H), 9.05 (d, *J* = 2.1 Hz, 1H), 7.83 (t, *J* = 7.1 Hz, 2H), 7.76 (s, 1H), 7.71-7.58 (m, 3H), 7.44 (d, *J* = 8.1 Hz, 2H), 7.34 (t, *J* = 7.4 Hz, 1H), 4.43-4.28 (m, 1H), 3.92 (d, *J* = 2.8 Hz, 3H), 3.16 (s, 1H), 2.87 (d, *J* = 7.9 Hz, 4H), 2.81-2.61 (m, 3H), 2.44-2.30 (m, 3H), 2.22 (s, 1H), 2.07 (s, 2H), 1.82 (d, *J* = 10.8 Hz, 4H), 1.60 (s, 2H), 1.46-1.34 (m, 1H), 1.25 (d, *J* = 6.0 Hz, 1H). |
| 138 | B86 | 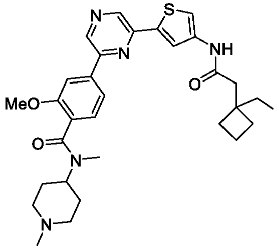 | 562.28/N/ A | 4-(6-(4-(2-(1-ethylcyclobutyl)ac etamido)thiophen-2-yl)pyrazin-2 -yl)-2-methoxy-N-methyl-N-(1-methylpiperidin-4-yl)benzamide |
| | | | | ¹H NMR (400 MHz, DMSO) δ 10.89 (s, 1H), 9.26 (d, *J* = 10.3 Hz, 1H), 9.16 (d, *J* = 2.2 Hz, 1H), 7.95 (dd, *J* = 3.5, 1.3 Hz, 1H), 7.84 (dd, *J* = 14.9, 8.4 Hz, 3H), 7.42-7.30 (m, 1H), 4.49-4.31 (m, 1H), 3.92 (t, *J* = 9.5 Hz, 3H), 3.23-3.11 (m, 1H), 2.96 (s, 1H), 2.88 (s, 3H), 2.79-2.63 (m, 3H), 2.31 (d, *J* = 16.7 Hz, 2H), 2.25-2.20 (m, 1H), 2.12 (s, 2H), 1.92-1.74 (m, 6H), 1.67 (dd, *J* = 14.6, 7.1 Hz, 2H), 1.45 (s, 1H), 1.28-1.21 (m, 2H), 0.97 (t, *J* = 7.4 Hz, 3H). |
| 139 | B87 | 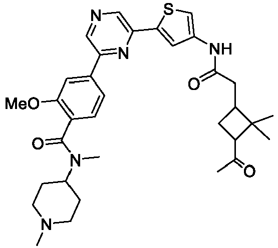 | 604.29/60 4.30 | 4-(6-(4-(2-(3-acetyl-2,2-dimethy lcyclobutyl)acetamido)thiophen-2-yl)pyrazin-2-yl)-2-methoxy-N-methyl-N-(1-methylpiperidin-4-yl)benzamide |
| | | | | ¹H NMR (400 MHz, DMSO) δ 10.43 (d, *J* = 8.8 Hz, 1H), 9.24 (d, *J* = 9.6 Hz, 1H), 9.10 (s, 1H), 7.85 (dd, *J* = 14.7, 7.0 Hz, 3H), 7.71 (s, 1H), 7.36 (t, *J* = 7.6 Hz, 1H), 4.51-4.35 (m, 1H), 3.93 (d, *J* = 2.0 Hz, 3H), 3.04 (d, *J* = 9.0 Hz, 2H), 3.00-2.62 (m, 6H), 2.45-2.10 (m, 6H), 2.08-1.96 (m, 3H), 1.96-1.73 (m, 4H), 1.36-1.19 (m, 5H), 0.90 (d, *J* = 51.9 Hz, 3H). |
| 140 | B88 | 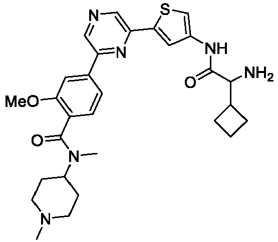 | 549.26/54 9.30 | 4-(6-(4-(2-amino-2-cyclobutylac etamido)thiophen-2-yl)pyrazin-2 -yl)-2-methoxy-N-methyl-N-(1-methylpiperidin-4-yl)benzamide |
| | | | | ¹H NMR (400 MHz, DMSO) δ 11.38 (d, *J* = 15.9 Hz, 1H), 9.24 (d, *J* = 9.2 Hz, 1H), 9.13 (d, *J* = 2.6 Hz, 1H), 8.11-7.96 (m, 1H), 7.85 (dd, *J* = 12.6, 4.4 Hz, 3H), 7.38 (dd, *J* = 10.1, 8.0 Hz, 1H), 4.74-4.56 (m, 1H), 3.99-3.95 (m, 1H), 3.94 (s, 3H), 3.34 (dd, *J* = 32.7, 12.0 Hz, 1H), 3.23-3.10 (m, 1H), 2.97 (t, *J* = 12.5 Hz, 1H), 2.88 (s, 1H), 2.81 (d, *J* = 12.6 Hz, 1H), 2.75 (d, *J* = 8.4 Hz, 2H), 2.64 (d, *J* = 23.1 Hz, 3H), 2.28-2.08 (m, 3H), 2.08-1.95 (m, 2H), 1.95-1.72 (m, 5H), 1.72- 1.61 (m, 1H). |
| 141 | B89 | 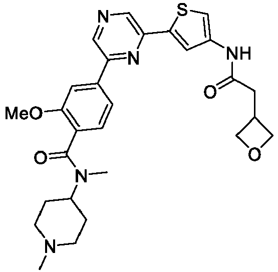 | 536.23/53 6.30 | 2-methoxy-N-methyl-N-(1-meth ylpiperidin-4-yl)-4-(6-(4-(2-(oxe tan-3-yl)acetamido)thiophen-2-y l)pyrazin-2-yl)benzamide |
| | | | | ¹H NMR (400 MHz, DMSO) δ 9.22 (dd, *J* = 11.2, 6.6 Hz, 2H), 8.35 (dd, *J* = 3.6, 1.2 Hz, 1H), 7.86 (dd, *J* = 13.3, 5.2 Hz, 2H), 7.74 (s, 1H), 7.38 (dd, *J* = 11.6, 8.0 Hz, 1H), 4.64 (t, *J* = 12.0 Hz, 1H), 3.98 (d, *J* = 9.6 Hz, 1H), 3.95 (s, 3H), 3.71 (dd, *J* = 9.7, 4.6 Hz, 1H), 3.60-3.45 (m, 4H), 3.25-3.11 (m, 1H), 3.09-2.94 (m, 1H), 2.84 (d, *J* = 29.7 Hz, 3H), 2.73-2.56 (m, 5H), 2.32 (q, *J* = 9.7 Hz, 1H), 2.07 (dd, *J* = 22.2, 14.3 Hz, 2H), 1.95-1.65 (m, 2H). |
| 142 | B90 | 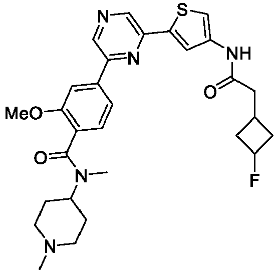 | 552.24/55 2.20 | 4-(6-(4-(2-(3-fluorocyclobutyl)a cetamido)thiophen-2-yl)pyrazin-2-yl)-2-methoxy-N-methyl-N-(1 -methylpiperidin-4-yl)benzamid e |
| | | | | ¹H NMR (400 MHz, DMSO) δ 10.44 (s, 1H), 9.24 (d, *J* = 8.8 Hz, 1H), 9.11 (d, *J* = 2.9 Hz, 1H), 7.88 (dt, *J* = 13.2, 5.8 Hz, 2H), 7.72 (s, 1H), 7.38 (dd, *J* = 11.3, 8.0 Hz, 1H), 4.62 (s, 1H), 3.94 (s, 3H), 3.53 (d, *J* = 11.3 Hz, 2H), 3.10 (d, *J* = 7.4 Hz, 2H), 2.99 (s, 1H), 2.84 (d, *J* = 29.7 Hz, 3H), 2.67 (s, 3H), 2.48 (d, *J* = 8.1 Hz, 2H), 2.40-2.27 (m, 2H), 2.24-2.13 (m, 2H), 2.09-1.95 (m, 2H), 1.91-1.79 (m, 2H), 1.71 (d, *J* = 12.5 Hz, 1H). |
| 143 | B91 | 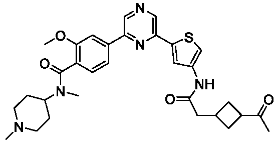 | 576.26/57 6.40 | 4-(6-(4-(2-(3-acetylcyclobutyl)a cetamido)thiophen-2-yl)pyrazin-2-yl)-2-methoxy-N-methyl-N-(1 -methylpiperidin-4-yl)benzamid e |
| | | | | ¹H NMR (400 MHz, DMSO) δ 10.40 (d, *J* = 14.9 Hz, 1H), 9.24 (d, *J* = 9.5 Hz, 1H), 9.10 (s, 1H), 7.94-7.80 (m, 3H), 7.71 (s, 1H), 7.36 (t, *J* = 7.7 Hz, 1H), 4.46 (s, 1H), 3.94 (d, *J* = 2.0 Hz, 3H), 3.21-3.12 (m, 2H), 2.89 (s, 3H), 2.70-2.60 (m, 3H), 2.39-2.20 (m, 6H), 2.05 (d, *J* = 15.2 Hz, 3H), 1.87 (dd, *J* = 20.7, 11.6 Hz, 4H), 1.69 (s, 2H), 1.48 (s, 2H). |
| 144 | B92 | 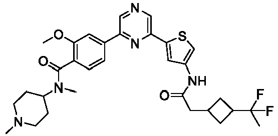 | 598.26/59 8.40 | 4-(6-(4-(2-(3-(1,1-difluoroethyl) cyclobutyl)acetamido)thiophen-2-yl)pyrazin-2-yl)-2-methoxy-N-methyl-N-(1-methylpiperidin-4-yl)benzamide |
| | | | | ¹H NMR (400 MHz, DMSO) δ 10.40 (s, 1H), 9.24 (d, *J* = 9.8 Hz, 1H), 9.09 (d, *J* = 2.0 Hz, 1H), 7.91-7.79 (m, 3H), 7.71 (s, 1H), 7.35 (t, *J* = 7.5 Hz, 1H), 4.41 (s, 1H), 3.93 (d, *J* = 2.4 Hz, 3H), 3.23-3.17 (m, 1H), 3.04 (s, 1H), 2.89 (s, 3H), 2.70-2.58 (m, 3H), 2.43-2.32 (m, 5H), 2.15 (dd, *J* = 19.7, 8.2 Hz, 4H), 1.83 (s, 3H), 1.65 (s, 2H), 1.49 (d, *J* = 18.6 Hz, 3H). |
| 145 | B93 | 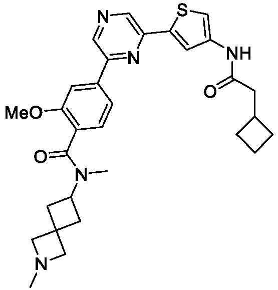 | 546.25/54 6.40 | 4-(6-(4-(2-cyclobutylacetamido)t hiophen-2-yl)pyrazin-2-yl)-2-me thoxy-N-methyl-N-(2-methyl-2-azaspiro[3.3]heptan-6-yl)benza mide |
| | | | | ¹H NMR (400 MHz, DMSO) δ 10.38 (s, 1H), 9.23 (d, *J* = 7.4 Hz, 1H), 9.09 (d, *J* = 2.4 Hz, 1H), 7.90 (d, *J* = 1.3 Hz, 1H), 7.87-7.79 (m, 2H), 7.71 (d, *J* = 1.4 Hz, 1H), 7.32 (t, *J* = 8.3 Hz, 1H), 4.87-4.76 (m, 1H), 3.92 (d, *J* = 5.0 Hz, 3H), 3.87 (d, *J* = 8.2 Hz, 1H), 3.20 (s, 1H), 3.06 (s, 1H), 3.01 (d, *J* = 3.4 Hz, 1H), 2.92 (d, *J* = 6.8 Hz, 3H), 2.78-2.64 (m, 3H), 2.43 (d, *J* = 7.6 Hz, 2H), 2.35-2.26 (m, 2H), 2.17 (s, 1H), 2.12-2.05 (m, 3H), 2.05-1.95 (m, 2H), 1.85-1.80 (m, 1H), 1.80-1.69 (m, 2H). |
| 146 | B94 | 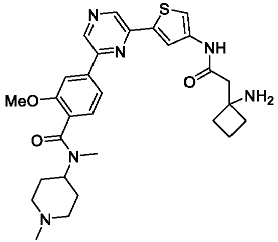 | 549.26/54 9.50 | 4-(6-(4-(2-(1-aminocyclobutyl)a cetamido)thiophen-2-yl)pyrazin-2-yl)-2-methoxy-N-methyl-N-(1 -methylpiperidin-4-yl)benzamid e |
| | | | | ¹H NMR (400 MHz, DMSO) δ 11.08 (d, *J* = 14.7 Hz, 1H), 9.26 (d, *J* = 9.5 Hz, 1H), 9.10 (d, *J* = 1.6 Hz, 1H), 7.97 (dd, *J* = 4.9, 1.2 Hz, 1H), 7.87 (dd, *J* = 13.1, 7.0 Hz, 2H), 7.78 (d, *J* = 1.1 Hz, 1H), 7.39 (dd, *J* = 12.3, 7.8 Hz, 1H), 4.66 (t, *J* = 12.2 Hz, 1H), 3.95 (s, 3H), 3.60-3.11 (m, 4H), 3.02-2.83 (m, 4H), 2.76-2.57 (m, 4H), 2.39-2.08 (m, 6H), 2.04-1.75 (m, 4H). |
| 147 | B95 | 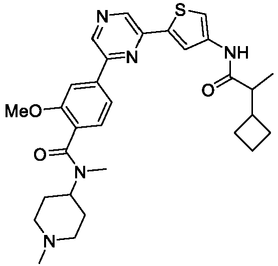 | 548.26/54 8.40 | 4-(6-(4-(2-cyclobutylpropanami do)thiophen-2-yl)pyrazin-2-yl)-2 -methoxy-N-methyl-N-(1-methy lpiperidin-4-yl)benzamide |
| | | | | ¹H NMR (400 MHz, DMSO) δ 10.35 (s, 1H), 9.24 (d, *J* = 10.2 Hz, 1H), 9.09 (d, *J* = 2.2 Hz, 1H), 8.00-7.79 (m, 3H), 7.73 (s, 1H), 7.35 (t, *J* = 7.4 Hz, 1H), 4.37 (s, 1H), 3.93 (d, *J* = 2.7 Hz, 3H), 3.19 (s, 1H), 2.92 (s, 1H), 2.90-2.62 (m, 6H), 2.44 (s, 3H), 2.33 (s, 1H), 2.25 (s, 2H), 2.09 (s, 4H), 1.92 (s, 1H), 1.62 (s, 2H), 1.44 (s, 1H), 1.02 (d, *J* = 5.8 Hz, 3H). |
| 148 | B96 | 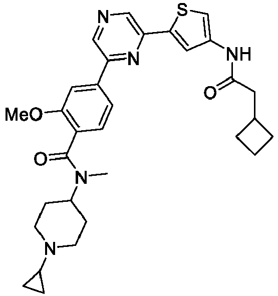 | 560.26/N/ A | 4-(6-(4-(2-cyclobutylacetamido)t hiophen-2-yl)pyrazin-2-yl)-N-(1 -cyclopropylpiperidin-4-yl)-2-m ethoxy-N-methylbenzamide |
| | | | | ¹H NMR (400 MHz, DMSO) δ 10.37 (s, 1H), 9.24 (d, *J* = 12.2 Hz, 1H), 9.09 (d, *J* = 3.7 Hz, 1H), 7.94-7.79 (m, 3H), 7.71 (s, 1H), 7.39-7.31 (m, 1H), 4.39 (s, 1H), 3.93 (s, 3H), 3.22-3.17 (m, 1H), 3.05 (s, 1H), 2.93 (s, 1H), 2.86 (s, 2H), 2.73-2.63 (m, 3H), 2.31 (d, *J* = 18.9 Hz, 2H), 2.08 (s, 2H), 1.98 (d, *J* = 11.3 Hz, 1H), 1.89-1.81 (m, 2H), 1.72 (d, *J* = 8.9 Hz, 2H), 1.61 (s, 2H), 1.48 (s, 2H), 0.45-0.21 (m, 4H). |
| 149 | B97 | 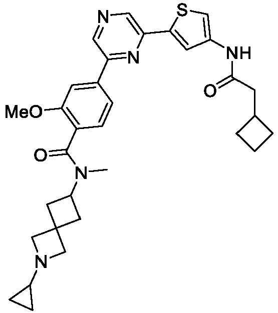 | 572.26/57 2.50 | 4-(6-(4-(2-cyclobutylacetamido)t hiophen-2-yl)pyrazin-2-yl)-N-(2 -cyclopropyl-2-azaspiro[3.3]hept an-6-yl)-2-methoxy-N-methylbe nzamide |
| | | | | ¹H NMR (400 MHz, DMSO) δ 10.38 (s, 1H), 9.23 (d, *J* = 7.3 Hz, 1H), 9.10 (d, *J* = 4.0 Hz, 1H), 7.91-7.88 (m, 1H), 7.84 (d, *J* = 8.0 Hz, 2H), 7.71 (s, 1H), 7.35-7.30 (m, 1H), 4.89-4.83 (m, 1H), 3.93 (d, *J* = 4.6 Hz, 3H), 3.00 (d, *J* = 30.4 Hz, 3H), 2.74-2.62 (m, 4H), 2.43 (d, *J* = 7.5 Hz, 2H), 2.34-2.32 (m, 1H), 2.11-2.05 (m, 2H), 1.91-1.82 (m, 2H), 1.78-1.69 (m, 2H), 1.51-1.41 (m, 2H), 1.30-1.21 (m, 3H), 0.79-0.62 (m, 4H). |
| 150 | B98 | 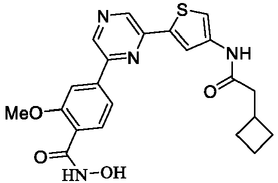 | 439.14/43 9.50 | 4-(6-(4-(2-cyclobutylacetamido)t hiophen-2-yl)pyrazin-2-yl)-N-hy droxy-2-methoxybenzamide |
| | | | | ¹H NMR (400 MHz, DMSO) δ 10.74 (s, 1H), 10.37 (s, 1H), 9.29-9.05 (m, 3H), 7.95-7.81 (m, 3H), 7.72 (dd, *J* = 11.3, 4.5 Hz, 2H), 3.97 (d, *J* = 11.4 Hz, 3H), 2.78-2.63 (m, 1H), 2.43 (d, *J* = 7.6 Hz, 2H), 2.14-1.98 (m, 2H), 1.92-1.66 (m, 4H). |
| 151 | B99 | 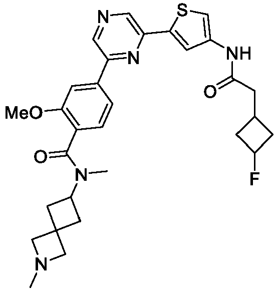 | 564.24/N/ A | 4-(6-(4-(2-(3-fluorocyclobutyl)a cetamido)thiophen-2-yl)pyrazin-2-yl)-2-methoxy-N-methyl-N-(2 -methyl-2-azaspiro[3.3]heptan-6 -yl)benzamide |
| | | | | ¹H NMR (400 MHz, CDCl₃) δ 8.85 (s, 1H), 8.80 (s, 1H), 7.67 (ddd, *J* = 20.0, 14.0, 7.2 Hz, 4H), 7.33 (d, *J* = 5.0 Hz, 1H), 4.09-4.02 (m, 1H), 3.95 (d, *J* = 12.3 Hz, 3H), 3.73-3.59 (m, 2H), 3.43-3.32 (m, 2H), 3.20-3.05 (m, 3H), 2.91-2.78 (m, 3H), 2.69-2.61 (m, 1H), 2.58-2.50 (m, 3H), 2.41-2.36 (m, 2H), 2.28-2.21 (m, 2H), 2.00-1.92 (m, 2H), 1.86-1.78 (m, 2H). |
| 152 | B100 | 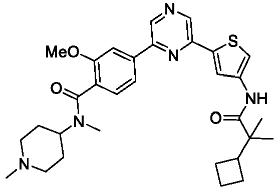 | 562.28/56 2.70 | 4-(6-(4-(2-cyclobutyl-2-methylp ropanamido)thiophen-2-yl)pyraz in-2-yl)-2-methoxy-N-methyl-N-(1-methylpiperidin-4-yl)benzami de |
| | | | | ¹H NMR (400 MHz, DMSO) δ 9.64 (s, 1H), 9.23 (d, *J* = 10.3 Hz, 1H), 9.04 (d, *J* = 1.9 Hz, 1H), 8.08 (s, 1H), 7.83 (dd, *J* = 20.7, 15.4 Hz, 3H), 7.38-7.29 (m, 1H), 4.44-4.32 (m, 1H), 3.93 (d, *J* = 2.7 Hz, 3H), 3.24-3.12 (m, 2H), 2.89 (s, 3H), 2.72-2.65 (m, 3H), 2.36-2.20 (m, 3H), 2.14- 2.04 (m, 2H), 1.68-1.56 (m, 3H), 1.48-1.38 (m, 1H), 1.32-1.21 (m, 4H), 1.16 (s, 6H). |

### Example 155: Preparation of 2-cyclopropyl-N - (5- (6- (3-methoxy-4-(N-methyl-N - (1-methylpiperidin-4-yl) sulfamoyl) phenyl) pyrazin-2-yl) thiophene-3-yl) acetamide (B103)

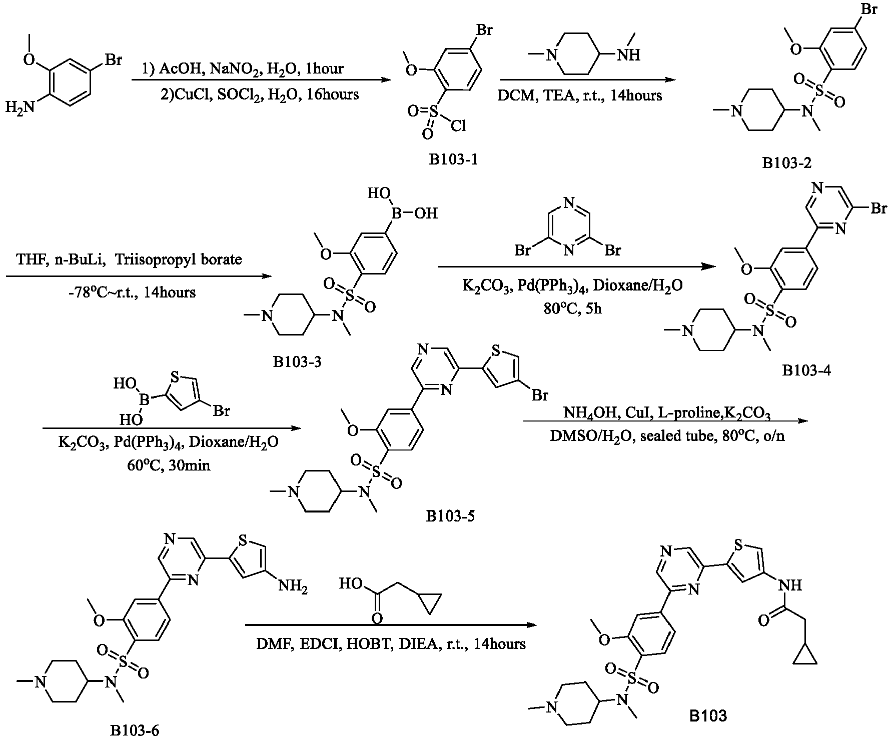

### Step 1: 4-bromo-2-methoxybenzenesulfonyl chloride (B103-1)

Cuprous chloride (20 mg, 0.20 mmol) was dissolved in water (30 mL), and the reaction system was cooled to 0-10 °C under iced-water bath. Dichloro sulfoxide (8.0 g, 67.24 mmol) was slowly added dropwise to the reaction system and the reaction system was naturally returned to room temperature and reacted for 16 hours to form a stable system A. 2-Methoxy-4-bromoaniline (3.0 g, 14.85 mmol) was dissolved in acetic acid (15mL), and the system was cooled to around 10°C under ice water bath. In addition, sodium nitrite (1.12 g, 16.23 mmol) was taken and dissolved in water (5mL) and then added dropwise to the reaction system. Upon addition, the reaction continued at 0°C for 1 hour to form a stable system B. The stable system A was cooled to 0-10°C, and the stable system B was slowly added to A. Upon addition, the mixture was naturally warmed to room temperature and continued to react for 16 hours. TLC detection showed that the reaction was complete. The reaction was extracted with ethyl acetate (50mL*3), and the organic phass were combined and washed with saturated saline (50mL*1), dried over anhydrous sodium sulfate, and spin-dried. The residue was purified by column chromatography to afford 4-bromo-2-methoxybenzenesulfonyl chloride (B103-1, 671 mg, 15.8%). ¹H NMR (400 MHz, CDC13) δ 7.84 (d, *J* = 8.4 Hz, 1H), 7.30-7.27 (m, 2H), 4.09 (s, 3H).

### Step 2: 4-bromo-2-methoxy-N-methyl-N - (1-methylpiperidin-4-yl) benzenesulfonamide (B103-2)

4-bromo-2-methoxybenzenesulfonyl chloride (B103-1, 200 mg, 0.7 mmol) was dissolved in dichloromethane (8mL), and 1-methyl-4- (methylamino) piperidine (99 mg, 0.77 mmol), and triethylamine (142 mg, 1.40 mmol) were sequentially added to the above reaction system. The reaction was carried out at room temperature for 14 hours, and TLC detection showed that the reaction was complete. The reaction solution was directly spin dried and purified by column chromatography to obtain 4-bromo-2-methoxy-N-methyl-N - (1-methylpiperidin-4-yl) benzenesulfonamide (B103-2, 264 mg, 100%). MS (ESI) m/z: calcd 377.05 (M+H⁺), found 377.40_{∘}

### Step 3: (3-Methoxy-4- (N-methyl-N - (1-methylpiperidin-4-yl) aminosulfonyl) phenyl) boronic acid (B103-3)

4-bromo-2-methoxy-N-methyl-N - (1-methylpiperidin-4-yl) benzenesulfonamide (B103-2, 264 mg, 0.70 mmol) and triisopropyl borate (396 mg, 2.10 mmol) were added to tetrahydrofuran (20mL), and the mixture was cooled to -78°C, and n-butyl lithium (0.84 mL, 2.10 mmol, 2.5 M in THF) was slowly added dropwise. Upon addition, the reaction was naturally warmed to room temperature and reacted for another 14 hours, and LCMS detected that the reaction was complete. An appropriate amount of water (1mL) was added to the system to quench the reaction, and the reaction was filtered to remove most of the inorganic salts, spin-dried, and then used directly in the next reaction without any purification. MS (ESI) m/z: calcd 343.14 (M+H⁺), found 343.60_{∘}

### Step 4: 4- (6-bromopyrazine 2-yl) -2-methoxy-N-methyl-N - (1-methylpiperidin-4-yl) benzenesulfonamide (B103-4)

(3-Methoxy-4- (N-methyl-N - (1-methylpiperidin-4-yl) aminosulfonyl) phenyl) boronic acid (B103-3, crude, theoretical 0.70 mmol), 2,6-dibromopyrazine (200 mg, 0.84 mmol), and tetrakis(triphenylphosphine)palladium (40 mg, 0.035 mmol) were added to 1,4-dioxane/water (4:1, 10 mL), and the mixture reacted under nitrogen protection at 80 °C for 5 hours. LCMS monitored that the reaction is complete. The reaction solution was directly spin dried and purified by column chromatography to obtain 4-(6-bromopyrazin-2-yl) -2-methoxy-N-methyl-N - (1-methylpiperidin-4-yl) benzenesulfonamide (B103-4, 216 mg, 47.9%). MS (ESI) m/z: calcd 455.07 (M+H⁺), found 454.90_{∘}

### Step 5: 4- (6- (4-bromothiophene-2-yl) pyrazine-2-yl) -2-methoxy-N-methyl-N - (1-methylpiperidin-4-yl) benzenesulfonamide (B103-5)

4- (6-bromopyrazin-2-yl) -2-methoxy-N-methyl-N - (1-methylpiperidin-4-yl) benzenesulfonamide (B103-4, 216mg, 0.48mmol), (4-bromothiophene-2-yl) boronic acid (98.5 mg, 0.48 mmol), tetrakis(triphenylphosphine)palladium (27 mg, 0.023mmol) and potassium carbonate (158 mg,1.14mmol) were added to 1,4-dioxane/water (4:1, 10 mL), and the mixture reacted under nitrogen protection at 60°C for 0.5 hour. TLC detected the completion of reaction. The reaction solution was directly spin-dried and purified by column chromatography to obtain 4- (6- (4-bromothiophene-2-yl) pyrazine-2-yl) -2-methoxy-N-methyl-N - (1-methylpiperidin-4-yl) benzenesulfonamide (B103-5, 182 mg, 71.4%). MS (ESI) m/z: calcd 537.06 (M+H), found 536.90_{∘}

### Step 6: 4- (6- (4-aminothiophene-2-yl) pyrazine-2-yl) -2-methoxy-N-methyl-N - (1-methylpiperidin-4-yl) benzenesulfonamide (B103-6)

4- (6- (4-bromothiophene-2-yl) pyrazine-2-yl) -2-methoxy-N-methyl-N - (1-methylpiperidin-4-yl) benzenesulfonamide (B103-5, 182 mg, 0.34 mmol), cuprous iodide (13 mg, 0.07 mmol), L-proline (16 mg, 0.14 mmol), dimethyl sulfoxide (2 mL), and ammonia (25% wt, 286 mg, 2.04 mmol) were sequentially added to the sealed tube, and the reaction was sealed and reacted at 80 °C for 10 hours. LCMS monitored that the reaction was complete. The reaction solution was cooled to room temperature, added with 20 mL of water, and extracted with ethyl acetate (10 mL * 3). The organic phases were combined , washed with saturated ammonium chloride (10 mL * 2), dried with anhydrous sodium sulfate, and spin-dried to obtain 4- (6- (4-aminothiophene-2-yl) pyrazine-2-yl) -2-methoxy-N-methyl-N - (1-methylpiperidin-4-yl) benzenesulfonamide (B103-6, crude, theoretical 0.34 mmol), which was directly used in the next reaction without any purification. MS (ESI) m/z: calcd 474.16 (M+H), found 474.10_{∘}

### Step 7: 2-cyclopropyl-N - (5- (6- (3-methoxy-4- (N-methyl-N - (1-methylpiperidin-4-yl) sulfamoyl) phenyl) pyrazin-2-yl) thiophene-3-yl) acetamide (B103)

4- (6- (4-aminothiophene-2-yl) pyrazine-2-yl) -2-methoxy-N-methyl-N - (1-methylpiperidin-4-yl) benzenesulfonamide (B103-6, crude, theoretical 0.17 mmol) was added to DMF (3mL). The flask was sequentially added with cyclopropyl acetic acid (25.4 mg, 0.25 mmol), EDCI (65 mg, 0.34 mmol), HOBT (46 mg, 0.34 mmol), and DIEA (109 mg, 0.84 mmol), and the mixture was reacted at room temperature for 14 hours. LCMS detection showed that the reaction was complete. The reaction solution was diluted with sufficient water and extracted with ethyl acetate (20mL*3), and the ethyl acetate phases were combined, washed with saturated ammonium chloride (10 mL*2), dried over anhydrous sodium sulfate, and spin-dried. The residue was purified by column chromatography to afford 2-cyclopropyl-N - (5- (6- (3-methoxy-4- (N-methyl-N - (1-methylpiperidin-4-yl) sulfamoyl) phenyl) pyrazin-2-yl) thiophene-3-yl) acetamide (B103, 25 mg, 26.4%). MS (ESI) m/z: calcd 556.20 (M+H⁺), found 556.00_{∘} ¹H NMR (400 MHz, DMSO) δ 10.35 (s, 1H), 9.27 (s, 1H), 9.15 (s, 1H), 8.01-7.86 (m, 4H), 7.75 (t, *J* = 2.4 Hz, 1H), 4.03 (s, 3H), 3.64-3.57 (m, 1H), 2.85-2.74 (m, 5H), 2.22 (d, *J* = 7.0 Hz, 2H), 2.12 (s, 3H), 1.89 (t, *J* = 11.0 Hz, 2H), 1.76-1.66 (m, 2H), 1.36 (d, *J* = 13.0 Hz, 2H), 1.29-1.20 (m, 1H), 0.55-0.46 (m, 2H), 0.26-0.17 (m, 2H).

### Examples 153-157 (compounds B101-105) were prepared using experimental steps similar to those in Example 155 above, and compounds B101-105 were summarized in Table 7.

**Table 7**

| Exam ple | Compou nd No. | Structure | MS(calcd) [M+H]⁺/ MS (found) | Name and characterization |
|---|---|---|---|---|
| 153 | B101 | 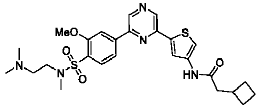 | 544.20/N/ A | 2-cyclobutyl-N-(5-(6-(4-(N-(2-( dimethylamino)ethyl)-N-methyls ulfamoyl)-3-methoxyphenyl)pyr azin-2-yl)thiophen-3-yl)acetami de |
| | | | | ¹H NMR (400 MHz, DMSO) δ 10.38 (s, 1H), 9.28 (d, *J* = 9.5 Hz, 1H), 9.15 (s, 1H), 8.00-7.86 (m, 4H), 7.72 (d*, J* = 1.3 Hz, 1H), 4.06 (s, 3H), 2.82 (d, *J* = 5.7 Hz, 3H), 2.62-2.55 (m, 2H), 2.43 (d, *J* = 7.6 Hz, 2H), 2.31 (d, *J* = 7.6 Hz, 2H), 2.29 (s, 6H), 2.09-2.04 (m, 2H), 1.88-1.81 (m, 2H), 1.79-1.72 (m, 2H), 1.69-1.61 (m, 1H). |
| 154 | B102 | 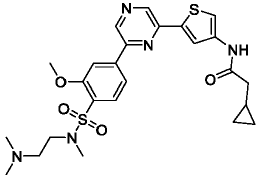 | 530.18/53 0.80 | 2-cyclopropyl-N-(5-(6-(4-(N-(2-(dimethylamino)ethyl)-N-methyl sulfamoyl)-3-methoxyphenyl)py razin-2-yl)thiophen-3-yl)acetami de |
| | | | | ¹H NMR (400 MHz, DMSO) δ 10.35 (s, 1H), 9.27 (s, 1H), 9.19 (d, *J* = 27.1 Hz, 1H), 8.02-7.87 (m, 4H), 7.75 (d, *J* = 1.3 Hz, 1H), 4.10-4.02 (m, 3H), 3.28-3.19 (m, 2H), 2.83 (s, 3H), 2.58 (s, 2H), 2.28 (s, 6H), 2.22 (d, *J* = 7.0 Hz, 2H), 1.14-1.03 (m, 1H), 0.58-0.45 (m, 2H), 0.30-0.18 (m, 2H). |
| 155 | B103 | 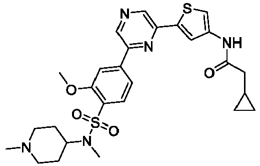 | 556.20/55 6.00 | 2-cyclopropyl-N-(5-(6-(3-metho xy-4-(N-methyl-N-(1-methylpip eridin-4-yl)sulfamoyl)phenyl)py razin-2-yl)thiophen-3-yl)acetami de |
| | | | | ¹H NMR (400 MHz, DMSO) δ 10.35 (s, 1H), 9.27 (s, 1H), 9.15 (s, 1H), 8.01-7.86 (m, 4H), 7.75 (t, *J* = 2.4 Hz, 1H), 4.03 (s, 3H), 3.64-3.57 (m, 1H), 2.85-2.74 (m, 5H), 2.22 (d, *J* = 7.0 Hz, 2H), 2.12 (s, 3H), 1.89 (t, *J* = 11.0 Hz, 2H), 1.76-1.66 (m, 2H), 1.36 (d, *J* = 13.0 Hz, 2H), 1.29-1.20 (m, 1H), 0.55-0.46 (m, 2H), 0.26-0.17 (m, 2H). |
| 156 | B104 | 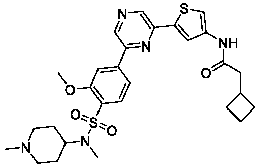 | 570.21/56 9.90 | 2-cyclobutyl-N-(5-(6-(3-methox y-4-(N-methyl-N-(1-methylpiper idin-4-yl)sulfamoyl)phenyl)pyra zin-2-yl)thiophen-3-yl)acetamid e |
| | | | | ¹H NMR (400 MHz, DMSO) δ 10.38 (s, 1H), 9.27 (s, 1H), 9.14 (s, 1H), 8.00-7.84 (m, 4H), 7.72 (d, *J* = 1.3 Hz, 1H), 4.10-4.01 (m, 3H), 2.78 (s, 3H), 2.75 (d, *J* = 7.5 Hz, 2H), 2.43 (d, *J* = 7.6 Hz, 2H), 2.31 (d, *J* = 7.6 Hz, 1H), 2.12 (s, 3H), 2.10-2.04 (m, 2H), 1.91-1.83 (m, 3H), 1.77-1.65 (m, 4H), 1.39-1.32 (m, 2H), 1.28-1.22 (m, 2H). |
| 157 | B105 | 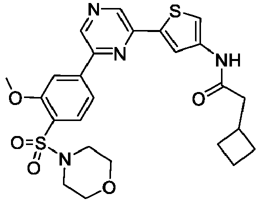 | 529.15/52 9.00 | 2-cyclobutyl-N-(5-(6-(3-methox y-4-(morpholinosulfonyl)phenyl )pyrazin-2-yl)thiophen-3-yl)acet amide |
| | | | | ¹H NMR (400 MHz, DMSO) δ 10.37 (s, 1H), 9.28 (s, 1H), 9.15 (s, 1H), 8.05-7.84 (m, 4H), 7.72 (d, *J* = 1.2 Hz, 1H), 4.04 (d, *J* = 11.8 Hz, 3H), 3.70-3.56 (m, 4H), 3.22-3.07 (m, 4H), 2.69 (dq, *J* = 15.2, 7.8 Hz, 1H), 2.43 (d, *J* = 7.5 Hz, 2H), 2.15-2.03 (m, 2H), 1.93-1.80 (m, 2H), 1.80-1.67 (m, 2H). |

### Example 158: Preparation of 2-cyclopropyl-N - (5- (6- (4-(1-methylpiperidin-4-carbonyl) -3,4-dihydro-2H-benzo [b] [1,4] oxazin-7-yl) pyrazine-2-yl) thiophene-3-yl) acetamide (E2)

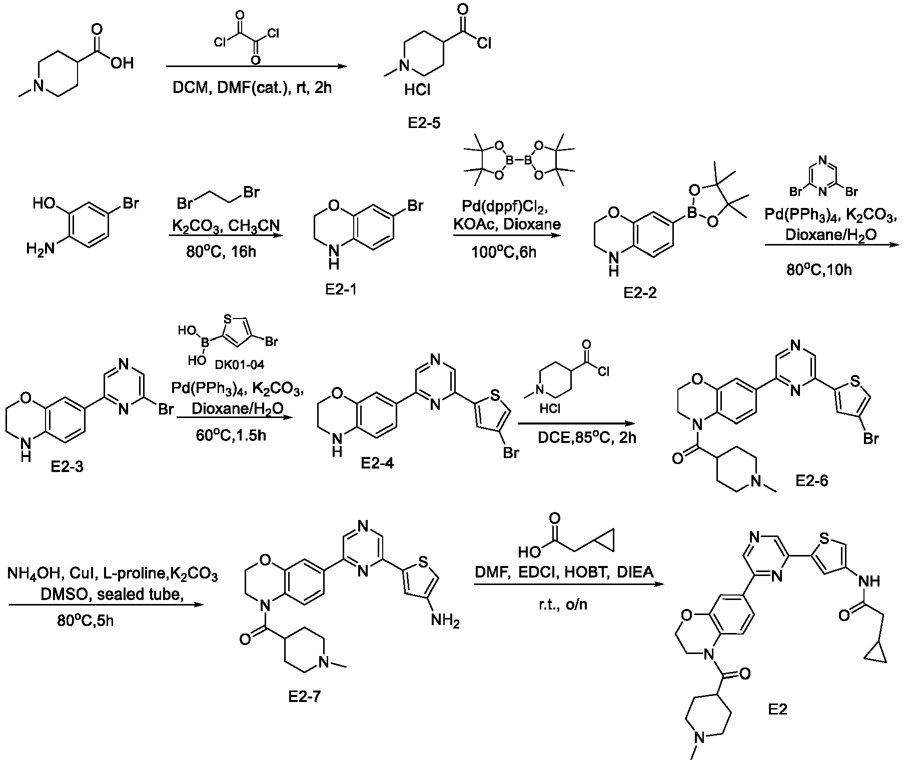

### Step 1: 7-bromo-3,4-dihydro-2H-benzo [b] [1,4] oxazine (E2-1)

2-Amino-5-bromophenol (500 mg, 2.66 mmol), 1,2-dibromoethane (1 g, 5.32 mmol), potassium carbonate (735 mg, 5.32 mmol), and acetonitrile (35 mL) were taken and placed sequentially in a reaction flask and the mixture was reacted at 80 ° C for 16 hours. LCMS monitoring showed that the reaction was completed. The reaction solution was concentrated, added with water and extracted with ethyl acetate. The aqueous phase was extracted again. The organic phases were combined, washed with saturated saline, and dried over anhydrous sodium sulfate. After concentration the residue was purified by column chromatography to afford 7-bromo-3,4-dihydro-2H-benzo [b] [1,4] oxazine (E2-1, 190 mg, 33.4%). MS (ESI) m/z: calcd216.1 (M+H), found 216.10_{∘}

### Step 2: 7- (4,4,5,5-tetramethyl-1,3,2-dioxaborolan-2-yl) -3,4-dihydro-2H-benzo [b] [1,4] oxazine (E2-2)

7-bromo-3,4-dihydro-2H-benzo [b] [1,4] oxazine (E2-1, 190 mg, 0.89 mmol), bis(pinacolato)diboron (271 mg, 1.07 mmol), potassium acetate (218 mg, 2.22 mmol), Pd (dppf)₂Cl₂ (33 mg, 0.05mmol), and 1,4-dioxane (10 mL) were taken and placed sequentially in a reaction flask. The mixture was reacted at 80°C for 6 hours under nitrogen protection. LCMS monitoring showed that the reaction was complete. The reaction solution was filtered, and the solid was washed with ethyl acetate. The filtrate was subjected to concentration. The residue was purified by column chromatography to afford 7- (4,4,5,5-tetramethyl-1,3,2-dioxaborolan-2-yl) -3,4-dihydro-2H-benzo [b] [1,4] oxazine (E2-2, 243 mg, 100%) MS (ESI) m/z: calcd262.15, (M+H),found 262.10_{∘}

### Step 3: 7- (6-bromopyrazin-2-yl) -3,4-dihydro-2H-benzo [b] [1,4] oxazine (E2-3)

7- (4,4,5,5-tetramethyl-1,3,2-dioxaborolan-2-yl) -3,4-dihydro-2H-benzo [b] [1,4] oxazine (E2-2, 243 mg,0.93 mmol), 2,6-dibromopyrazine (266 mg, 1.12 mmol), potassium carbonate (309 mg, 2.24 mmol), Pd (dppf)₂ (54 mg, 0.05 mmol), and 1,4-dioxane:water=4:1(15 mL) were taken and placed sequentially in a reaction flask. The mixture was reacted at 80°C for 6 hours under nitrogen protection. LCMS monitoring showed that the reaction was completed. The reaction solution was diluted with water and extracted with EA, and the aqueous phase was extracted again. The organic phases were combined, washed with saturated saline, and dried over anhydrous sodium sulfate. After concentration, the residue was purified by column chromatography to afford 7-(6-bromopyrazine 2-yl) -3,4-dihydro-2H-benzo [b] [1,4] oxazine (E2-3, 120 mg, 44.12%). MS (ESI) m/z: calcd292.00 (M+H), found 293.90_{∘}

### Step 4: 7- (6- (4-bromothiophene-2-yl) pyrazine-2-yl) -3,4-dihydro-2H-benzo [b] [1,4] oxazine (E2-4)

7- (6-bromopyrazin-2-yl) -3,4-dihydro-2H-benzo [b] [1,4] oxazine (E2-3, 120 mg, 0.41mmol), (4-bromothiophene-2-yl) boronic acid (85 mg, 0.41 mmol), potassium carbonate (136 mg, 0.98 mmol), Pd(PPh₃)₄ (24 mg, 0.02 mmol), and 1,4-dioxane:water=4:1 (10 mL) were taken and added sequentially in a reaction flask. The mixture was reacted at 60°C for 40 min under nitrogen protection. LCMS monitoring showed that the reaction was completed. The reaction solution was extracted with water and EA and the aqueous phase was extracted again. The organic phases were combined, washed with saturated saline, and dried over anhydrous sodium sulfate. After concentration, the residue was purified by column chromatography to afford 7- (6-(4-bromothiophene-2-yl) pyrazine-2-yl) -3,4-dihydro-2H-benzo [b] [1,4] oxazine (E2-4, 85 mg, 55.19%). MS (ESI) m/z: calcd373.99 (M+H), found 376.00_{∘}

### Step 5: 1-methylpiperidin-4-carbonyl chloride (E2-5)

1-Methylpiperidin-4-carboxylic acid (150 mg, 1.05 mmol) was taken and placed in a flask. Dichloromethane (10 mL) was added and oxalyl chloride (266 mg, 2.10 mmol) was added slowly dropwise. Upon addition, N,N-dimethylformamide (0.5mL) was added dropwise and the resulting solution was reacted at room temperature for 3 hours. The reaction solution was spin-dried directly to obtain 1-methylpiperidin-4-carbonyl chloride (E2-5, 167 mg, 99.82%).

### Step 6: (7- (6- (4-bromothiophene-2-yl) pyrazin-2-yl) -2,3-dihydro-4H-benzo [b] [1,4] oxazin-4-yl) (1-methylpiperidin-4-yl) ketone (E2-6)

7- (6- (4-bromothiophene-2-yl) pyrazine-2-yl) -3,4-dihydro-2H-benzo [b] [1,4] oxazine (E2-4, 85 mg, 0.23 mmol), 1-methylpiperidin-4-carbonyl chloride (E2-5, 55 mg, 0.34 mmol), and 1,2-dichloroethane (8 mL) were taken and added sequentially to a reaction flask. The mixture was reacted at 85 °C for 2 hours, and LCMS monitoring showed that the reaction was complete. The reaction solution was diluted with water and extracted with DCM, and the aqueous phase was extracted again. The organic phases were combined, washed with saturated saline, and dried over anhydrous sodium sulfate, and concentrated to afford (7- (6- (4-bromothiophene-2-yl) pyrazin-2-yl) -2,3-dihydro-4H-benzo [b] [1,4] oxazin-4-yl) (1-methylpiperidin-4-yl) ketone (E2-6, 60 mg, 53.1%). MS (ESI) m/z: calcd 499.07 (M+H), found 501.20_{∘}

### Step 7: (7- (6- (4-aminothiophene-2-yl) pyrazin-2-yl) -2,3-dihydro-4H-benzo [b] [1,4] oxazin-4-yl) (1-methylpiperidin-4-yl) ketone (E2-7)

(7- (6- (4-bromothiophene-2-yl) pyrazin-2-yl) -2,3-dihydro-4H-benzo [b] [1,4] oxazin-4-yl) (1-methylpiperidin-4-yl) ketone (E2-6, 60 mg, 0.12 mmol), cuprous iodide (5 mg, 0.03 mmol), L-proline (6 mg, 0.18 mmol), potassium carbonate (25 mg, 0.18 mmol), dimethyl sulfoxide (4 mL), and ammonia (42 mg, 1.2 mmol) were taken and added sequentially to a sealed tube, and the mixture was reacted at 80°C for 10 hours. LCMS monitoring showed that the reaction was complete. The reaction solution was added with water and extracted with EA, and the aqueous phase was extracted again. The organic phases were combined, washed with saturated saline, dried over anhydrous sodium sulfate and concentrated to afford crude product (7- (6- (4-aminothiophene-2-yl) pyrazin-2-yl) -2,3-dihydro-4H-benzo [b] [1,4] oxazin-4-yl) (1-methylpiperidin-4-yl) ketone (E2-7, 20 mg, 38.5%). MS (ESI) m/z: calcd 436.17(M+H), found 436.30_{∘}

### Step 8: 2-cyclopropyl-N - (5- (6- (4- (1-methylpiperidin-4-carbonyl) -3,4-dihydro-2H-benzo [b] [1,4] oxazin-7-yl) pyrazin-2-yl) thiophen-3-yl) acetamide(E2)

1-Methylenecyclopropanoic acid (7 mg, 0.07 mmol), EDCI (18 mg, 0.09 mmol), HOBT (12mg, 0.09 mmol), N, N-dimethylformamide (5 mL) were taken and placed in a reaction flask and the mixture was reacted at room temperature for 5 minutes. Then, the reaction was added with N, N-diisopropylethylamine (30 mg, 0.23 mmol), and (7- (6-(4-aminothiophene-2-yl) pyrazin-2-yl) -2,3-dihydro-4H-benzo [b] [1,4] oxazin-4-yl) (1-methylpiperidin-4-yl) ketone (E2-7, 20 mg, 0.05mmol), and then reacted overnight at room temperature. MS detection showed that the reaction was complete. The reaction solution was added with water and extracted with EA, and the aqueous phase was extracted again. The organic phases were combined, washed with saturated saline, dried over anhydrous sodium sulfate and concentrated. The residue was purified by column chromatography to afford 2-cyclopropyl-N - (5- (6- (4- (1-methylpiperidin-4-carbonyl) -3,4-dihydro-2H-benzo [b] [1,4] oxazin-7-yl) pyrazin-2-yl) thiophen-3-yl) acetamide (E2, 5.18 mg, 21.7%). MS (ESI) m/z: calcd 518.21 (M+H), found 518.50; 1H NMR (400 MHz, DMSO) δ 10.33 (s, 1H), 9.12 (s, 1H), 9.02 (s, 1H), 7.89 (d, J = 1.4 Hz, 1H), 7.79-7.71 (m, 3H), 4.38-4.31 (m, 2H), 4.01-3.93 (m, 2H), 2.92-2.79 (m, 3H), 2.22 (d, J = 7.1 Hz, 2H), 2.20 (s, 2H), 2.12 (d, J = 7.0 Hz, 1H), 2.02-1.92 (m, 2H), 1.82-1.65 (m, 4H), 1.11-1.05 (m, 1H), 0.52-0.43 (m, 2H), 0.23-0.19 (m, 1H), 0.14-0.08 (m, 1H).

### Example 159 was prepared using experimental steps similar to those in Example 158 above to afford compound E1, see Table 8.

**Table 8**

| Exam ple | Compo und No. | Structure | MS(cald) [M+H]⁺/ MS (found) | Name and characterization |
|---|---|---|---|---|
| 158 | E2 | 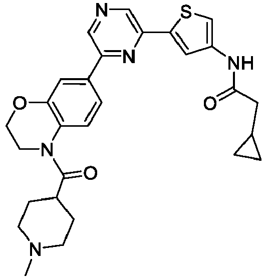 | 518.21/518 .50 | 2-cyclopropyl-N - (5- (6- (4-(1-methylpiperidin-4-carbonyl) -3,4-dihydro-2H-benzo [b] [1,4] oxazin-7-yl) pyrazine-2-yl) thiophene-3-yl) acetamide |
| | | | | 1H NMR (400 MHz, DMSO) δ 10.33 (s, 1H), 9.12 (s, 1H), 9.02 (s, 1H), 7.89 (d, J = 1.4 Hz, 1H), 7.79-7.71 (m, 3H), 4.38-4.31 (m, 2H), 4.01-3.93 (m, 2H), 2.92-2.79 (m, 3H), 2.22 (d, J = 7.1 Hz, 2H), 2.20 (s, 2H), 2.12 (d, J = 7.0 Hz, 1H), 2.02-1.92 (m, 2H), 1.82-1.65 (m, 4H), 1.11-1.05 (m, 1H), 0.52-0.43 (m, 2H), 0.23-0.19 (m, 1H), 0.14-0.08 (m, 1H). |
| 159 | E1 | 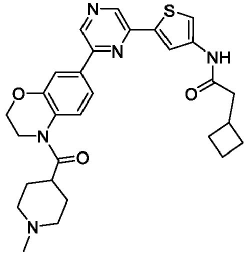 | 532.23/532. 40 | 2-cyclobutyl-N-(5-(6-(4-(1-meth ylpiperidine-4-carbonyl)-3,4-dih ydro-2H-benzo[b][1,4] oxazin-7-yl)pyrazin-2-yl)thiophen-3-yl)ac etamide |
| | | | | ¹H NMR (400 MHz, DMSO) δ 10.36 (s, 1H), 9.11 (s, 1H), 9.01 (s, 1H), 7.88 (d, *J* = 1.4 Hz, 1H), 7.71 (dd, *J* = 17.2, 4.7 Hz, 3H), 4.42-4.30 (m, 2H), 4.05-3.91 (m, 2H), 2.94-2.79 (m, 3H), 2.74-2.65 (m, 1H), 2.43 (d, *J* = 7.6 Hz, 2H), 2.20 (s, 3H), 2.14-2.03 (m, 2H), 2.03-1.93 (m, 2H), 1.89-1.80 (m, 2H), 1.80-1.69 (m, 4H), 1.38-1.29 (m, 2H). |

### Example 160: Preparation of 4- (6- (4- (3-cyclobutylurea) thiophen-2-yl) pyrazin-2-yl) -2-methoxy-N-methyl-N - (1-methylpiperidin-4-yl) benzamide (F1)

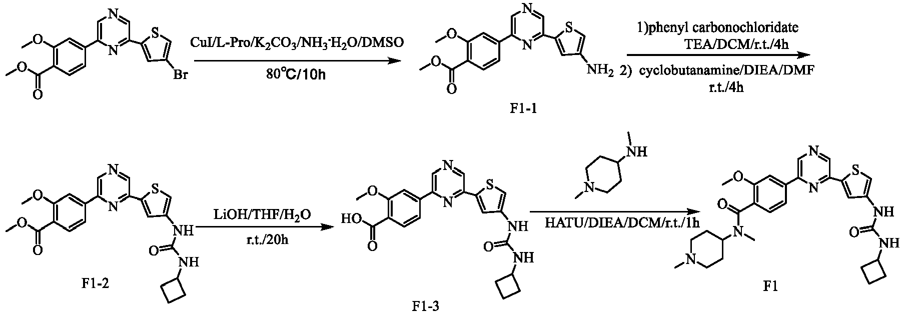

### Step 1: methyl 4- (6- (4-aminothiophen-2-yl) pyrazin-2-yl-2-methoxybenzoate (F1-1)

Methyl 4- (6- (4-bromothiophen-2-yl) pyrazine-2-yl-2-methoxybenzoate (B4-3, 200 mg, 0.49 mmol), cuprous iodide (19 mg, 0.1 mmol), L-proline (23 mg, 0.2 mmol), and potassium carbonate (102 mg, 0.74 mmol) were added to dimethyl sulfoxide (4 mL) followed by the addition of ammonia (1 mL). The tube was sealed, and heated to 80 °C and stirred for 8 hours. After TLC confirmed completion of the reaction, the reaction was poured into water and extracted with ethyl acetate. The organic phase was washed three times with deionized water, dried over anhydrous sodium sulfate, and concentrated to remove ethyl acetate to obtain methyl 4- (6- (4-aminothiophen-2-yl) pyrazin-2-yl-2-methoxybenzoate (F1-1, 150mg, 89%). MS (ESI) m/z: calcd 342.08 (M+H), found 342.1_{∘}

### Step 2: methyl 4- (6- (4- (3-cyclobutylurea) thiophen-2-yl -pyrazin-2-yl) -2-methoxybenzoate (F1-2)

Methyl 4- (6- (4-aminothiophen-2-yl) pyrazin-2-yl-2-methoxybenzoate (F1-1, 148mg, 0.43 mmol) was dissolved in dichloromethane (10 mL). The solution was added with triethylamine (53 mg, 0.52 mmol) and phenyl chloroformate (75 mg, 0.48 mmol) and the mixture was stirred at room temperature for 4 hours. LCMS monitoring showed that the reaction was completed. The reaction solution was washed for three times by adding water; the organic phase was dried over anhydrous sodium sulfate and concentrated. The product and cyclobutylamine (34 mg, 0.48 mmol) were added to N,N-dimethylformamide (8 ml), followed by the addition of N,N-diisopropylethylamine (181 mg, 1.4 mmol). The mixture was stirred at room temperature for 4 hours. LCMS monitoring showed that the reaction was completed. The reaction solution was poured into water, and extracted with ethyl acetate. The organic phase was washed three times with deionized water, dried over anhydrous sodium sulfate, and concentrated to remove ethyl acetate. The residue was purified by column chromatography to afford methyl 4- (6- (4- (3-cyclobutylurea) thiophen-2-yl -pyrazin-2-yl) -2-methoxybenzoate (F1-2, 75 mg, 39.5%). MS (ESI) m/z: calcd 439.14 (M+H), found 349.3_{∘}

### Step 3: 4- (6- (4- (3-cyclobutylurea) thiophen-2-yl -pyrazin-2-yl) -2-methoxybenzoic acid (F1-3)

Methyl 4- (6- (4- (3-cyclobutylurea) thiophen-2-yl -pyrazin-2-yl) -2-methoxybenzoate (F1-2, 75 mg, 0.17 mmol) was added to a mixed solvent of tetrahydrofuran and water (8 mL, 2mL). Then the mixture was added with lithium hydroxide (29 mg, 0.69 mmol), and stirred at room temperature for 16 hours. LCMS monitoring showed that the reaction was completed. The reaction solution was adjusted to weak acidity with 2M HCl and extracted with ethyl acetate three times. The organic phases were combined and dried over anhydrous sodium sulfate, and concentrated to afford 4- (6- (4- (3-cyclobutylurea) thiophen-2-yl -pyrazin-2-yl) -2-methoxybenzoic acid (F1-3, 40 mg, 55.3%). MS (ESI) m/z: calcd 425.12, (M+H), found 452.2_{∘}

### Step 4: 4- (6- (4- (3-cyclobutylurea) thiophen-2-yl -pyrazin-2-yl) -2-methoxy-N-methyl-N - (1-methylpiperidin-4-yl) benzamide (F1-4)

4- (6- (4- (3-cyclobutylurea) thiophen-2-yl -pyrazin-2-yl) -2-methoxybenzoic acid (F1-3, 40 mg, 0.094 mmol) and 1-methyl-4 (methylamino) piperidine (15 mg, 0.12 mmol), and o-(7-aza-benzotriazol-1-yl)-N,N,N',N'-tetramethyluronium hexafluorophosphate (72 mg, 0.19 mmol) were added to dichloromethane. The mixture was then added with N, N-diisopropylethylamine (49 mg, 0.38 mmol) and stirred at room temperature for 1 hour. TLC detection showed that the reaction was completed. The reaction solution was washed with water and the organic phase was dried over anhydrous sodium sulfate and concentrated. The residue was purified by column chromatography to afford 4- (6- (4-(3-cyclobutylurea) thiophen-2-yl -pyrazin-2-yl) -2-methoxy-N-methyl-N - (1-methylpiperidin-4-yl) benzamide (F1, 25 mg, 49.8%). MS (ESI) m/z: calcd 535.24, (M+H), found 535.4_{∘} ¹H NMR (400 MHz, DMSO) δ 9.21 (d, *J* = 10.7 Hz, 1H), 9.10 (d, *J* = 2.6 Hz, 1H), 8.69 (s, 1H), 7.84 (d, *J* = 8.2 Hz, 3H), 7.40 (s, 1H), 7.34 (t, *J* = 7.3 Hz, 1H), 6.51 (d, *J* = 7.8 Hz, 1H), 4.21-4.07 (m, 1H), 3.93 (d, *J* = 2.7 Hz, 3H), 3.16 (s, 1H), 2.95-2.61 (m, 6H), 2.22-2.17 (m, 2H), 2.09-1.94 (m, 4H), 1.92-1.75 (m, 4H), 1.69-1.54 (m, 4H).

### Example 161: Preparation of (S) -4- (6- (4- (4-amino-3-methylbutoxy) thiophen-2-yl) pyrazin-2-yl) -2-methoxy-N-methyl-N - (1-methylpiperidin-4-yl) benzamide (G1)

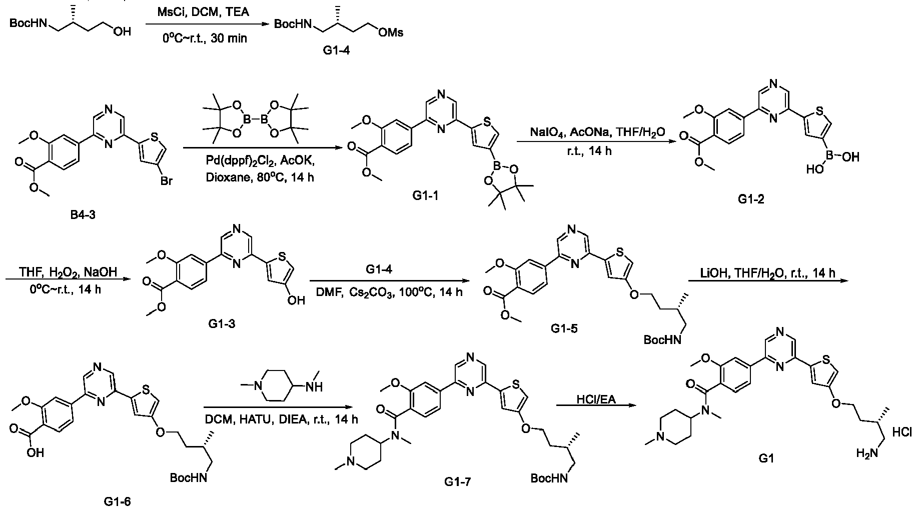

### Step 1: Methyl 2-Methoxy-4- (6- (4- (4,4,5,5-tetramethyl-1,3,2-dioxoborolan-2-yl) thiophen-2-yl) pyrazin-2-ylbenzoate (G1-1)

Methyl 4- (6- (4- bromothiophen-2-yl) pyrazin-2-ylmethoxybenzoate (B4-3, 200 mg, 0.49mmol), bis(pinacolato)diboron (150 mg, 0.59 mmol), Pd (dppf)₂Cl₂ (18mg, 0.02mmol), potassium acetate (121 mg, 1.23 mmol), and dioxane (10 mL) were placed sequentially into a reaction flask. The mixture was reacted at 80°C for 6 hours under nitrogen protection. MS monitoring showed that the reaction was completed. The reaction solution was filtered. The solid was washed with ethyl acetate and the filtrate was concentrated and purified by column chromatography to afford methyl 2-methoxy-4- (6-(4- (4,4,5,5-tetramethyl-1,3,2-dioxoborolan-2-yl) thiophen-2-yl) pyrazin-2-ylbenzoate (G1-1, 197 mg, 89.8%). MS (ESI) m/z: calcd 453.16(M+H), found 453.50_{∘}

### Step 2: (5- (6- (3-methoxy-4- (methoxycarbonyl) phenyl) pyrazin-2-yl) thiophen-3-yl) boronic acid (G1-2)

2-Methoxy-4- (6- (4- (4,4,5,5-tetramethyl-1,3,2-dioxoborolan-2-yl) thiophen-2-yl) pyrazin-2-ylbenzoate (G1-1,197 mg, 0.44 mmol), sodium periodate (189 mg, 0.88 mmol), sodium acetate (171 mg, 2.21 mmol), and tetrahydrofuran: water=3:1 (10 mL) were sequentially added to a reaction flask and the mixture was reacted at room temperature for 14 hours. LCMS detection showed that the reaction was completed. The reaction solution was filtered and the solid was collected, washed with water and dried to afford yellow product (5- (6- (3-methoxy-4- (methoxycarbonyl) phenyl) pyrazin-2-yl) thiophen-3-yl) boronic acid (G1-2, 110 mg, 67.6%). MS (ESI) m/z: calcd 371.08(M+H), found 371.30.

### Step 3: methyl 4- (6- (4-hydroxythiophen-2-yl)pyrazin-2-yl)-2-methoxybenzoate (G1-3)

To a flask containing (5- (6- (3-methoxy-4- (methoxycarbonyl) phenyl) pyrazin-2-yl) thiophen-3-yl) boronic acid (G1-2, 110mg, 0.30 mmol) was added tetrahydrofuran (10 mL). The reaction was placed in an ice bath and added slowly with hydrogen peroxide (35%, 230 mg, 2.37 mmol), followed by the addition of a 2mol/L sodium hydroxide solution (5 mg). Upon addition, the ice bath was removed and the reaction was carried out at room temperature for 14 hours. LCMS monitoring showed that the reaction was complete. The reaction solution was filtered and the solid was collected, washed with water and dried to afford methyl 4- (6-(4-hydroxythiophen-2-yl)pyrazin-2-yl)-2-methoxybenzoate (G1-3, 54mg, 48.9%). MS (ESI) m/z: calcd 342.07(M+H), found 343.30_{∘}

### Step 4: butyl (R) -4- ((tert butoxycarbonyl) amino) -3-methylmethane sulfonate (G1-4)

(R) - (4-hydroxy-2-methylbutyl) carbamate (100 mg, 0.49 mmol) was taken and placed in a flask. Dichloromethane (10mL) was added and the reaction was placed in an ice bath. Then the reaction was added with triethylamine (149 mg, 1.47 mmol) and slowly added with methylsulfonyl chloride (68 mg, 0.59 mmol). Upon addition, the ice bath was removed and the reaction was carried out at room temperature for 30 min. LCMS monitoring showed that the reaction was completed. The reaction was quenched with water and extracted with dichloromethane, and the aqueous phase was extracted again. The organic phases were combined, washed with saturated saline, dried over anhydrous sodium sulfate, and concentrated to afford butyl (R) -4- ((tert butoxycarbonyl) amino) -3-methyl methane sulfonate (G1-4, 131mg, 94.93%). MS (ESI) m/z: calcd 282.13 found 282.40.

### Step 5: methyl (S) -4- (6- (4- (4- (tert butoxycarbonyl) amino) -3-methylbutoxy) thiophen-2-yl) pyrazin-2-yl-2-methoxybenzoate (G1-5)

Methyl 4- (6- (4-hydroxythiophen-2-yl)pyrazin-2-yl)-2-methoxybenzoate (G1-3, 54 mg, 0.16 mmol), butyl (R) -4- ((tert butoxycarbonyl) amino) -3-methyl methane sulfonate (G1-4, 53 mg, 0.19 mmol), cesium carbonate (103 mg, 0.32 mmol), and N, N-dimethylformamide (10 mL) were sequentially added to a flask and the mixture was reacted at 100 ° C for 14 hours. MS monitoring showed that the reaction was completed. The reaction solution was added with water and extracted with ethyl acetate. The aqueous phase was extracted again. The organic phases were combined, washed with saturated saline, and dried over anhydrous sodium sulfate. After concentration, the residue was purified by column chromatography to afford methyl (S) -4- (6- (4- (4- (tert butoxycarbonyl) amino) -3-methylbutoxy) thiophen-2-yl) pyrazin-2-yl-2-methoxybenzoate (G1-5, 40 mg, 48.2%). MS (ESI) m/z: calcd 528.21(M+H), found 528.40.

### Step 6: (S) -4- (6- (4- (4- (tert butoxycarbonyl) amino) -3-methylbutoxy) thiophen-2-yl) pyrazin-2-yl-2-methoxybenzoic acid (A1-06)

Methyl (S) -4- (6- (4- (4- (tert butoxycarbonyl) amino) -3-methylbutoxy) thiophen-2-yl) pyrazin-2-yl-2-methoxybenzoate (G1-5, 40 mg, 0.08 mmol), lithium hydroxide (13 mg, 0.31 mmol) and tetrahydrofuran: water=4:1 (10 mL) were sequentially added to a reaction flask and the mixture was reacted at room temperature for 14 hours. MS monitoring showed that the reaction was completed. The reaction solution was adjusted to around pH 6 with citric acid, added with water, and extracted with ethyl acetate. The aqueous phase was extracted again. The organic phase was combined, washed with saturated salt water, dried over anhydrous sodium sulfate, and concentrated to obtain crude (S) -4- (6- (4- (4- (tert butoxycarbonyl) amino) -3-methylbutoxy) thiophen-2-yl) pyrazin-2-yl-2-methoxybenzoic acid (G1-6, 45mg). MS (ESI) m/z: calcd 514.19 (M+H), found 514.40.

### Step 7: Tert-butyl (S) - (4- (5- (6- (3-methoxy-4- (methyl (1-methylpiperidin-4-ylcarbamoyl) phenyl) pyrazin-2-yl) thiophen-3-yl -oxy) -2-methylbutyl) carbamate (G1-7)

(S) -4- (6- (4- (4- (tert butoxycarbonyl) amino) -3-methylbutoxy) thiophen-2-yl) pyrazin-2-yl-2-methoxybenzoic acid (G1-6, 45 mg, 0.09 mmol) was mixed with 2-(7-aza-benzotriazol-1-yl)-N,N,N',N'-tetramethyluronium hexafluorophosphate (67 mg, 0.18 mmol) and dichloromethane(10mL). The mixture was reacted at room temperature for 5min and then added with 1-methyl-4- (methylamino) piperidine (14 mg, 0.11 mmol), and N, N-diisopropylethylamine (45 mg, 0.35 mmol). The resulting solution was reacted at room temperature for 2 hours. LCMS detection showed that the reaction was completed. The reaction solution was added with water and extracted with DCM, and the aqueous phase was extracted again. The organic phases were combined, washed with saturated saline, and dried over anhydrous sodium sulfate. After concentration, the residue was purified by column chromatography to afford tert-butyl (S) - (4- (5- (6- (3-methoxy-4-(methyl (1-methylpiperidin-4-ylcarbamoyl) phenyl) pyrazin-2-yl) thiophen-3-yl -oxy) -2-methylbutyl) carbamate (G1-7, 21 mg, 38.2%). MS (ESI) m/z: calcd 624.31, (M+H), found 624.50.

### Step 8: (S) -4- (6- (4- (4-amino-3-methylbutoxy) thiophen-2-yl) pyrazin-2-yl) -2-methoxy-N-methyl-N - (1-methylpiperidin-4-yl)benzamide (G1)

Tert-butyl (S) - (4- (5- (6- (3-methoxy-4- (methyl (1-methylpiperidin-4-ylcarbamoyl) phenyl) pyrazin-2-yl) thiophen-3-yloxy) -2-methylbutyl) carbamate (G1-7, 21 mg, 0.03mmol) was taken and added with hydrochloric acid ethyl acetate solution (3mol/L, 3mL) and ethyl acetate (1mL). The mixture was reacted at room temperature for 30min. LCMS monitoring showed that the reaction was complete. The reaction solution was directly dried by rotary evaporation to afford yellow solid product (S) -4- (6- (4-(4-amino-3-methylbutoxy) thiophen-2-yl) pyrazin-2-yl) -2-methoxy-N-methyl-N - (1-methylpiperidin-4-yl)benzamide hydrochloride (G1, 12 mg, 66.67%). MS (ESI) m/z: calcd 524.26 (M+H), found 524.50; ¹H NMR (400 MHz, DMSO) δ 9.21 (dd, *J* = 15.2, 7.4 Hz, 2H), 8.10 (s, 3H), 7.95-7.74 (m, 3H), 7.46-7.33 (m, 1H), 6.96-6.88 (m, 1H), 4.12-4.06 (m, 1H), 3.94 (s, 3H), 3.65-3.53 (m, 2H), 3.47 (d, *J* = 11.2 Hz, 1H), 3.41-3.23 (m, 2H), 3.23-3.07 (m, 2H), 2.91-2.81 (m, 3H), 2.74-2.67 (m, 3H), 2.59 (d, *J* = 4.6 Hz, 2H), 2.38-2.17 (m, 2H), 2.08-1.96 (m, 1H), 1.95-1.87 (m, 1H), 1.80 (t, *J* = 12.1 Hz, 1H), 1.70-1.56 (m, 2H), 1.02 (d, *J* = 6.7 Hz, 3H).

### Examples 162-164 (compounds A46-A48) were prepared using experimental steps similar to those in Example 35 above, and compounds A46-A48 were summarized in Table 9.

**Table 9**

| Examp le | Comp ound No. | Structure | MS(cald) [M+H]⁺/ MS (found) | Name and characterization |
|---|---|---|---|---|
| 162 | A46 | 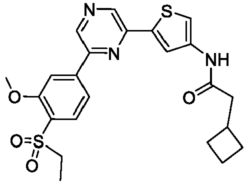 | 472.13/471. 90 | 2-cyclobutyl-N-(5-(6-(4-(ethyl sulfonyl)-3-methoxyphenyl)p yrazin-2-yl)thiophen-3-yl)acet amide |
| | | | | ¹H NMR (400 MHz, DMSO) δ 10.38 (s, 1H), 9.29 (s, 1H), 9.16 (s, 1H), 8.01-7.91 (m, 4H), 7.72 (d, *J*=1.3 Hz, 1H), 4.10 (s, 3H), 3.44 (q, *J*=7.4 Hz, 2H), 2.70 (dt, *J*=15.2, 7.7 Hz, 2H), 2.43 (d, *J*=7.5 Hz, 2H), 2.12-2.04 (m, 2H), 1.94-1.65 (m, 4H), 1.13 (t, *J*=7.4 Hz, 3H). |
| 163 | A47 | 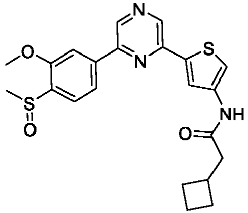 | 442.12/442. 30 | 2-cyclobutyl-N-(5-(6-(3-meth oxy-4-(methylsulfinyl)phenyl) pyrazin-2-yl)thiophen-3-yl)ac etamide |
| | | | | ¹H NMR (400 MHz, DMSO) δ 10.38 (s, 1H), 9.25 (s, 1H), 9.12 (s, 1H), 8.03 (dd, *J* = 8.0, 1.3 Hz, 1H), 7.89 (dd, *J* = 7.8, 1.3 Hz, 2H), 7.82 (d, *J* = 8.0 Hz, 1H), 7.72 (d, *J* = 1.3 Hz, 1H), 4.02 (s, 3H), 2.79 (s, 3H), 2.70 (dt, *J* = 15.5, 7.8 Hz, 1H), 2.43 (d, *J* = 7.6 Hz, 2H), 2.12-2.04 (m, 2H), 1.91-1.68 (m, 4H). |
| 164 | A48 | 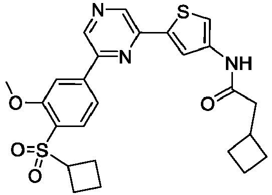 | 498.14/498. 30 | 2-cyclobutyl-N-(5-(6-(4-(cycl obutylsulfonyl)-3-methoxyphe nyl)pyrazin-2-yl)thiophen-3-y l)acetamide |

### Example 166: Preparation of 2-cyclobutyl-N - (5- (6- (2- (1-methylpiperidin-4-yl) methyl) -2H-indazol-6-yl) pyrazin-2-yl) thiophene-3-yl) acetamide (A50)

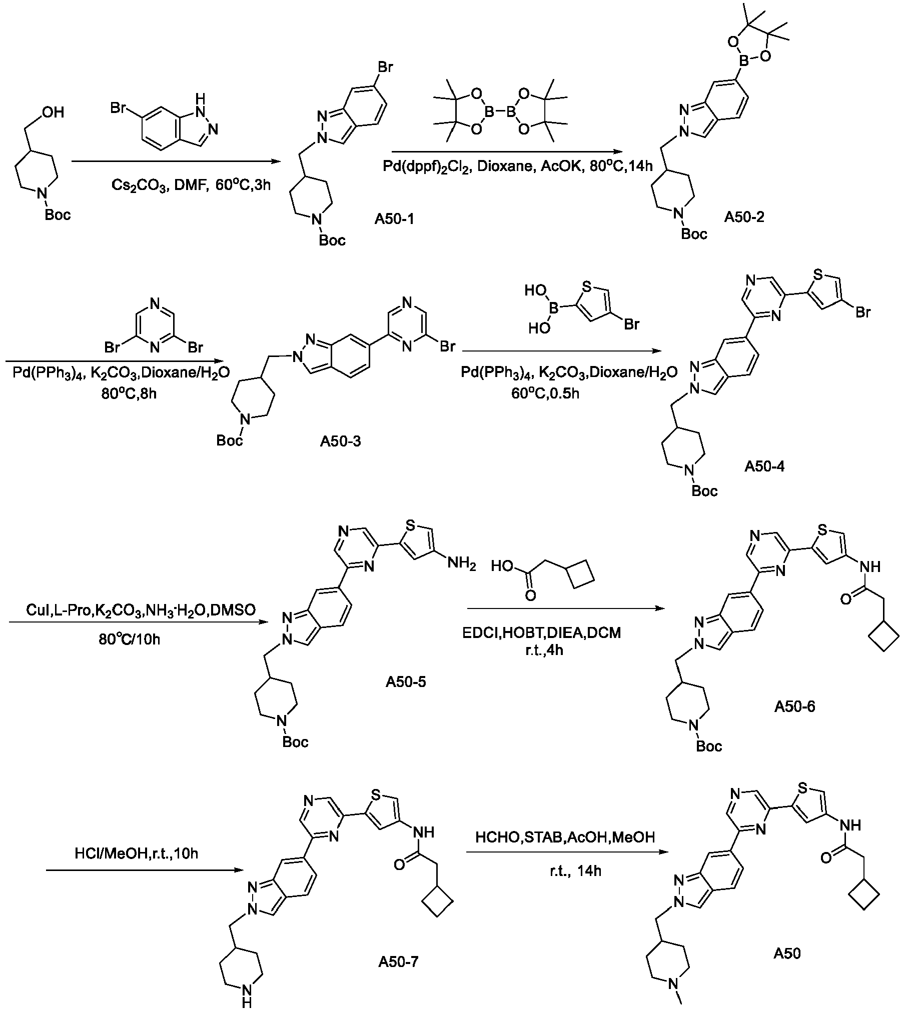

### Step 1: Tert butyl 4- (6-bromo-2H-indazol-2-yl) methyl) piperidin-1-carboxylate (A50-1)

6-bromo-1H-indazole (600 mg, 3.04 mmol), tert butyl 4- (hydroxymethyl) piperidin-1-carboxylate (1.0 g, 3.6 mmol), and cesium carbonate (1.98 g, 6.08 mmol) were added to N,N-dimethylformamide (10ml), and the mixture was heated to 60 ° C and stirred for 3 hours. After LCMS and TLC confirmed completion of the reaction, the reaction solution was poured into water, and extracted with ethyl acetate. The organic phase was washed three times with water, dried over anhydrous sodium sulfate, and concentrated to remove ethyl acetate. The residue was purified by column chromatography to afford tert butyl 4- (6-bromo-2H-indazol-2-yl) methyl) piperidin-1-carboxylate (A50-1, 630 mg, 52.3%). MS (ESI) m/z: calcd 394.11 (M+H), found 338.2.

### Step 2: Tert butyl 4- ((6- (4,4,5,5-tetramethyl-1,3,2-dioxoboran-2-yl) -2H-indazol-2-ylmethyl) piperidin-1-carboxylate (A50-2)

Tert butyl 4- (6-bromo-2H-indazol-2-yl) methyl) piperidin-1-carboxylate (A50-1, 630 mg, 1.59 mmol), bis(pinacolato)diboron (487 mg, 1.90 mmol), [1,1'-Bis(diphenylphosphino)ferrocene]dichloropalladium(II) (58 mg, 0.08 mmol), and potassium acetate (392 mg, 3.98 mmol) were added to dioxane (10mL), and the mixture was heated to 80 °C under nitrogen protection, and stirred for 14 hours. LCMS and TLC confirmed completion of the reaction, and the crude product was used directly in the next step to afford tert butyl 4- ((6- (4,4,5,5-tetramethyl-1,3,2-dioxoboran-2-yl) -2H-indazol-2-ylmethyl) piperidin-1-carboxylate (A50-2, 705 mg Crude, theoretical 1.59 mmo). MS (ESI) m/z: calcd 442.28 (M+H), found 442.5.

### Step 3: Tert butyl 4- ((6- (6-bromopyrazin-2-yl)-2H-indazol-2-ylmethyl) piperidin-1-carboxylate (A50-3)

Tert butyl 4- ((6- (4,4,5,5-tetramethyl-1,3,2-dioxoboran-2-yl) -2H-indazol-2-ylmethyl) piperidin-1-carboxylate (A50-2, 705 mg Crude, theoretical 1.59 mmol), 2,6-dibromopyrazine (456 mg, 1.90 mmol), tetrakis(triphenylphosphine)palladium (92 mg, 0.08 mmol), and potassium carbonate (530 mg, 3.80 mmol) were added to a mixed solvent of dioxane/water (12 mL/3 mL), and the mxiture was heated to 80 °C under nitrogen protection, and stirred for 8 hours. After MS and TLC confirmed completion of the reaction, the reaction solution was poured into water, and extracted three times with ethyl acetate. The organic phase was dried over anhydrous sodium sulfate, and concentrated. The residue was purified by column chromatography to afford tert butyl 4- ((6-(6-bromopyrazin-2-yl)-2H-indazol-2-ylmethyl) piperidin-1-carboxylate (A50-3, 280 mg, 37.1%). MS (ESI) m/z: calcd 472.13 (M+H), found 374.2.

### Step 4: Tert butyl 4- (6- (6- (4-bromothiophen-2-yl) pyrazin-2-yl) -2H-indazol-2-yl) methylpiperidin-1-carboxylate (A50-4)

Tert butyl 4- ((6- (6-bromopyrazin-2-yl)-2H-indazol-2-ylmethyl) piperidin-1-carboxylate (A50-3, 280mg, 0.59 mmol), (4-bromothiophen-2-yl) boronic acid (122 mg, 0.59 mmol), tetrakis(triphenylphosphine)palladium (35 mg, 0.03 mmol) and potassium carbonate (197 mg,1.44 mmol) were added to dioxane/water (12mL/3 mL), and the mixture was heated to 60°C and stirred fo 0.5 h under nitrogen protection. After MS and TLC confirmed completion of the reaction, the reaction solution was poured into water, and extracted three times with ethyl acetate. The organic phase was dried over anhydrous sodium sulfate, and concentrated. The residue was purified by column chromatography to afford tert butyl 4- (6- (6- (4-bromothiophen-2-yl) pyrazin-2-yl) -2H-indazol-2-yl) methylpiperidin-1-carboxylate (A50-4, 240 mg, 83.0%). MS (ESI) m/z: calcd 554.11 found 456.2.

### Step 5: Tert butyl 4- (6- (6- (4-aminothiophen-2-yl) pyrazin-2-yl) -2H-indazol-2-yl) methyl) piperidin-1-carboxylate (A50-5)

Tert butyl 4- (6- (6- (4-bromothiophen-2-yl) pyrazin-2-yl) -2H-indazol-2-yl) methylpiperidin-1-carboxylate (A50-4, 240 mg, 0.49 mmol), cuprous iodide (16 mg, 0.10 mmol), L-proline (19mg, 0.20 mmol), and potassium carbonate (85 mg, 1.18 mmol) were added to dimethyl sulfoxide (4 mL), and the ammonia (1 mL) was added. The tube was sealed, and heated to 80 °C and stirred for 8 hours. TLC confirmed completion of the reaction. The reaction solution was poured into water, and extracted with ethyl acetate. The organic phase was washed three times with water, dried over anhydrous sodium sulfate, and concentrated to remove ethyl acetate to afford tert butyl 4- (6- (6-(4-aminothiophen-2-yl) pyrazin-2-yl) -2H-indazol-2-yl) methyl) piperidin-1-carboxylate (A50-5, 220 mg crude, 0.49 mmol theoretical), which was used directly in the next step. MS (ESI) m/z: calcd 491.22 found 491.6.

### Step 6: Tert butyl 4- (6- (6-(4- (2-cyclobutylacetamido) thiophen-2-yl)pyrazin-2-yl) -2H-indazol-2-yl)methyl)piperidin-1-carboxylate (A50-6)

Tert butyl 4- (6- (6- (4-aminothiophen-2-yl) pyrazin-2-yl) -2H-indazol-2-yl) methyl) piperidin-1-carboxylate (A50-5, 220 mg crude, 0.49 mmol theoretical), 2-cyclobutyl acetic acid (62 mg, 0.54 mmol), 1-hydroxybenzotriazole (122 mg, 0.90 mmol), and 1-(3-dimethylaminopropyl) -3-ethylcarbodiimide hydrochloride (172 mg, 0.90 mmol) were added to dichloromethane (12mL), and then the mixture was added with N, N-diisopropylethylamine (232 mg, 1.80 mmol) and stirred at room temperature for 4 hours. LCMS and TLC confirmed completion of the reaction. The reaction solution was poured into water, and extracted with ethyl acetate. The organic phase was washed three times with water, dried over anhydrous sodium sulfate, and concentrated to remove ethyl acetate. The residue was purified by column chromatography to afford tert butyl 4- (6- (6-(4-(2-cyclobutylacetamido) thiophen-2-yl)pyrazin-2-yl) -2H-indazol-2-yl)methyl)piperidin-1-carboxylate (A50-6, 70 mg, 20.4%). MS (ESI) m/z: calcd 586.27 found 487.6.

### Step 7: 2-cyclobutyl-N - (5- (6- (2- (2-piperidin-4-ylmethyl) -2H-indazol-6-yl) pyrazin-2-yl) thiophen-3-yl) acetamide (A50-7)

Tert butyl 4- (6- (6-(4- (2-cyclobutylacetamido) thiophen-2-yl)pyrazin-2-yl) -2H-indazol-2-yl)methyl)piperidin-1-carboxylate (A50-6, 70 mg, 0.1 mmol) was dissolved in 2 mL of ethyl acetate. The mixture was addded with 4 mL of 3M hydrogen chloride in ethyl acetate and then stirred at room temperature for 10 hours. TLC detection showed that the reaction was completed. The solvent was dried by rotary evaporation to afford 2-cyclobutyl-N - (5- (6- (2- (2-piperidin-4-ylmethyl) -2H-indazol-6-yl) pyrazin-2-yl) thiophen-3-yl) acetamide (A50-7, 90 mg crude product, theoretical 0.1 mmol) and the crude product was directly used in the next step. MS (ESI) m/z: calcd 486.22 found 487.5.

### Step 8: 2-cyclobutyl-N - (5- (6- (2- (1-methylpiperidin-4-yl) methyl) -2H-indazol-6-yl) pyrazin-2-yl) thiophene-3-yl) acetamide (A50)

2-cyclobutyl-N - (5- (6- (2- (2-piperidin-4-ylmethyl) -2H-indazol-6-yl) pyrazin-2-yl) thiophen-3-yl) acetamide (A50-7, 90 mg crude product, theoretical 0.1 mmol), formaldehyde (55 mg, 1.90 mmol) and acetic acid (34.2 mg, 0.57 mmol) were added to methanol (12mL). After stirring at room temperature for 0.5 hours, the mixture was added with sodium triacetoxyborohydride (196 mg, 0.93 mmol) and stirred at room temperature for 14 hours. After LCMS and TLC confirmed completion of the reaction, the reaction was dried to remove solvent. The residue was added with water, and extracted with ethyl acetate. The organic phase was dried over anhydrous sodium sulfate, and concentrated to remove ethyl acetate. The residue was purified by column chromatography to afford 2-cyclobutyl-N - (5- (6- (2- (1-methylpiperidin-4-yl) methyl) -2H-indazol-6-yl) pyrazin-2-yl) thiophene-3-yl) acetamide (A50, 20 mg, 39.9%). MS (ESI) m/z: calcd 501.24 found 501.40_{∘} ¹H NMR (400 MHz, DMSO) δ 10.40 (s, 1H), 9.23 (s, 1H), 9.03 (s, 1H), 8.50 (s, 1H), 8.45 (s, 1H), 7.90 (dd, *J* = 5.0, 1.1 Hz, 3H), 7.71 (d, *J* = 1.2 Hz, 1H), 4.37 (d, *J* = 7.2 Hz, 2H), 2.79-2.69 (m, 2H), 2.69-2.65 (m, 1H), 2.44 (d, *J* = 7.6 Hz, 2H), 2.13 (s, 2H), 2.10-2.05 (m, 1H), 2.02-1.93 (m, 1H), 1.91-1.82 (m, 2H), 1.80 (s, 3H), 1.78-1.69 (m, 2H), 1.45 (d, *J* = 10.8 Hz, 2H), 1.35-1.26 (m, 3H).

### Examples 165-168 (compounds A49-A52) were prepared using experimental steps similar to those in above Example A50, and compounds A49-A52 were summarized in Table 10.

**Table 10**

| Example | Compo und No. | Structure | MS(cald) [M+H]⁺/ MS (found) | Name and characterization |
|---|---|---|---|---|
| 165 | A49 | 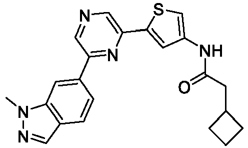 | 404.15/404. 40 | 2-cyclobutyl-N-(5-(6-(1-met hyl-1H-indazol-6-yl)pyrazin-2-yl)thiophen-3-yl)acetamide ¹H NMR (400 MHz, DMSO) δ 10.37 (s, 1H), 9.30 (s, 1H), 9.07 (s, 1H), 8.49 (s, 1H), 8.14 (s, 1H), 8.01 (d, *J* = 8.5 Hz, 1H), 7.94 (d, *J* = 8.5 Hz, 1H), 7.90 (d, *J* = 1.1 Hz, 1H), 7.72 (s, 1H), 4.17 (s, 3H), 2.77-2.65 (m, 1H), 2.44 (d, *J* = 7.6 Hz, 2H), 2.15-2.03 (m, 2H), 1.94-1.81 (m, 2H), 1.81-1.67 (m, 2H). |
| 166 | A50 | 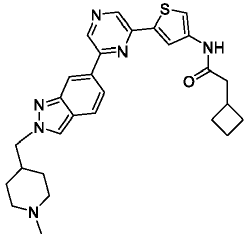 | 501.24/501. 40 | 2-cyclobutyl-N-(5-(6-(2-((1 -methylpiperidin-4-yl)meth yl)-2H-indazol-6-yl)pyrazi n-2-yl)thiophen-3-yl)aceta mide |
| | | | | ¹H NMR (400 MHz, DMSO) δ 10.40 (s, 1H), 9.23 (s, 1H), 9.03 (s, 1H), 8.50 (s, 1H), 8.45 (s, 1H), 7.90 (dd, *J* = 5.0, 1.1 Hz, 3H), 7.71 (d, *J* = 1.2 Hz, 1H), 4.37 (d, *J* = 7.2 Hz, 2H), 2.79-2.69 (m, 2H), 2.69-2.65 (m, 1H), 2.44 (d, *J* = 7.6 Hz, 2H), 2.13 (s, 2H), 2.10-2.05 (m, 1H), 2.02-1.93 (m, 1H), 1.91-1.82 (m, 2H), 1.80 (s, 3H), 1.78-1.69 (m, 2H), 1.45 (d, *J* = 10.8 Hz, 2H), 1.35-1.26 (m, 3H). |
| 167 | A51 | 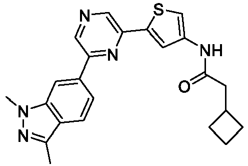 | 418.16/418. 30 | 2-cyclobutyl-N-(5-(6-(1,3-di methyl-1H-indazol-6-yl)pyra zin-2-yl)thiophen-3-yl)aceta mide |
| | | | | ¹H NMR (400 MHz, DMSO) δ 10.38 (s, 1H), 9.29 (s, 1H), 9.06 (s, 1H), 8.41 (s, 1H), 7.96 (d, *J* = 9.5 Hz, 1H), 7.89 (d, *J* = 9.1 Hz, 2H), 7.72 (s, 1H), 4.08 (s, 3H), 2.73-2.67 (m, 1H), 2.53 (s, 3H), 2.44 (d, *J* = 7.6 Hz, 2H), 2.16-2.04 (m, 2H), 1.91-1.81 (m, 2H), 1.81-1.70 (m, 2H). |
| 168 | A52 | 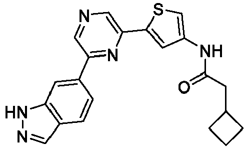 | 390.13/390. 40 | N-(5-(6-(1H-indazol-6-yl)py razin-2-yl)thiophen-3-yl)-2-c yclobutylacetamide |
| | | | | ¹H NMR (400 MHz, DMSO) δ 13.35 (s, 1H), 10.38 (s, 1H), 9.22 (s, 1H), 9.06 (s, 1H), 8.37 (s, 1H), 8.17 (s, 1H), 7.95 (s, 2H), 7.90 (d, *J* = 1.4 Hz, 1H), 7.72 (d, *J* = 1.3 Hz, 1H), 2.78-2.64 (m, 1H), 2.44 (d, *J* = 7.6 Hz, 2H), 2.15-2.03 (m, 2H), 1.92-1.81 (m, 2H), 1.81-1.69 (m, 2H). |

### Effect example 1

### Drak2 activity testing method

The activity of Drak2 was detected using the ADP-Glo kinase assay kit from Promega. The reaction for this method was in a 384 white shallow well plate and the total volume of the reaction was 5 µL. Specifically, including 1 µ L of the compound to be tested (2% DMSO), 2 µ L of Drak2, and 2 µ L of ATP. The reaction buffer system includes 50 mM Na₃PO₄, 0.02% NaN₃, 0.1 mM Na₃VO₄, 5 mM MgCl₂, 0.01% (w/v) BSA, and 50 mM HEPES with pH value of 7.0 (the above reaction buffer system was used to dilute Drak2 and ATP, and the above concentrations were their respective concentrations in the total reaction volume of 5µL). After incubating at room temperature for two hours, 5 µ L of ADP Glo termination buffer (reagent in the kit) was added to terminate the kinase reaction and consume the remaining ATP. After one hour of reaction, a kinase detection reagent (reagent in the kit) was added and incubated for half an hour. It not only converted ADP to ATP, but also used a coupled luciferase/luciferin (reagent in the kit) reaction to detect newly synthesized ATP. An Envision multifunctional microplate enzyme-linked immunosorbent assay instrument was used to read the value and detect the effect of the compounds to be tested on Drak2 kinase activity. Envision parameter was set to Aperture, and the specific selection was 384 plate US Lminescence Aperture-In. Background pores without Drak2 and Drak2 full enzyme active pores without compounds were set up in the reaction .

The IC50 value of the compound inhibiting Drak2 kinase activity was calculated using Graphpad Prism 7.00 software, using the formula: Y=100/(1+10 ^ (LogIC50-X) * HillSlope). Activity range: A: <10 nM; B: 11-100 nM; C: 101-1000 nM; D: 1001-10000 nM; E: > 10000 nM.

**Table 11**

| **Examples** | **Compounds No.** | **IC50_Drak2 (nM)** |
|---|---|---|
| 1 | A1 | C |
| 2 | A2 | D |
| 3 | A3 | C |
| 4 | A4 | C |
| 5 | A5 | C |
| 6 | A6 | C |
| 7 | A7 | C |
| 8 | A8 | C |
| 9 | A9 | C |
| 10 | A10 | D |
| 11 | A11 | E |
| 12 | A12 | D |
| 13 | A13 | C |
| 14 | A14 | D |
| 15 | A15 | C |
| 16 | A16 | D |
| 17 | A17 | C |
| 18 | A18 | C |
| 19 | A19 | C |
| 20 | A20 | C |
| 21 | A21 | C |
| 22 | A22 | C |
| 23 | A23 | C |
| 24 | A24 | D |
| 25 | A25 | E |
| 26 | A26 | D |
| 27 | A27 | D |
| 28 | A28 | D |
| 29 | A29 | C |
| 30 | A30 | E |
| 31 | A31 | B |
| 32 | A32 | B |
| 33 | A33 | C |
| 34 | A34 | B |
| 35 | A35 | B |
| 36 | A36 | C |
| 37 | A37 | C |
| 38 | A38 | D |
| 39 | A39 | C |
| 40 | A40 | C |
| 41 | A41 | C |
| 42 | A42 | C |
| 43 | A43 | B |
| 44 | A44 | B |
| 45 | A45 | D |
| 46 | B1 | C |
| 46 | B2 | B |
| 47 | B3 | C |
| 46 | B4 | B |
| 48 | B5 | C |
| 49 | B6 | C |
| 50 | B7 | C |
| 51 | B8 | D |
| 52 | B9 | C |
| 53 | B10 | C |
| 54 | B11 | C |
| 55 | B12 | C |
| 56 | B13 | C |
| 57 | B14 | C |
| 58 | B15 | B |
| 59 | B16 | C |
| 60 | B17 | D |
| 61 | B18 | E |
| 62 | B19 | E |
| 63 | B20 | E |
| 64 | B21 | E |
| 65 | B22 | C |
| 66 | B23 | B |
| 67 | B24 | D |
| 68 | B25 | B |
| 69 | B26 | B |
| 70 | B27 | B |
| 71 | B28 | C |
| 72 | B29 | B |
| 73 | B30 | C |
| 74 | B31 | B |
| 75 | B32 | B |
| 76 | B33 | B |
| 77 | B34 | C |
| 78 | B35 | C |
| 79 | B36 | C |
| 80 | B37 | B |
| 81 | B38 | B |
| 82 | B39 | B |
| 83 | B40 | B |
| 84 | B41 | C |
| 85 | B42 | C |
| 86 | B43 | C |
| 87 | B44 | C |
| 88 | B45 | B |
| 89 | B46 | B |
| 90 | B47 | B |
| 91 | B48 | B |
| 92 | B49 | B |
| 93 | B50 | B |
| 94 | B51 | B |
| 95 | B52 | B |
| 96 | B53 | B |
| 97 | B54 | B |
| 98 | B55 | B |
| 99 | B56 | A |
| 100 | B57 | B |
| 101 | B58 | B |
| 102 | B59 | A |
| 103 | B60 | B |
| 104 | B61 | B |
| 105 | B62 | B |
| 106 | B63 | A |
| 107 | B64 | B |
| 108 | B65 | C |
| 109 | B66 | D |
| 110 | B67 | C |
| 111 | B68 | A |
| 112 | B69 | A |
| 113 | B70 | C |
| 114 | B71 | B |
| 115 | B72 | C |
| 116 | B73 | C |
| 117 | B74 | C |
| 118 | B75 | E |
| 119 | B76 | B |
| 120 | B77 | B |
| 121 | B78 | B |
| 122 | C1 | E |
| 122 | C2 | D |
| 123 | C3 | D |
| 124 | C4 | D |
| 125 | D1 | E |
| 125 | D2 | E |
| 126 | D3 | E |
| 126 | D4 | D |
| 127 | D5 | D |
| 128 | D6 | C |
| 129 | D7 | D |
| 130 | D8 | D |
| 131 | B79 | B |
| 132 | B80 | B |
| 133 | B81 | B |
| 134 | B82 | C |
| 135 | B83 | B |
| 136 | B84 | B |
| 137 | B85 | C |
| 138 | B86 | C |
| 139 | B87 | C |
| 140 | B88 | C |
| 141 | B89 | C |
| 142 | B90 | A |
| 143 | B91 | C |
| 144 | B92 | B |
| 145 | B93 | B |
| 146 | B94 | C |
| 147 | B95 | B |
| 148 | B96 | B |
| 149 | B97 | B |
| 150 | B98 | A |
| 151 | B99 | A |
| 152 | B100 | B |
| 153 | B101 | B |
| 154 | B102 | B |
| 155 | B103 | B |
| 156 | B104 | B |
| 157 | B105 | B |
| 158 | E2 | B |
| 159 | E1 | B |
| 160 | F1 | B |
| 161 | G1 | C |
| 162 | A46 | C |
| 163 | A47 | C |
| 164 | A48 | C |
| 165 | A49 | B |
| 166 | A50 | B |
| 167 | A51 | C |
| 168 | A52 | B |

It can be concluded from the activity data from above table that the compound of the present invention possess Drak2 kinase inhibitory activity.

All documents mentioned in the present invention are cited as references in this application, just as each document is individually cited as a reference. In addition, it should be understood that, after reading the above teaching content of the present invention, those skilled in the art can make various changes or modifications to the present invention, and these equivalent forms also fall within the scope defined by the appended claims of the present application.

## Claims

1. A compound of formula I, or a stereoisomer or optical isomer, a pharmaceutically acceptable salt, a prodrug or a solvate thereof, 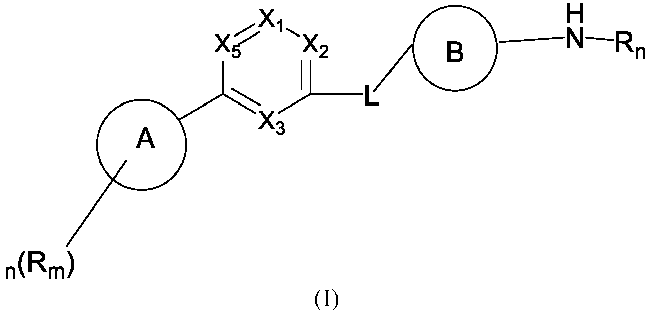 wherein,
X₁ is selected from N and CRₘ₁;
X₂ is selected from N and CRₘ₂;
X₃ is selected from N and CRₘ₃;
X₅ is selected from N and CR'ₘ₃;
L is selected from: bond, -O-, -S-, NRₘ₄-, -C(O)- and -C(O)NRₘ₄-;
ring A and ring B are each independently selected from: C3-C12 cycloalkyl, 3-12 membered heterocyclyl, C6-C10 aryl, and 5-12 membered heteroaryl; wherein the C3-C12 cycloalkyl, 3-12 membered heterocyclyl, C6-C10 aryl, or 5-12 membered heteroaryl is optionally substituted with 1 to 3 R;
each Rₘ is independently selected from: H, D, halogen, cyano, hydroxyl, carboxyl, - S(O)ₜRₘ₅, -C(O)Rₘ₅, -C(O)ORₘ₅, -C(O)NRₘ₆Rₘ₇, -S(O)ₜNRₘ₆Rₘ₇, -C(O)NRₘ₈S(O)ₜNRₘ₆Rₘ₇, -(CH₂)_{q}S(O)ₜRₘ₅, -(CH₂)_{q}C(O)Rₘ₅, -(CH₂)_{q}C(O)ORₘ₅, -(CH₂)_{q}C(O)NRₘ₆Rₘ₇, -(CH₂)_{q}(CH₂)_{q}S (O)ₜNRₘ₆Rₘ₇, -C(O)NRₘ₈S(O)ₜNRₘ₆Rₘ₇, -(CH₂)_{q}Rₘ₉, -C1-C6 alkyl, -C1-C6 alkoxy, -C1-C6 alkylamino, C3-C12 cycloalkyl, C2-C6 alkenyl, C2-C6 alkynyl, 3-12 membered heterocyclyl, C6-C10 aryl and 5-12 membered heteroaryl; wherein the C1-C6 alkyl, C1-C6 alkoxy, C3-C12 cycloalkyl, 3-12 membered heterocyclyl, C6-C10 aryl or 5-12 membered heteroaryl is optionally substituted with 1 to 3 R;
alternatively, two Rₘ located on adjacent ring atoms together with their adjacent ring atoms form a C3-C12 cycloalkyl or a 3-12 membered heterocyclyl, wherein the C3-C12 cycloalkyl or 3-12 membered heterocyclyl is optionally substituted with 1 to 3 R;
Rₙ is selected from: H, C1-C15 alkyl, C2-C15 alkenyl, C2-C15 alkynyl, and C(O)Rₚ; wherein, Rₚ is selected from: NH₂, C1-C15 alkyl, C1-C15 alkoxy, C1-C15 alkylamino, C2-C15 alkenyl, C2-C15 alkynyl, C3-C12 cycloalkyl, 3-12 membered heterocyclyl, C6-C10 aryl and 5-12 membered heteroaryl; wherein the C1-C15 alkyl, C1-C15 alkoxy, C1-C15 alkylamino, C3-C12 cycloalkyl, 3-12 membered heterocyclyl, C6-C10 aryl or 5-12 membered heteroaryl is optionally substituted with 1 to 3 R;
Rₘ₁, Rₘ₂, Rₘ₃, and R'ₘ₃ are each independently selected from: H, cyano, halogen, C(O)NH₂, carboxyl, S(O)₂NH₂, C(O)OC1-C6 alkyl and S(O)₂C1-C6 alkyl;
Rₘ₄ is selected from: H and C1-C6 alkyl;
Rₘ₅ is selected from: C1-C6 alkyl, C1-C6 alkoxy, C1-C6 alkylamino, C3-C12 cycloalkyl, 3-12 membered heterocyclyl, C6-C10 aryl and 5-12 membered heteroaryl; wherein the C1-C6 alkyl, C1-C6 alkoxy, C3-C12 cycloalkyl, 3-12 membered heterocyclyl, C6-C10 aryl or 5-12 membered heteroaryl is optionally substituted with 1 to 3 R;
Rₘ₆, Rₘ₇, Rₘ₈, and Rₘ₉ are each independently selected from: H, C1-C6 alkyl, C1-C6 alkoxy, C1-C6 alkylamino, C3-C12 cycloalkyl, 3-12 membered heterocyclyl, C6-C10 aryl, and 5-12 membered heteroaryl; or, Rₘ₆ and Rₘ₇ together with the N atom to which they are adjacent form a 3-12 membered heterocyclyl; wherein the C1-C6 alkyl, C1-C6 alkoxy, C3-C12 cycloalkyl, 3-12 membered heterocyclyl, C6-C10 aryl, or 5-12 membered heteroaryl is optionally substituted with 1-3 R;
the H atoms in (CH₂)_{q} are optionally substituted with R;
R is selected from: H, D, halogen, cyano, hydroxyl, carboxyl, NH₂, oxo- (=O), C1-C6 alkyl, C1-C6 alkoxy, C1-C6 alkylamino, C(O)OC1-C6 alkyl, S(O)₂C1-C6 alkyl, C2-C6 alkenyl, C2-C6 alkynyl, C3-C12 cycloalkyl, 3-12 membered heterocyclyl, C6-C10 aryl and 5-12 membered heteroaryl; wherein the C1-C6 alkyl, C1-C6 alkoxy, C3-C12 cycloalkyl, 3-12 membered heterocyclyl, C6-C10 aryl or 5-12 membered heteroaryl is optionally substituted with 1 to 3 substituents selected from the group consisting of: halogen, cyano, hydroxyl, carboxyl, C1-C6 alkyl, C1-C6 alkoxy, C1-C6 alkylamino, C(O)OC1-C6 alkyl, and S(O)₂C1-C6 alkyl;
t is 0, 1 or 2;
q is 1, 2, 3, 4, 5 or 6; and
n is 1, 2, 3, 4, 5 or 6.

2. The compound of claim 1, or the stereoisomer or optical isomer, the pharmaceutically acceptable salt, the prodrug or the solvate thereof, wherein the compound has a structure of formula II 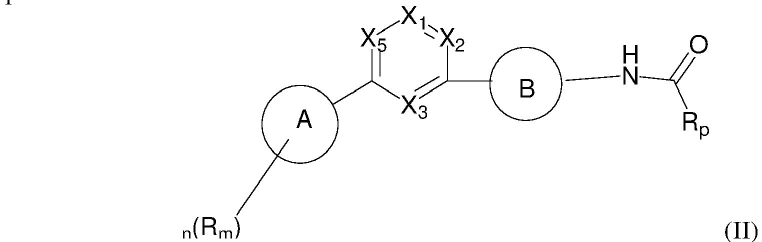
wherein, X₁, X₂, X₃, X₅, ring A, ring B, Rₘ, Rₚ, and n are as defined in claim 1;
particularly, X₁, X₂, X₃, X₅, ring A, ring B, and Rₘ are as defined in claim 8; n is as defined in claim 1; 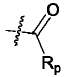 is defined as Rn of claim 8;
more particularly, 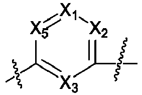 is as defined in claim 8.

3. The compound of claim 1, or the stereoisomer or optical isomer, the pharmaceutically acceptable salt, the prodrug or the solvate thereof, wherein the compound has a structure of formula III 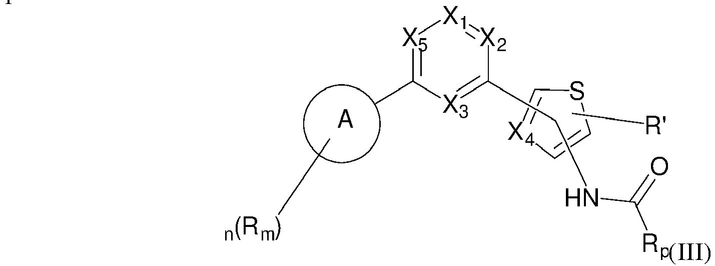
wherein, X₄ is selected from: N and CRₘ₁₀; wherein Rₘ₁₀ is selected from: H, C1-C6 alkyl, C1-C6 alkoxy, C1-C6 alkylamino, C3-C12 cycloalkyl, 3-12 membered heterocyclyl, C6-C10 aryl and 5-12 membered heteroaryl; or, Rₘ₆ and Rₘ₇ together with the N atom to which they are adjacent form a 3-12 membered heterocyclyl; wherein the C1-C6 alkyl, C1-C6 alkoxy, C3-C12 cycloalkyl, 3-12 membered heterocyclyl, C6-C10 aryl, or 5-12 membered heteroaryl is optionally substituted with 1 to 3 R;
R' is selected from: H, D, halogen, cyano, hydroxyl, carboxyl, NH₂, C1-C6 alkyl, C1-C6 alkoxy, C1-C6 alkylamino, preferably is H;
R, X₁, X₂, X₃, X₅, ring A, Rₘ, Rₚ, and n are as defined in claim 1;
particularly, X₁, X₂, X₃, X₅, ring A, and Rₘ are as defined in claim 8; n is as defined in claim 1; 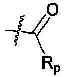 is defined as Rn of claim 8;
more particularly, 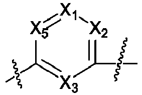 is as defined in claim 8.

4. The compound of any one of claims 1 to 3, or the stereoisomer or optical isomer, the pharmaceutically acceptable salt, the prodrug or the solvate thereof, wherein, the compound has a structure of formula IV 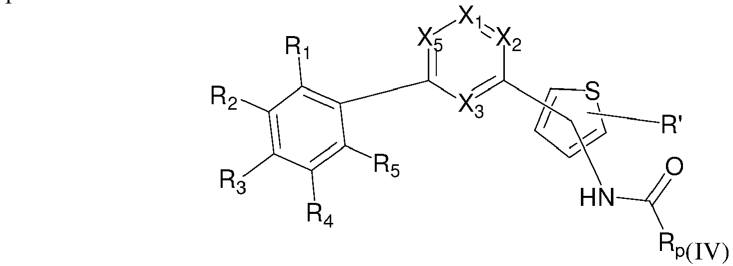 wherein,
R₁, R₂, R₃, R₄, and R₅ are each independently selected from: H, D, halogen, cyano, hydroxyl, carboxyl, S(O)ₜRₘ₅, C(O)Rₘ₅, C(O)ORₘ₅, C(O)NRₘ₆Rₘ₇, S(O)ₜNRₘ₆Rₘ₇, C(O)NRₘ₈S(O)ₜNRₘ₆Rₘ₇, (CH₂)_{q}S(O)ₜRₘ₅, (CH₂)_{q}C(O)Rₘ₅, (CH₂)_{q}C(O)ORₘ₅, (CH₂)_{q}C(O)NRₘ₆Rₘ₇, (CH₂)_{q}(CH₂)_{q}S(O)ₜNRₘ₆Rₘ₇, C(O)NRₘ₈S(O)ₜNRₘ₆Rₘ₇, (CH₂)_{q}Rₘ₉, C1-C6 alkyl, C1-C6 alkoxy, C1-C6 alkylamino, C3-C12 cycloalkyl, 3-12 membered heterocyclyl, C6-C10 aryl and 5-12 membered heteroaryl; wherein the C1-C6 alkyl, C1-C6 alkoxy, C3-C12 cycloalkyl, 3-12 membered heterocyclyl, C6-C10 aryl or 5-12 membered heteroaryl is optionally substituted with 1 to 3 R;
or, R₁ and R₂, or R₂ and R₃, or R₃ and R₄, or R₄ and R₅ together with the C atoms to which they are adjacent form a 5-6-membered heterocyclyl, wherein the 5-6-membered heterocyclyl is optionally substituted with 1 to 3 R;
R' is selected from: H, D, halogen, cyano, hydroxyl, carboxyl, NH₂, C1-C6 alkyl, C1-C6 alkoxy, C1-C6 alkylamino, preferably is H;
t, q, R, Rₘ₅, Rₘ₆, Rₘ₇, Rₘ₈, Rₘ₉, X₁, X₂, X₃, X₅ and Rₚ are as defined in claim 1.
particularly, X₁, X₂, X₃, and X₅ are as defined in claim 8; R₁, R₂, R₃, R₄, and R₅ are defined as Rm of claim 8; 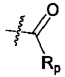 is defined as Rn of claim 8;
more particularly, 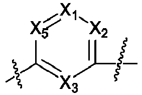 is as defined in claim 8.

5. The compound of any one of claims 1 to 4, or the stereoisomer or optical isomer, the pharmaceutically acceptable salt, the prodrug or the solvate thereof, wherein the compound has a structure of formula A 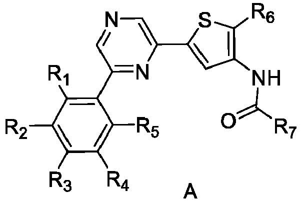 wherein
R₆ is selected from: H, D, halogen, cyano, hydroxyl, carboxyl, NH₂, C1-C6 alkyl, C1-C6 alkoxy, C1-C6 alkylamino, preferably is H;
R₇ is selected from: C1-C15 alkyl, C1-C15 alkoxy, C1-C15 alkylamino, C3-C12 cycloalkyl, 3-12 membered heterocyclyl, C6-C10 aryl and 5-12 membered heteroaryl; wherein the C1-C15 alkyl, C1-C15 alkoxy, C1-C15 alkylamino, C3-C12 cycloalkyl, 3-12 membered heterocyclyl, C6-C10 aryl is 5-12 membered heteroaryl is optionally substituted with 1 to 3 substituents selected from the group consisting of: D, halogen, cyano, hydroxyl, carboxyl, NH₂, oxo-(=O), C1-C6 alkyl, C1-C6 alkoxy, C1-C6 alkylamino, C(O)OC1-C6 alkyl, S(O)₂C1-C6 alkyl, C3-C12 cycloalkyl, 3-12 membered heterocyclyl, C6-C10 aryl and 5-12 membered heteroaryl;
R₁, R₂, R₃, R₄, and R₅ are as defined in claim 4;
particularly, R₁, R₂, R₃, R₄, and R₅ are defined as Rm of claim 8; 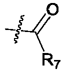 is defined as Rn of claim 8;

6. The compound of any one of claims 1 to 4, or the stereoisomer or optical isomer, the pharmaceutically acceptable salt, the prodrug or the solvate thereof, wherein the compound has a structure of formula B 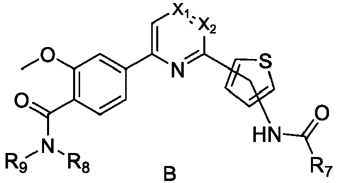 wherein
R₇ is selected from: C1-C15 alkyl, C1-C15 alkoxy, C1-C15 alkylamino, C3-C12 cycloalkyl, 3-12 membered heterocyclyl, C6-C10 aryl and 5-12 membered heteroaryl; wherein the C1-C15 alkyl, C1-C15 alkoxy, C1-C15 alkylamino, C3-C12 cycloalkyl, 3-12 membered heterocyclyl, C6-C10 aryl is 5-12 membered heteroaryl is optionally substituted with 1 to 3 substituents selected from the group consisting of: D, halogen, cyano, hydroxyl, carboxyl, NH₂, oxo-(=O), C1-C6 alkyl, C1-C6 alkoxy, C1-C6 alkylamino, C(O)OC1-C6 alkyl, S(O)₂C1-C6 alkyl, C3-C12 cycloalkyl, 3-12 membered heterocyclyl, C6-C10 aryl and 5-12 membered heteroaryl;
R₈ and R₉ are each independently selected from: H, S(O)₂NR₁₁R₁₂, C1-C6 alkyl, C1-C6 alkoxy, C1-C6 alkylamino, C3-C12 cycloalkyl, 3-12 membered heterocyclyl, C6-C10 aryl, and 5-12 membered heteroaryl; or, R₈ and R₉ together with the N atom to which they are adjacent form a 3-12 membered heterocyclyl, preferably 5-6 membered heterocyclyl; wherein the C1-C6 alkyl, C1-C6 alkoxy, C3-C12 cycloalkyl, 3-12 membered heterocyclyl, 5-6 membered heterocyclyl, C6-C10 aryl, or 5-12 membered heteroaryl is optionally substituted with 1 to 3 substituents selected from the group consisting of: C1-C6 alkyl, C1-C6 alkoxy, C1-C6 alkylamino, C(O)OC1-C6 alkyl, S(O)₂C1-C6 alkyl, C3-C12 cycloalkyl, C1-C6 alkyl-C3-C12 cycloalkyl, 3-12 membered heterocyclyl, C1-C6 alkyl-3-12 membered heterocyclyl, C6-C10 aryl and 5-12 membered heteroaryl;
R₁₁ and R₁₂ are each independently selected from: H, C1-C6 alkyl, C3-C12 cycloalkyl, 3-12 membered heterocyclyl, C6-C10 aryl and 5-12 membered heteroaryl;
particularly,
X₁ and X₂ are as defined in claim 8; 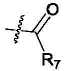 is defined as Rn of claim 8; 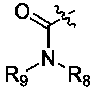 is formamide,

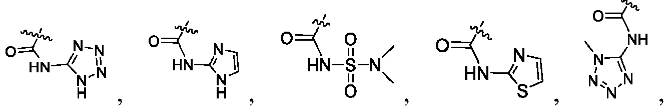

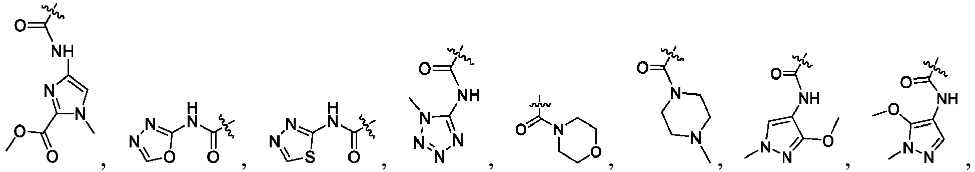

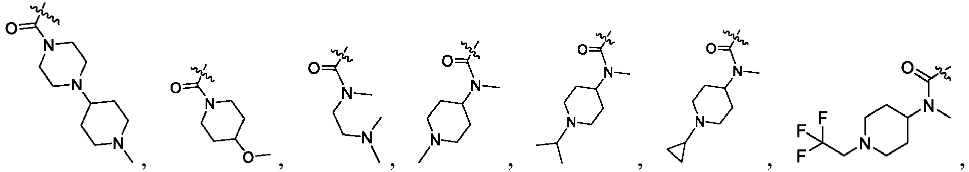

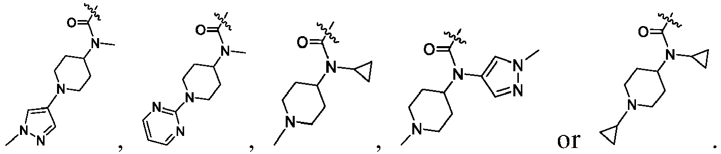

7. The compound of any one of claims 1 to 4, or the stereoisomer or optical isomer, the pharmaceutically acceptable salt, the prodrug or the solvate thereof, wherein, the compound has a structure of formula C 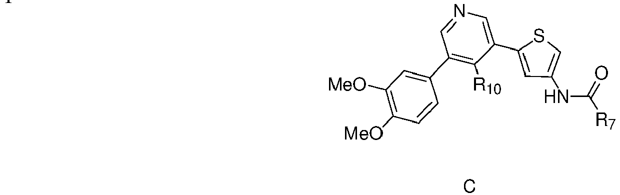 wherein,
R₁₀ is selected from: H, cyano, and CONH₂;
R₇ is selected from: C1-C15 alkyl, C1-C15 alkoxy, C1-C15 alkylamino, C3-C12 cycloalkyl, 3-12 membered heterocyclyl, C6-C10 aryl and 5-12 membered heteroaryl; wherein the C1-C15 alkyl, C1-C15 alkoxy, C1-C15 alkylamino, C3-C12 cycloalkyl, 3-12 membered heterocyclyl, C6-C10 aryl or 5-12 membered heteroaryl is optionally substituted with 1 to 3 substituents selected from the group consisting of: D, halogen, cyano, hydroxyl, carboxyl, NH₂, oxo-(=O), C1-C6 alkyl, C1-C6 alkoxy, C1-C6 alkylamino, C(O)OC1-C6 alkyl, S(O)₂C1-C6 alkyl, C3-C12 cycloalkyl, 3-12 membered heterocyclyl, C6-C10 aryl and 5-12 membered heteroaryl;
particularly, 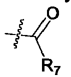 is defined as Rn of claim 8.

8. The compound of claim 1, or the stereoisomer or optical isomer, the pharmaceutically acceptable salt, the prodrug or the solvate thereof, wherein the compound satisfies one or more of the following conditions,
(1) X₁ is N or CH;
(2) X₂ is N or CH;
(3) X₃ is N, C-C(=O)NH₂ or C-CN;
(4) X₅ is N or CH;
(5) L is bond or NH;
(6) ring A is C6-C10 aryl, or 3-12 membered heterocyclyl; wherein the 3-12 membered heterocyclyl is preferably 5-7 membered heterocyclyl, and more preferably 6-membered heterocyclyl; preferably, ring A is phenyl, 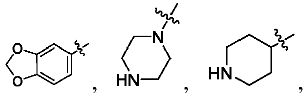 or 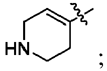 the substituents on ring A is defined as Rₘ of claim 1;
(7) ring B is 5-12 membered heteroaryl or C3-C12 cycloalkyl, preferably 5-membered heteroaryl or 6-membered cycloalkyl; preferably, ring B is 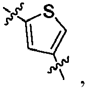 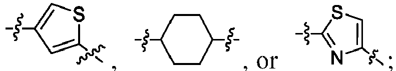 the substituents on ring B is defined as R of claim 1;
(8) Rₘ is methoxy, F, Cl, cyano, hydroxyl, carboxyl, formamide, 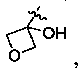 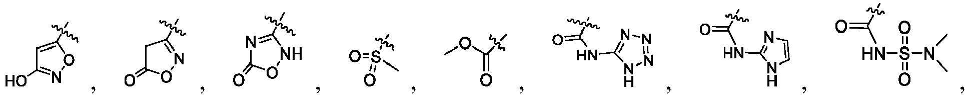 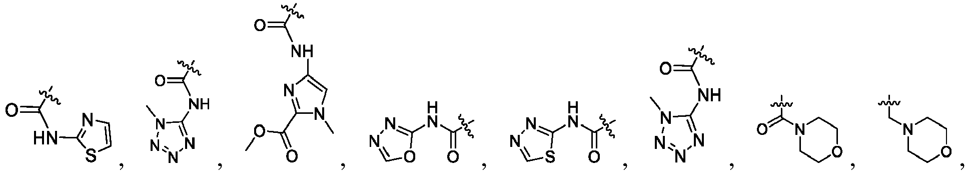 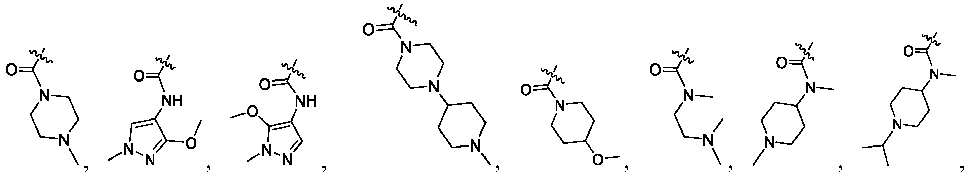 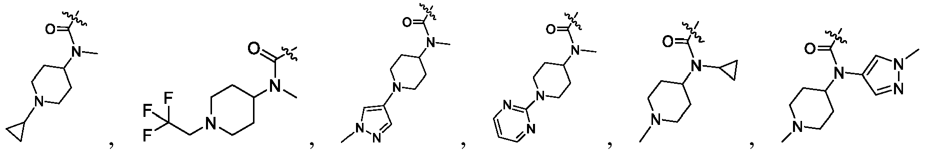 or 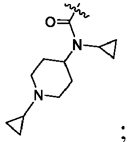
(9) Rₙ is 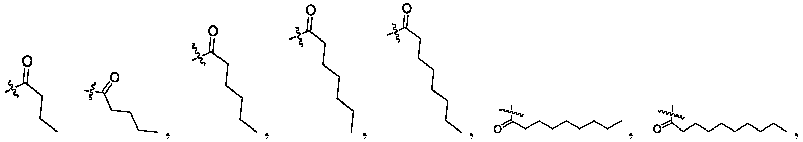 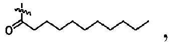 acetyl, isobutyryl, 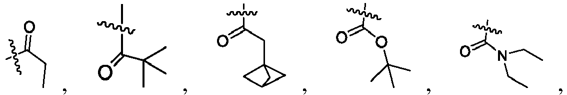 cyclopropylcarbonyl, cyclobutylcarbonyl, cyclopentyl carbonyl, cyclohexyl carbonyl, phenyl carbonyl, cyclopropyl methylene carbonyl, cyclobutyl methylene carbonyl, cyclopentyl methylene carbonyl, cyclohexyl methylene carbonyl, or 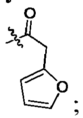 or Rₙ is H;
particularly,

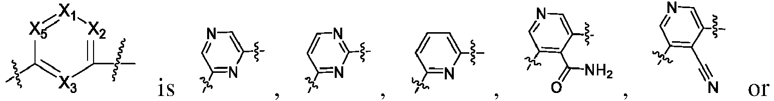

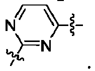

9. The compound of any one of claims 1 to 8, or the stereoisomer or optical isomer, the pharmaceutically acceptable salt, the prodrug or the solvate thereof, wherein Rn is selected from: C(O)Rₚ; wherein Rₚ is selected from: C1-C15 alkyl; wherein the C1-C15 alkyl is optionally substituted with 1, 2, or 3 R; R is selected from: C3-C12 cycloalkyl; wherein the C3-C12 cycloalkyl is optionally substituted with 1, 2, or 3 substituents selected from the group consisting of: C1-C6 alkyl, halogenated C1-C6 alkyl, C6-C10 aryl substituted with halogenated C1-C6 alkyl, halogenated C1-C6 alkoxy, C6-C10 aryl substituted with halogenated C1-C6 alkoxy, and C(O)C1-C6 alkyl;
or, Rn is selected from: ORₚ; wherein Rₚ is selected from: C1-C15 alkyl; wherein C1-C15 alkyl is optionally substituted with 1, 2, or 3 R; R is selected from: C1-C15 alkylamino; the C1-C15 alkylamino is optionally substituted with 1, 2, or 3 substituents selected from the group consisting of: halogen, cyano, hydroxyl, carboxyl, C1-C6 alkyl, halogenated C1-C6 alkyl, C6-C10 aryl substituted with halogenated C1-C6 alkyl, halogenated C1-C6 alkoxy, C6-C10 aryl substituted with halogenated C1-C6 alkoxy, C(O)C1-C6 alkyl, C1-C6 alkoxy, C1-C6 alkylamino, C(O)OC1-C6 alkyl, and S(O)₂C1-C6 alkyl;
or, 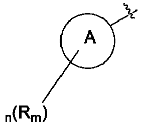 is selected from 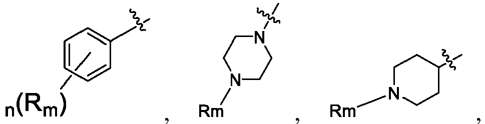 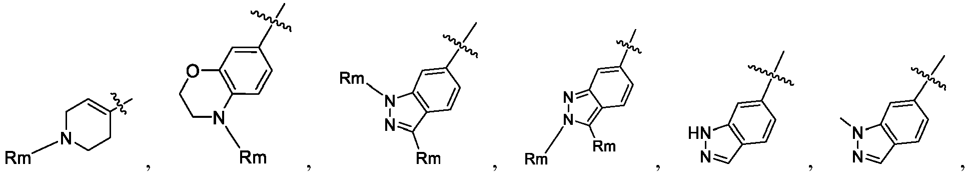 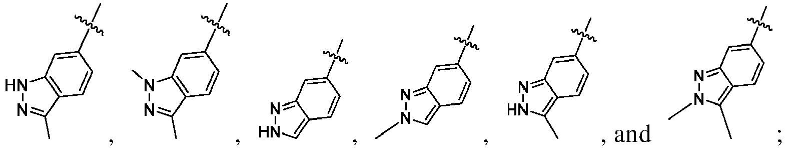 wherein Rₘ is as defined in claim 1;
or, ring B selected from 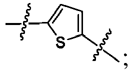 the substituents on ring B is defined as R of claim 1;
or, Rm is selected from: 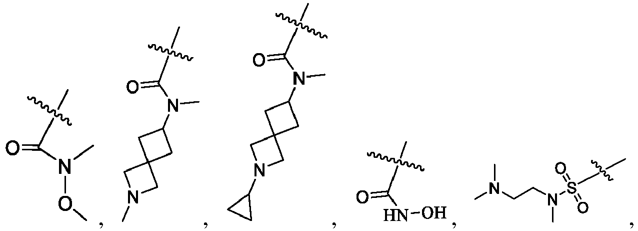 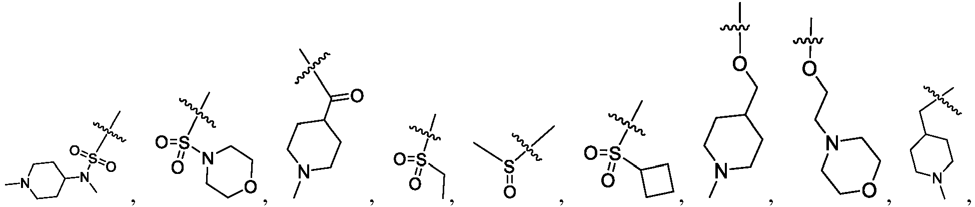 methyl and 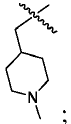
or Rn is selected from: 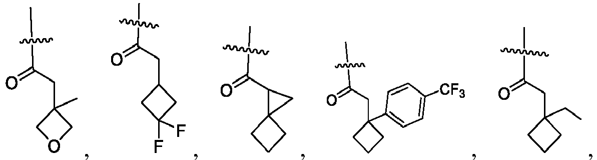 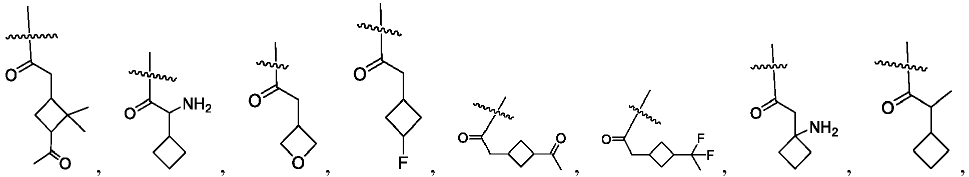 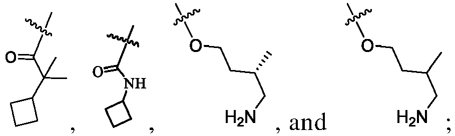
or, n is 0.

10. A compound of formula I', or a stereoisomer or optical isomer, a pharmaceutically acceptable salt, a prodrug or a solvate thereof, 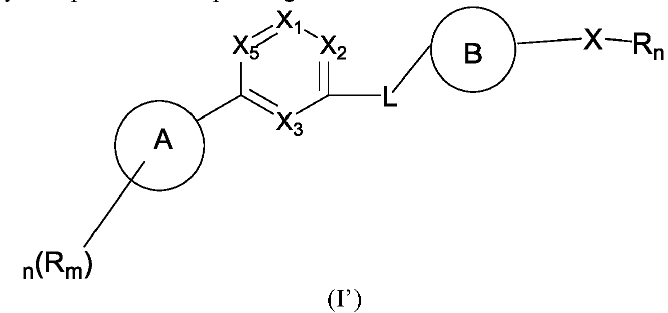 wherein,
X is selected from O and CH₂;
X₁ is selected from N and CRₘ₁;
X₂ is selected from N and CRₘ₂;
X₃ is selected from N and CRₘ₃;
X₅ is selected from N and CR'ₘ₃;
L is selected from: bond, -O-, -S-, NRₘ₄-, -C(O)- and -C(O)NRₘ₄-;
ring A and ring B are each independently selected from: C3-C12 cycloalkyl, 3-12 membered heterocyclyl, C6-C10 aryl, and 5-12 membered heteroaryl; wherein the C3-C12 cycloalkyl, 3-12 membered heterocyclyl, C6-C10 aryl, and 5-12 membered heteroaryl are optionally substituted with 1-3 R;
each Rₘ is independently selected from: H, D, halogen, cyano, hydroxyl, carboxyl, - S(O)ₜRₘ₅, -C(O)Rₘ₅, -C(O)ORₘ₅, -C(O)NRₘ₆Rₘ₇, -S(O)ₜNRₘ₆Rₘ₇, -C(O)NRₘ₈S(O)ₜNRₘ₆Rₘ₇, -(CH₂)_{q}S(O)ₜRₘ₅, -(CH₂)_{q}C(O)Rₘ₅, -(CH₂)_{q}C(O)ORₘ₅, -(CH₂)_{q}C(O)NRₘ₆Rₘ₇, -(CH₂)_{q}(CH₂)_{q}S(O)ₜNRₘ₆Rₘ₇, -C(O)NRₘ₈S(O)ₜNRₘ₆Rₘ₇, -(CH₂)_{q}Rₘ₉, -C1-C6 alkyl, -C1-C6 alkoxy, -C1-C6 alkylamino, C3-C12 cycloalkyl, C2-C6 alkenyl, C2-C6 alkynyl, 3-12 membered heterocyclyl, C6-C10 aryl and 5-12 membered heteroaryl; wherein the C1-C6 alkyl, C1-C6 alkoxy, C3-C12 cycloalkyl, 3-12 membered heterocyclyl, C6-C10 aryl or 5-12 membered heteroaryl is optionally substituted with 1 to 3 R;
or, two Rₘ on adjacent ring atoms together with the ring atoms to which they are adjacent form a C3-C12 cycloalkyl or a 3-12 membered heterocyclyl, wherein the C3-C12 cycloalkyl or 3-12 membered heterocyclyl is optionally substituted with 1 to 3 R;
Rₙ is selected from: H, C1-C15 alkyl, C2-C15 alkenyl, C2-C15 alkynyl, and C(O)Rₚ; wherein Rₚ is selected from: NH₂, C1-C15 alkyl, C1-C15 alkoxy, C1-C15 alkylamino, C2-C15 alkenyl, C2-C15 alkynyl, C3-C12 cycloalkyl, 3-12 membered heterocyclyl, C6-C10 aryl and 5-12 membered heteroaryl; wherein the C1-C15 alkyl, C1-C15 alkoxy, C1-C15 alkylamino, C3-C12 cycloalkyl, 3-12 membered heterocyclyl, C6-C10 aryl or 5-12 membered heteroaryl is optionally substituted with 1-3 R;
Rₘ₁, Rₘ₂, Rₘ₃, and R'ₘ₃ are each independently selected from: H, cyano, halogen, C(O)NH₂, carboxyl, S(O)₂NH₂, C(O)OC1-C6 alkyl and S(O)₂C1-C6 alkyl;
Rₘ₄ is selected from: H and C1-C6 alkyl;
Rₘ₅ is selected from: C1-C6 alkyl, C1-C6 alkoxy, C1-C6 alkylamino, C3-C12 cycloalkyl, 3-12 membered heterocyclyl, C6-C10 aryl and 5-12 membered heteroaryl; wherein the C1-C6 alkyl, C1-C6 alkoxy, C3-C12 cycloalkyl, 3-12 membered heterocyclyl, C6-C10 aryl or 5-12 membered heteroaryl is optionally substituted with 1 to 3 R;
Rₘ₆, Rₘ₇, Rₘ₈, and Rₘ₉ are each independently selected from: H, C1-C6 alkyl, C1-C6 alkoxy, C1-C6 alkylamino, C3-C12 cycloalkyl, 3-12 membered heterocyclyl, C6-C10 aryl, and 5-12 membered heteroaryl; or, Rₘ₆ and Rₘ₇ together with the N atom to which they are adjacent form a 3-12 membered heterocyclyl; wherein the C1-C6 alkyl, C1-C6 alkoxy, C3-C12 cycloalkyl, 3-12 membered heterocyclyl, C6-C10 aryl, or 5-12 membered heteroaryl is optionally substituted with 1 to 3 R;
the H atoms in (CH₂)_{q} are optionally substituted with R;
R is selected from: H, D, halogen, cyano, hydroxyl, carboxyl, NH₂, oxo- (=O), C1-C6 alkyl, C1-C6 alkoxy, C1-C6 alkylamino, C(O)OC1-C6 alkyl, S(O)₂C1-C6 alkyl, C2-C6 alkenyl, C2-C6 alkynyl, C3-C12 cycloalkyl, 3-12 membered heterocyclyl, C6-C10 aryl and 5-12 membered heteroaryl; wherein the C1-C6 alkyl, C1-C6 alkoxy, C3-C12 cycloalkyl, 3-12 membered heterocyclyl, C6-C10 aryl or 5-12 membered heteroaryl is optionally substituted with 1 to 3 substituents selected from the group consisting of: halogen, cyano, hydroxyl, carboxyl, C1-C6 alkyl, C1-C6 alkoxy, C1-C6 alkylamino, C(O)OC1-C6 alkyl, and S(O)₂C1-C6 alkyl;
t is 0, 1 or 2;
q is 1, 2, 3, 4, 5 or 6; and
n is 1, 2, 3, 4, 5 or 6.

11. The compound of claim 1, or the stereoisomer or optical isomer, the pharmaceutically acceptable salt, the prodrug or the solvate thereof, wherein the compound is selected from:

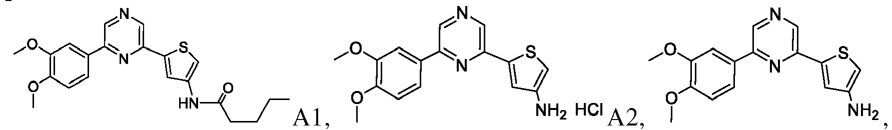

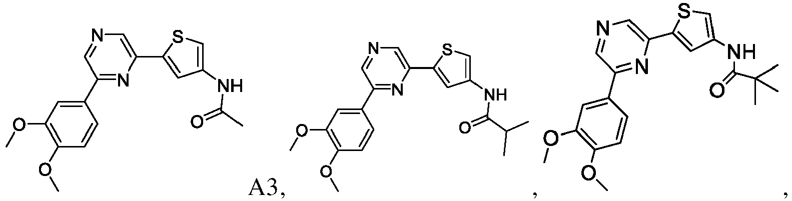

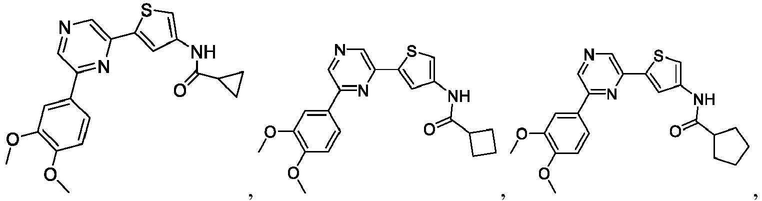

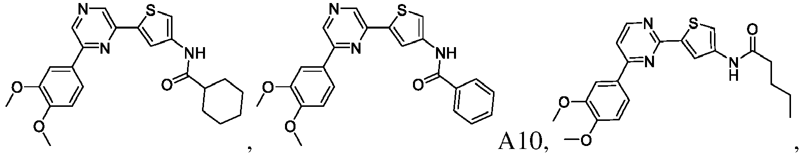

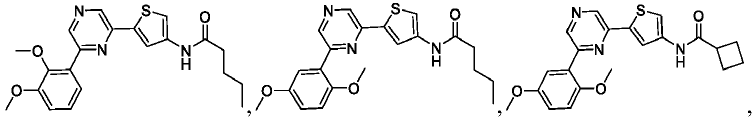

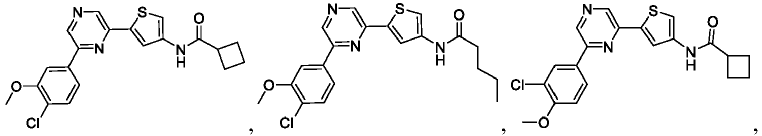

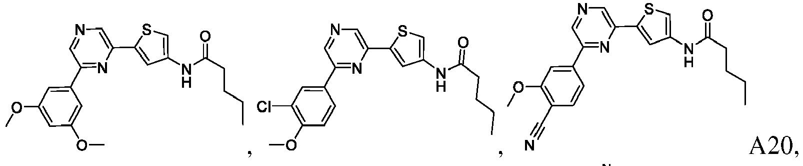

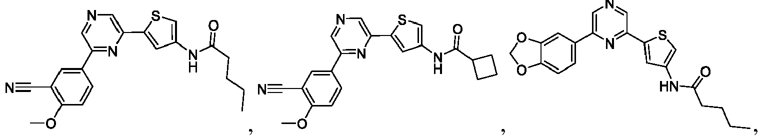

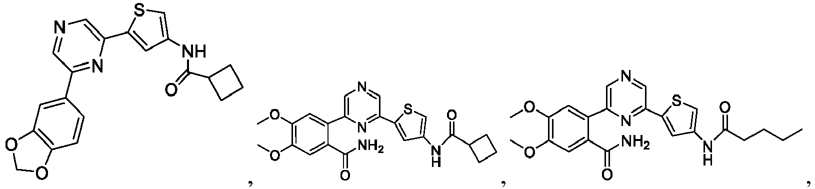

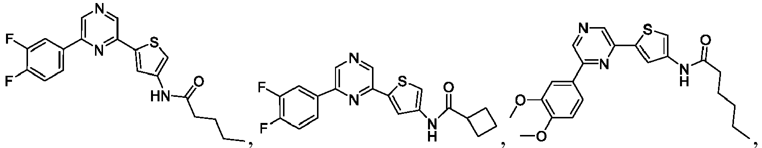

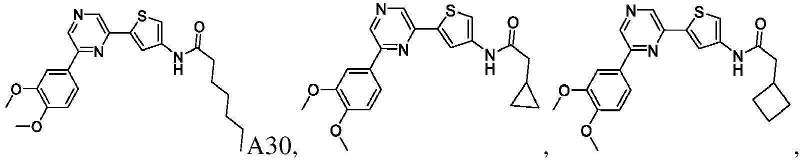

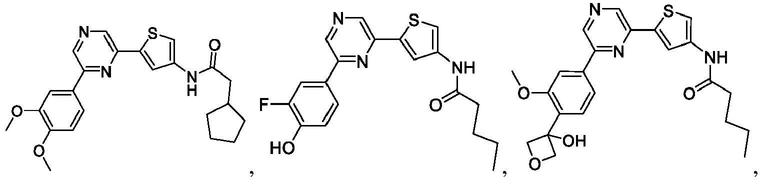

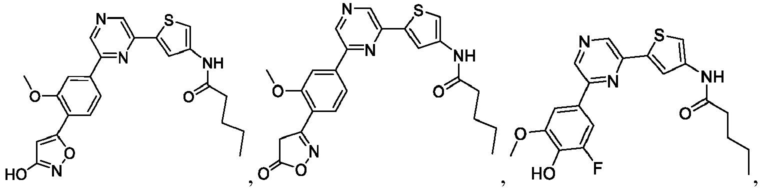

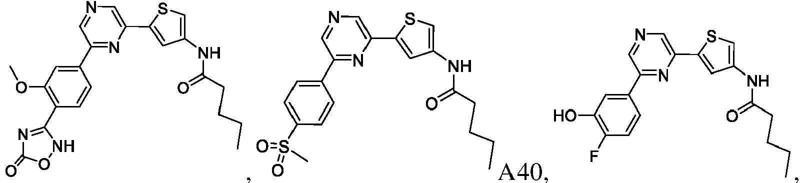

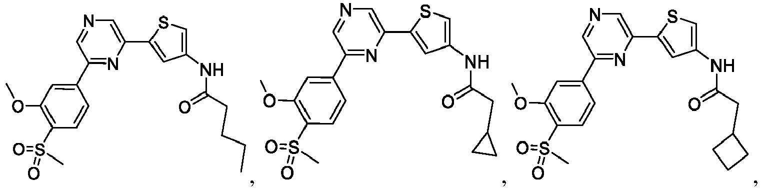

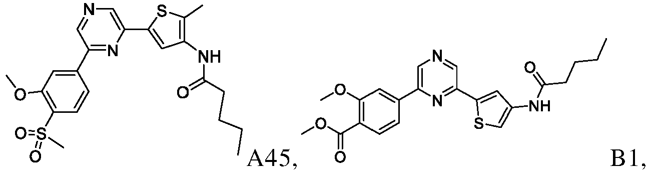

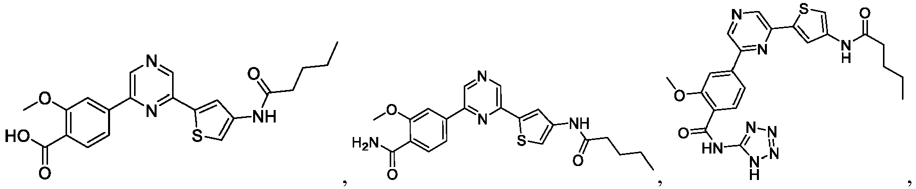

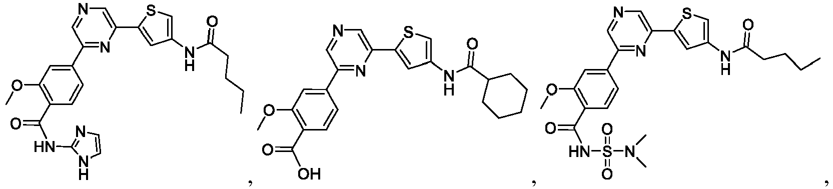

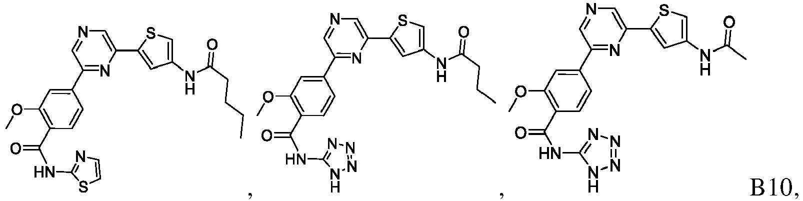

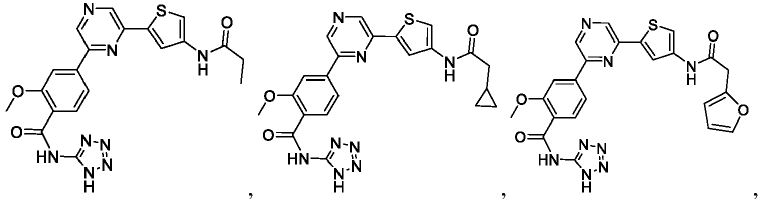

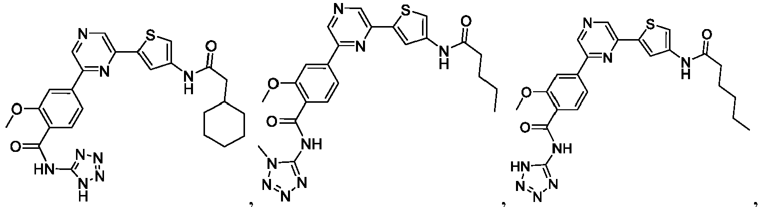

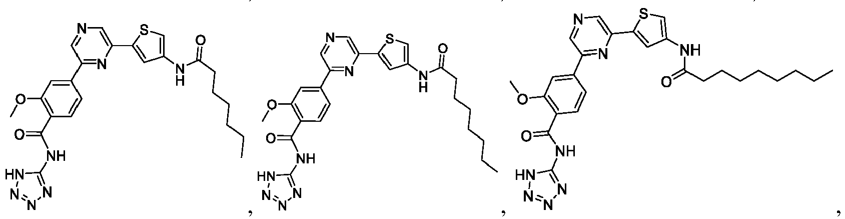

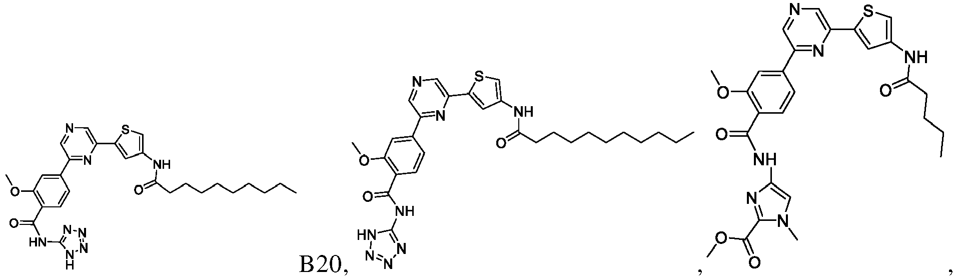

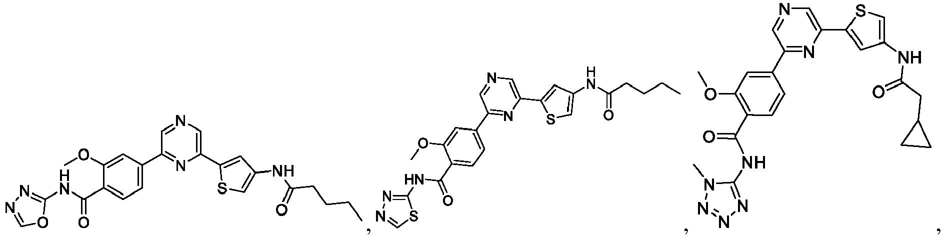

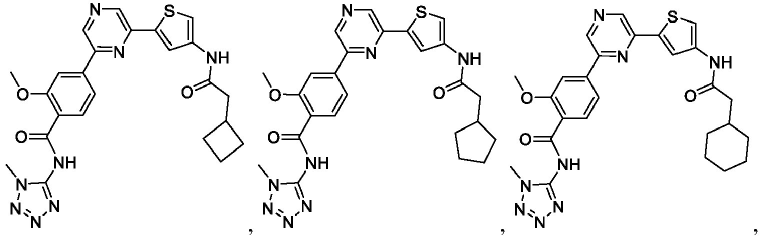

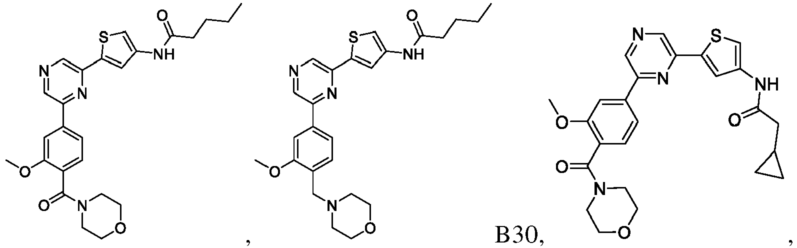

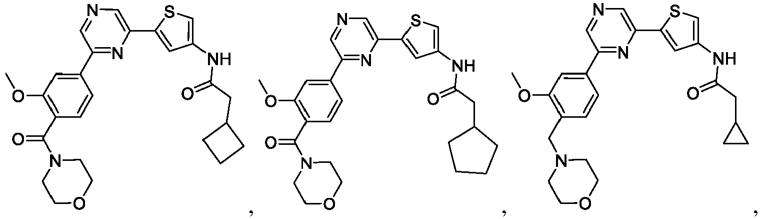

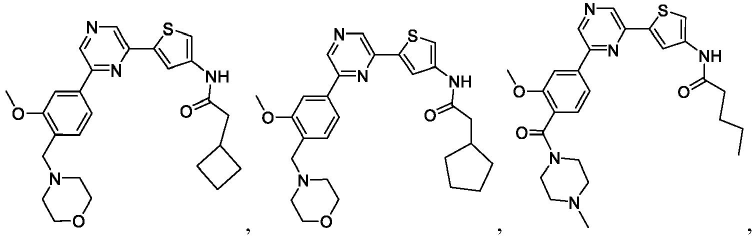

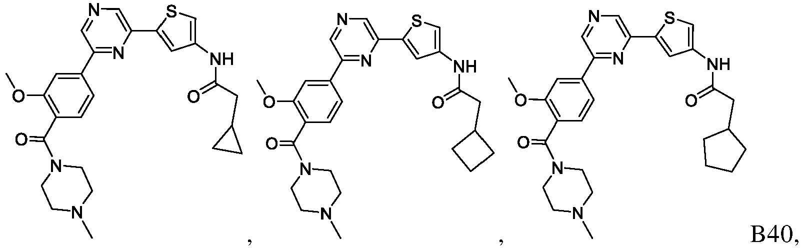

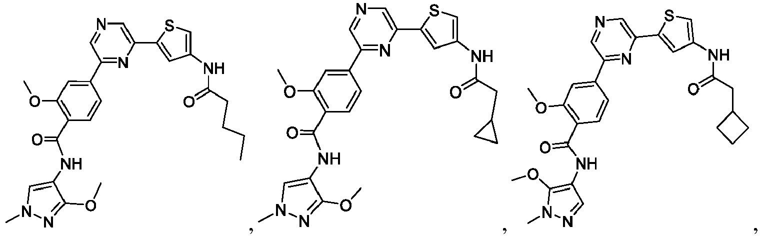

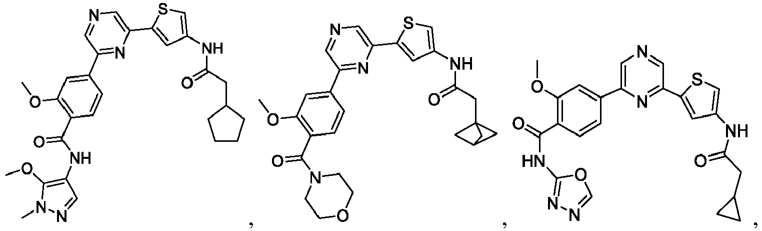

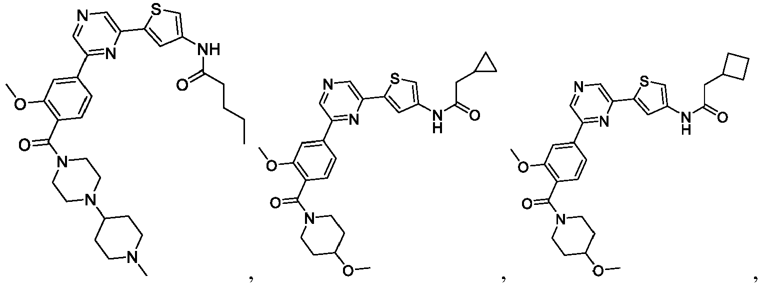

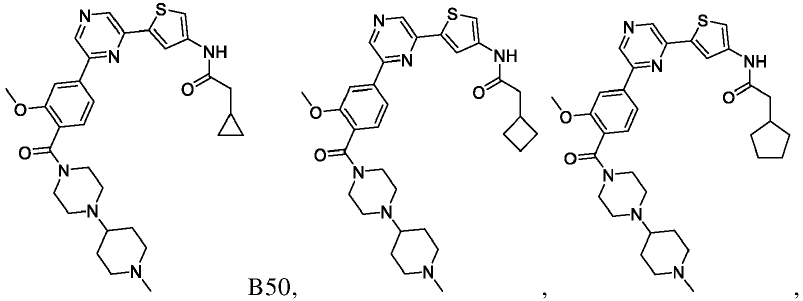

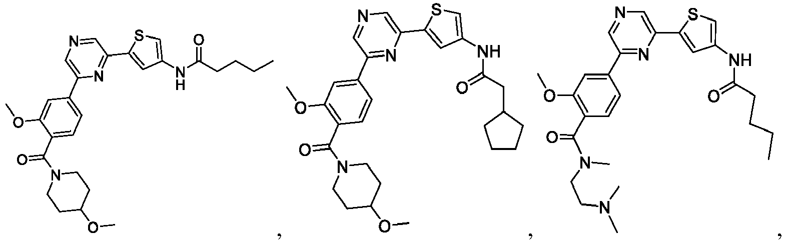

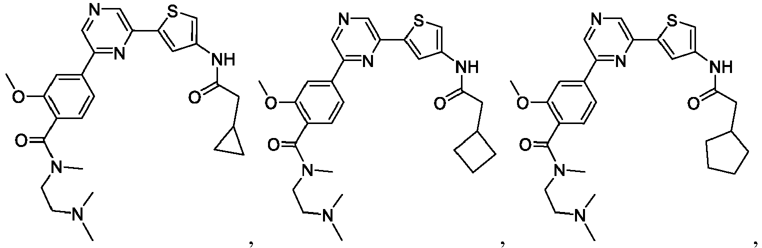

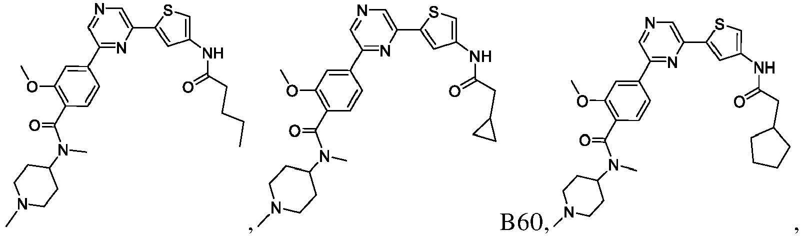

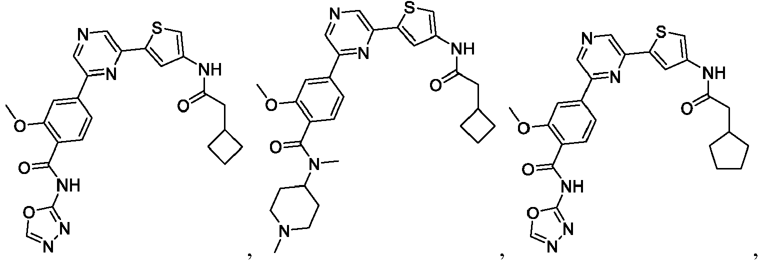

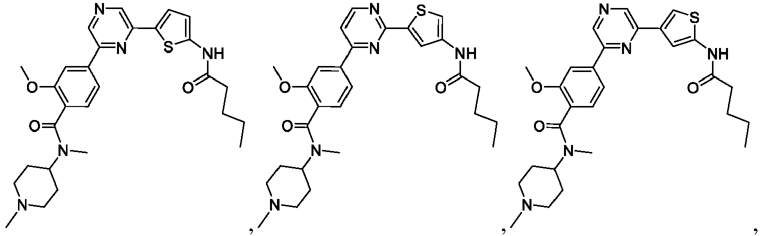

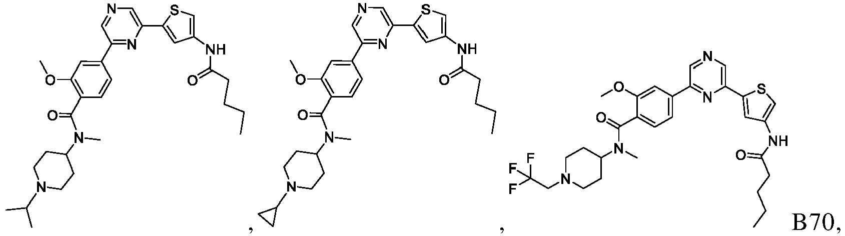

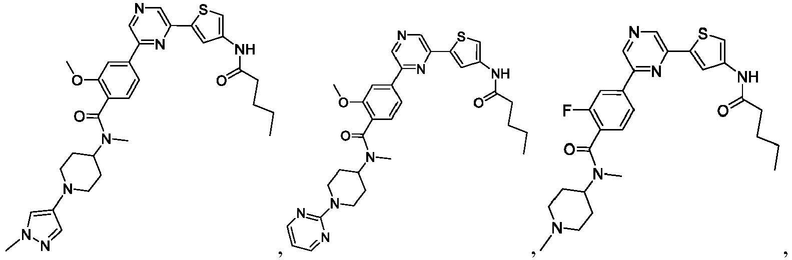

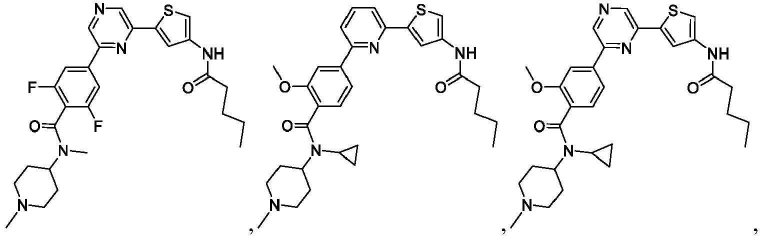

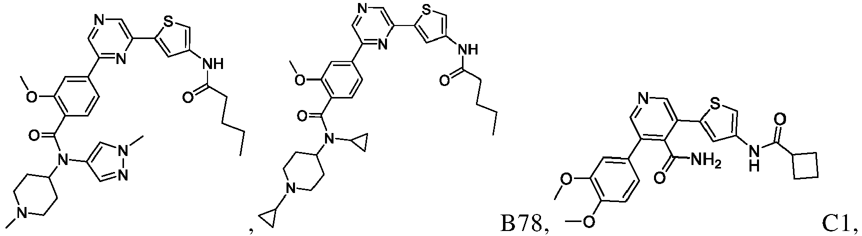

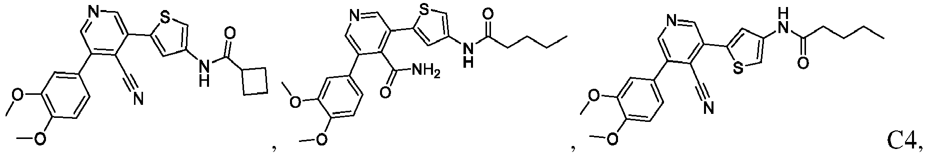

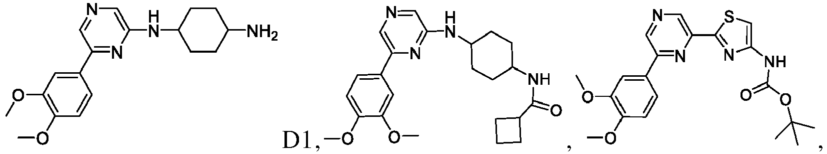

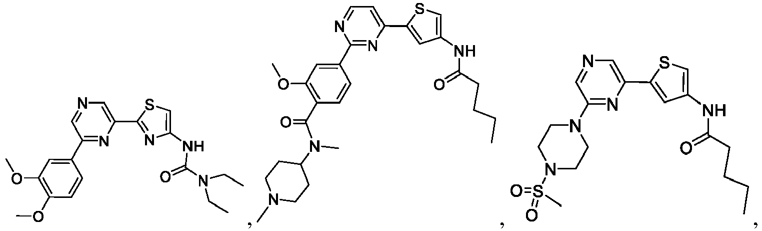

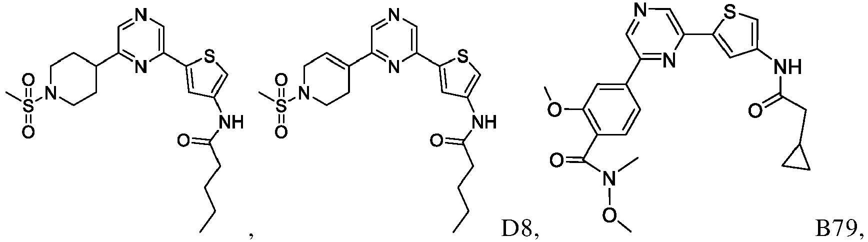

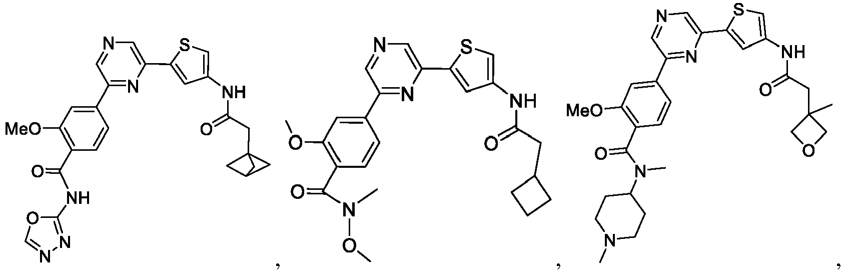

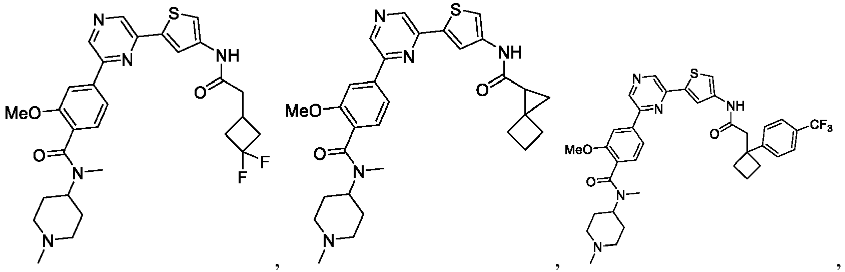

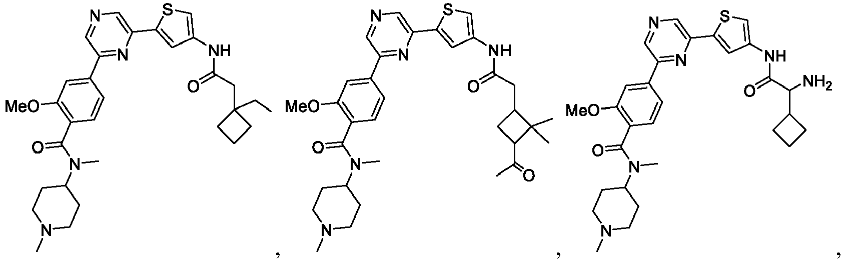

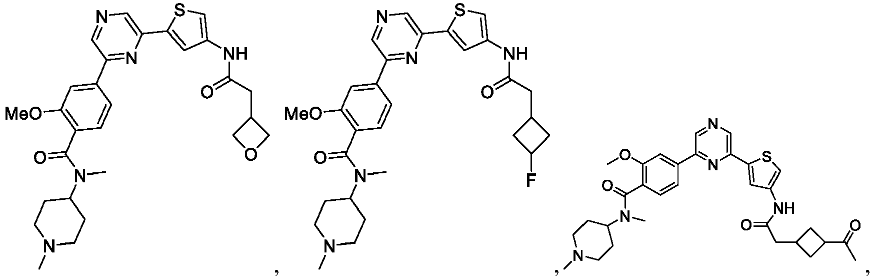

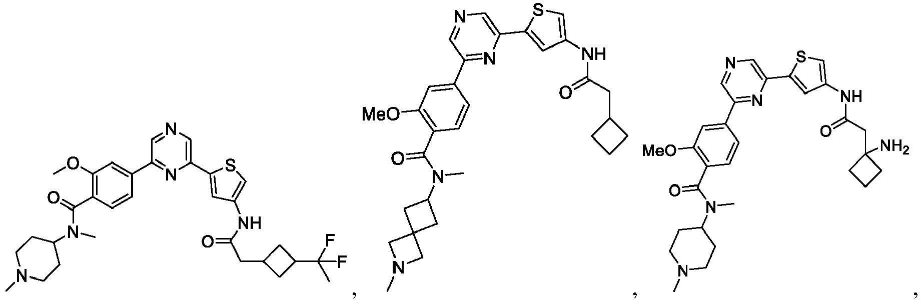

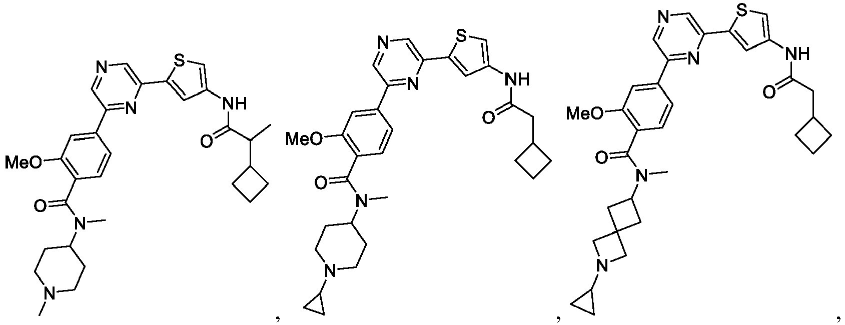

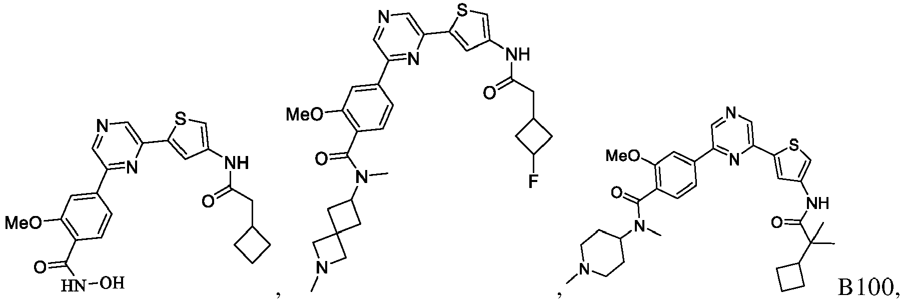

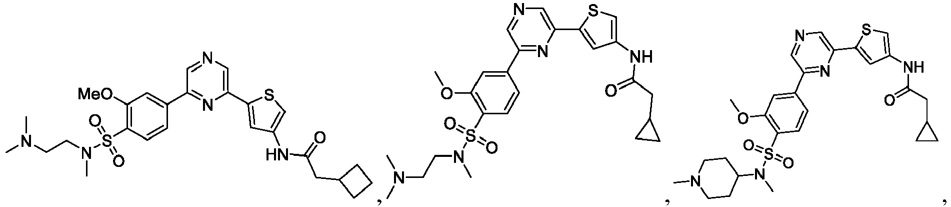

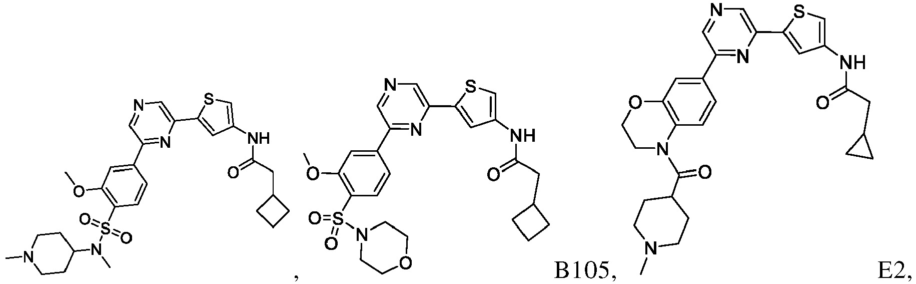

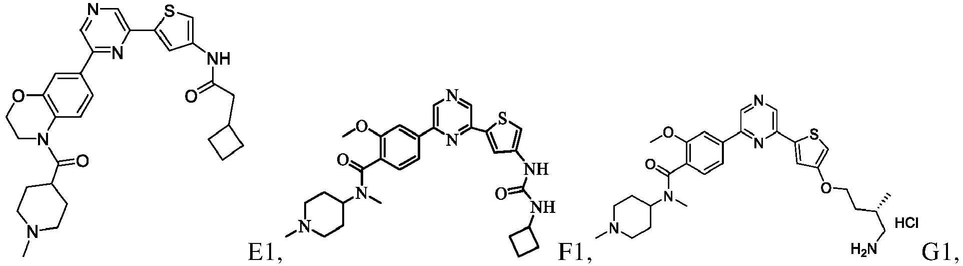

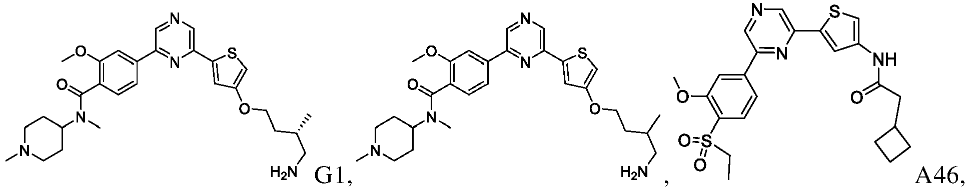

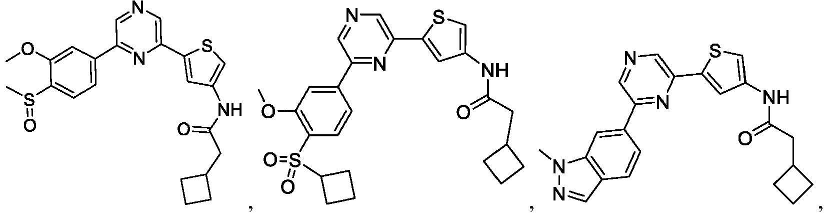

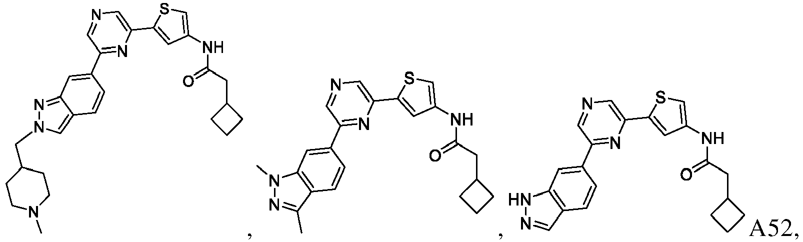

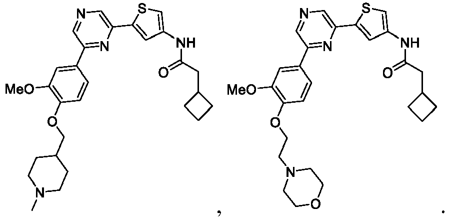

12. A pharmaceutical composition, comprising the compound of any one of claims 1 to 11, or the stereisomer or optical isomer, the pharmaceutically acceptable salt, the prodrug, or the solvate thereof; and pharmaceutically acceptable carriers.

13. A use of the compound of any one of claims 1 to 11, or the stereoisomer or optical isomer, the pharmaceutically acceptable salt, the prodrug or the solvate thereof, or the pharmaceutical composition of claim 12 in the preparation of a drug for reating or preventing a disease associated with the activity or expression of Drak2 kinase, or in the preparation of a drug for inhibiting the activity of Drak2 kinase.
preferably, the disease is cancer, autoimmune disease, or metabolic disease;
wherein, the cancer is preferably selected from: malignant lymphoma, acute myeloid leukemia, acute lymphocytic leukemia, chronic lymphocytic leukemia, chronic myeloid pseudomyxoma, intrahepatic cholangiocarcinoma, hepatoblastoma, liver cancer, thyroid cancer, colon cancer, testicular cancer, myelodyplastic syndrome, glioblastoma, basal cell carcinoma, breast cancer, brain cancer, adrenal cancer, renal cancer, nephroblastoma, stomach cancer, gastrointestinal stromal cancer, pityitary adenoma, pancreatic cancer, gallbladder cance, cholangiocarcinoma, colon cancer, rectal cancer, small intestine cancer, duodenal carcinoma, retinoblastoma, choroidal nelanoma, ampullary cancer, bladder cancer, peritoneal cancer, parathyroid carcinoma, non sinus cancer, small cell lung cancer, non small cell lung cancer, astrocytima, esophageal cancer, glioma, neuroblastoma, malignant soft tissue tumor, malignant bone tumor, malignant mesothelioma, malignant melanoma, eye cancer, vulvar cancer, ureteral cancer, urethral cancer, tumors with unknown primary sites, carcinoma of penis, oral cancer, lip cancer, pharyngeal cancer, epithelial ovarian cancer, ovarian gernital carcinoma, cervical cancer, endometrial cancer, uterine sarcoma, prostate cancer, vaginal cancer, Paget's disease, tonsil cancer, anal cancer, rhabdomyosarcoma, Kaposi sarcoma, sarcoma, tongue cancer, laryngeal cancer, pleural cancer, thymic cancer, and combinations thereof; wherein the non-small cell lung cancer is preferably lung adenocarcinoma, or lung squamous cell carcinoma;
the autoimmune disease is preferably selected from: inflammatory colitis, Crohn's disease, Behcet's disease, multiple sclerosis, macular degeneration, arthritis, encephalitis, viral meningitis, and combinations thereof;
the metabolic disease is preferably selected from diabetes, and the diabetes is preferably type I diabetes or type II diabetes, more preferably is type II diabetes.
